# EUROPEAN PATENT SPECIFICATION

(11) **EP 1 320 523 B1**
(45) Date of publication and mention of the grant of the patent: **22.06.2005**
(21) Application number: 01964182.8
(22) Date of filing: 17.08.2001
(51) Int. Cl.: C07D 209/18

(54) **BRANCHED CHAIN AMINO ACID-DEPENDENT AMINOTRANSFERASE INHIBITORS AND THEIR USE IN THE TREATMENT OF NEURODEGENERATIVE DISEASES**
HEMMER DER VERZWEIGTENAMINOSÄURE-ABHÄNGIGEN AMINOTRANSFERASE UND DEREN VERWENDUNG IN DER BEHANDLUNG VON NEURODEGENERATIVEN ERKRANKUNGEN
INHIBITEURS D'AMINOTRANSFERASE DEPENDANT D'AMINOACIDES A CHAINE RAMIFIEE, ET LEUR UTILISATION DANS LE TRAITEMENT DE MALADIES NEURODEGENERATIVES

(30) Priority: 19.09.2000 US 233786 P
(43) Date of publication of application: 25.06.2003
(73) Proprietor: Warner-Lambert Company LLC, Morris Plains, New Jersey 07950 (US)
(72) Inventor: BORA, Keenan, Martin, Philadelphia, PA 19129 (US); HU, Lain-Yen, Ann Arbor, MI 48105 (US); KESTEN, Suzanne, Ross, Ann Arbor, MI 48105 (US); LEI, Huanyshu, Ann Arbor, MI 48105 (US); MORELAND, David, Winslow, Ann Arbor, MI 48105 (US); RAFFERTY, Michael, Francis, Ann Arbor, MI 48105 (US); RYDER, Todd, Robert, Rochester, NY 14608 (US); SCHOLTEN, Jeffrey, David, Ann Arbor, MI 48105 (US); WUSTROW, David, Juergen, Ann Arbor, MI 48105 (US)
(74) Representative: Modiano, Micaela Nadia
(86) International application number: PCT/US2001/025892
(87) International publication number: WO 2002/024672

(56) References cited:
- DE-A- 3 606 949
- KORUNCEV D ET AL: "ANTIBACTERIAL AND ANTIVIRAL EFFECTS IN A SERIES OF HYDRAZIDES AND HYDRAZONES DERIVED FROM QUINOLINE-2-CARBOXYLIC ACID" ACTA PHARMACEUTICA JUGOSLAVICA, SAVEZ FARMACEUTSKIH DRUSTAVA JUGOSLAVIJE, ZAGREB, YU, vol. 25, no. 4, October 1975 (1975-10), pages 241-246, XP001055780 ISSN: 0001-6667
- BHAT G A ET AL: "SYNTHESIS OF INDOLE-2-CARBALDEHYDES, 2-(2-AMINOETHYL) - AND 2-(2-AMION-PROPYL)-INDOLES" JOURNAL OF THE CHEMICAL SOCIETY, SECTION C: ORGANIC CHEMISTRY, CHEMICAL SOCIETY. LETCHWORTH, GB, no. 1, 1971, pages 178-181, XP001055788
- RIED W ET AL: "ZUR KENNTNIS DES 7.80DIOXY-CHINOLINS UND SEINER ABKOEMMLINGE" CHEMISCHE BERICHTE, VERLAG CHEMIE GMBH. WEINHEIM, DE, vol. 85, no. 3, 1952, pages 204-216, XP000571675 ISSN: 0009-2940 & DATABASE BEILSTEIN [Online]
- H.M. NOUR EL-DIN AND H.M. SAFWAT: "Synthesis of bergapten and some of its derivatives of expected molluscicidal activity, startintg from visnagin" U.A.R.J.PHARM.SCI., vol. 11, no. 1, 1970, pages 45-51, XP002202625
- J.ORG.CHEM., vol. 21, 1956, pages 530-531, XP002202626 & DATABASE BEILSTEIN [Online]
- ALLEN G R: "SELECTIVITY IN THE FISCHER INDOLIZATION OF PHENYTHYDRAZONES DERIVEDFROM 3-KETOCYCLOHEXANECARBOXYLIC ACID" JOURNAL OF HETEROCYCLIC CHEMISTRY, HETEROCORPORATION. PROVO, US, vol. 7, no. 1, February 1970 (1970-02), pages 239-241, XP001055783 ISSN: 0022-152X

## Description

### FIELD OF THE INVENTION

This invention is related to branched chain amino acid-dependent amino transferase (BCAT) inhibitors. The invention is also directed to the use of BCAT inhibitors as neuro-protective agents for treating conditions such as stroke, cerebral ischemia, central nervous system trauma, hypoglycemia, anxiety, convulsions, aminoglycoside antibiotics-induced hearing loss, migraine headaches, chronic pain, neuropathic pain, glaucoma, CMV retinitis, diabetic retinopathy, psychosis, urinary incontinence, opioid tolerance or withdrawal, or neuro-degenerative disorders such as lathyrism, Alzheimer's disease, Parkinsonism, amyotrophic lateral sclerosis (ALS), and Huntington's Disease.

### RELATED BACKGROUND ART

Excessive excitation by neurotransmitters can cause the degeneration and death of neurons. It is believed that this degeneration is in part mediated by the excitotoxic actions of the excitatory amino acids (EAA) glutamate and aspartate at the N-methyl-D-aspartate (NMDA) receptor. This excitotoxic action is considered responsible for the loss of neurons in cerebrovascular disorders such as cerebral ischemia or cerebral infarction resulting from a range of conditions, such as thromboembolic or hemorrhagic stroke, cerebral vasospasms, hypoglycemia, cardiac arrest, status epilepticus, perinatal asphyxia, anoxia such as from drowning, pulmonary surgery and cerebral trauma, as well as lathyrism, Alzheimer's disease, Parkinson's disease, and Huntington's disease.

Excitatory amino acid receptor antagonists that block NMDA receptors are recognized for usefulness in the treatment of disorders. NMDA receptors are intimately involved in the phenomenon of excitotoxicity, which may be a critical determinant of outcome of several neurological disorders. Disorders known to be responsive to blockade of the NMDA receptor include acute cerebral ischemia (stroke or cerebral trauma, for example), muscular spasm, convulsive disorders, neuropathic pain and anxiety, and may be a significant causal factor in chronic neurodegenerative disorders such as Parkinson's disease (Klockgether T., Turski L., *Ann. Neurol*., 1993;34:585-593), human immunodeficiency virus (HIV) related neuronal injury, amyotrophic lateral sclerosis (ALS), Alzheimer's disease (Francis P.T., Sims N.R., Procter A.W., Bowen D.M., *J. Neurochem*., 1993;60(5):1589-1604, and Huntington's disease (see Lipton S., *TINS*, 1993;16(12):527-532; Lipton S.A., Rosenberg P.A., *New Eng. J. Med*., 1994;330(9):613-622; and Bigge C.F., *Biochem. Pharmacol*., 1993;45:1547-1561, and referenced cited therein.) NMDA receptor antagonists may also be used to prevent tolerance to opiate analgesia or to help control withdrawal symptoms from addictive drugs (Eur. Pat. Appl. 488,959A).

U.S. Pat. No. 5,352,683 discloses the treatment of chronic pain with a compound which is an antagonist of the NMDA receptor.

U.S. Pat. No. 4,902,695 discloses certain competitive NMDA antagonists that are useful for the treatment of neurological disorders, including epilepsy, stroke, anxiety, cerebral ischemia, muscular spasms, and neurodegenerative diseases such as Alzheimer's disease and Huntington's disease.

U.S. Pat No. 5,192,751 discloses a method of treating urinary incontinence in a mammal which comprises administering an effective amount of a competitive NMDA antagonist.

### SUMMARY OF THE INVENTION

The invention relates BCAT inhibitor compounds of Formula I 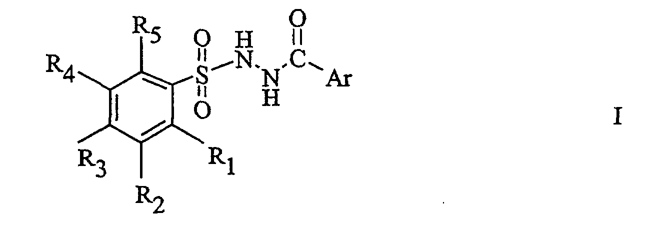 wherein:
- R₃: is H, or F, Br, alkyl, carboxy, alkoxy, substituted alkoxy,
- R₁, R₂, R₄, and R₅: are independently, H, halogen, alkyl, substituted alkyl, alkoxy, substituted alkoxy, cyano, nitro, amino, alkylamine, and thioalkyl, Ar is bicyclic heteroaryl, tricyclic heteroaryl, substituted bicyclic heteroaryl or substituted tricyclic heteroaryl, except (6-methoxybenzofuran)-2-nyl, 3-quinolinyl; or a pharmaceutically acceptable salt, ester, prodrug, or amide thereof;
- Ar: is bicyclic heteroaryl, tricyclic heteroaryl, substituted bicyclic heteroaryl, substituted tricyclic heteroaryl, except (6-methoxybenzofuran)-2-nyl or 3-quinolinyl, selected from one of the group as listed later on;
where there is more than one stereoisomer, each chiral center may be independently R or S; or a pharmaceutically acceptable salt, ester, or amide thereof.

The invention also relates to compounds of Formula I, wherein R₁, R₂, R₃, R₄, and R₅ are hydrogen; R₂ or R₄ are alkyl; R₂ and R₄ are halogen; R₁ is alkyl and R₂ is halogen; R₅ is halogen; and Ar is a tricyclic heteroaryl, bicyclic heteroaryl, substituted bicyclic heteroaryl and substituted tricyclic heteroaryl.

The invention also relates to compounds of Formula I, wherein R₁, R₂, R₃, R₄, and R₅ are hydrogen, and Ar is a bicyclic heteroaryl; R₁, R₂, R₃, R₄ and R₅ are hydrogen, and Ar is a tricyclic heteroaryl; one of R₁, R₂, R₃, R₄, and R₅ are halogen, and Ar is a tricyclic heteroaryl; and two of R₁, R₂, R₃, R₄, and R₅ are halogen, and Ar is a tricyclic heteroaryl.

The invention also relates to compounds selected from:
Dibenzofuran-2-carboxylic acid, 2-(phenylsulfonyl)hydrazide;
2,3,4,9-Tetrahydro-1H-carbazole-6-carboxylic acid, 2-(phenylsulfonyl)-hydrazide;
Benzofuran-2-carboxylic acid, 2-(phenylsulfonyl)hydrazide;
5-Methoxy-2-benzofurancarboylic acid, 2-(phenylsulfonyl)hydrazide;
7-Methoxy-2-benzofurancarboxylic acid, 2-(phenylsulfonyl)hydrazide;
7-Ethoxy-2-benzofurancarboxylic acid, 2-(phenylsulfonyl)hydrazide;
7-Chloro-benzofuran-2-carboxylic acid, 2-(phenylsulfonyl)hydrazide;
5-Chloro-benzofuran-2-carboxylic acid, 2-(phenylsulfonyl)hydrazide;
2,3-Dihydro-benzofuran-5-carboxylic acid, 2-(phenylsulfonyl)hydrazide;
Benzo[1,3]dioxole-5-carboxylic acid, 2-(phenylsulfonyl)hydrazide;
1H-Indole-5-carboxylic acid, 2-(phenylsulfonyl)hydrazide;
1-Dibenzofurancarboxylic acid, 2-(phenylsulfonyl)hydrazide;
Quinoline-6-carboxylic acid, 2-(phenylsulfonyl)hydrazide;
2-Dibenzofurancarboxylic acid, 2-[(2-methylphenyl)sulfonyl]hydrazide;
2-Dibenzofurancarboxylic acid, 2-[(3-methylphenyl)sulfonyl]hydrazide;
2-Dibenofurancarboxylic acid, 2-[(3-chlorophenyl)sulfonyl]hydrazide;
2-Dibenzofurancarboxylic acid, 2-[(2-chlorophenyl)sulfonyl]hydrazide;
Dibenzofuran-2-carboxylic acid, 2-(3,5-dichlorophenylsulfonyl)hydrazide;
Dibenzofuran-2-carboxylic acid, 2-(3-chloro-2-methyl-phenylsulfonyl)-hydrazide;
Quinoline-6-carboxylic acid, 2-(phenylsulfonyl)hydrazide;
Quinoline-8-carboxylic acid, 2-(phenylsulfonyl)hydrazide;
2-Benzofurancarboxylic acid, 5-chloro-2-[(4-fluorophenyl)]sulfonyl hydrazide;
2-Benzofurancarboxylic acid, 7-methoxy-, 2-[(4-fluoro-phenyl)sulfonyl]hydrazide;
2-Dibenzofurancarboxylic acid, 2-[(4-fluorophenyl)sulfonyl]hydrazide;
2-Dibenzofurancarboxylic acid, 2-[(2-chloro-4-fluorophenyl)sulfonyl]hydrazide;
2-Dibenzofurancarboxylic acid, 2-[(3-cyanophenyl)sulfonyl]hydrazide;
2-Benzofurancarboxylic acid, 5-chloro-, 2-[(3-cyanophenyl)sulfonyl]hydrazide;
2-Dibenzofurancarboxylic acid, 2-[(2,6-dichlorophenyl) sulfonyl]hydrazide;
2-Benzofurancarboxylic acid, 5-bromo-, 2-(phenylsulfonyl)hydrazide;
2-Benzofurancarboxylic acid, 5-chloro-, 2-[(2-chloro-4-fluorophenyl)sulfonyl]hydrazide;
2-Benzofurancarboxylic acid, 5-chloro-, 2-[(3,5-dichlorophenyl)sulfonyl]hydrazide;
2-Dibenzofurancarboxylic acid, 2-[[3,5-bis(trifluoromethyl)phenyl]sulfonyl]hydrazide;
2-Benzofurancarboxylic acid, 5-chloro, 2-[(3-chloro-4-fluorophenyl)sulfonyl]hydrazide;
2-Benzofurancarboxylic acid, 5-nitro-, 2-(phenylsulfonyl)hydrazide;
2-Dibenzofurancarboxylic acid, 2-[(3-fluorophenyl)sulfonyl]hydrazide;
2-Dibenzofurancarboxylic acid, 2-[(5-fluoro-2-methylphenyl)sulfonyl]hydrazide;
2-Dibenzofurancarboxylic acid, 2-[(3,4-difluorophenyl)sulfonyl]-hydrazide;
2-Dibenzofurancarboxylic acid, 2-[(3-chloro-4-fluorophenyl)sulfonyl]hydrazide;
2-Dibenzofurancarboxylic acid, 2-[(3-methoxyphenyl)sulfonyl]-hydrazide;
2-Benzofurancarboxylic acid, 5-chloro-2-[(3-fluorophenyl)sulfonyl]-hydrazide;
2-Benzofurancarboxylic acid, 5-chloro-2-[(3,4-difluorophenyl)sulfonyl]hydrazide;
2-Benzofurancarboxylic acid, 5-chloro-2-[(2-chlorophenyl)sulfonyl]hydrazide;
2-Benzofurancarboxylic acid, 5-chloro-2-[(3-chlorophenyl)sulfonyl]hydrazide;
2-Benzofurancarboxylic acid, 5-chloro-2-[(2-bromophenyl)sulfonyl]hydrazide;
2-Benzofurancarboxylic acid, 5-chloro-2-[(3-bromophenyl)sulfonyl]hydrazide;
2-Benzofurancarboxylic acid, 5-chloro-2-[(2-methylphenyl)sulfonyl]hydrazide;
2-Benzofurancarboxylic acid, 5-chloro-2-[(3-methylphenyl)sulfonyl]hydrazide;
2-Benzofurancarboxylic acid, 5-chloro-2-[(3-methoxyphenyl)sulfonyl]hydrazide;
2-Benzofurancarboxylic acid, 5-chloro-2-[(3-chloro-2-methylphenyl)sulfonyl]hydrazide;
2-Benzofurancarboxylic acid, 5-chloro-2-[(5-fluoro-2-methylphenyl)sulfonyl]hydrazide;
2-Benzofurancarboxylic acid, 5-chloro-2-[[(2-(trifluoromethyl)phenyl]sulfonyl]hydrazide;
2-Benzofurancarboxylic acid, 5-chloro-2-[[2-(trifluoromethoxy)phenyl]sulfonyl]hydrazide;
2-Benzofurancarboxylic acid, 7-methoxy-, 2-[[2-(trifluoromethyl)phenyl]sulfonyl]hydrazide;
2-Benzofurancarboxylic acid, 7-methoxy-, 2-[(3-methylphenyl)sulfonyl]hydrazide;
2-Benzofurancarboxylic acid, 6-methoxy-, 2-(phenylsulfonyl)hydrazide;
2-Benzofurancarboxylic acid, 5-methoxy-3-(1-methylethoxy), 2-(phenylsulfonyl)hydrazide;
2-Benzofurancarboxylic acid, 3,5-dimethoxy-, 2-(phenylsulfonyl)-hydrazide;
2-Benzofurancarboxylic acid, 3,6-dimethoxy-, 2-(phenylsulfonyl)-hydrazide;
2-Benzofurancarboxylic acid, 4,6-dimethoxy-, 2-(phenylsulfonyl)-hydrazide;
2-Benzofurancarboxylic acid, 3,7-dimethoxy-, 2-(phenylsulfonyl)-hydrazide;
2-Benzofurancarboxylic acid, 3,7-dimethoxy-,
2-[(2-trifluoromethylphenyl)sulfonyl]hydrazide;
2-Benzofurancarboxylic acid, 3,7-dimethoxy-, 2-[(4-fluorophenyl)sulfonyl]hydrazide;
2-Benzofurancarboxylic acid, 3,7-dimethoxy-, 2-[(3-methylphenyl)sulfonyl]hydrazide;
2-Benzofurancarboxylic acid, 3-methoxy-5-phenyl-, 2-(phenylsulfonyl)hydrazide;
2-Benzofurancarboxylic acid, 3-ethoxy-5-phenyl-, 2-(phenylsulfonyl)hydrazide;
2-Benzofurancarboxylic acid, 3-methoxy-7-phenyl-, 2-(phenylsulfonyl)hydrazide;
Naptho[2,3-*b*]furan-2-carboxylic acid, 3-methoxy-, 2-(phenylsulfonyl)hydrazide;
2-Benzofurancarboxylic acid, 5,7-dichloro 2-(phenylsulfonyl)sulfonyl hydrazide;
2-Benzofurancarboxylic acid, 5,7-dichloro 2-[[2-(trifluoromethyl)phenyl)]sulfonyl]hydrazide;
2-Benzofurancarboxylic acid, 5,7-dichloro 2-[(2-methylphenyl)sulfonyl]hydrazide;
2-Benzofurancarboxylic acid, 5,7-dichloro 2-[(3-methylphenyl) sulfonyl]hydrazide;
2-Benzofurancarboxylic acid, 5,7-dichloro 2-[(2-bromophenyl)sulfonyl]hydrazide;
2-Benzofurancarboxylic acid, 5,7-dichloro 2-[(3-bromophenyl)sulfonyl]hydrazide;
2-Benzofurancarboxylic acid, 5,7-dichloro 2-[(3-chlorophenyl)sulfonyl]hydrazide;
2-Benzofurancarboxylic acid, 5,7-dichloro 2-[[2-(trifluoromethoxy)phenyl]sulfonyl]hydrazide;
Benzofurancarboxylic acid, 5-methyl-, 2-(phenylsulfonyl)hydrazide;
2-Benzofurancarboxylic acid, 5-chloro-3-methoxy, 2-(phenylsulfonyl)hydrazide;
2-Benzofurancarboxylic acid, 5-methyl-, 2-[[2-(trifluoromethyl)phenyl]sulfonyl]hydrazide;
2-Benzofurancarboxylic acid, 5-methyl-, 2-([3-methylphenyl)sulfonyl]hydrazide;
2-Benzofurancarboxylic acid, 5-methyl-, 2-[2-(methylphenyl)sulfonyl]hydrazide;
2-Benzofurancarboxylic acid, 5-methyl-, 2-[(3-chloro-2-methylphenyl)sulfonyl]hydrazide;
2-Benzofurancarboxylic acid, 5-methyl-, 2-[3-(bromophenyl)sulfonyl]hydrazide;
2-Benzofurancarboxylic acid, 5-methyl-, 2-[(3-chlorophenyl)sulfonyl]hydrazide;
2-Benzofurancarboxylic acid, 5-methyl-, 2-[[2-(trifluoromethoxy)phenyl]sulfonyl]hydrazide;
2-Benzofurancarboxylic acid, 7-methyl-, 2-(phenylsulfonyl)hydrazide;
2-Benzofurancarboxylic acid, 4-methyl-, 2-(phenylsulfonyl)hydrazide;
2-Benzofurancarboxylic acid, 3-methyl-, 2-(phenylsulfonyl)hydrazide;
2-Benzofurancarboxylic acid, 3,5-dimethyl-, 2-(phenylsulfonyl)hydrazide;
2-Benzofurancarboxylic acid, 5-chloro-3-methyl, 2-(phenylsulfonyl)hydrazide;
2-Benzofurancarboxylic acid, 5-amino-, 2-(phenylsulfonyl)hydrazide;
2-Dibenzofurancarboxylic acid, 2-[(2-fluorophenyl)sulfonyl]hydrazide;
2-Dibenzofurancarboxylic acid, 2-(2,6-difluorophenylsulfonyl)hydrazide;
2-Dibenzofurancarboxylic acid, 2-[(2-bromophenyl)sulfonyl]hydrazide;
2-Dibenzofurancarboxylic acid, 2-[(3-bromophenyl)sulfonyl]hydrazide;
2-Dibenzofurancarboxylic acid, 2-[(4-methyl-3-nitrophenyl)sulfonyl]hydrazide;
2-Dibenzofurancarboxylic acid, 2-[2-chloro-5-(trifluoromethyl)phenyl]sulfonyl)hydrazide;
2-Dibenzofurancarboxylic acid, 2-[(2-trifluoromethoxyphenyl)sulfonyl]hydrazide;
2-Dibenzofurancarboxylic acid, 2-[(4-bromo-2-trifluoromethoxyphenyl)sulfonyl]hydrazide;
2-Dibenzofurancarboxylic acid, 2-[(2-nitrophenyl)sulfonyl]hydrazide;
2-Dibenzofurancarboxylic acid, 2-[(4-carboxyphenyl)sulfonyl]hydrazide;
2-Dibenzofurancarboxylic acid, 2-[(4,5-dichloro-2-thienyl)sulfonyl]-hydrazide;
2-Benzofurancarboxylic acid, 7-methoxy-, 2-[(3-chlorophenyl)sulfonyl]hydrazide;
2-Benzofurancarboxylic acid, 7-methoxy-, 2-[(3-fluorophenyl)sulfonyl]hydrazide;
2-Benzofurancarboxylic acid, 7-methoxy-, 2-[(2,3-dichlorophenyl)sulfonyl]hydrazide;
2-Benzofurancarboxylic acid, 7-methoxy-, 2-[(3-chloro-4-fluorophenyl)sulfonyl]hydrazide;
2-Benzofurancarboxylic acid, 7-methoxy-, 2-[(5-chloro-2. methoxyphenyl)sulfonyl]hydrazide;
2-Benzofurancarboxylic acid, 7-methoxy-, 2-[(5-bromo-2methoxyphenyl)sulfonyl]hydrazide;
2-Benzofurancarboxylic acid, 7-methoxy-, 2-[(4-ethylphenyl)sulfonyl]hydrazide;
2-Benzofurancarboxylic acid, 7-methoxy-, 2-[(3-methoxyphenyl)sulfonyl]hydrazide;
2-Dibenzofurancarboxylic acid, 2-[(2,3-dichlorophenyl)sulfonyl]-hydrazide;
2-Naphthalenecarboxylic acid, 2-[(3-methylphenyl)sulfonyl]hydrazide;
2-Naphthalenecarboxylic acid, 2-[(4,5-dibromo-2-thienyl)sulfonyl]-hydrazide;
2-Naphthalenecarboxylic acid, 2-[(4-methylphenyl)sulfonyl]hydrazide;
2-Naphthalenecarboxylic acid, 2-[[(2-(trifluoromethyl)phenyl]sulfonyl]hydrazide;
2-Naphthalenecarboxylic acid, 2-[[(4-(trifluoromethyl)phenyl]sulfonyl]hydrazide;
2-Naphthalenecarboxylic acid, 2-[(3-chloro-4-fluorophenyl)sulfonyl]hydrazide;
2-Naphthalenecarboxylic acid, 2-[(3-methoxyphenyl)sulfonyl]hydrazide;
2-Naphthalenecarboxylic acid, 2-[(3-chlorophenyl)sulfonyl]hydrazide;
2-Naphthalenecarboxylic acid, 2-[(2-chlorophenyl)sulfonyl]hydrazide;
2-Naphthalenecarboxylic acid, 2-[(3-fluorophenyl)sulfonyl]hydrazide;
2-Naphthalenecarboxylic acid, 2-[(2,3-dichlorophenyl)sulfonyl]hydrazide;
2-Naphthalenecarboxylic acid, 2-[(4-ethylphenyl)sulfonyl]hydrazide;
3-Quinolinecarboxylic acid, 2-[(4-pentylphenyl)sulfonyl)hydrazide;
3-Quinolinecarboxylic acid, 2-[[2(trifluoromethyl)phenyl]sulfonyl]-hydrazide;
3-Quinolinecarboxylic acid, 2-[[2,5-bis(2,2,2-trifluoroethoxyl)phenyl]sulfonyl]hydrazide;
3-Quinolinecarboxylic acid, 2-[(3-methylphenyl)sulfonyl]hydrazide;
3-Quinolinecarboxylic acid, 2-[[2-(trifluoromethoxy)phenyl]sulfonyl]hydrazide;
3-Quinolinecarboxylic acid, 2-[(4-methylphenyl)sulfonyl]hydrazide;
6-Quinolinecarboxylic acid, 2-[(3-methylphenyl)sulfonyl]hydrazide;
3-Quinolinecarboxylic acid, 2-[(3-methylphenyl)sulfonyl]hydrazide;
6-Quinolinecarboxylic acid, 2-[(3-chlorophenyl)sulfonyl]hydrazide;
3-Quinolinecarboxylic acid, 2-[(3-chlorophenyl)sulfonyl]hydrazide;
6-Quinolinecarboxylic acid, 2-[(3-fluorophenyl)sulfonyl]hydrazide;
3-Quinolinecarboxylic acid, 2-[(3-fluorophenyl)sulfonyl]hydrazide;
6-Quinolinecarboxylic acid, 2-[(2-trifluoromethylphenyl)sulfonyl]-hydrazide;
3-Quinolinecarboxylic acid, 2-[(2-trifluoromethylphenyl)sulfonyl]-hydrazide;
6-Quinolinecarboxylic acid, 2-[(2-trifluoromethoxyphenyl)-sulfonyl]hydrazide;
3-Quinolinecarboxylic acid, 2-[(2-trifluoromethoxyphenyl)-sulfonyl]hydrazide;
6-Quinolinecarboxylic acid, 2-[(3,5-dichlorophenyl)sulfonyl]hydrazide;
3-Quinolinecarboxylic acid, 2-[(3,5-dichlorophenyl)sulfonyl]hydrazide;
6-Quinolinecarboxylic acid, 2-[(3-chloro-4-fluorophenyl)sulfonyl]-hydrazide;
3-Quinolinecarboxylic acid, 2-[(3-chloro-4-fluorophenyl)sulfonyl]-hydrazide;
6-Quinolinecarboxylic acid, 2-[(3-methoxyphenyl)sulfonyl]hydrazide;
3-Quinolinecarboxylic acid, 2-[(3-methoxyphenyl)sulfonyl]hydrazide;
6-Quinolinecarboxylic acid, 2-[(3-bromophenyl)sulfonyl]hydrazide;
3-Quinolinecarboxylic acid, 2-[(3-bromophenyl)sulfonyl]hydrazide;
Benzofuran-2-carboxylic acid, 2-(phenylsulfonyl)hydrazide;
7-Ethoxy-2-benzofurancarboxylic acid, 2-(phenylsulfonyl)hydrazide;
7-Chloro-benzofuran-2-carboxylic acid, 2-(phenylsulfonyl)hydrazide;
2,3-Dihydro-benzofuran-5-carboxylic acid, 2-(phenylsulfonyl)hydrazide;
5-Chloro-benzofuran-2-carboxylic acid, 2-(phenylsulfonyl)hydrazide;
Isoquinoline-1-carboxylic acid, 2-(phenylsulfonyl)hydrazide;
1*H*-Indole-5-carboxylic acid, 2-(phenylsulfonyl)hydrazide;
Quinoline-3-carboxylic acid, 2-(phenylsulfonyl)hydrazide;
Quinoline-4-carboxylic acid, 2-(phenylsulfonyl)hydrazide;
1*H*-Indole-5-carboxylic acid, 2-[(3-chlorophenyl)sulfonyl]hydrazide;
1*H*-Indole-5-carboxylic acid, 2-[(2-chlorophenyl)sulfonyl]hydrazide;
1*H*-Indole-5-carboxylic acid, 2-[(3-fluorophenyl)sulfonyl]hydrazide;
1*H*-Indole-5-carboxylic acid, 2-[(3-trifluoromethylphenyl)sulfonyl]-hydrazide;
1*H*-Indole-5-carboxylic acid, 2-[(2-trifluoromethylphenyl)sulfonyl]-hydrazide;
1*H*-Indole-5-carboxylic acid, 2-[(2-trifluoromethoxyphenyl)sulfonyl]hydrazide;
1*H*-Indole-5-carboxylic acid, 2-[(3trifluoromethoxyphenyl)sulfonyl]hydrazide;
1*H*-Indole-5-carboxylic acid, 2-[(3-bromophenyl)sulfonyl]hydrazide;
1*H*-Indole-5-carboxylic acid, 2-[(3-methylphenyl)sulfonyl]hydrazide;
1*H*-Indole-5-carboxylic acid, 2-[(2-methylphenyl)sulfonyl]hydrazide;
6-Quinolinecarboxylic acid, 2-[(2-chlorophenyl)sulfonyl]hydrazide;
6-Quinolinecarboxylic acid, 2-[(3-trifluoromethylphenyl)sulfonyl]-hydrazide;
6-Quinolinecarboxylic acid, 2-[(3-trifluoromethoxyphenyl)sulfonyl]-hydrazide;
6-Quinolinecarboxylic acid, 2-[(2-methylphenyl)sulfonyl]hydrazide;
Dibenzofuran-2-carboxylic acid, 2-(4-fluoro-3-methylphenylsulfonyl)hydrazide;
7-Methoxy-1H-indole-2-carboxylic acid, 2-(phenylsulfonyl)-hydrazide;
7-Chloro-1H-indole-2-carboxylic acid, 2-(phenylsulfonyl)-hydrazide;
5-Methoxy-1H-indole-2-carboxylic acid, 2-(phenylsulfonyl)-hydrazide;
5-Chloro-1H-indole-2-carboxylic acid, 2-(phenylsulfonyl)-hydrazide;
5-Chloro-1H-indole-2-carboxylic acid, 2-[(2-trifluoromethyl)phenylsulfonyl]hydrazide;
5-Methyl-1H-indole-2-carboxylic acid, 2-(phenylsulfonyl)-hydrazide;
5-Methyl-1H-indole-2-carboxylic acid, 2-[(2-trifluoromethyl)phenylsulfonyl]hydrazide; and
2-Naphthalenecarboxylic acid, 2-(phenylsulfonyl)hydrazide.

The invention also relates to the use of a compound of formula I for preparation of a medicament for treatment or preventing neuronal loss associated with stroke, ischemia, CNS trauma, hypoglycemia and surgery, as well as treating neurodegenerative diseases including Alzheimer's disease, amyotrophic lateral sclerosis, Huntington's disease, Parkinson's disease and Down's syndrome, treating or preventing the adverse consequences of the overstimulation of the excitatory amino acids, treating anxiety, psychosis, convulsions, chronic pain, neuropathic pain, diabetic retinopathy, glaucoma, CMV retinitis, urinary incontinence, and inducing anesthesia, . as well as enhancing cognition, and preventing opiate tolerance and withdrawal symptoms, comprising administering to an animal in need of such treatment an effective amount of any one of the BCAT inhibitors of the present invention, or a pharmaceutically acceptable salt thereof.

The present invention also includes a pharmaceutical composition comprising a therapeutically effective amount of one or more compounds of Formula I and a pharmaceutically acceptable carrier.

### DETAILED DESCRIPTION OF THE INVENTION

The present invention is predicated on the discovery that intraocular glutamate injection causes ganglion cell loss which is related to the early stages of diabetic retinopathy (Vorwerk et al., *IOVS*, 1996;37:1618-1624 and that an inhibitor of the branched chain amino acid-dependent aminotransferase pathway, specifically gabapentin, is effective in inhibiting the synthesis of glutamate (see example below), thus preventing diabetic retinopathy. Accordingly, the present invention provides the use of a compound as referred to above, for the prophylactic and therapeutic treatment of diabetic retinopathy, including treatment at the pre-diabetic retinopathy stage, the nonproliferative diabetic retinopathy stage, and the proliferative diabetic retinopathy stage. By "prophylactic" is meant the protection, in whole or in part, against diabetic retinopathy, in particular diabetic macular edema. By "therapeutic" is meant the amelioration of diabetic retinopathy, itself, and the protection, in whole or in part, against further diabetic retinopathy, in particular diabetic macular edema.

The use comprises the administration of an inhibitor of the branched chain amino acid-dependent aminotransferase pathway in an amount sufficient to treat the neurodegenerative disease or condition, for example, to treat the retina for retinopathy prophylactically or therapeutically. Any inhibitor of the branched chain amino acid-dependent aminotransferase pathway can be used in the use of the present invention as long as it is safe and efficacious. Herein, "branch chain amino acid-dependent aminotransferase (BCAT) inhibitor" will be used to refer to such compounds and is intended to encompass all compounds that affect the branch chain amino acid-dependent aminotransferase pathway at any and all points in the pathway.

Preferably, the BCAT inhibitor is a compound of Formula I as described above, or a pharmaceutically acceptable, BCAT pathway-inhibiting analogue or prodrug thereof or a pharmaceutically acceptable salt, ester, or amide of any of the foregoing.

Unless otherwise expressly stated, the following definitions are adhered to throughout this disclosure.

"Alkyl" means a straight or branched hydrocarbon radical having from 1 to 10 carbon atoms (unless stated otherwise) and includes, for example, methyl, ethyl, n-propyl, isopropyl, n-butyl, sec-butyl, isobutyl, tert-butyl, n-pentyl, iso-pentyl, n-hexyl, and the like. "Halogen" includes fluoro, chloro, bromo, and iodo.

"Alkenyl" means straight and branched hydrocarbon radicals having from 2 to 6 carbon atoms and one double bond and includes ethenyl, 3-buten-1-yl, 2-ethenylbutyl, 3-hexen-1-yl, and the like.

"Alkynyl" means straight and branched hydrocarbon radicals having from 2 to 6 carbon atoms and one triple bond and includes ethynyl, 3-butyn-1-yl, propynyl, 2-butyn-1-yl, 3-pentyn-1-yl, and the like.

"Cycloalkyl" means a monocyclic or polycyclic hydrocarbyl group such as cyclopropyl, cycloheptyl, cyclooctyl, cyclodecyl, cyclobutyl, adamantyl, norpinanyl, decalinyl, norbornyl, cyclohexyl, and cyclopentyl. Such groups can be substituted with groups such as hydroxy, keto, alkoxy, hydroxyalkyl and the like. Also included are rings in which 1 to 3 heteroatoms replace carbons. Such groups are termed "heterocyclyl," which means a cycloalkyl group also bearing at least one heteroatom selected from O, S, or NR₂, wherein R₂ is as defined above with respect to substiuents of the phenyl group of Formula I.. Suitable examples include substituted or unsubstituted oxiranyl, pyrrolidinyl, piperidyl, tetrahydropyran, piperazinyl, acylpiperazinyl, pyrrolidinyl, and morpholine.

"Alkoxy" refers to the alkyl groups mentioned above bound through oxygen, examples of which include methoxy, ethoxy, isopropoxy, tert-butoxy, and the like. In addition, alkoxy refers to polyethers such as -O-(CH₂)₂-O-CH₃, and the like.

"Alkanoyl" groups are alkyl linked through a carbonyl, ie, C₁-C₅-C(O)-. Such groups include formyl, acetyl, propionyl, butyryl, and isobutyryl.

"Acyl" means an alkyl or aryl (Ar) group bonded through a carbonyl group, ie, R-C(O)-. For example, acyl includes a C₁-C₆ alkanoyl, including substituted alkanoyl, wherein the alkyl portion can be substituted by NR₄R₅ or a carboxylic or heterocyclic group. R₄ and R₅ are as defined above with respect to substiuents of the phenyl group of Formula I. Typical acyl groups include acetyl, benzoyl, and the like.

The alkyl, alkenyl, alkoxy, and alkynyl groups described above are optionally substituted, preferably by 1 to 3 groups selected from NR₄R₅, phenyl, substituted phenyl (wherein the substituent on the phenyl group may be selected from 1 to 3 groups selected from NR₄R₅, phenyl, substituted phenyl, thio C₁-C₆ alkyl, C₁-C₆ alkoxy, hydroxy, carboxy, C₁-C₆ alkoxycarbonyl, halo, nitrile, cycloalkyl, and a 5- or 6-membered carbocyclic ring or heterocyclic ring having 1 or 2 heteroatoms selected from nitrogen, substituted nitrogen, oxygen, and sulfur), thio C₁-C₆ alkyl, C₁-C₆ alkoxy, hydroxy, carboxy, C₁-C₆ alkoxycarbonyl, halo, nitrile, cycloalkyl, and a 5- or 6-membered carbocyclic ring or heterocyclic ring having 1 or 2 heteroatoms selected from nitrogen, substituted nitrogen, oxygen, and sulfur. R₄ and R₅ are as defined above with respect to substiuents of the phenyl group of Formula I. "Substituted nitrogen" means nitrogen bearing C₁-C₆ alkyl or (CH₂)ₙPh where n is 1, 2, or 3. Perhalo and polyhalo substitution is also embraced.

Examples of substituted alkyl groups include 2-aminoethyl, pentachloroethyl, trifluoromethyl, 2-diethylaminoethyl, 2-dimethylaminopropyl, ethoxycarbonylmethyl, 3-phenylbutyl, methanylsulfanylmethyl, methoxymethyl, 3-hydroxypentyl, 2-carboxybutyl, 4-chlorobutyl, 3-cyclopropylpropyl, pentafluoroethyl, 3-morpholinopropyl, piperazinylmethyl, and 2-(4-methylpiperazinyl)ethyl.

Examples of substituted alkynyl groups include 2-methoxyethynyl, 2-ethylsulfanyethynyl, 4-(1-piperazinyl)-3-(butynyl), 3-phenyl-5-hexynyl, 3-diethylamino-3-butynyl, 4-chloro-3-butynyl, 4-cyclobutyl-4-hexenyl, and the like.

Typical substituted alkoxy groups include aminomethoxy, trifluoromethoxy, 2-diethylaminoethoxy, 2-ethoxycarbonylethoxy, 3-hydroxypropoxy, 6-carboxhexyloxy, and the like.

Further, examples of substituted alkyl, alkenyl, and alkynyl groups include dimethylaminomethyl, carboxymethyl, 4-dimethylamino-3-buten-1-yl, 5-ethylmethylamino-3-pentyn-1-yl, 4-morpholinobutyl, 4-tetrahydropyrinidylbutyl, 3-imidazolidin-1-ylpropyl, 4-tetrahydrothiazol-3-yl-butyl, phenylmethyl, 3-chlorophenylmethyl, and the like.

The terms "Ar" and "aryl" refer to unsubstituted and substituted aromatic groups. Heteroaryl groups have from 4 to 9 ring atoms, from I to 4 of which are independently selected from the group consisting of O, S, and N. Preferred heteroaryl groups have 1 or 2 heteroatoms in a 5- or 6-membered aromatic ring. Mono and bicyclic aromatic ring systems are included in the definition of aryl and heteroaryl. Typical aryl and heteroaryl groups include phenyl, 3-chlorophenyl, 2,6-dibromophenyl, pyridyl, 3-methylpyridyl, benzothienyl, 2,4,6-tribromophenyl, 4-ethylbenzothienyl, furanyl, 3,4-diethylfuranyl, naphthyl, 4,7-dichloronaphthyl, pyrrole, pyrazole, imidazole, thiazole, and the like.

Ar being selected from the group consisting of : 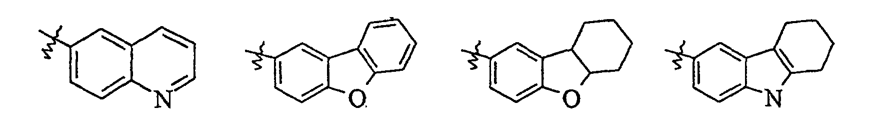 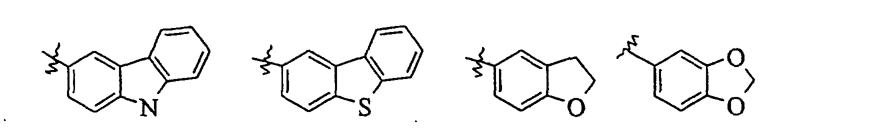 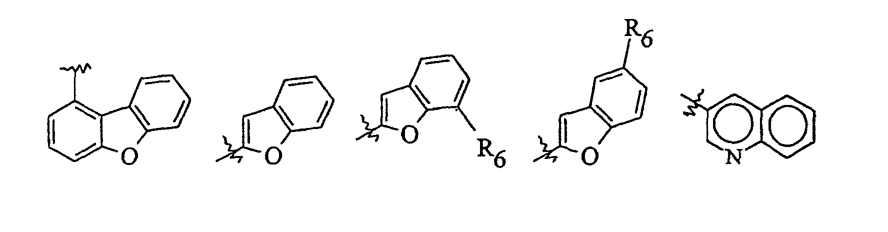 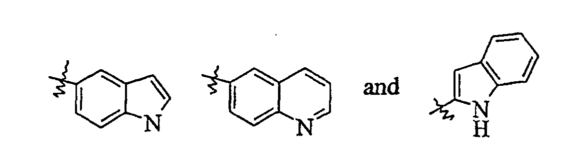 wherein R₆ is alkoxy, such as methoxy or ethoxy, substituted alkoxy, halogen, such as chloro, aryloxy, substituted aryloxy, alkylamino, substituted alkylamino, arylalkylamino, substituted arylalkylamino, amide or substituted amide where in the substitents may be as defined above.

Preferred Ar groups are phenyl and phenyl substituted by 1, 2, or 3 groups independently selected from the group consisting of alkyl, alkoxy, thio, thioalkyl, hydroxy, -COOR₇, amino of the formula -NR₄R₅, and T(CH₂)ₘQR₄ or T(CH₂)ₘCO₂R₄ wherein m is 1 to 6, T is O, S, NR₄, N(O)R₄, or CR₄R₅, Q is O, S, NR₅, N(O)R₅, wherein R₄ and R₅ are as described above, and R₇ is alkyl or substituted alkyl, for example, methyl, trichloroethyl, diphenylmethyl, and the like. The alkyl and alkoxy groups can be substituted as defined above. For example, typical groups are carboxyalkyl, alkoxycarbonylalkyl, hydroxyalkyl, hydroxyalkoxy, and alkoxyalkyl.

The symbol "-" means a bond.

The term "patient" means all animals including humans. Examples of patients include humans, cows, dogs, cats, goats, sheep, and pigs.

The compounds of the present invention can exist in unsolvated forms as well as solvated forms, including hydrated forms. In general, the solvated forms, including hydrated forms, are equivalent to unsolvated forms and are intended to be encompassed within the scope of the present invention.

Certain of the compounds of the present invention possess one or more chiral centers and each center may exist in the R(D) or S(L) configuration. The present invention includes all enantiomeric and epimeric forms as well as the appropriate mixtures thereof.

The compounds of Formula I are capable of further forming both pharmaceutically acceptable formulations comprising salts, esters, amides, and prodrugs. As used herein, the term "pharmaceutically acceptable salts, esters, amides, and prodrugs" refers to those carboxylate salts, amino acid addition salts, esters, amides, and prodrugs of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively non-toxic, inorganic and organic acid addition salts of compounds of the present invention. These salts can be prepared *in situ* during the final isolation and purification of the compounds or by separately reacting the purified compound in its free base form with a suitable organic or inorganic acid and isolating the salt thus formed and including, but not limited to, acid addition and/or base salts, solvents and N-oxides of a compound of Formula I. This invention also provides pharmaceutical formulations comprising a compound of Formula I together with a pharmaceutically acceptable carrier, diluent, or excipient therefor. All of these forms are within the present invention.

Pharmaceutically acceptable acid addition salts of the compounds of Formula I include salts derived form inorganic acids such as hydrochloric, nitric, phosphoric, sulfuric, hydrobromic, hydriodic, phosphorus, and the like, as well as the salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are the salts of amino acids such as arginate, gluconate, galacturonate, and the like; see, for example, Berge, et al., "Pharmaceutical Salts," *J. of Pharmaceutical Science,* 1977;66:1-19.

The acid addition salts of the basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts are formed with metals or amines, such as alkali and alkaline earth metal hydroxides, or of organic amines. Examples of metals used as cations are sodium, potassium, magnesium, calcium, and the like. Examples of suitable amines are N,N'-dibenzylethylenediamine, chloroprocaine, choline, diethanolamine, ethylenediamine, N-methylglucamine, and procaine; see, for example, Berge et al., supra., 1977.

The base addition salts of acidic compounds are prepared by contacting the free acid form with a sufficient amount of the desired base to produce the salt in the conventional manner. The free acid form may be regenerated by contacting the salt form with an acid and isolating the free acid in a conventional manner. The free acid forms differ from their respective salt forms somewhat in certain physical properties such as solubility in polar solvents, but otherwise the salts are equivalent to their respective free acid for purposes of the present invention.

Examples of pharmaceutically acceptable, non-toxic esters of the compounds of this invention include C₁-C₆alkyl esters wherein the alkyl group is a straight or branched chain. Acceptable esters also include C₅-C₇cycloalkyl esters as well as arylalkyl esters such as, but not limited to benzyl. C₁-C₄alkyl esters are preferred. Esters of the compounds of the present invention may be prepared according to conventional methods.

Examples of pharmaceutically acceptable, non-toxic amides of the compounds of this invention include amides derived from ammonia, primary C₁-C₆alkyl amines and secondary C₁-C₆dialkyl amines wherein the alkyl groups are straight or branched chain. In the case of secondary amines the amine may also be in the form of a 5- or 6-membered heterocycle containing one nitrogen atom. Amides derived from ammonia, C₁-C₃alkyl primary amines, and C₁-C₂dialkyl secondary amines are preferred. Amides of the compounds of the invention may be prepared according to conventional methods.

A therapeutically effective amount is an amount of a compound of Formula I that when administered to a patient, ameliorates a symptom of the disease.

The BCAT inhibitor, which is preferably a compound of Formula I, a BCAT pathway-inhibiting analogue of Formula I, a BCAT pathway-inhibiting prodrug of Formula I, or a pharmaceutically acceptable salt of any of the foregoing, can be administered in accordance with the present inventive method by any suitable route. Suitable routes of administration include systemic, such as orally or by injection, topical, intraocular, periocular (e.g., subTenon's), subconjunctival, subretinal, suprachoroidal and retrobulbar. The manner in which the BCAT inhibitor is administered is dependent, in part, upon whether the treatment of retinopathy is prophylactic or therapeutic. The manner in which the BCAT inhibitor is administered for therapeutic treatment of retinopathy is dependent, in part, upon the cause of the retinopathy.

For example, given that diabetes is the leading cause of retinopathy, the BCAT inhibitor can be administered prophylactically as soon as the pre-diabetic retinopathy state is detected. For the prophylactic treatment of retinopathy that can result from diabetes, the BCAT inhibitor is preferably administered systemically, e.g., orally or by injection. For the therapeutic treatment of nonproliferative diabetic retinopathy, the BCAT inhibitor can be administered systemically, e.g., orally or by injection, or intraocularly. Proliferative diabetic retinopathy can be therapeutically treated by the administration of the BCAT inhibitor intraocularly, topically, subconjunctivally or periocularly (e.g., subTenon's), for example. The BCAT inhibitor is preferably administered intraocularly, topically, subconjunctivally or periocularly (e.g., subTenon's) for the prophylactic or therapeutic treatment of retinopathy before, during or after surgical removal from an eye of scar tissue generated during neovascularization during the proliferative diabetic stage.

The BCAT inhibitor is preferably administered as soon as possible after it has been determined that an animal, such as a mammal, specifically a human, is at risk for retinopathy (prophylactic treatment) or has begun to develop retinopathy (therapeutic treatment). Treatment will depend, in part, upon the particular BCAT inhibitor used, the amount of the BCAT inhibitor administered, the route of administration, and the cause and extent, if any, of retinopathy realized.

One skilled in the art will appreciate that suitable methods of administering a BCAT inhibitor, which is useful in the present inventive method, are available. Although more than one route can be used to administer a particular BCAT inhibitor, a particular route can provide a more immediate and more effective reaction than another route. Accordingly, the described routes of administration are merely exemplary and are in no way limiting.

The dose administered to an animal, particularly a human, in accordance with the present invention should be sufficient to effect the desired response in the animal over a reasonable time frame. One skilled in the art will recognize that dosage will depend upon a variety of factors, including the strength of the particular BCAT inhibitor employed, the age, species, condition or disease state, and body weight of the animal, as well as the amount of the retina about to be affected or actually affected by retinopathy. The size of the dose also will be determined by the route, timing and frequency of administration as well as the existence, nature, and extent of any adverse side effects that might accompany the administration of a particular BCAT inhibitor and the desired physiological effect. It will be appreciated by one of ordinary skill in the art that various conditions or disease states, in particular, chronic conditions or disease states, may require prolonged treatment involving multiple administrations.

Suitable doses and dosage regimens can be determined by conventional range-finding techniques known to those of ordinary skill in the art. Generally, treatment is initiated with smaller dosages, which are less than the optimum dose of the compound. Thereafter, the dosage is increased by small increments until the optimum effect under the circumstances is reached. The present inventive method will typically involve the administration of from about 1 mg/kg/day to about 100 mg/kg/day, preferably from about 15 mg/kg/day to about 50 mg/kg/day, if administered systemically. Intraocular administration typically will involve the administration of from about 0.1 mg total to about 5 mg total, preferably from about 0.5 mg total to about 1 mg total. A preferred concentration for topical administration is 100 mu M.

Compositions for use in the present inventive method preferably comprise a pharmaceutically acceptable carrier and an amount of a BCAT inhibitor sufficient to treat retinopathy prophylactically or therapeutically. The carrier can be any of those conventionally used and is limited only by chemico-physical considerations, such as solubility and lack of reactivity with the compound, and by the route of administration. It will be appreciated by one of ordinary skill in the art that, in addition to the following described pharmaceutical compositions, the BCAT inhibitor can be formulated as polymeric compositions, inclusion complexes, such as cyclodextrin inclusion complexes, liposomes, microspheres, microcapsules and the like (see, e.g., U.S. Pat. Nos. *4,997,652*; *5,185,152*; and *5,718,922*).

The BCAT inhibitor can be formulated as a pharmaceutically acceptable acid addition salt. Examples of pharmaceutically acceptable acid addition salts for use in the pharmaceutical composition include those derived from mineral acids, such as hydrochloric, hydrobromic, phosphoric, metaphosphoric, nitric and sulfuric acids, and organic acids, such as tartaric, acetic, citric, malic, lactic, fumaric, benzoic, glycolic, gluconic, succinic, and arylsulphonic, for example p-toluenesulphonic, acids.

The pharmaceutically acceptable excipients described herein, for example, vehicles, adjuvants, carriers or diluents, are well-known to those who are skilled in the art and are readily available to the public. It is preferred that the pharmaceutically acceptable carrier be one which is chemically inert to the BCAT inhibitor and one which has no detrimental side effects or toxicity under the conditions of use.

The choice of excipient will be determined in part by the particular BCAT inhibitor, as well as by the particular method used to administer the composition. Accordingly, there is a wide variety of suitable formulations of the pharmaceutical composition of the present invention. The following formulations are merely exemplary and are in no way limiting.

Injectable formulations are among those that are preferred in accordance with the present inventive method. The requirements for effective pharmaceutically carriers for injectable compositions are well-known to those of ordinary skill in the art (see Pharmaceutics and Pharmacy Practice, J. B. Lippincott Co., Philadelphia, PA, Banker and Chalmers, eds., pages 238-250 (1982), and ASHP Handbook on Injectable Drugs, Toissel, 4th ed., pages 622-630 (1986). It is preferred that such injectable compositions be administered intramuscularly, intravenously, or intraperitoneally.

Topical formulations are well-known to those of skill in the art. Such formulations are suitable in the context of the present invention for application to the skin. The use of patches, corneal shields (see, e.g., U.S. Pat. No. 5,185,152), and ophthalmic solutions (see, e.g., U.S. Pat. No. *5,710,182)* and ointments, e.g., eye drops, is also within the skill in the art.

Formulations suitable for oral administration can consist of (a) liquid solutions, such as an effective amount of the compound dissolved in diluents, such as water, saline, or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and corn starch. Tablet forms can include one or more of lactose, sucrose, mannitol, com starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

The effectiveness of an orally administered drug is dependent upon the drug's efficient transport across the mucosal epithelium and its stability in entero-hepatic circulation. Drugs that are effective after parenteral administration but less effective orally, or whose plasma half-life is considered too short, may be chemically modified into a prodrug form.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The inhibitor can be administered in a physiologically acceptable diluent in a pharmaceutical carrier, such as a sterile liquid or mixture of liquids, including water, saline, aqueous dextrose and related sugar solutions, an alcohol, such as ethanol, isopropanol, or hexadecyl alcohol, glycols, such as propylene glycol or polyethylene glycol, dimethylsulfoxide, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride, with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants. Oils, which can be used in parenteral formulations include petroleum, animal, vegetable, or synthetic oils. Specific examples of oils include peanut, soybean, sesame, cottonseed, corn, olive, petrolatum, and mineral.

Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metals, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylenepolypropylene copolymers, (d) amphoteric detergents such as, for example, alkyl-p-aminopropionates, and 2-alkyl-imidazoline quaternary ammonium salts, and (e) mixtures thereof.

The parenteral formulations will typically contain from about 0.5 to about 25% by weight of the active ingredient in solution. Preservatives and buffers may be used. In order to minimize or eliminate irritation at the site of injection, such compositions may contain one or more nonionic surfactants having a hydrophile-lipophile balance (HLB) of from about 12 to about 17.

The quantity of surfactant in such formulations will typically range from about 5 to about 15% by weight. Suitable surfactants include polyethylene sorbitan fatty acid esters, such as sorbitan monooleate and the high molecular weight adducts of ethylene oxide with a hydrophobic base, formed by the condensation of propylene oxide with propylene glycol. The parenteral formulations can be presented in unit-dose or multidose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

Such compositions can be formulated as intraocular formulations, sustained-release formulations or devices (see, e.g., U.S. Pat. No. *5,378,475*). For example, gelantin, chondroitin sulfate, a polyphosphoester, such as bis-2-hydroxyethylterephthalate (BHET), or a polylactic-glycolic acid (in various proportions) can be used to formulate sustained-release formulations. Implants (see, e.g., U.S. Pat. Nos. *5,443,505*; *4,853,224*; and *4,997,652*), devices (see, e.g., U.S. Pat. Nos. *5,554,187*; *4,863,457*; *5,098,443*; and *5,725,493*), such as an implantable device, e. g., a mechanical reservoir, an intraocular device or an extraocular device with an intraocular conduit (e.g., 100 mu -1 mm in diameter), or an implant or a device comprised of a polymeric composition as described above, can be used.

The present inventive method also can involve the co-administration of other pharmaceutically active compounds. By "co-administration" is meant administration before, concurrently with, e.g., in combination with the BCAT inhibitor in the same formulation or in separate formulations, or after administration of a BCAT inhibitor as described above. For example, corticosteroids, e.g., prednisone, methylprednisolone, dexamethasone, or triamcinalone acetinide, or noncorticosteroid anti-inflammatory compounds, such as ibuprofen or flubiproben, can be co-administered. Similarly, vitamins and minerals, e.g., zinc, antioxidants, e.g., carotenoids (such as a xanthophyll carotenoid like zeaxanthin or lutein), and micronutrients can be co-administered.

Compounds of the present invention may be obtained by reaction between a hydrazide of the formula ArC(O)NH₂NH₂ with a sulfonyl halide of the formula:

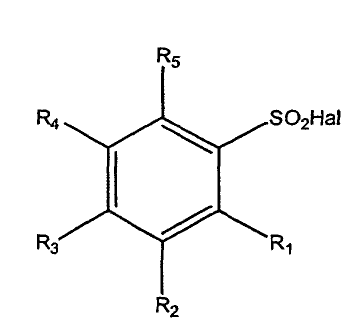

Where R₁, R₂, R₃, R₄, R₅ and Ar are as defined above and Hal is halogen, preferably chlorine. The hydrazide staring materials may be prepared in conventional manner by reaction of the corresponding carboxylic acid with hydrazine. The reaction between the hydrazide and the sulfonyl halide may be carried out in manner known for reaction between these classes of compounds and may conveniently be carried out in an organic solvent and where desired using a catalyst such as an amine catalyst, for example dimethylaminopyridine or N,N-diisopropylethylamine. Such a sequence of reactions is illustrated by the following reaction scheme:

General synthetic scheme for preparing compounds of Formula I

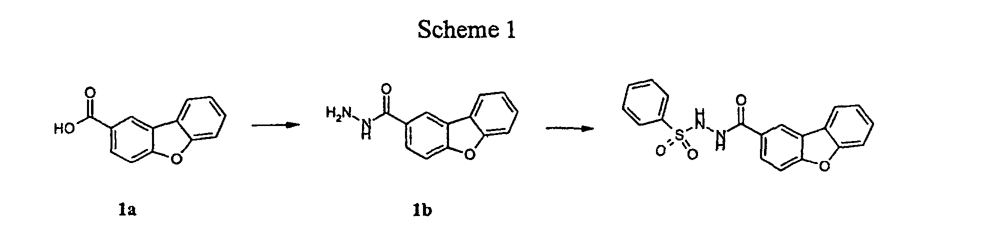

### EXAMPLE 1

### Dibenzofuran-2-carboxylic acid 2-(phenylsulfonyl)hydrazide

### Step 1. Dibenzofuran-2-carboxylic acid (1a) was prepared in accordance with the methods of Ames et al., Synthesis, 1983:234.

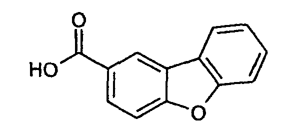

MS: 212.0 (M+1 for C₁₃H₈O₃); mp: 248-249°C; IR (KBr, cm⁻¹): 2825, 1668, 1434, 1310. ¹H NMR (DMSO-d₆) δ 7.39-7.43 (m, 1H], 7.51-7.56 (m, 1H), 7.69-7.77 (m, 2H), 8.06-8.10 (m, 1H), 8.26 (d, 1H, J = 7.6 Hz), 8.74 (d, 1H, J = 1.7 Hz).

### Step 2. Dibenzofuran-2-carboxylic acid hydrazide (1b)

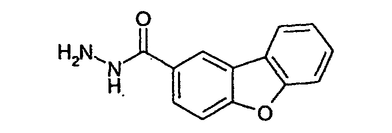

Synthesis of dibenzofuran-2-carboxylic acid hydrazide: Dibenzofuran-2-carboxylic acid (3.50 g, 16.5 mmol) was dissolved in DMF (160 mL), treated with N-methyl-morpholine (4.0 mL, 36.3 mmol), cooled to 0°C, treated with isobutyl chloroformate (2.35 mL, 18.1 mmol), and stirred for 10 minutes. Hydrazine (10.4 mL, 330.0 mmol) was added, and the reaction was allowed to warm to room temperature and stir for 3 hours. The reaction was then diluted with EtOAc (400 mL), washed with saturated sodium bicarbonate solution and brine, dried over Na₂SO₄, and concentrated in vacuo to give 3.40 g (91%) of the desired product as a white solid which was used without further purification.
MS: 227.0 (M+1 for C₁₃H₁₀N₂O₃); mp: 204-205°C; TLC (SiO₂) R_{f}= 0.34 (8% MeOH/CH₂Cl₂); HPLC (C18 column, 1:1 CH₃CN/H₂O + 0.1% TFA) 94.76%, RT = 2.196 min. HRMS (calc): 227.0820 (found): 227.0827. IR (KBr, cm⁻¹): 3303, 1620, 1542, 1472, 1192. ¹H NMR (DMSO-d₆) δ 4.45 (br, 2H), 7.39-7.43 (m, 1H), 7.51-7.55 (m, 1H), 7.72 (t, 2H, J = 8.5 Hz), 7.96 (dd, 1H, J = 8.5, 1.7 Hz), 8.15 (d, 1H, J = 8.3 Hz), 8.61 (d, 1H, J = 1.7 Hz), 9.83 (s, 1H).

### Step 3. Dibenzofuran-2-carboxylic acid 2-(phenylsulfonyl)hydrazide

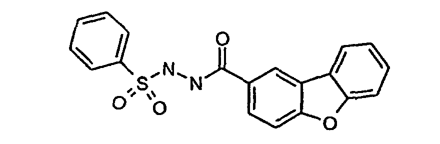

Dibenzofuran-2-carboxylic acid hydrazide (**1b**, 0.3 g, 1.33 mmol) was dissolved in DMF (9 mL) and treated with Hunig's base (0.93 mL, 5.32 mmol). The reaction was cooled to 0°C, treated with benzenesulfonyl chloride (0.186 mL, 1.46 mmol) and a catalytic amount of dimethylaminopyridine (5 mg), then allowed to warm to room temperature and stir overnight. The reaction was then diluted with EtOAc (125 mL), washed with saturated sodium bicarbonate solution and brine, dried over Na₂SO₄, and concentrated in vacuo. The crude material was chromatographed on silica gel eluting with 6% MeOH/CH₂Cl₂ to give 0.25 g (50%) of the desired product.

MS: 367.0 (M+1 for C₁₉H₁₄N₂O₄S); mp: 216-218°C. TLC: SiO₂, R_{f}= 0.58 (1:1 hexane/EtOAc); Analysis (C₁₉H₁₄N₂O₄S) (calc) C: 62.29, H: 3.85, N: 7.65; (found) C: 62.16, H: 3.88, N: 7.60. IR (KBr, cm⁻¹): 3334, 3157, 1659, 1414, 1203, 1172. ¹H NMR (DMSO-d₆) δ 7.41 (t, 1H, J = 7.6 Hz), 7.49-7.61 (m, 4H), 7.71 (t, 2H, J = 8.5 Hz), 7.82-7.83 (m, 3H), 8.16 (d, 1H, J = 7.8 Hz), 8.49 (s, 1H).

### EXAMPLE 2

### 2,3,4,9-Tetrahydro-1H-carbazole-6-carboxylic acid 2-(phenylsulfonyl)hydrazide

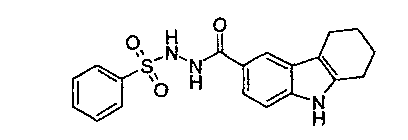

Synthesis of 2,3,4,9-tetrahydro-1*H*-carbazole-6-carboxylic acid 2-(phenylsulfonyl)hydrazide: Example 2 was synthesized in accordance with the methods of Example 1 except that 6,7,8,9-tetrahydro-5*H*-carbazole-3-carboxylic acid hydrazide was used instead of dibenzofuran-2-carboxylic acid hydrazide (56% yield). MS: 370.1 (M+1 for C₁₉H₁₉N₃O₃S₁); mp: >250°C. TLC (SiO₂) R_{f}= 0.28 (1:1 hexane/EtOAc); HPLC (C18 column, 1:1 CH₃CN/H₂O+0.1% TFA) 99.25%, RT = 7.748 min. IR (KBr, cm⁻¹): 3403, 3340, 3124, 2941, 1657, 1422, 1165, Analysis (C₁₉H₁₉N₃O₃S₁); (calc) C: 61.77, H: 5.18, N: 11.37; (found) C: 61.56, H: 5.15, N: 11.17. ¹H NMR (DMSO-d₆) δ 1.76-1.80 (m, 4H), 2.46-2.65 (m, 4H), 7.17 (d, 1H, J = 8.3 Hz), 7.33 (d, 1H, J = 8.5 Hz), 7.44-7.48 (m, 2H), 7.56 (t, 1H, J = 7.1 Hz), 7.76-7.81 (m, 3H), 9.80 (s, 1H), 10.38 (s, 1H), 10.91 (s,1H).

### EXAMPLE 3

### Benzofuran-2-carboxylic acid 2-(phenylsulfonyl)hydrazide

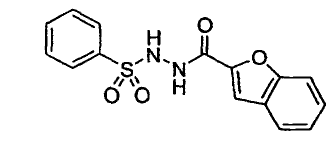

Benzofuran-2-carboxylic acid hydrazide (0.4 g, 2.27 mmol) was suspended in THF (25 mL). N,N-diisopropylethyl amine (1.58 mL, 9.08 mmol) was added, and the reaction was cooled to 0°C. Phenylsulfonyl chloride (0.32 mL, 2.50 mmol) was added followed by DMAP (5 mg, 0.04 mmol). The reaction was allowed to warm to room temperature. The solution was orange after it stirred for 1 hour, and DMF (20 mL) was added and the solution became clear. After 4 hours of stirring, the solution was diluted with EtOAc (200 mL). It was then washed with saturated sodium bicarbonate and brine and dried over Na₂SO₄. The reaction was then concentrated down and purified by flash chromatography eluting with 3:1 hexane/EtOAc. The desired product was isolated (0.38 g, 53.0%).
MS: 317.0 (M+1 for C₁₅H₁₂N₂O₄S). TLC: SiO₂, R_{f}= 0.23 (1:1 hexane/EtOAc). mp: 175-180°C; HRMS: 317.0598 (M+1 for C₁₅H₁₂N₂O₄S), HPLC: (30% H₂O/70% CH₃CN/0.1% TFA), Ret. Time 2.911, Purity: 93.12%. IR (KBr, cm⁻¹) 3338, 3131, 1676, 1581, 1349, 1163. ¹H NMR (400 MHz, CDCl₃) δ 7.23-7.67 (m, 9H), 7.93 (d; . 2H, J = 5.6 Hz), 8.52 (s, 1H).

### EXAMPLE 4

### 5-Methoxy-2-benzofurancarboylic acid 2-(phenylsulfonyl)hydrazide

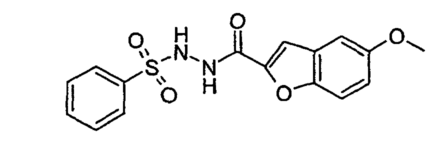

Synthesis of 5-methoxy-2-benzofurancarboxylic acid 2-(phenylsulfonyl)-hydrazide: Example 4 was synthesized in accordance with the methods of Example 1 except that 5-methoxy-benzofuran-2-carboxylic acid was used instead of dibenzofuran-2-carboxylic acid hydrazide (3.2% yield).
MS: 347.0 (M+1 for C₁₆H₁₄N₂O₅S₁); glassy solid; TLC (SiO₂) R_{f}= 0.16 (3:2 hexane/EtOAc); HPLC (C18 column, 1:1 CH₃CN/H₂O+0.1% TFA) 99.12%, RT = 5.813 min. HRMS (calc for M+1) 347.0701 (found) 347.0696. IR (KBr, cm⁻¹): 3302, 3139, 2927, 1666, 1582, 1168. ¹H NMR (DMSO-d₆) δ 3.75 (s, 3H), 7.01 (dd, 1H, J = 9.0, 2.4 Hz), 7.21 (d, 1H, J = 2.4 Hz), 7.49-7.53 (m, 4H), 7.61 (t, 1H, J = 7.6 Hz), 7.79-7.81 (m, 2H), 10.13 (br, 1H), 10.85 (br, 1H).

### EXAMPLE 5

### 7-Methoxy-2-benzofurancarboxylic acid 2-(phenylsulfonyl)hydrazide

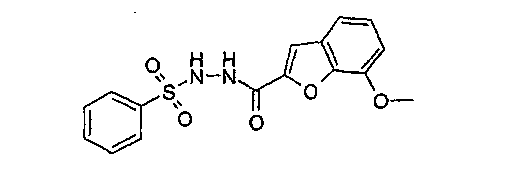

Synthesis of 7-methoxy-2-benzofurancarboxylic acid 2-(phenylsulfonyl)-hydrazide: Example 5 was synthesized in accordance with the methods of Example 1 except that 7-methoxy-benzofuran-2-carboxylic acid was used instead of dibenzofuran-4-carboxylic acid (64% yield).
MS: 347.0 (M+1 for C₁₆H₁₄N₂O₅S₁); mp: 164-165°C. TLC (SiO₂) R_{f}= 0.35 (1:1 hexane/EtOAc); HPLC (C18 column, 40:60 CH₃CN/H₂O+0.05% TFA) 99.87%, RT = 11.355 min. HRMS (calc for M+1) 347.0701 (found) 347.0702. IR (KBr, cm⁻¹): 3232, 1675, 1588, 1490, 1166. ¹H NMR (CDCl₃) δ 4.03 (s, 3H), 6.94 (dd, 1H, J = 7.1, 2.0 Hz), 7.21-7.26 (m, 2H), 7.34-7.37 (m, 2H), 7.45-7.50 (m, 2H), 7.58-7.63 (m, 1H), 7.93-7.96 (m, 2H), 8.61 (d, 1H, J = 6.1 Hz).

### EXAMPLE 6

### 7-Ethoxy-2-benzofurancarboxylic acid 2-(phenylsulfonyl)hydrazide

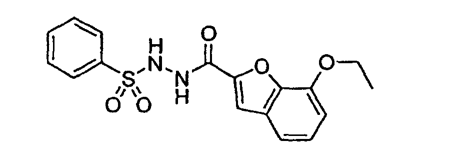

7-Ethoxy-benzofuran-2-carboxylic acid hydrazide (0.5 g, 2.27 mmol) was dissolved in THF (25 mL). N,N-diisopropylethylamine (1.58 mL, 9.08 mmol) was added, and the reaction was cooled to 0°C. Phenylsulfonyl chloride (0.32 mL, 2.50 mmol) was added followed by DMAP (5 mg, 0.04 mmol). The reaction was allowed to warm to room temperature. The solution was orange after it stirred for 1 hour. After 3 hours of stirring, the solution was washed with saturated sodium bicarbonate and brine and dried over Na₂SO₄. The reaction was then concentrated down and purified by flash chromatography eluting with 1:1 hexane/EtOAc. The desired product was isolated (0.05 g, 5.6%).
MS: 361.0 (M+1 for C₁₇H₁₆N₂O₅S). TLC: SiO₂, R_{f}= 0.17 (1:1 hexane/EtOAc); mp: 223-224°C. HRMS: (calc) 361.0558, (found) 361.0853 (M+1 for C₁₇H₁₆N₂O₅S) HPLC: (50% H₂O/50% CH₃CN/0.1% TFA) Ret. Time: 2.596, Purity: 98.71%. IR (KBr, cm⁻¹) 3268, 3124, 2982, 1666, 1586, 1344, 1166, 1087, 724. ¹H NMR (400 MHz, DMSO-d₆) δ 1.37 (t, 3H, J = 6.8 Hz), 4.19 (q, 2H, J = 7.1 Hz), 7.01 (d, 1H,

J = 8.0 Hz), 7.16-7.26 (m, 2H), 7.49-7.62 (m, 4H), 7.81 (d, 1H, J = 7.3 Hz), 10.17 (s, 1H), 10.92 (s, 1H).

### EXAMPLE 7

### 7-Chloro-benzofuran-2-carboxylic acid 2-(phenylsulfonyl)hydrazide

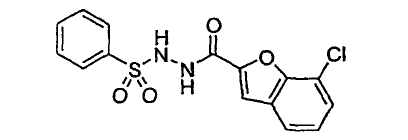

7-Chloro-benzofuran-2-carboxylic acid hydrazide (0.5 g, 2.38 mmol) was suspended in THF (26 mL). N,N-diisopropylethylamine (1.66 mL, 9.52 mmol) was added, and the reaction was cooled to 0°C. Phenylsulfonyl chloride (0.33 mL, 2.62 mmol) was added followed by DMAP (5 mg, 0.04 mmol). The reaction was allowed to warm to room temperature. The solution was yellow after it stirred for 2 hours, and DMF (10 mL) was added. After another hour of stirring, the solution was diluted with EtOAc (150 mL), and washed with saturated sodium bicarbonate and brine and dried over Na₂SO₄. The reaction was then concentrated down and purified by flash chromatography eluting with 3:1 hexane/EtOAc. The desired product was isolated (0.04 g, 4.6%).
MS: 351.0 (M+1 for C₁₅H₁₁N₂O₄SCl). TLC: SiO₂, R_{f} = 0.57 (1:1 hexane/EtOAc); mp: 223-224°C; HRMS: (calc) 351.0206, (found) 351.0215 (M+1 for C₁₅H₁₁N₂O₄SCl) HPLC: Ret. Time 9.862, Purity: 98.26%. IR (KBr, cm⁻¹) 3291, 3033, 1670, 1344, 1164. ¹H NMR (400 MHz, CDCl₃) δ 7.24-7.64 (m, 7H), 7.94 (d, 2H, J = 7.1 Hz), 8.62 (s, 1H), 9.12 (s, 1H).

### EXAMPLE 8

### 5-Chloro-benzofuran-2-carboxylic acid 2-(phenylsulfonyl)hydrazide

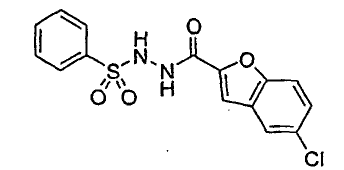

5-Chloro-benzofuran-2-carboxylic acid hydrazide (0.4 g, 1.90 mmol) was suspended in THF (21 mL). N,N-diisopropylethylamine (1.32 mL, 7.6 mmol) was added, and the reaction was cooled to 0°C. Phenylsulfonyl chloride (0.27 mL, 2.09 mmol) was added followed by DMAP (5 mg, 0.04 mmol). The reaction was allowed to warm to room temperature. The solution was orange after it stirred for 2 hours, and DMF (10 mL) was added and the solution became clear. After 15 minutes of stirring, the solution was diluted with EtOAc (200 mL). It was then washed with saturated sodium bicarbonate and brine and dried over Na₂SO₄. The reaction was then concentrated down and purified by flash chromatography eluting with 2:1 hexane/EtOAc. The desired product was isolated (0.03 g, 3.8%).
MS: 350.9 (M+1 for C₁₅H₁₁N₂O₄SCl). TLC: SiO₂, R_{f}= 0.55 (1:1 hexane/EtOAc);
mp: 181-183°C; HRMS: (calc) 351.0206, (found) 351.0206 (M+1 for C₁₅H₁₁N₂O₄SCl) HPLC: (50% H₂O/50% CH₃CN/0.1% TFA) Ret. Time 8.755,
Purity: 97.13%. IR (KBr, cm⁻¹) 3291, 3066, 1670, 1344, 1164. ¹H NMR (400 MHz, CDCl₃) δ 4.79 (br, 1H), 7.24-7.69 (m, 7H), 7.92 (d, 2H, J = 7.6 Hz), 8.51 (br, 1H).

### EXAMPLE 9

### 2,3-Dihydro-benzofuran-5-carboxylic acid 2-(phenylsulfonyl)hydrazide

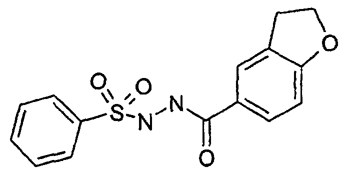

2,3-Dihydro-benzofuran-5-carboxylic acid hydrazide (0.25 g, 1.40 mmol) was dissolved in THF (16 mL). N,N-diisopropylethylamine (0.98 mL, 5.6 mmol) was added, and the reaction was cooled to 0°C. Phenylsulfonyl chloride (0.20 mL, 1.54 mmol) was added followed by DMAP (5 mg, 0.04 mmol). The reaction was allowed to warm to room temperature. After 2 hours of stirring, the solution was diluted with EtOAc (200 mL), and washed with saturated sodium bicarbonate and brine and dried over Na₂SO₄. The reaction was then concentrated down and purified by flash chromatography eluting with 1:1 hexane/EtOAc. The desired product was isolated (0.09 g, 20.0%).
MS: 319.0 (M+1 for C₁₅H₁₄N₂O₄S). mp: 181-183 °C; TLC: SiO₂, R_{f} = 0.46 (1:1 hexane/EtOAc); HRMS: (calc) 319.0752, (found) 319.0761 (M+1 for C₁₅H₁₄N₂O₄S)
HPLC: (50% H₂O/50% CH₃CN/0.1% TFA) Ret. Time 9.862, Purity: 95.04%. ¹H NMR (400 MHz, DMSO-d₆) δ 3.13 (t, 2H, J = 8.8 Hz), 4.54 (t, 2H, J = 8.8 Hz), 6.74 (d, 1H, J = 8.3 Hz), 7.46-7.57 (m, 5H), 7.77 (d, 2H, J = 7.0 Hz), 9.85 (s, 1H), 10.42 (s, 1H).

### EXAMPLE 10

### Benzo[1,3] dioxole-5-carboxylic acid 2-(phenylsulfonyl)hydrazide

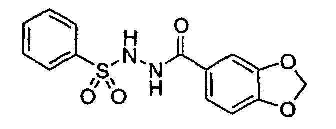

Benzo[1,3] dioxole-5-carboxylic acid hydrazide (0.5 g, 3.33 mmol) was suspended in THF (37 mL). N,N-diisopropylethylamine (2.32 mL, 13.32 mmol) was added, and the reaction was cooled to 0°C. Phenylsulfonyl chloride (0.47 mL, 3.66 mmol) was added followed by DMAP (5 mg, 0.04 mmol). The reaction was allowed to warm to room temperature. The solution was orange after it stirred for 1 hour, and DMF (5 mL) was added and the solution became clear. After 2 hours of stirring, the solution was diluted with EtOAc (100 niL). It was then washed with saturated sodium bicarbonate and brine and dried over Na₂SO₄. The reaction was then concentrated down and purified by flash chromatography eluting with 3:1 hexane/EtOAc. The desired product was isolated (0.09 g, 8.8%).
MS: 321.0 (M+1 for C₁₄H₁₂N₂O₅S). TLC: SiO₂, R_{f} = 0.59 (1:1 hexane/EtOAc); mp: 156-158°C. HPLC: (50% H₂O/50% CH₃CN/0.1% TFA) Ret. Time: 5.359, Purity: 95.27%. Analysis: (C₁₄H₁₂N₂O₅S) (calc) C: 52.49, H: 3.78, N: 8.75 (found) C: 52.61, H: 4.06, N: 8.35. IR (KBr, cm⁻¹) 3210, 2920, 1643, 1502, 1390, 1251, 1168, 1029. ¹H NMR (400 MHz, DMSO-d₆) δ 6.05 (s, 2H), 6.92 (d, 1H, J = 8.1 Hz), 7.15 (s, 1H), 7.24 (d, 1H, J = 8.1 Hz), 7.48 (t, 2H, J = 7.8 Hz), 7.58 (t, 1H, J = 7.6 Hz), 7.77 (d, 2H, J = 7.1 Hz), 9.92 (s, 1H), 10.49 (s, 1H).

### EXAMPLE 11

### 1H-Indole-5-carboxylic acid 2-(phenylsulfonyl)hydrazide

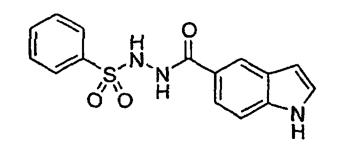

1*H*-Indole-5-carboxylic acid hydrazide (0.5 g, 2.85 mmol) was suspended in THF (32 mL). N,N-diisopropylethylamine (1.99 mL, 11.40 mmol) was added, and the reaction was cooled to 0°C. Phenylsulfonyl chloride (0.36 mL, 2.85 mmol) was added followed by DMAP (5 mg, 0.04 mmol). The reaction was allowed to warm to room temperature, and DMF (3 mL) was added. After 3 hours of stirring, the solution was diluted with EtOAc (200 mL). It was then washed with saturated sodium bicarbonate and brine and dried over Na₂SO₄. The reaction was then concentrated down and purified by flash chromatography eluting with 2:1 hexane/EtOAc. The desired product was isolated (0.41 g, 23.8%).
MS: 316.0 (M+1 for C₁₅H₁₃N₃O₃S). TLC: SiO₂, R_{f}= 0.38 (1:1 hexane/EtOAc); mp: 209-211°C. HPLC: (50% H₂O/50% CH₃CN/0.1% TFA) Ret. Time: 4.102, Purity: 96.75%. Analysis (C₁₅H₁₃N₃O₃S) (calc) C: 57.13, H: 4.16, N: 13.32 (found) C: 57.28, H: 4.08, N: 13.06. IR(KBr, cm⁻¹) 3293, 3168, 1649, 1312, 1168. ¹H NMR (400 MHz, DMSO-d₆) δ 6.48 (s, 1H), 7.33-7.41 (m, 3H), 7.47 (t, 2H, J = 7.3 Hz), 7.57 (t, 1H, J = 7.4 Hz), 7.80 (d, 2H, J = 7.3 Hz), 7.94 (s, 1H), 9.84 (s, 1H), 10.45 (s, 1H), 11.33 (s, 1H).

### EXAMPLE 12

### 1-Dibenzofurancarboxylic acid 2-(phenylsulfonyl)hydrazide

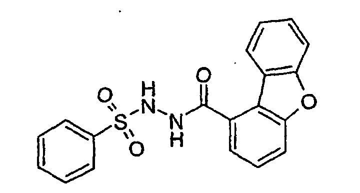

Synthesis of 1-dibenzofurancarboxylic acid 2-(phenylsulfonyl)hydrazide: Example 12 was synthesized in accordance with the methods of Example 1 except that dibenzofuran-1-carboxylic acid hydrazide was used instead of dibenzofuran-2-carboxylic acid hydrazide (8.4%).
MS: 367.0 (M+1 for C₁₉H₁₄N₂O₄S₁); glassy solid; TLC (SiO₂) R_{f} = 0.77 (8% MeOH/CH₂Cl₂); HPLC (C18 column, 7:3 CH₃CN/H₂O+0.1% TFA) 91.94%, RT = 4.008 min. HRMS (calc for M+1) 367.0752 (found) 367.0744. IR (KBr, cm⁻¹): 3231, 1653, 1341, 1161. ¹H NMR (DMSO-d₆) δ 7.20-7.23 (m, 1H), 7.47-7.92 (m, 11H), 10.32 (d, 1H, J = 3.6 Hz), 10.90 (d, 1H, J = 3.6 Hz).

### EXAMPLE 13

### Quinoline-6-carboxylic acid 2-(phenylsulfonyl)hydrazide

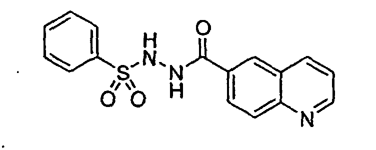

Quinoline-6-carboxylic acid hydrazide (1.0 g, 5.34 mmol) was dissolved in THF (60 mL). N,N-diisopropylethylamine (3.72 mL, 21.36 mmol) was added, and the reaction was cooled to 0°C. Phenylsulfonyl chloride (0.67 mL, 5.34 mmol) was added followed by DMAP (5 mg, 0.04 mmol). The reaction was allowed to warm to room temperature. After 3.5 hours of stirring, the red solution was washed with saturated sodium bicarbonate and brine and dried over Na₂SO₄. The reaction was then concentrated down and recrystallized from dichloromethane. The desired product was isolated (0.155 g, 8.9%).
MS: 328.0 (M+1 for C₁₆H₁₃N₃O₃S). TLC: SiO₂, R_{f} = 0.42 (1:1 hexane/EtOAc); mp: 211-214°C. HPLC: (50% H₂O/50% CH₃CN/0.1% TFA) Ret. Time: 1.985, Purity: 90.33%. Analysis (C₁₆H₁₃N₃O₃S·0.2H₂O) (calc) C: 58.06, H: 4.08, N: 12.70; (found) C: 57.72, H: 4.04, N: 12.49. IR (KBr, cm⁻¹) 3308, 3033, 2817, 1690, 1329, 1164. ¹H NMR (400 MHz, DMSO-d₆) δ 7.48-7.61 (m, 4H), 7.84 (d, 2H, J = 7.3 Hz), 7.93 (dd, 1H, J = 8.8, 1.7 Hz), 8.01 (d, 1H, J = 8.8 Hz), 8.35 (s, 1H), 8.44 (d, 1H, J = 7.6 Hz), 8.94-8.96 (m, 1H), 10.57 (br, 2H).

### EXAMPLE 14

### 2-Dibenzofurancarboxylic acid 2-[(2-methylphenyl)sulfonyl]hydrazide

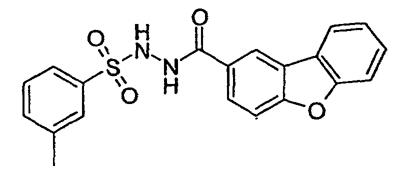

Synthesis of 2-dibenzofurancarboxylic acid 2-[(2-methylphenyl)sulfonyl]-hydrazide: Example 14 was synthesized in accordance with the methods of Example 1 except that 3-methylbenzenesulfonyl chloride was used instead of benzenesulfonyl chloride (33% yield).
MS: 381.0 (M+1 for C₂₀H₁₆N₂O₄S₁); mp 194-195°C. TLC (SiO₂) R_{f}= 0.36 (3:2 hexane/EtOAc); HPLC (C18 column, 70:30 CH₃CN/H₂O+0.1% TFA) 98.78%, RT = 4.423 min. HRMS (calc for M+1) 381.0909 (found) 381.0915; IR (KBr, cm⁻¹): 3300, 3147, 2964, 1664, 1435, 1157. ¹H NMR (DMSO-d₆) δ 2.29 (s, 3H), 7.35-7.43 (m, 3H), 7.52-7.55 (m, 1H), 7.62-7.74 (m, 4H), 7.84 (d, 1H, J = 8.5 Hz), 8.16 (d, 1H, J = 7.8 Hz), 8.49 (s, 1H), 10.00 (s, 1H), 10.74 (s, 1H).

### EXAMPLE 15

### 2-Dibenzofurancarboxylic acid 2-[(3-methylphenyl)sulfonyl]hydrazide

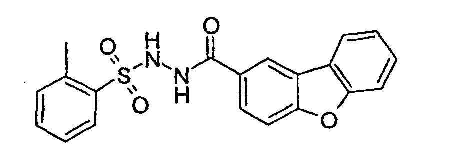

Synthesis of 2-dibenzofurancarboxylic acid 2-[(3-methylphenyl)sulfonyl]-hydrazide: Example 15 was synthesized in accordance with the methods of Example 1 except that 2-methylbenzenesulfonyl chloride was used instead of benzenesulfonyl chloride (11% yield).
MS: 381.0 (M+1 for C₂₀H₁₆N₂O₄S₁); mp 234-235°C. TLC (SiO₂) R_{f}= 0.43 (3:2 hexane/EtOAc); HPLC (C18 column, 70:30 CH₃CN/H₂O+0.1% TFA) 97.30%, RT = 4.434 min. HRMS (calc for M+1) 381.0909 (found) 381.0911; IR (KBr, cm⁻¹): 3324, 3144, 1657, 1330, 1164. ¹H NMR (DMSO-d₆) δ 2.71 (s, 3H), 7.21-7.55 (m, 5H), 7.68-7.72 (m, 2H), 7.80-7.85 (m, 2H), 8.14 (d, 1H, J = 7.8 Hz), 8.47 (s, 1H), 9.99 (s, 1H), 10.69 (s, 1H).

### EXAMPLE 16

### 2-Dibenofurancarboxylic acid 2-[(3-chlorophenyl)sulfonyl]hydrazide

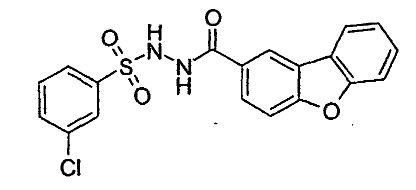

Synthesis of 2-dibenofurancarboxylic acid 2-[(3-chlorophenyl)sulfonyl]-hydrazide: Example 16 was synthesized in accordance with the methods of Example 1 except that 3-chlorobenzenesulfonyl chloride was used instead of benzenesulfonyl chloride (3.0% yield).
MS: 401.0 (M+1 for C₁₉H₁₃N₂O₄S₁Cl₁); mp 208-209°C. TLC (SiO₂) R_{f}= 0.30 (3:2 hexane/EtOAc); HPLC (C 18 column, 70:30 CH₃CN/H₂O+0.1% TFA) 100%, RT = 4.741 min. HRMS (calc for M+1) 401.0363 (found) 401.0367. IR (KBr, cm⁻¹): 3330, 3004, 2804, 1655, 1344, 1192, 1160. ¹H NMR (DMSO-d₆) δ 7.42 (t, 1H, J = 7.6 Hz), 7.54 (t, 2H, J = 7.8 Hz), 7.68-7.85 (m, 6H), 8.16 (d, 1H, J = 7.6 Hz), 8.50 (s, 1H), 10.31 (s, 1H), 10.82 (s, 1H).

### EXAMPLE 17

### 2-Dibenzofurancarboxylic acid 2-[(2-chlorophenyl)sulfonyl]hydrazide

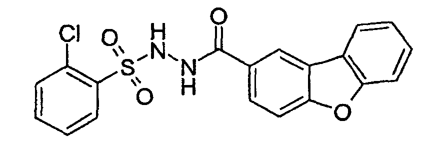

Synthesis of 2-dibenzofurancarboxylic acid 2-[(2-chlorophenyl)sulfonyl]-hydrazide: Example 17 was synthesized in accordance with the methods of Example 1 except that 2-chlorobenzenesulfonyl chloride was used instead of benzenesulfonyl chloride (25% yield).
MS: 401.1 (M+1 for C₁₉H₁₃N₂O₄S₁Cl₁); mp 235-236°C. TLC (SiO₂) R_{f}= 0.38 (2:1 hexane/EtOAc); HPLC (C18 column, 70:30 CH₃CN/H₂O+0.1% TFA) 97.79%, RT = 4.390 min. HRMS (calc for M+1) 401.0363 (found) 401.0359; IR (KBr, cm⁻¹): 3338, 3124, 2959, 1719, 1171. ¹H NMR (DMSO-d₆) δ 7.38-7.73 (m, 7H), 7.85 (d, 1H, J = 8.5 Hz), 7.97 (d, 1H, J = 8.1 Hz), 8.14 (d, 1H, J = 7.6 Hz), 8.51 (s, 1H), 10.15 (s, 1H), 10.75 (s, 1H).

### EXAMPLE 18

### Dibenzofuran-2-carboxylic acid 2-(3,5-dichlorophenylsulfonyl) hydrazide

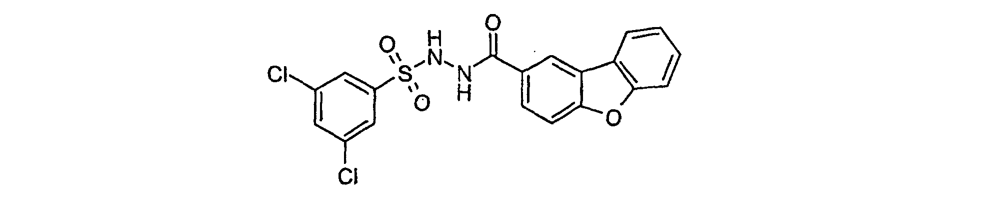

Synthesis of dibenzofuran-2-carboxylic acid 2-(3,5-dichlorophenylsulfonyl)hydrazide: Example 18 was synthesized in accordance with the methods of Example 1 except that 3,5-dichlorobenzenesulfonyl chloride was used instead of benzenesulfonyl chloride (6.7% yield).
MS: 435.0 (M+1 for C₁₉H₁₂N₂O₄S₁Cl₂); mp: 231-233°C. TLC (SiO₂) R_{f}= 0.26 (8% MeOH/CH₂Cl₂); HPLC (C18 column, 1:1 CH₃CN/H₂O+0.1% TFA) 94.51%, RT = 34.421 min. HRMS (calc for M+1) 434.9973 (found) 434.9981. IR (KBr, cm⁻¹): 3327, 3079, 2754, 1657, 1349, 1167. ¹H NMR (DMSO-d₆) δ 7.42-7.46 (m, 2H), 7.52-7.58 (m, 2H), 7.71-7.87 (m, 5H), 8.17-8.22 (m, 1H), 10.51 (br, 1H), 10.87 (br, 1H).

### EXAMPLE 19

### Dibenzofuran-2-carboxylic acid 2-(3-chloro-2-methyl-phenylsulfonyl)hydrazide

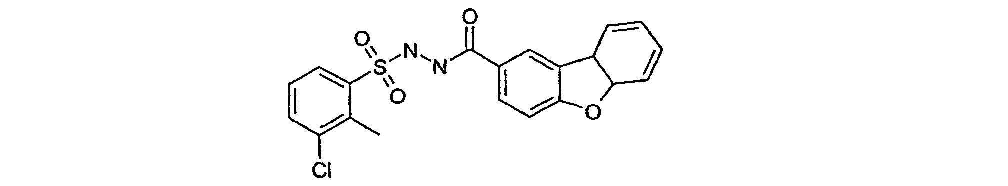

Synthesis of dibenzofuran-2-carboxylic acid 2-(3-chloro-2-methylphenylsulfonyl)hydrazide: Example 19 was synthesized in accordance with the methods of Example 1 except that 3-chloro-2-methyl-benzenesulfonyl chloride was used instead of benzenesulfonyl chloride (4.8% yield).
MS: 415.0 (M+1 for C₂₀H₁₅N₂O₄S₁Cl₁); mp: 192-194°C; TLC (SiO₂) R_{f}= 0.41 (8% MeOH/CH₂Cl₂); HRMS (calc for M+1): 415.0519 (found): 415.0510. HPLC (C18 column, 7:3 CH₃CN/H₂O+0.1% TFA) 96.38%, RT = 11.222 min. IR (KBr, cm⁻¹): 3316, 3042, 1665, 1346, 1191, 1148. ¹H NMR (DMSO-d₆) δ 2.74 (s, 3H), 7.27 (t, 1H, J = 8.1 Hz), 7.41 (t, 1H, J = 7.6 Hz), 7.53 (t, 1H, J = 7.3 Hz), 7.65-7.73 (m, 3H), 7.80-7.87 (m, 2H), 8.15 (d, 1H, J = 7.3 Hz), 8.48 (s, 1H), 10.27 (br, 1H), 10.73 (br, 1H).

### EXAMPLE 20

### Quinoline-6-carboxylic acid 2-(phenylsulfonyl)hydrazide

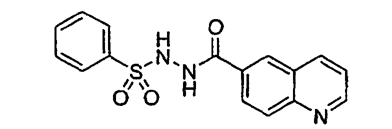

Quinoline-6-carboxylic acid hydrazide (1.0 g, 5.34 mmol) was dissolved in THF (60 mL). N,N-diisopropylethylamine (3.72 mL, 21.36 mmol) was added, and the reaction was cooled to 0°C. Phenylsulfonyl chloride (0.67 mL, 5.34 mmol) was added followed by DMAP (5 mg, 0.04 mmol). The reaction was allowed to warm to room temperature. After 3.5 hours of stirring, the red solution was washed with saturated sodium bicarbonate and brine and dried over Na₂SO₄. The reaction was then concentrated down and recrystallized from dichloromethane. The desired product was isolated (0.155 g, 8.9%).
MS: 328.0 (M+1 for C₁₆H₁₃N₃O₃S). TLC: SiO₂, R_{f} = 0.42 (1:1 hexane/EtOAc); mp 211-214°C. HPLC: (50% H₂O/50% CH₃CN/0.1% TFA) Ret. Time: 1.985, Purity: 90.33%. Analysis (C₁₆H₁₃N₃O₃S·0.2H₂O) (calc) C: 58.06, H: 4.08, N: 12.70 (found) C: 57.72, H: 4.04, N: 12.49. IR (KBr, cm⁻¹) 3308, 3033, 2817, 1690, 1329, 1164. ¹H NMR (400 MHz, DMSO) δ 7.48-7.61 (m, 4H), 7.84 (d, 2H, J = 7.3 Hz), 7.93 (dd, 1H, J = 8.8, 1.7 Hz), 8.01 (d, 1H, J = 8.8 Hz), 8.35 (s, 1H), 8.44 (d, 1H, J = 7.6 Hz), 8.94-8.96 (m, 1H), 10.57 (br, 2H).

### EXAMPLE 21

### Quinoline-8-carboxylic acid 2-(phenylsulfonyl)hydrazide

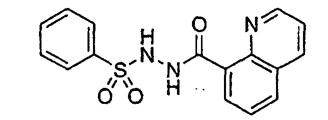

Quinoline-8-carboxylic acid hydrazide (0.3 g, 1.6 mmol) was dissolved in THF (18 mL). N,N-diisopropylethylamine (1.12 mL, 6.41 mmol) was added, and the reaction was cooled to 0°C. Phenylsulfonyl chloride (0.20 mL, 1.6 mmol) was added followed by DMAP (5 mg, 0.04 mmol). The reaction was allowed to warm to room temperature. After 1.5 hours of stirring, the red solution was washed with saturated sodium bicarbonate and brine and dried over Na₂SO₄. The reaction was then concentrated down and purified by flash chromatography eluting with 3% MeOH/CH₂Cl₂. The desired product was isolated (0.158 g, 30.2%).
MS: 328.0 (M+1 for C₁₆H₁₃N₃O₃S). TLC: SiO₂, R_{f}=0.42 (1:1 hexane/EtOAc); mp: 173-175°C. HPLC: (50% H₂O/50% CH₃CN/0.1% TFA) Ret. Time: 3.952, Purity: 96.23%. Analysis (C₁₆H₁₃N₃O₃S) (calc) C: 58.70, H: 4.00, N: 12.84 (found) C: 58.80, H: 4.21, N: 12.66. IR (KBr, cm⁻¹) 3190, 1665, 1341, 1172. ¹H NMR (400 MHz, DMSO) δ 7.56 (t, 2H, J = 7.8 Hz), 7.63-6.71 (m, 3H), 7.85 (d, 2H, 7.6 Hz), 8.20 (dd, 1H, J = 1.2, 8.1 Hz), 8.32 (d, 1H, J = 7.3 Hz), 8.55 (dd, 1H, J = 1.8, 8.4 Hz), 8.81-8.82 (m, 1H), 10.24 (s, 1H), 12.34 (s, 1H).

### EXAMPLE 22

### 2-Benzofurancarboxylic acid, 5-chloro-2-[(4-fluorophenyl)]sulfonyl hydrazide

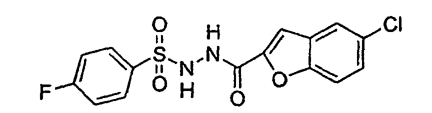

This was run in accordance with the Example 1, Step 3 except that 4-fluorophenyl sulfonyl chloride and 5-chloro-benzofuran-2-carboxylic acid hydrazide were used instead of the benzenesulfonyl chloride and dibenzofuran-2-carboxylic acid hydrazide. The desired product was purified by columning over SiO₂ eluting with 1:2 EtOAc/hexane and triturating the combined and concentrated fractions in diethyl ether (7.9% yield).
MS: 369.0 (M+1 for C₁₅H₁₀N₂O₄SFCl); mp: 229.4-230.5°C. TLC: SiO₂,
R_{f} = 0.75 (EtOAc/hexane; 1:1); HPLC (C18 column, 1:1 MeCN/H₂O+0.1% TFA) 99.1%, RT = 6.8 min. ¹H NMR (DMSO-d₆) δ 7.36 (t, 2H, J = 9.03 Hz), 7.46 (dd, 1H, J = 8.79 and 2.2 Hz), 7.56 (s, 1H), 7.65 (d, 1H, J = 8.79 Hz), 7.85-7.87 (m, 3H), 10.27 (s, 1H), and 11.01 (s, 1H).

### EXAMPLE 23

### 2-Benzofurancarboxylic acid, 7-methoxy-, 2-[(4-fluoro-phenyl)sulfonyl]hydrazide

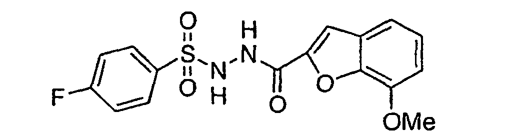

This was run in accordance with the Example 22 except that 7-methoxybenzofuran-2-carboxylic acid hydrazide was used instead of 5-chloro-benzofuran-2-carboxylic acid hydrazide. The desired product was purified by columning over SiO₂ eluting with 1:2 EtOAc/hexane and triturating the combined and concentrated fractions in diethyl ether (8.2% yield).
MS: 365.0 (M+1 for C₁₆H₁₃N₂O₅SF); mp: 182.7-183.3°C. TLC: SiO₂,
R_{f}= 0.75 (EtOAc/hexane; 1:1); HPLC (C18 column, 1:1 MeCN/H₂O+0.1% TFA) 95.7%, RT = 5.3 min. ¹H NMR (DMSO-d₆) δ 3.89 (s, 3H), 7.01 (d, 1H, J = 7.81 Hz), 7.20 (t, 1H, J = 7.87 Hz), 7.25 (d, 1H, J = 7.57 Hz), 7.35 (t, 2H, J = 8.54 Hz), 7.56 (s, 1H), 7.83 (dd, 2H, J = 8.30 and 6.59 Hz), 10.27 (s, 1H), and 11.01 (s, 1H).

### EXAMPLE 24

### 2-Dibenzofurancarboxylic acid 2-[(4-fluorophenyl)sulfonyl]hydrazide

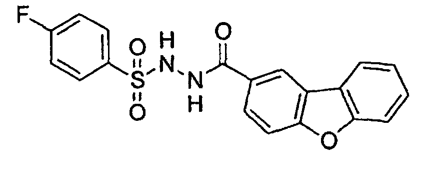

This was run in accordance with Example 1, Step 3 except that 4-fluorophenyl sulfonyl chloride was used instead of benzenesulfonyl chloride (23.1% yield).
MS: 385.0 (M+1 for C₁₉H₁₃N₂O₄FS); mp: 206.3-207.0°C. TLC: SiO₂,
R_{f}= 0.85 (EtOAc/hexane; 1:1); HPLC (C18 column, 1:1 MeCN/H₂O+0.1% TFA) 98.0%, RT = 9.72 min. ¹H NMR (CDCl₃) δ 7.08 (t, 2H, J = 8.30 Hz), 7.35 (t, 1H, J = 7.33 Hz), 7.45-7.55 (m, 4H), 7.17 (dd, 1H, J = 8.55 and 1.71 Hz), 7.9-7.96 (m, 3H), 8.19 (d, 1H, J = 5.86 Hz), 8.24 (d, 1H, J = 1.47 Hz).

### EXAMPLE 25

### 2-Dibenzofurancarboxylic acid 2-[(2-chloro-4-fluorophenyl)sulfonyl]hydrazide

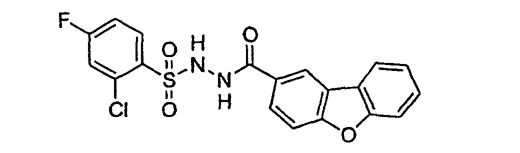

This was run in accordance with Example 1, Step 3 except that 2-chloro-4-fluorophenyl sulfonyl chloride was used instead of benzenesulfonyl chloride (4.3% yield).
MS: 419.0 (M+1 for C₁₉H₁₂N₂O₄FSCl); mp: 227.5-227.8°C. TLC: SiO₂,
R_{f}= 0.85 (EtOAc/hexane; 1:1); HPLC (C18 column, 1:1 MeCN/H₂O+0.1% TFA) 97.7%, RT = 13.2 min. ¹H NMR (CDCl₃) δ 6.94 (dd, 1H, J = 10.0 and 2.44 Hz), 7.25 (dd, 1H, J = 7. 81 and 1.95 Hz), 7.34 (t, 1H, J = 8.06 Hz), 7.46 (t, 1H, J = 7.33 Hz), 7.55-7.70 (m, 2H), 7.69 (d, 1H, J = 8.55 Hz), 7.89 (d,1H, J = 7.82 Hz), 7.99-8.08 (m, 3H), 8.23 (s, 1H).

### EXAMPLE 26

### 2-Dibenzofurancarboxylic acid 2-[(3-cyanophenyl)sulfonyl]hydrazide

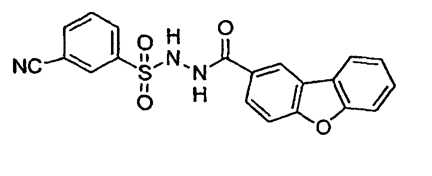

This was run in accordance with Example 1, Step 3 except that 3-cyanobenzene sulfonyl chloride was used instead of benzenesulfonyl chloride (7.2% yield).
MS: 392.0.0 (M+1 for C₂₀H₁₃N₃O₄S); mp: 234-236°C. TLC: SiO₂, R_{f}= 0.20 (EtOAc/hexane; 1:2); HPLC (C18 column, 1:1 MeCN/H₂O+0.1% TFA) 98.8%, RT = 8.12 min. ¹H NMR (DMSO-d₆) δ 7.39 (m, 1H), 7.52 (m, 1H), 7.55-7.70 (m, 1H), 7.68-7.72 (m, 3H), 7.81 (dd, 1H, J = 8.79 and 1.95 Hz), 8.06-8.16 (m, 3H), 8.23 (s, 1H), 8.47 (d, 1H, J = 1.46 Hz), 10.41 (s, 1H), 10.84 (s, 1H).

### EXAMPLE 27

### 2-Benzofurancarboxylic acid, 5-chloro-, 2-[(3-cyanophenyl)sulfonyl]hydrazide

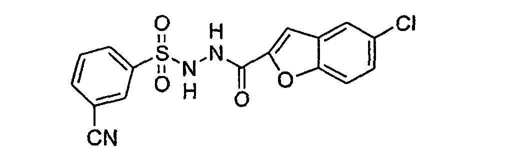

This was run in accordance with Example 8 except 3-cyanobenzene sulfonyl chloride was used instead of benzenesulfonyl chloride (2.65% yield).
MS: 375.9 (M+1 for C₁₆H₁₀N₃O₄SCl); mp: 208.5-210°C. TLC: SiO₂, R_{f}= 0.25 (EtOAc/hexane; 1:2); HPLC (C18 column, 1:1 MeCN/H₂O+0.1% TFA) 95.3%, RT = 6.12 min. ¹H NMR (DMSO-d₆) δ 7.45 (dd, 1H, J = 8.79 and 1.71 Hz), 7.57 (s, 1H), 7.65 (d, 1H, J = 8.79 Hz), 7.74 (t, 1H, J = 8.06 Hz), 7.84 (s, 1H), 8.10 (t, 2H, J = 8.06 Hz), 8.22 (s, 1H).

### EXAMPLE 28

### 2-Dibenzofurancarboxylic acid 2-[(2,6-dichlorophenyl)sulfonyl]hydrazide

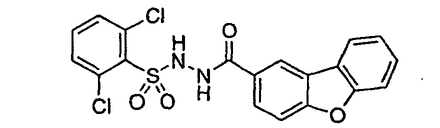

Dibenzofuran-2-carboxylic acid hydrazide (0.2 g, 0.88 mmol) was dissolved in THF (10 mL). N,N-diisopropylethylamine (0.61 mL, 3.52 mmol) was added, and the reaction was cooled to 0°C. 2,6-Dichloro-benzenesulfonyl chloride (0.22 g, 0.88 mmol) was added followed by DMAP (5 mg, 0.04 mmol). The reaction was allowed to warm to room temperature and stirred overnight. The reaction was then warmed to 40°C and stirred overnight. The solution was diluted with EtOAc (20 mL) and was washed with saturated sodium bicarbonate and brine and dried over Na₂SO₄. The reaction was then concentrated down and purified by flash chromatography eluting with 3% MeOH/CH₂Cl₂. The desired product was isolated (0.035 g, 9.14%).
MS: 437.0 (M+1 for C₁₉H₁₂N₂O₄Cl₂S). TLC: SiO₂, R_{f}= 0.60 (1:1 hexane/EtOAc); mp: 202-203°C; HRMS: (calc) 434.9973 (found) 434.9977 (M+1 for C₁₉H₁₂N₂O₄Cl₂S) HPLC: (50% H₂O/50% CH₃CN/0.1% TFA) Ret. Time: 17.828, Purity: 98.79%. IR (KBr, cm⁻¹) 3494, 3286, 3091, 1673, 1430, 1181, 738. ¹H NMR (400 MHz, DMSO) δ 7.39-7.94 (m, 6H), 8.08-8.15 (m, 1H), 8.25 (t, 1H, J = 5.3 Hz), 8.52 (s, 1H), 8.63-8.73 (m, 1H), 10.33 (s, 1H), 10.87 (s, 1H).

### EXAMPLE 29

### 2-Benzofurancarboxylic acid, 5-bromo-, 2-(phenylsulfonyl)hydrazide

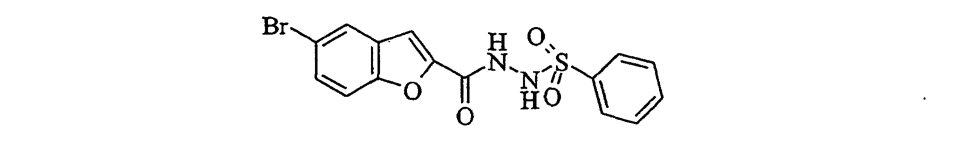

### Step 1. 5-Bromo-benzofuran-2-carboxylic acid hydrazide

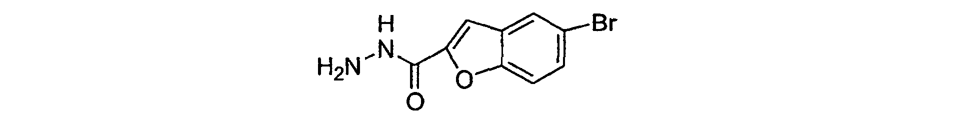

This was run in accordance with Example 1, Step 2 except 5-bromobenzofuran-2-carboxylic acid, which was prepared in accordance with the methods of Perkin, *J. Chem. Soc,* 1951:101-103, was used instead of dibenzofuran-2-carboxylic acid (76% yield).
MS: 254.9 (M+1 for C₉H₇N₂O₂Br); mp: 183.5-183.7°C. HPLC (C18 column, 1:1 MeCN/H₂O+0.1% TFA) 97.4%, RT = 1.93 min. Micro: (calc) (C₉H₇N₂O₂Br) C: 42.38, H: 2.77, N: 10.98, Br: 31.33; (found) C: 42.33, H: 2.71, N: 10.65, Br: 31.04. ¹H NMR (DMSO-d₆) δ 4.57 (s, 2H), 7.45 (d, 1H, J = 0.73 Hz), 7.55 (dd, 1H, J = 8.80 and 1.96 Hz), 7.60 (d, 1H, J = 8.80 Hz), 7.96 (t, 1H, J = 1.46 Hz), and 10.08 (s, 1H).

### Step 2. 2-Benzofurancarboxylic acid, 5-bromo-, 2-(phenylsulfonyl)-hydrazide

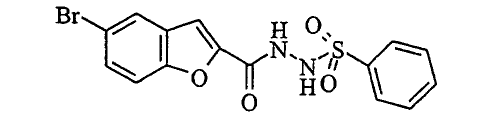

This was run in accordance with Example 35 except 5-bromo-benzofuran-2-carboxylic acid hydrazide was used instead of dibenzofuran-2-carboxylic acid hydrazide, and phenyl sulfonyl chloride was used instead of 3-fluorophenyl chloride (28.5% yield).
MS: 396.9 (M+1 for C₁₅H₁₁N₂O₄SBr); mp: 168.1-171.1°C. HPLC (C18 column, 1:1 MeCN/H₂O+0.1% TFA) 96.7%, RT = 6.38 min. Micro: (calc) (C₁₅H₁₁N₂O₄SBr) C: 45.59, H: 2.81, N: 7.09, S: 8.11, Br: 20.22; (found) C: 45.76, H: 2.79, N: 7.13, S: 8.26, Br: 20.23. ¹H NMR (DMSO-d₆) δ 7.50-7.56 (m, 3H), 7.58-7.64 (m, 3H), 7.81 (d, 2H, J = 7.09 Hz), 7.81 (d, 1H, J = 1.71 Hz), 10.21 (d, 1H, J = 2.8 Hz), and 11.00 (d, 1H, J = 2.8 Hz).

### EXAMPLE 30

### 2-Benzofurancarboxylic acid, 5-chloro-, 2-[(2-chloro-4-fluorophenyl)sulfonyl]-hydrazide

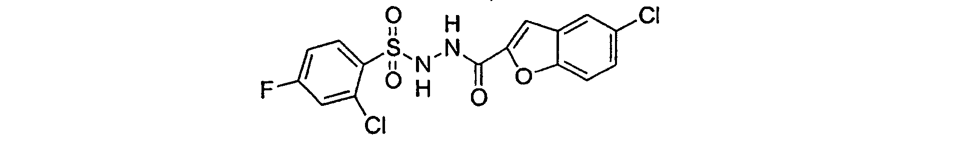

This was run in accordance with the Example 8 except that 2-chloro-4-fluorophenyl sulfonyl chloride was used instead of benzenesulfonyl chloride. The desired product was purified by column chromatography over SiO₂ eluting with 1:2 EtOAc/hexanes and triturating the combined and concentrated fractions in diethyl ether (2.6% yield).
MS: 404 (M+1 for C₁₅H₉N₂O₄SFCl₂); mp: 204.6-206.2°C. TLC: SiO₂,
R_{f}= 0.7 (EtOAc/hexane; 1:1); HPLC (C 18 column, 1:1 MeCN/H₂O+0.1% TFA) 99.1%, RT = 10.04 min. ¹H NMR (DMSO-d₆) δ 7.26-7.31 (m, 1H), 7.43 (dd, 1H, J = 9.04 and 2.44 Hz), 7.53 (s, 1H), 7.62 (d, 1H, J = 9.04 Hz), 7.66 (dd, 1H, J = 9.04 and 2.44 Hz), 7.82 (d, 1H, J = 2.20 Hz), 7.93-7.97 (m, 1H), 10.36 (s, 1H), and 10.97 (s, 1H).

### EXAMPLE 31

### 2-Benzofurancarboxylic acid, 5-chloro-, 2-[(3,5-dichlorophenyl)sulfonyl]-hydrazide

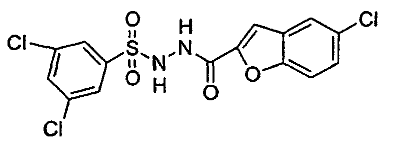

This was run in accordance with Example 8 except that 3,5-dichlorophenyl sulfonyl chloride was used instead of benzenesulfonyl chloride. The desired product was purified by column chromatography over SiO₂ eluting with 1:2 EtOAc/hexane and triturating the combined and concentrated fractions in diethyl ether (4.0% yield).
MS: 418.9 (M+1 for C₁₅H₉N₂O₄SCl₃); mp: 242.5-243.3°C. TLC: SiO₂,
R_{f} = 0.8(EtOAc/hexane; 1:1); HPLC (C18 column, 1:1 MeCN/H₂O+0.1% TFA) 99.3%, RT = 17.22 min. ¹H NMR (DMSO-d₆) δ 7.47 (dd, 1H, J = 8.79 and 2.20 Hz), 7.59 (s, 1H), 7.67 (d, 1H, J = 8.79 Hz), 7.75 (d, 2H, J = 1.96 Hz), 7.86 (d, 1H, J = 2.20 Hz), 7.97 (s, 1H), 10.63 (s, 1H), and 11.12 (s, 1H).

### EXAMPLE 32

### 2-Dibenzofurancarboxylic acid, 2-[[3,5-bis(trifluoromethyl)phenyl]sulfonyl]-hydrazide

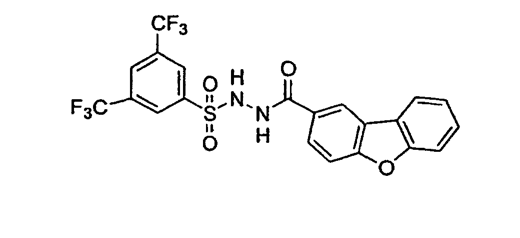

This was run in accordance with Example 1, Step 3 except that 2-(3,5-ditrifluoromethylphenyl)sulfonyl chloride was used instead of benzenesulfonyl chloride (2.3% yield).
MS: 503.0 (M+1 for C₂₁H₁₂N₂O₄SF₆); mp: 244.7-245.5°C. TLC: SiO₂, R_{f}= 0.85 (EtOAc/hexane; 1:2); HPLC (cyclohexyl-phenyl column, 1:1 MeCN/H₂O+0.1% TFA) 98.9%, RT = 13.82 min. ¹H NMR (DMSO-d₆) δ 7.41 (t, 1H, J = 7.57 Hz), 7.53 (t, 1H, J = 7.57 Hz), 7.69-7.82 (m, 2H), 8.13 (d, 2H, J = 7.57 Hz), 8.36 (s, 2H), 8.47 (d, 2H, J = 12.21 Hz), 8.65 (s, 1H), 10.72 (s, 1H), 10.99 (s, 1H).

### EXAMPLE 33

### 2-Benzofurancarboxylic acid, 5-chloro 2-[(3-chloro-4-fluorophenyl)sulfonyl]-hydrazide

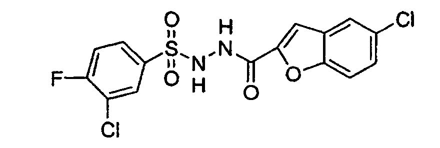

This was run in accordance with Example 8 except that 3-chloro-4-fluorophenyl sulfonyl chloride was used instead of benzenesulfonyl chloride. The desired product was purified by column chromatography over SiO₂ eluting with 1:2 EtOAc/hexane and triturating the combined and concentrated fractions in diethyl ether (4.2% yield).
MS: 404.9 (M+1 for C₁₅H₉N₂O₄SFCl₂); mp: 208.2-208.5°C. TLC: SiO₂,
R_{f}= 0.7 (EtOAc/hexane; 1:1); HPLC (cyclohexyl-phenyl column, 1:1 MeCN/H₂O+0.1% TFA) 99.3%, RT = 10.33 min. ¹H NMR (DMSO-d₆) δ 7.45 (dd, 1H, J = 8.79 and 2.20 Hz), 7.55-7.59 (m, 2H), 7.65 (d, 1H, J = 9.03 Hz), 7.66-7.80 (m, 1H), 7.84 (d, 1H, J = 2.20 Hz), 7.96 (dd, 1H, J = 6.84 and 2.20 Hz), 10.46 (s, 1H), and 11.06 (s, 1H).

### EXAMPLE 34

### 2-Benzofurancarboxylic acid, 5-nitro-, 2-(phenylsulfonyl)hydrazide

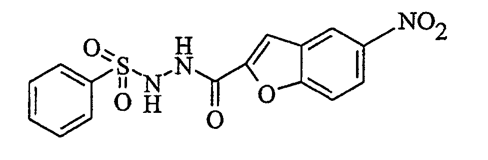

### Step 1. 5-Nitro-benzofuran-2-carboxylic acid hydrazide

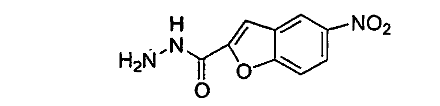

This was run in accordance with Example 1, Step 2 except 5-nitro-benzofuran-2-carboxylic acid was used instead of dibenzofuran-2-carboxylic acid (99% yield). MS: 222.0 (M+1 for C₉H₇N₃O₄); HPLC (C18 column, 1:1 MeCN/H₂O+0.1% TFA) 97.1%, RT = 1.85 min. ¹H NMR (DMSO-d₆) δ 4.60 (s, 2H), 7.67 (s, 1H), 7.84 (d, 1H, J = 9.27 Hz), 8.27 (dd, 1H, J = 2.46 and 9.28 Hz), 8.72 (d, 1H, J = 1.95 Hz), and 10.21 (s, 1H).

### Step 2. 2-Benzofurancarboxylic acid, 5-nitro-, 2-(phenylsulfonyl)hydrazide

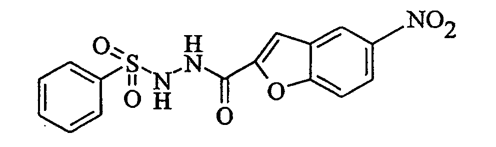

This was run in accordance with Example 35 except 5-nitro-benzofuran-2-carboxylic acid hydrazide was used instead of dibenzofuran-2-carboxylic acid hydrazide, and phenyl sulfonyl chloride was used instead of 3-fluorophenyl chloride (31% yield).
MS: 362.0 (M+1 for C₁₅H₁₁N₃O₆S); mp: 207.7-208.1°C. HPLC (C18 column, 1:1 MeCN/H₂O+0.1% TFA) 98.6%, RT = 4.38 min. Micro: (calc) (C₁₅H₁₁N₃O₆S·0.25EtOAc) C: 49.93, H: 3.40, N: 10.92, S: 8.33; (found) C: 50.06, H: 3.29, N: 11.29, S: 8.40. ¹H 14MR (DMSO-d₆) δ 7.53 (dt, 2H, J = 8.06 and 1.71 Hz), 7.60-7.65 (m, 1H), 7.79-7.81 (m, 3H), 7.87 (d, 1H, J = 9.28 Hz), 8.30 (dd, 1H, J = 2.44 and 9.28 Hz), 8.76 (d, 1H, J = 2.44 Hz), 10.28 (d, 1H, J = 2.8 Hz), and 11.14 (d, 1H, J = 2.8 Hz).

### EXAMPLE 35

### 2-Dibenzofurancarboxylic acid 2-[(3-fluorophenyl)sulfonyl]hydrazide

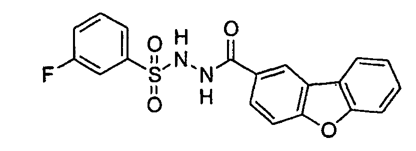

A solution of 3-fluorophenyl sulfonyl chloride (0.19 g, 1 mmol) and dibenzofuran-2-carboxylic acid hydrazide (0.22 g, 1 mmol) was stirred 18 hours at RT in pyridine (10 mL). After the solvent was removed in vacuo, the residue was triturated in water, and dried. Recrystallization of this solid from ethyl acetate provided the title compound, 250 mg, 65% yield.
MS: 385.0 (M+1 for C₁₉H₁₃N₂O₄FS); mp: 222.6-223.3°C; Analysis: (C₁₉H₁₃N₂O₄FS) (calc) C: 59.37, H: 3.41, N: 7.29, S: 8.34, F: 4.94; (found) C: 59.17, H: 3.45, N: 7.21, S: 8.02, F: 5.03. HPLC (C18 column, 1:1 MeCN/H₂O+0.1% TFA) 97.9%, RT = 8.67 min. ¹H NMR (DMSO-d₆) δ 7.39 (t, 1H, J = 7.32 Hz), 7.43-7.75 (m, 7H), 7.84 (dd, 1H, J = 8.79 and 1.71 Hz), 8.16 (d, 1H, J = 7.82 Hz), 8.50 (d, 1H, J = 1.22 Hz), 10.28 (d, 1H, J = 2.8 Hz), 10.812 (d, 1H, J = 2.8 Hz).

### EXAMPLE 36

### 2-Dibenzofurancarboxylic acid 2-[(5-fluoro-2-methylphenyl)sulfonyl]hydrazide

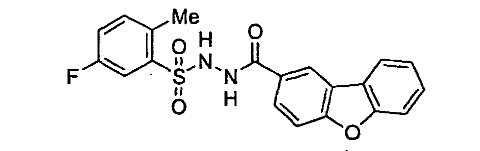

This was run in accordance with Example 35 except 5-fluoro-2-methylphenylsulfonyl chloride was used instead of 3-fluorophenyl sulfonyl chloride (55.3% yield).
MS: 399.0 (M+1 for C₂₀H₁₅N₂O₄FS); mp: 210.1-210.5°C; Analysis:
(C₂₀H₁₅N₂O₄FS) (calc) C: 59.62, H: 3.88, N: 6.95, S: 7.96, F: 4.4.71; (found) C: 59.54, H: 3.74, N: 6.99, S :7.78, F: 4.84. HPLC (C18 column, 1:1 MeCN/H₂O+0.1% TFA) 97.2%, RT = 9.25 min. ¹H NMR (DMSO-d₆) δ 2.65 (s, 3H), 7.32-7.39 (m, 1H), 7.45 (dd, 2H, J = 7.57 and 1.22 Hz), 7.55 (dd, 1H, J = 7.57 and 1.22 Hz), 7.60 (dd, 1H, J = 9.04 and 2.69 Hz), 7.72 (t, 1H, J = 8.79 Hz), 7.83 (dd, 1H, J = 8.54 and 1.46 Hz), 8.15 (d, 1H, J = 7.58 Hz), 8.50 (s, 1H), 10.24 (d, 1H, J = 2.8 Hz), 10.76 (d, 1H, J = 2.8 Hz).

### EXAMPLE 37

### 2-Dibenzofurancarboxylic acid 2-[(3,4-difluorophenyl) sulfonyl]hydrazide

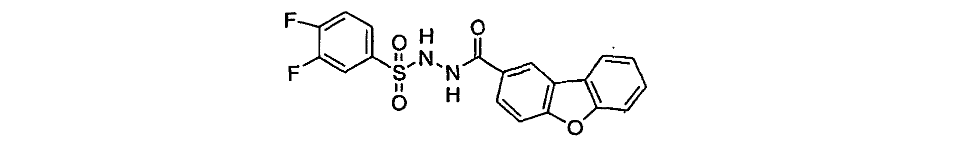

This was run in accordance with Example 35 except 3,4-difluorophenylsulfonyl chloride was used instead of 3-fluorophenyl sulfonyl chloride (42.4% yield), and the material was purified through column chromatography eluting with 1:2 ethyl acetate/hexanes.
MS: 402.9 (M+1 for C₁₉H₁₂N₂O₄F₂S); mp: 210.1-210.5°C; Analysis: (C₁₉H₁₂N₂O₄F₂S) (calc) C: 56.72, H: 3.01, N: 6.96, S: 7.97, F: 9.44; (found) C: 56.64, H: 3.00, N: 6.88, S: 7.71, F: 9.48. HPLC (C18 column, 1:1 MeCN/H₂O+0.1% TFA) 99.2%, RT = 11.06 min. ¹H NMR (DMSO-d₆) δ 7.37-7.41 (m, 1H), 7.52 (dd, 2H, J = 7.33 and 1.22 Hz), 7.55-7.73 (m, 3H), 7.82-7.90 (m, 2H), 8.14 (d, 1H, J = 7.82 Hz), 8.15 (d, 1H, J = 7.58 Hz), 8.49 (d, 1H, J = 1.22 Hz), 10.3 (s, 1H), 10.8 (s, 1H).

### EXAMPLE 38

### 2-Dibenzofurancarboxylic acid 2-[(3-chloro-4-fluorophenyl)sulfonyl]hydrazide

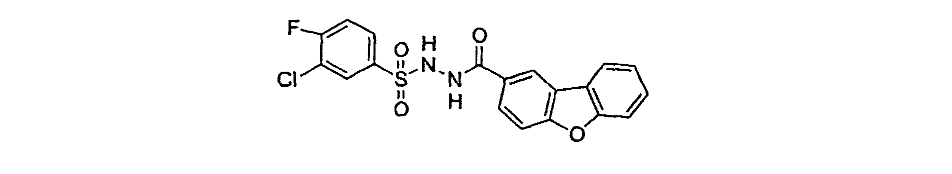

This was run in accordance with Example 35 except 3-chloro-4-fluorophenylsulfonyl chloride was used instead of 3-fluorophenyl sulfonyl chloride (28.5% yield), and the material was purified through column chromatography eluting with 1:2 ethyl acetate/hexanes.
MS: 418.9 (M+1 for C₁₉H₁₂N₂O₄ClFS); mp: 210.8-211.6°C; Analysis:
(C₁₉H₁₂N₂O₄ClFS) (calc) C: 54.49, H: 2.89, N: 6.69, S: 7.66, F: 4.54; (found) C: 53.97, H: 3.02, N: 6.53, S: 7.41, F: 4.64. HPLC (C18 column, 1:1 MeCN/H₂O+0.1% TFA) 97.9%, RT = 15.05 min. ¹H NMR (DMSO-d₆) δ 7.40 (dd, 1H, J = 7.57 and 0.73 Hz), 7.49-7.57 (m, 2H), 7.68 (d, 1H, J = 8.30 Hz), 7.71 (d, 1H, J = 8.30 Hz), 7.79-7.84 (m, 2H), 7.99 (dd, 1H, J = 6.84 and 1.95 Hz), 8.14 (d, 1H, J = 7.57 Hz), 8.48 (d, 1H, J = 0.68 Hz), 10.33 (s, 1H), 10.81 (s, 1H).

### EXAMPLE 39

### 2-Dibenzofurancarboxylic acid 2-[(3-methoxyphenyl)sulfonyl]hydrazide

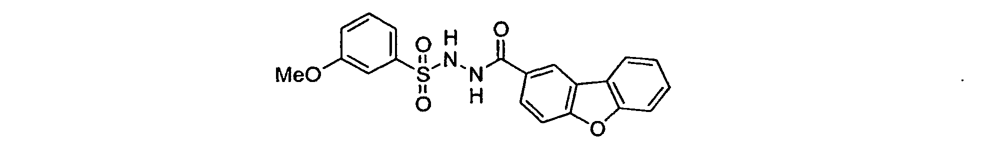

This was run in accordance with Example 35 except 3-methoxy phenylsulfonyl chloride was used instead of 3-fluorophenyl sulfonyl chloride (63.1 % yield).
MS: 397.0 (M+1 for C₂₀H₁₆N₂O₅S); mp: 199.5-200.5°C; Analysis:
(C₂₀H₁₆N₂O₅S) (calc) C: 60.60, H: 4.07, N: 7.07, S: 8.09; (found) C: 60.24, H: 3.96, N: 6.95, S: 7.86. HPLC (C18 column, 1:1 MeCN/H₂O+0.1% TFA) 98.7%,
RT = 8.60 min. ¹H NMR (DMSO-d₆) δ 3.72 (s, 3H), 7.14-7.17 (m, 1H), 7.34-7.39 (m, 1H), 7.40-7.43 (m, 3H), 7.55 (t, 1H, J = 5.86 Hz), 7.84 (dd, 1H, J = 9.08 and 2.0 Hz), 8.15 (d, 1H, J = 6.84 Hz), 8.50 (s, 1H), 10.07 (d, 1H, J = 2.80 Hz), and 10.75 (d, 1H, J = 2.80 Hz).

### EXAMPLE 40

### 2-Benzofurancarboxylic acid, 5-chloro-2-[(3-fluorophenyl)sulfonyl] hydrazide

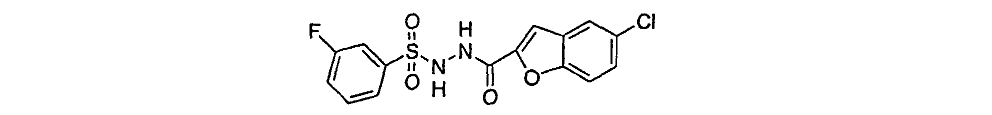

This was run in accordance with the Example 35 except that 5-chlorobenzofuran-2-carboxylic acid hydrazide was used instead of dibenzofuran-2-carboxyclic acid hydrazide. The desired product was purified by columning over SiO₂ eluting with 1:2 EtOAc/hexane and triturating the combined and concentrated fractions in diethyl ether (46.2% yield).
MS: 368.9 (M+1 for C₁₅H₁₀N₂O₄SFCl); mp: 174.4-174.7°C; Micro: (calc) (C₁₅H₁₀N₂O₄SFCl) C: 48.86, H: 2.73, N: 7.60, S:8.69, F: 5.15; (found) C: 48.56, H: 2.61, N: 7.45, S: 8.64, F: 5.19. HPLC (C18 column, 1:1 MeCN/H₂O+0.1% TFA) 98.9%, RT = 7.73 min. ¹H NMR (DMSO-d₆) δ 7.46 (dd, 2H, J = 9.04 and 2.2 Hz), 7.50-7.67 (m, 5H), 7.85 (d, 1H, J = 1.96 Hz), 10.41 (s, 1H), and 11.05 (s, 1H).

### EXAMPLE 41

### 2-Benzofurancarboxylic acid, 5-chloro-2-[(3,4-difluorophenyl)sulfonyl]hydrazide

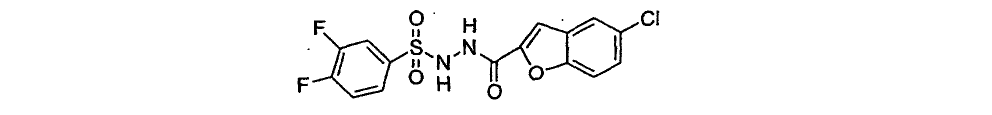

This was run in accordance with Example 40 except that 3,4-difluorophenyl sulfonyl chloride was used instead of 3-fluorophenyl sulfonyl chloride. The desired product was purified by columning over SiO₂ eluting with 1:2 EtOAc/hexanes (51.7% yield).
MS: 386.9 (M+1 for C₁₅H₉N₂O₄SF₂Cl); mp: 193.8-195°C; Micro: (calc) (C₁₅H₉N₂O₄SF₂Cl) C: 46.58, H: 2.35, N: 7.25, S: 8.29, F: 9.82; (found) C: 46.58, H: 2.44, N: 7.08, S: 8.18, F: 9.54. HPLC (C18 column, 1:1 MeCN/H₂O+0.1% TFA) 97.5%, RT = 8.34 min. ¹H NMR (DMSO-d₆) δ 7.46 (dd, 1H, J = 9.04 and 2.2 Hz), 7.57 (s, 1H), 7.61 (dd, 1H, J = 10.25 and 2.68 Hz), 7.65-7.83 (m, 2H), 7.84-7.88 (m, 3H), 10.45 (s, 1H), and 11.06 (s, 1H).

### EXAMPLE 42

### 2-Benzofurancarboxylic acid, 5-chloro-2-[(2-chlorophenyl)sulfonyl]hydrazide

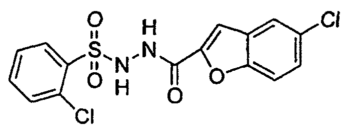

This was run in accordance with Example 40 except that 2-chlorophenyl sulfonyl chloride was used instead of 3-fluorophenyl sulfonyl chloride. The desired product was purified by columning over SiO₂ eluting with 1:2 EtOAc/hexanes (51.9% yield).
MS: 384.9 (M+1 for C₁₅H₁₀N₂O₄SCl₂); mp: 200.3-200.5°C; Micro: (calc) (C₁₅H₁₀N₂O₄SCl₂) C: 46.77, H: 2.62, N:7.27, S: 8.32, Cl: 18.41; (found) C: 47.09, H: 2.98, N: 6.58, S: 7.57, Cl: 18.62. HPLC (C18 column, 1:1 MeCN/H₂O+0.1% TFA) 99.2%, RT = 7.49 min. ¹H NMR (DMSO-d₆) δ 7.41 (dd, 1H, J = 6.59 and 1.71 Hz), 7.45 (dd, 1H, J = 8.79 and 2.2 Hz), 7.54 (s, 1H), 7.58-7.65 (m, 3H), 7.82 (d, 1H, J = 1.96), 7.92 (dd, 1H, J = 9.03 and 1.22 Hz), 10.30 (s, 1H), and 10.98 (s, 1H).

### EXAMPLE 43

### 2-Benzofurancarboxylic acid, 5-chloro-2-[(3-chlorophenyl) sulfonyl]hydrazide

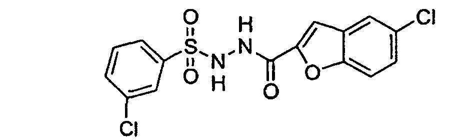

This was run in accordance with Example 40 except that 3-chlorophenyl sulfonyl chloride was used instead of3-fluorophenyl sulfonyl chloride. The desired product was purified by columning over SiO₂ eluting with 1:2 EtOAc/hexanes (42.4% yield).
MS: 384.9 (M+1 for C₁₅H₁₀N₂O₄SCl₂); mp: 202.9-205.2°C; Micro: (calc) for (C₁₅H₁₀N₂O₄SCl₂) C: 46.77, H: 2.62, N: 7.27, S: 8.32, Cl: 18.41; (found): C: 46.71, H: 2.43, N: 7.18, S: 8.25, Cl: 18.35; HPLC (C18 column, 1:1 MeCN/H₂O+0.1% TFA) 98.7%, RT = 9.3 min. ¹H NMR (DMSO-d₆) δ 7.46 (dd, 1H, J = 8.79 and 2.19 Hz), 7.55 (d+s, 2H, J = 7.82 Hz), 7.54 (s, 1H), 7.70-7.75 (m, 2H), 7.80 (t, 1H, J = 1.96), 7.85 (d, 1H, J = 2.20 Hz), 10.44 (s, 1H), and 11.07 (s, 1H).

### EXAMPLE 44

### 2-Benzofurancarboxylic acid, 5-chloro-2-[(2-bromophenyl)sulfonyl]hydrazide

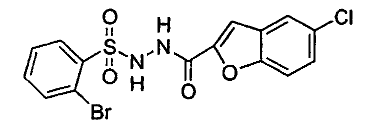

This was run in accordance with Example 40 except that 2-bromophenyl sulfonyl chloride was used instead of 3-fluorophenyl sulfonyl chloride. The desired product was purified by columning over SiO₂ eluting with 1:2 EtOAc/hexanes (59.8% yield).
MS: 426.9 (M+1 for C₁₅H₁₀N₂O₄SBrCl); mp: 206.4-207.3°C; Micro: (calc) (C₁₅H₁₀N₂O₄SBrCl·0.5H₂O) C: 41.68, H: 2.62, N: 5.96; (found) C: 41.07, H: 2.53, N: 6.39. HPLC (C18 column, 1:1 MeCN/H₂O+0.1% TFA) 93.7%,
RT = 8.17 min. ¹H NMR (DMSO-d₆) δ 7.44-7.52 (m, 3H), 7.55 (d, 1H, J = 0.73 Hz), 7.64 (d, 1H, J = 8.80 Hz), 7.79-7.82 (m, 1H), 7.84 (d, 1H, J = 1.95 Hz), 7.97-7.99 (m, 1H), 10.30 (br s, 1H), and 10.95 (br s, 1H).

### EXAMPLE 45

### 2-Benzofurancarboxylic acid, 5-chloro-2-[(3-bromophenyl)sulfonyl]hydrazide

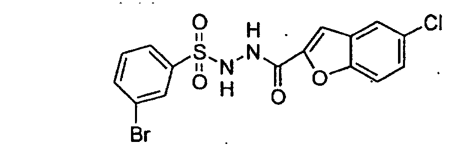

This was run in accordance with Example 40 except that 3-bromophenyl sulfonyl chloride was used instead of 3-fluorophenyl sulfonyl chloride. The desired product was purified by recrystallization in ethyl acetate (58.3% yield).
MS: 428.9 (M+1 for C₁₅H₁₀N₂O₄SBrCl); mp: 218.1-220.0°C; Micro: (calc) for (C₁₅H₁₀N₂O₄SBrCl) C: 42.06, H: 2.36, N: 6.54, S: 7.47; (found) C: 41.99, H: 2.31, N:6.51, S: 7.21. HPLC (C18 column, 1:1 MeCN/H₂O+0.1% TFA) 95.8%,
RT = 10.17 min. ¹H NMR (DMSO-d₆) δ 7.45 (t, 2H, J = 8.06 Hz), 7.57 (s, 1H), 7.66 (d, 1H, J = 9.04 Hz), 7.76 (dd, 1H, J = 1.71 and 0.98 Hz), 7.78 (dd, 1H, J = 1.71 and 0.98 Hz), 7.85 (s, 1H), 7.92 (t, 1H, J = 1.71 Hz), 10.44 (d, 1H, J = 2.80 Hz), and 11.08 (d, 1H, J = 2.80 Hz).

### EXAMPLE 46

### 2-Benzofurancarboxylic acid, 5-chloro-2-[(2-methylphenyl)sulfonyl ]hydrazide

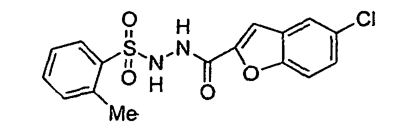

This was run in accordance with Example 40 except that 2-methylphenyl sulfonyl chloride was used instead of 3-fluorophenyl sulfonyl chloride. The desired product was purified by columning over SiO₂ eluting with 1:2 EtOAc/hexanes (63.0% yield).
MS: 364.9 (M+1 for C₁₆H₁₃N₂O₄SCl); mp: 190.5-192.0°C; Micro: (calc) (C₁₆H₁₃N₂O₄SCl·0.5 EtOAc) C: 52.88, H: 4.19, N: 6.85, S:7.84; (found): C: 52.90, H: 3.91, N: 7.00, S: 8.03. HPLC (C18 column, 1:1 MeCN/H₂O+0.1% TFA) 98.8%, RT = 13.1 min. ¹H NMR (DMSO-d₆) δ 2.66 (s, 3H), 7.25 (t, 1H, J = 7.57 Hz), 7.35 (d, 1H, J = 7.57 Hz), 7.43-7.49 (m, 2H), 7.52 (s, 1H), 7.63 (d, 1H, J = 9.04 Hz), 7.78 (d, 1H, J = 6.84 Hz), 7.83 (d, 1H, J = 2.20 Hz), 10.12 (s, 1H), and 10.91 (s, 1H).

### EXAMPLE 47

### 2-Benzofurancarboxylic acid, 5-chloro-2-[(3-methylphenyl)sulfonyl]hydrazide

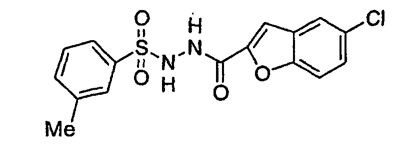

This was run in accordance with Example 40 except that 3-methylphenyl sulfonyl chloride was used instead of 4-fluorophenyl sulfonyl chloride. The desired product was purified by columning over SiO₂ eluting with 1:2 EtOAc/hexanes (83.0% yield).
MS: 364.9 (M+1 for C₁₆H₁₃N₂O₄SCl); mp: 201.2-201.6°C; Micro: (calc) (C₁₆H₁₃N₂O₄SCl) C: 52.68, H: 3.59, N: 7.68, S: 8.74. (found) C: 52.61, H: 3.61, N: 7.57, S: 8.67. HPLC (C18 column, 1:1 MeCN/H₂O+0.1% TFA) 98.8%, RT = 12.3 min. ¹H NMR (DMSO-d₆) δ 2.31 (s, 3H), 7.37-7.48 (m, 3H), 7.56 (d, 1H, J = 0.73 Hz), 7.59 (d, 1H, J = 7.33 Hz), 7.65 (t, 2H, J = 11.23 Hz), 7.85 (d, 1H, J = 2.20 Hz), 10.17 (s, 1H), and 10.97 (s, 1H).

### EXAMPLE 48

### 2-Benzofurancarboxylic acid, 5-chloro-2-[(3-methoxyphenyl)sulfonyl] hydrazide

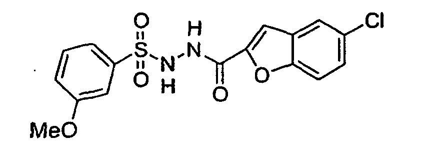

This was run in accordance with Example 40 except that 3-methoxyphenyl sulfonyl chloride was used instead of 3-fluorophenyl sulfonyl chloride. The desired product was purified by columning over SiO₂ eluting with 1:2 EtOAc/hexanes (68.2% yield).
MS: 380.9 (M+1 for C₁₆H₁₃N₂O₅SCl); mp: 204.3-204.6°C; Micro: (calc) (C₁₆H₁₃N₂O₅SCl·0.3H₂O) C: 49.76, H: 3.43, N: 7.13, S: 8.19, Cl: 9.17; (found) C: 49.75, H: 3.55, N: 7.25, S: 8.30, Cl: 9.18; HPLC (C18 column, 1:1 MeCN/H₂O+0.1% TFA) 98.5%, RT = 6.35 min. ¹H NMR (DMSO-d₆) δ 3.74 (s, 3H), 7.18 (dd, 1H, J = 7.08 and 0.98 Hz), 7.30 (s, 1H), 7.36 (t, 1H, J = 7.08 Hz), 7.41 (d, 1H, J = 7.82 Hz), 7.47 (d, 1H, J = 2.20 Hz), 7.55 (d, 1H, J = 0.74 Hz), 7.65 (d, 1H, J = 8.79 Hz), 7.85 (d, 1H, J = 1.95 Hz), 10.17 (s, 1H), and 10.71 (s, 1H).

### EXAMPLE 49

### 2-Benzofurancarboxylic acid, 5-chloro-2-[(3-chloro-2-methylphenyl)sulfonyl]hydrazide

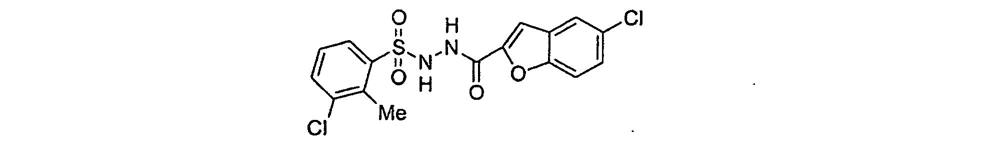

This was run in accordance with Example 40 except that 3-chloro-2-methylphenyl sulfonyl chloride was used instead of 3-fluorophenyl sulfonyl chloride. The desired product was purified by columning over SiO₂ eluting with 1:2 EtOAc/hexanes and triturating the combined pure fractions in ethyl ether (62.7% yield).
MS: 398.9 (M+1 for C₁₆H₁₂N₂O₄SCl₂); mp: 198.2-198.5°C; Micro: (calc) (C₁₆H₁₂N₂O₄SCl₂) C: 48.13, H: 3.03, N: 7.02, S: 8.03, Cl: 17.76; (found) C: 48.09, H: 3.11, N: 6.90, S: 8.02, Cl: 17.80; HPLC (C18 column, 1:1 MeCN/H₂O+0.1% TFA) 93.25%, RT = 7.65 min. ¹H NMR (DMSO-d₆) δ 2.70 (s, 3H), 7.28 (t, 1H, J = 7.81 Hz), 7.45 (dd, 1H, J = 8.79 and 2.20 Hz), 7.54 (s, 1H), 7.64 (d, 1H, J = 9.04 Hz), 7.69 (d, 1H, J = 7.81 Hz), 7.80 (d, 1H, J = 7.81 Hz), 7.83 (d, 1H, J = 2.20 Hz), 10.39 (s, 1H), and 10.96 (s, 1H).

### EXAMPLE 50

### 2-Benzofurancarboxylic acid, 5-chloro-2-[(5-fluoro-2-methylphenyl)sulfonyl]hydrazide

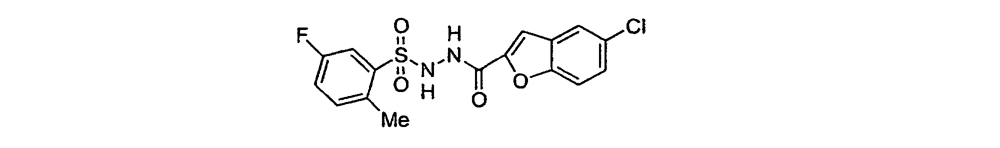

This was run in accordance with Example 40 except that 5-fluoro-2-methylphenyl sulfonyl chloride was used instead of 3-fluorophenyl sulfonyl chloride. The desired product was purified by columning over SiO₂ eluting with 1:2 EtOAc/hexane and triturating the combined pure fractions in ethyl ether (52.2% yield).
MS: 382.9 (M+1 for C₁₆H₁₂N₂O₄SFCl); mp: 209.6-211.2°C; Micro: (calc) (C₁₆H₁₂N₂O₄SFCl) C: 50.20, H: 3.16, N: 7.32, S: 8.38, F: 4.96; (found) C: 49.83, H: 3.04, N: 7.17, S: 8.15, F: 4.93; HPLC (C 18 column, 1:1 MeCN/H₂O+0.1% TFA) 97.8%, RT = 11.77 min. ¹H NMR (DMSO-d₆) δ 2.61 (s, 3H), 7.34-7.43 (m, 2H), 7.45 (dd, 1H, J = 8.79 and 2.20 Hz), 7.55 (s, 1H), 7.57 (d, 1H, J = 2.68 Hz), 7.65 (d, 1H, J = 8.79 Hz), 7.84 (d, 1H, J = 2.2 Hz), 10.39 (s, 1H), and 10.96 (s, 1H).

### EXAMPLE 51

### 2-Benzofurancarboxylic acid, 5-chloro-2-[[(2-(trifluoromethyl)phenyl]sulfonyl]hydrazide

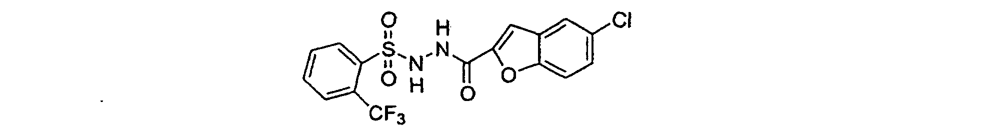

This was run in accordance with Example 40 except that 2-trifluoromethylphenyl sulfonyl chloride was used instead of 3-fluorophenyl sulfonyl chloride. The desired product was purified by columning over SiO₂ eluting with 1:2 EtOAc/hexane (60.3% yield).
MS: 419.9 (M+1 for C₁₆H₁₀N₂O₄SF₃Cl); mp: 192.0-193.4°C; Micro: (calc) (C₁₆H₁₀N₂O₄SF₃Cl) C: 45.89, H: 2.41, N: 6.69, S: 7.66, F: 13.61; (found) C: 46.16, H: 2.41, N: 6.60, S: 7.38, F: 13.30; HPLC (C18 column, 1:1 MeCN/H₂O+0.1% TFA) 99.2%, RT = 9.63 min. ¹H NMR (DMSO-d₆) δ 7.44 (dd, 1H, J = 9.04 and 2.20 Hz), 7.53 (s, 1H), 7.63 (d, 1H, J = 8.79 Hz), 7.75-7.82 (m, 2H), 7.84 (d, 1H, J = 1.95 Hz), 7.93 (dd, 1H, J = 2.2 and 9.03 Hz), 8.13 (dd, 1H, J = 2.44 and 6.59 Hz), 10.19 (s, 1H), and 11.06 (s, 1H).

### EXAMPLE 52

### 2-Benzofurancarboxylic acid, 5-chloro-2-[[2-(trifluoromethoxy)phenyl]sulfonyl]hydrazide

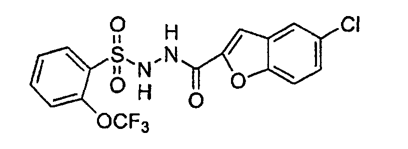

This was run in accordance with Example 40 except that 2-trifluoromethoxyphenyl sulfonyl chloride was used instead of 3-fluorophenyl sulfonyl chloride. The desired product was purified by recrystallizing in ethyl acetate and then columning the resulting solid over SiO₂ eluting with 1:2 EtOAc/hexane (34.6% yield).
MS: 434.9 (M+1 for C₁₆H₁₀N₂O₅SF₃Cl); mp: 218.2-219.8°C; Micro: (calc) (C₁₆H₁₀N₂O₅SF₃Cl) C: 44.20, H: 2.32, N: 6.44, S:7.37, F: 13.11; (found) C: 44.10, H: 2.46, N: 6.27, S: 7.23, F: 13.22; HPLC (C18 column, 1:1 MeCN/H₂O+0.1% TFA) 98.4%, RT = 11.9 min. ¹H NMR (DMSO-d₆) δ 7.54-7.58 (m, 2H), 7.62 (d, 1H, J = 8.31 Hz), 7.65 (d, 1H, J = 0.97 Hz), 7.76 (d, 1H, J = 9.03 Hz), 7.84 (dt, 1H, J = 1.71 and 8.31 Hz), 7.96 (d, 1H, J = 1.96 Hz), 8.02 (dd, 1H, J = 1.70 and 8.06 Hz), 10.46 (s, 1H), and 11.15 (s, 1H).

### EXAMPLE 53

### 2-Dibenzofurancarboxylic acid, 2-[(4-N,N-dimethylaminophenyl)-sulfonyl]hydrazide hydrochloride salt

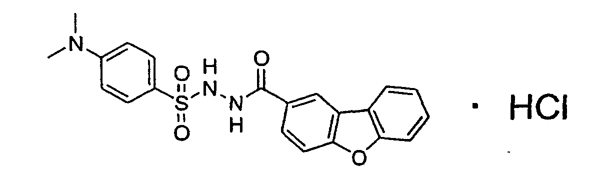

Synthesis of 2-Dibenzofurancarboxylic acid, 2-[(4-N,N-dimethylaminophenyl)sulfonyl] hydrazide hydrochloride salt: Example 53 was synthesized in accordance with the methods of Example 167, Step 2, except that 2-dibenzofurancarboxylic acid, 2-[(4-N,N-dimethylaminophenyl)sulfonyl]-hydrazide was used instead of 2-dibenzofurancarboxylic acid, 2-[(2-N,N-dimethylaminophenyl)sulfonyl]hydrazide (yield 98%).
MS: 409.1 (M for C₂₁H₁₉N₃O₄S); mp: 187.6-188.8°C; HPLC (C18 column, 1:1 MeCN/H₂O = 0.1% TFA) 88.97%, RT = 8.3 min.; ¹HNMR (DMSO-d₆) δ 3.15 (S, 6H), 7.00 (t, 1H, J = 7.1 Hz), 7.18 (d, 1H, J = 8.1 Hz), 7.40-7.80 (m, 6H), 7.92 (d, 1H, J = 7.8 Hz), 8.18 (d, 1H, J = 7.4 Hz), 8.55 (s, 1H), 10.75 (s, 2H).

### EXAMPLE 54

### 2-Benzofurancarboxylic acid, 7-methoxy-, 2-[[2-(trifluoromethyl)phenyl]sulfonyl]hydrazide

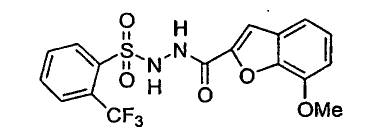

This was run in accordance with Example 40 except that 7-methoxybenzofuran-2-carboxylic acid hydrazide was used instead of 5-chlorobenzofuran-2-carboxylic acid hydrazide, and 2-trifluoromethylphenyl sulfonyl chloride was used instead of 3-fluorophenyl sulfonyl chloride. The desired product was purified by columning over SiO₂ eluting with 1:2 EtOAc/hexane and triturating the combined and concentrated fractions in diethyl ether (41.6% yield).
MS: 415.1 (M+1 for C₁₇H₁₃N₂O₅SF₃); mp: 156.8-157.6°C. HPLC (C18 column, 1:1 MeCN/H₂O+0.1% TFA) 99.0%, RT = 6.49 min. Micro: (calc) (C₁₇H₁₃N₂O₅SF₃) C: 49.28, H: 3.16, N: 6.76, S: 7.74, F: 13.75; (found) C: 49.31, H: 3.12, N: 6.67, S: 7.70, F: 13.76; ¹H NMR (DMSO-d₆) δ 3.89 (s, 3H), 7.02 (d, 1H, J = 7.81 Hz), 7.21 (t, 1H, J = 7.58 Hz), 7.26 (d, 1H, J = 7.08 Hz), 7.54 (s, 1H), 7.76-7.83 (m, 3H), 7.93 (d, 1H, J = 7.08 Hz), 8.14 (d, 1H, J = 6.84 Hz), 10.12 (s, 1H), and 10.89 (s, 1H).

### EXAMPLE 55

### 2-Benzofurancarboxylic acid, 7-methoxy-, 2-[(3-methylphenyl) sulfonyl] hydrazide

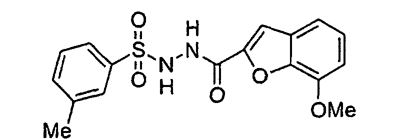

This was run in accordance with Example 54 except 3-methylphenyl sulfonyl chloride was used instead of 2-trifluoromethylphenyl sulfonyl chloride. The desired product was purified by columning over SiO₂ eluting with 1:2 EtOAc/hexane and triturating the combined and concentrated fractions in diethyl ether (33.3% yield). MS: 361.0 (M+1 for C₁₇H₁₆N₂O₅S); mp: 170.8-171.8°C. HPLC (C18 column, 1:1 MeCN/H₂O+0.1% TFA) 98.7%, RT = 5.08 min. Micro: (calc) (C₁₇H₁₆N₂O₅S) C: 56.66 H: 4.48, N: 7.77, S: 8.90; (found) C: 56.45, H: 4.49, N: 7.55, S: 8.68; ¹H NMR (DMSO-d₆) δ 2.31 (s, 3H), 3.90 (s, 3H) 7.02 (d, 1H, J = 7.57 Hz), 7.20 (t, 1H, J = 7.81 Hz), 7.26 (d, 1H, J = 7.82 Hz), 7.36-7.43 (m, 2H), 7.55 (s, 1H), 7.57-7.62 (m, 2H), 10.12 (s, 1H), and 10.89 (s, 1H).

### EXAMPLE 56

### 2-Benzofurancarboxylic acid, 6-methoxy-, 2-(phenylsulfonyl)hydrazide

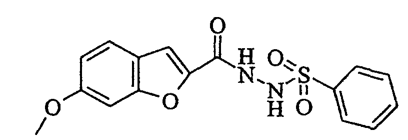

### Step 1. 6-Methoxy-benzofuran-2-carboxylic acid hydrazide

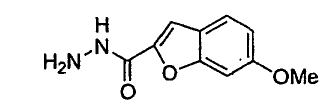

The hydrazide was prepared in accordance with Example 1, Step 2 except 6-methoxy-benzofuran-2-carboxylic acid, prepared in accordance with the methods of Tanaka et al., *J. Amer. Chem. Soc.,* 1951:872, was used instead of the dibenzofuran-2-carboxylic acid (yield 58.9%).
MS: 207.0 (M+1 for C₁₀H₁₀N₂O₄); mp: 124.1-125.0°C. HPLC (C18 column, 1:1 MeCN/H₂O+0.1% TFA) 98.7%, RT = 1.67 min. ¹H NMR (DMSO-d₆) δ 3.79 (s, 3H), 4.49 (s, 2H), 6.90 (dd, 1H, J = 8.54 and 2.19 Hz), 7.16 (d, 1H, J = 1.95 Hz), 7.40 (d, 1H, J = 0.73 Hz), 7.58 (d, 1H, J = 8.79 Hz), and 9.84 (s, 1H).

### Step 2. 2-Benzofurancarboxylic acid, 6-methoxy-, 2-(phenylsulfonyl) hydrazide

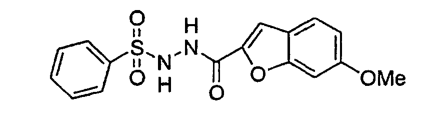

This was run in accordance with Example 35 except 6-methoxy-benzofuran-2-carboxylic acid hydrazide was used instead of dibenzofuran-2-carboxylic acid hydrazide, and benzenesulfonyl chloride was used instead of 3-fluorophenyl sulfonyl chloride. The desired product was purified by recrystallization in ethyl acetate/methanol (66.5% yield).
MS: 347.0 (M+1 for C₁₆H₁₄N₂O₅S); mp: 224.9-225.8°C. HPLC (C18 column, 1:1 MeCN/H₂O+0.1% TFA) 99.0%, RT =4.18 min. Micro: (calc) (C₁₆H₁₄N₂O₅S) C: 55.48, H: 4.07, N: 8.09; (found) C: 55.37, H: 4.00, N: 8.07. ¹H NMR (DMSO-d₆) δ 3.78 (s, 3H) 6.92 (dd, 1H, J = 8.55 and 2.20 Hz), 7.16 (d, 1H, J = 1.95 Hz), 7.50-7.53 (m, 3H), 7.59-7.61 (m, 2H), 7.57-7.62 (m, 2H), 7.79 (dd, 1H, J = 7.08 and 1.46 Hz), 10.12 (s, 1H), and 10.77 (s, 1H).

### EXAMPLE 57

### 2-Benzofurancarboxylic acid, 5-methoxy-3-(1-methylethoxy), 2-(phenylsulfonyl)-hydrazide

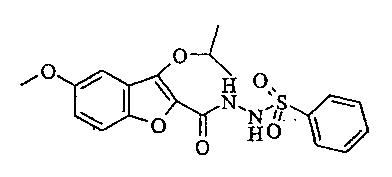

### Step 1. 5-Methoxy-3-(1-methylethoxy)-benzofuran-2-carboxylic acid hydrazide

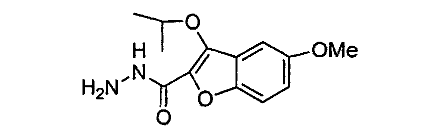

The hydrazide was prepared in accordance with Example 1, Step 2 except that 5-methoxy-3-(1-methylethoxy)-benzofuran-2-carboxylic acid was used instead of dibenzofuran-2-carboxylic acid (yield 59.9%).
MS: 265.3 (M+1 for C₁₃H₁₆N₂O₄); HPLC (C18 column, 1:1
MeCN/H₂O+0.1% TFA) 95.5%, RT = 1.88 min. ¹H NMR (DMSO-d₆) δ 1.39 (d, 6H, J = 6.10 Hz), 3.88 (s, 3H), 4.58 (d, 2H, J = 3.18 Hz), 5.00 (quintet, 1H, J = 6.11 Hz), 7.12 (dd, 1H, J = 9.09 and 2.49 Hz), 7.19 (d, 1H, J = 2.45 Hz), 7.53 (d, 1H, J = 9.03 Hz), and 9.29 (s, 1H).

### Step 2. 2-Benzofurancarboxylic acid, 5-methoxy-3-(1-methylethoxy), 2-(phenylsulfonyl) hydrazide

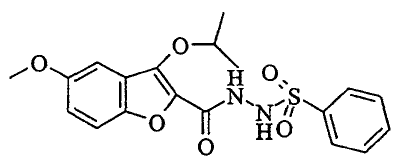

This was run in accordance with Example 35 except 3-isopropoxy-5-methoxybenzofuran-2-carboxylic acid hydrazide was used instead of dibenzofuran-2-carboxylic acid hydrazide, and phenyl sulfonyl chloride was used instead of 3-fluorophenyl chloride. The desired product was purified by column chromatography with ethyl acetate/hexanes (45.6% yield).
MS: 405.3 (M+1 for C₁₉H₂₀N₂O₆S); mp: 127-129°C. HPLC (C18 column, 1:1 MeCN/H₂O+0.1% TFA) 98.6%, RT = 6.14 min. ¹H NMR (DMSO-d₆) δ 3.29 (s, 6H), 3.76 (s, 3H), 4.64 (quintet, 1H), 7.03-7.06 (s+m, 2H), 7.43 (d, 1H, J = 8.54 Hz), 7.52 (t, 2H, J = 8.06 Hz), 7.59-7.63 (m, 1H), 7.80 (dd, 2H, J = 7.32 and 1.47 Hz), 10.00 (s, 1H), and 10.06 (s, 1H).

### EXAMPLE 58

### 2-Benzofurancarboxylic acid, 3, 5-dimethoxy-, 2-(phenylsulfonyl)hydrazide

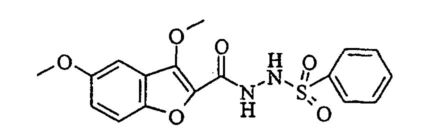

### Step 1. 3,5-Dimethoxy-benzofuran-2-carboxylic acid hydrazide

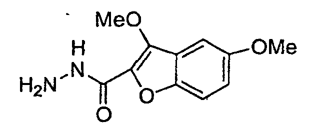

The hydrazide was prepared in accordance with Example 1, Step 2 except that 3,5-dimethoxy-benzofuran-2-carboxylic acid, which was prepared in accordance with the methods of Lamotte et al., *Eur. J. Med Chem. Chim. Ther*., 1986:379-383, was used instead of dibenzofuran-2-carboxylic acid (yield 89.9%).
MS: 237.0 (M+1 for C₁₁H₁₂N₂O₄); mp: 176.2-179.1°C. ¹H NMR (DMSO-d₆) δ 3.78 (s, 3H), 4.11 (s, 3H), 4.83 (s, 2H), 7.02 (dd, 1H, J = 9.04 and 2.69 Hz), 7.21 (d, 1H, J = 2.45 Hz), 7.43 (d, 1H, J = 9.03 Hz), and 9.25 (s, 1H).

### Step 2. 2-Benzofurancarboxylic acid, 3,5-dimethoxy-, 2-(phenylsulfonyl)-hydrazide

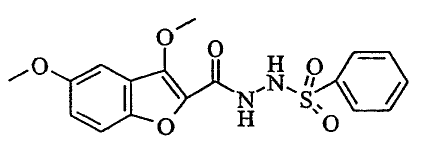

This was run in accordance with Example 56 except 3,5-dimethoxy-benzofuran-2-carboxylic acid hydrazide was used instead of 6-methoxy-benzofuran-2-carboxylic acid hydrazide.
MS: 377.0 (M+1 for C₁₇H₁₆N₂O₆S); mp: 172.2-173.8°C; HPLC (C18 column, 1:1 MeCN/H₂O+0.1% TFA) 99.3%, RT = 4.18 min. Micro: (calc) (C₁₇H₁₆N₂O₆S·0.5H₂O) C: 52.77 H: 4.43, N: 7.24; (found) C: 53.03, H: 4.22, N: 7.09. ¹H NMR (DMSO-d₆) δ 3.78 (s, 3H), 3.87 (s, 3H), 7.06 (dd, 2H, J = 9.04 and 2.69 Hz), 7.18 (d, 1H, J = 2.45 Hz), 7.44 (d, 1H, J = 9.04 Hz), 7.52 (t, 2H, J = 7.09 Hz), 7.61 (t, 1H, J = 7.33 Hz), 7.80 (d, 2H, J = 7.33 Hz), 10.04 (d, 1H, J = 3.20 Hz), and 10.18 (d, 1H, J = 3.20 Hz).

### EXAMPLE 59

### 2-Benzofurancarboxylic acid, 3,6-dimethoxy-, 2-(phenylsulfonyl)hydrazide

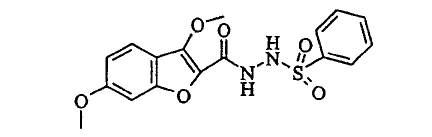

### Step 1. 3,6-Dimethoxy-benzofuran-2-carboxylic acid hydrazide

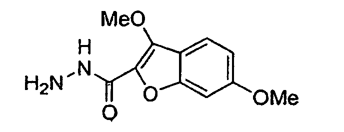

The hydrazide was prepared in accordance with Example 1, Step 2 except that 3,6-dimethoxy-benzofuran-2-carboxylic acid, which was prepared in accordance with Lele, *J. Sci. Ind. Res.,* 1955:101-103, was used instead of dibenzofuran-2-carboxylic acid (yield 85.9%).
MS: 237.0 (M+1 for C₁₁H₁₂N₂O₄); mp: 178.9-181.5°C. ¹H NMR (DMSO-d₆) δ 3.79 (s, 3H), 4.13 (s, 3H), 4.44 (s, 2H), 6.90 (dd, 1H, J = 8.80 and 2.20 Hz), 7.09 (d, 1H, J = 1.95 Hz), 7.71 (d, 1H, J = 8.79 Hz), and 8.97 (s, 1H).

### Step 2. 2-Benzofurancarboxylic acid, 3,6-dimethoxy-, 2-(phenylsulfonyl)-hydrazide

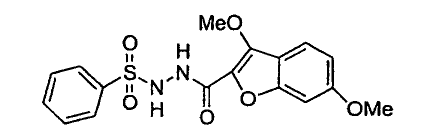

This was run in accordance with Example 56 except 3,6-dimethoxy-benzofuran-2-carboxylic acid hydrazide was used instead of 6-methoxy-benzofuran-2-carboxylic acid hydrazide. The desired product was purified by column chromatography eluting with ethyl acetate/hexanes (37.4% yield).
MS: 377.0 (M+1 for C₁₇H₁₆N₂O₆S); mp: 174.8-175.2°C; HPLC (C18 column, 1:1 MeCN/H₂O+0.1% TFA) 99.7%, RT = 4.20 min. Micro: (calc) (C₁₇H₁₆N₂O₆S·0.2H₂O) C: 53.72, H: 4.35, N: 7.37; (found) C: 53.77, H: 4.02, N: 7.11. ¹H NMR (DMSO-d₆) δ 3.79, (s, 3H), 4.02 (s, 3H), 6.90 (dd, 2H, J = 8.80 and 2.20 Hz), 7.08 (d, 1H, J = 1.95 Hz), 7.52 (t, 2H, J = 7.33 Hz), 7.61 (t, 1H, J = 7.33 Hz), 7.72 (d, 1H, J = 8.79 Hz), 7.81 (d, 2H, J = 7.33 Hz), 9.85 (s, 1H), and 9.99 (s, 1H).

### EXAMPLE 60

### 2-Benzofurancarboxylic acid, 4,6-dimethoxy-, 2-(phenylsulfonyl)hydrazide

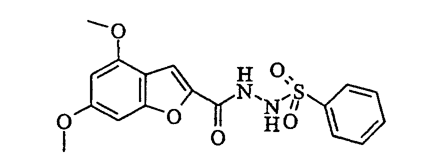

### Step 1. 4,6-Dimethoxy-benzofuran-2-carboxylic acid hydrazide

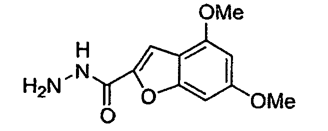

The hydrazide was prepared in accordance with Example 1, Step 2 except that 4,6-dimethoxy-benzofuran-2-carboxylic acid, which was prepared in accordance with Reichstein et al., *Helv. Chim. Acta,* 1935:816, 828, was used instead of dibenzofuran-2-carboxylic acid (yield 50%).
MS: 237.0 (M+1 for C₁₁H₁₂N₂O₄); mp: 185.8-186.6°C. HPLC (C18 column, 1:1 MeCN/H₂O+0.1% TFA) 99.7%, RT = 1.72 min. ¹H NMR (DMSO-d₆) δ 3.78 (s, 3H), 3.86 (s, 3H), 4.51 (s, 2H), 6.42 (d, 1H, J = 1.96 Hz), 7.37 (t, 1H, J = 0.73 Hz), 7.37 (d, 1H, J = 0.73 Hz), and 9.78 (s, 1H).

### Step 2. 2-Benzofurancarboxylic acid, 4,6-dimethoxy-, 2-(phenylsulfonyl-hydrazide

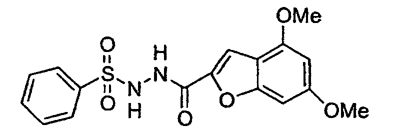

This was run in accordance with Example 56 except 4,6-dimethoxy-benzofuran-2-carboxylic acid hydrazide was used instead of 6-methoxy-benzofuran-2-carboxylic acid hydrazide. The desired product was purified by recrystallization in ethyl acetate/methanol (83.8% yield).
MS: 377.0 (M+1 for C₁₇H₁₆N₂O₆S); mp: 230.6-231.0°C. HPLC (C18 column, 1:1 MeCN/H₂O+0.1% TFA) 99.1%, RT = 5.73 min. Micro: (calc) (C₁₇H₁₆N₂O₆S) C: 54.25, H: 4.28, N: 7.44, S: 8.52; (found) C: 54.34, H: 4.06, N: 7.38, S: 8.60. ¹H NMR (DMSO-d₆) δ 3.77 (s, 3H), 3.84 (s, 3H), 6.42 (d, 1H, J = 1.95 Hz), 6.76 (d, 1H, J = 0.73 Hz), 7.50 (dd, 2H, J = 8.06 and 1.95 Hz), 7.61 (t, 1H, J = 7.33 Hz), 7.78 (dd, 2H, J = 7.08 and 1.46 Hz), 10.67 (s, 1H), and 10.67 (s, 1H).

### EXAMPLE 61

### 2-Benzofurancarboxylic acid, 3,7-dimethoxy-, 2-(phenylsulfonyl)hydrazide

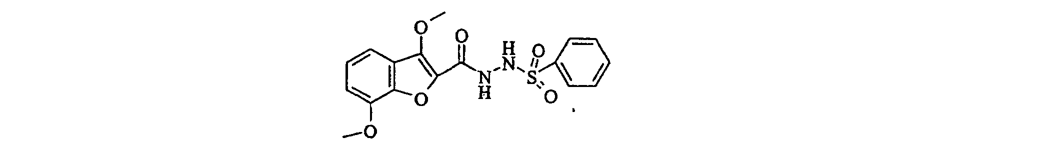

### Step 1. 3,7-Dimethoxy-benzofuran-2-carboxylic acid hydrazide

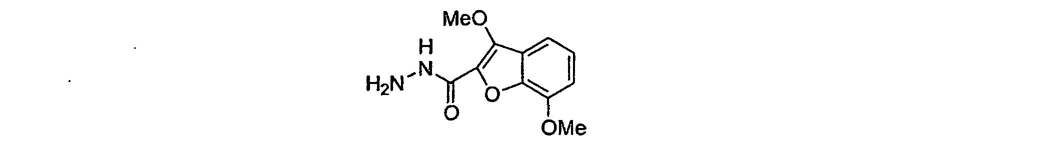

The hydrazide was prepared in accordance with Example 1, Step 2 except that 3,7-dimethoxy-2-benzofurancarboxylic acid, prepared in accordance with the methods of Lamotte et al., *Eur. J. Med Chem. Chim. Ther*., 1986:379-383, was used instead of dibenzofuran-2-carboxylic acid (yield 71 %).
MS: 237.0 (M+1 for C₁₁H₁₂N₂O₄); mp: 172.1-173.0°C. HPLC (C18 column, 1:1 MeCN/H₂O+0.1% TFA) 94.7%, RT = 1.64 min. Micro: (calc) (C₁₁H₁₂N₂O₄·0.2H₂O) C: 55.09, H: 5.21, N: 11.68; (found) C: 55.08, H: 5.04, N: 11.16. ¹H NMR (DMSO-d₆) δ 3.91(s, 3H), 4.11 (s, 3H), 4.49 (s, 2H), 7.04 (d, 1H, J = 7.81 Hz), 7.20 (t, 1H, J = 7.81 Hz), 7.36 (d, 1H, J = 7.81 Hz), and 9.15 (s, 1H).

### Step 2. 2-Benzofurancarboxylic acid, 3,7-dimethoxy-, 2-(phenylsulfonyl-hydrazide

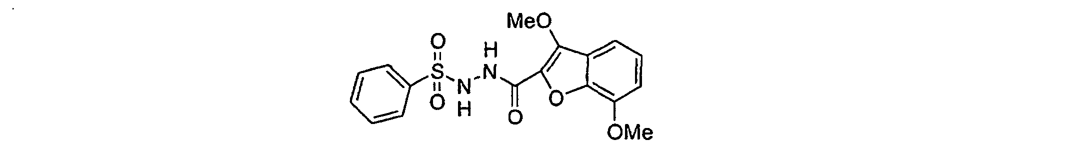

This was run in accordance with Example 56 except 3,7-dimethoxy-benzofuran-2-carboxylic acid hydrazide was used instead of except 6-methoxy-benzofuran-2-carboxylic acid hydrazide. The desired product was purified by recrystallization in ethyl acetate/hexanes (66.5% yield).
MS: 377.0 (M+1 for C₁₇H₁₆N₂O₆S); mp: 177.9-179.6°C. HPLC (C18 column, 1:1 MeCN/H₂O+0.1% TFA) 94.1%, RT = 4.35 min. Micro: (calc) (C₁₇H₁₆N₂O₆S) C: 54.25, H: 4.28, N: 7.44, S: 8.52; (found) C: 54.01, H: 4.23, N: 6.98, S: 8.64. ¹H NMR (DMSO-d₆) δ 3.91 (s, 3H), 4.00 (s, 3H), 7.07 (d, 1H, J = 7.82 Hz), 7.20 (t, 1H, J = 8.06 Hz), 7.35 (d, 1H, J = 7.82 Hz), 7.62 (t, 2H, J = 8.06 Hz), 7.59-7.62 (m, 1H), 7.81 (d, 2H, J = 7.33 Hz), 10.05 (d, 1H, J = 3.2 Hz), and 10.13 (d, 1H, J = 3.2 Hz).

### EXAMPLE 62

### 2-Benzofurancarboxylic acid, 3,7-dimethoxy-, 2-[(2-trifluoromethylphenyl)-sulfonyl]hydrazide

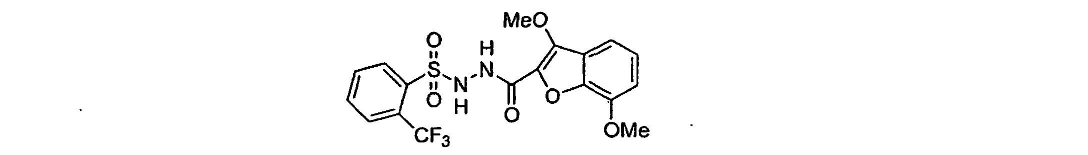

This was run in accordance with Example 61 except 2-trifluoromethylphenyl sulfonyl chloride was used instead of benzenesulfonyl chloride. The desired product was purified by column chromatography eluting with ethyl acetate/hexanes (46.5% yield).
MS: 445.0 (M+1 for C₁₈H₁₅N₂O₆SF₃); mp: 190.6-192.3°C. HPLC (C18 column, 1:1 MeCN/H₂O+0.1% TFA) 97.1%, RT = 6.17 min. Micro: (calc) (C₁₈H₁₅N₂O₆SF₃) C: 48.65, H: 3.40, N: 6.30, S: 7.22; (found) C: 48.59, H: 3.34, N: 6.17, S: 7.26. ¹H NMR (DMSO-d₆) δ 3.90 (s, 3H), 3.98 (s, 3H), 7.07 (d, 1H, J = 7.33 Hz), 7.21 (t, 1H, J = 8.06 Hz), 7.35 (d, 1H, J = 7.08 Hz), 7.70-7.81 (m, 2H), 7.94 (t, 2H, J = 4.15 Hz), 8.16 (t, 2H, J = 4.88 Hz), 10.04 (d, 1H, J = 2.0 Hz), and 10.31 (d, 1H, J = 2.0 Hz).

### EXAMPLE 63

### 2-Benzofurancarboxylic acid, 3,7-dimethoxy-, 2-[(4-fluorophenyl)sulibnyl]-hydrazide

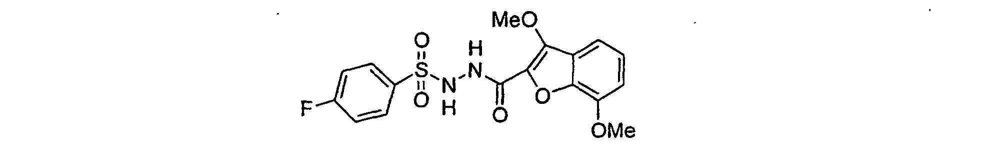

This was run in accordance with Example 61 except 4-fluorophenyl sulfonyl chloride was used instead of benzenesulfonyl chloride. The desired product was purified by column chromatography eluting with ethyl acetate/hexanes (72% yield).
MS: 395.0 (M+1 for C₁₇H₁₅N₂O₆SF); mp: 190.9-191.1°C. HPLC (C18 column, 1:1 MeCN/H₂O+0.1% TFA) 96.8%, RT = 4.90 min. Micro: (calc) (C₁₇H₁₅N₂O₆SF) C: 51.77, H: 3.83, N: 7.10, S: 8.13, F: 4.82; (found) C: 51.73, H: 3.73, N: 7.02, S: 8.04, F: 4.88. ¹H NMR (DMSO-d₆) δ 3.91 (s, 3H), 4.01 (s, 3H), 7.07 (d, 1H, J = 8.06 Hz), 7.21 (t, 1H, J = 8.06 Hz), 7.37 (t, 3H, J = 9.04 Hz), 7.84-7.88 (m, 2H), 10.12 (s, 1H), and 10.20 (s, 1H).

### EXAMPLE 64

### 2-Benzofurancarboxylic acid, 3,7-dimethoxy-, 2-[(3-methylphenyl)sulfonyl]-hydrazide

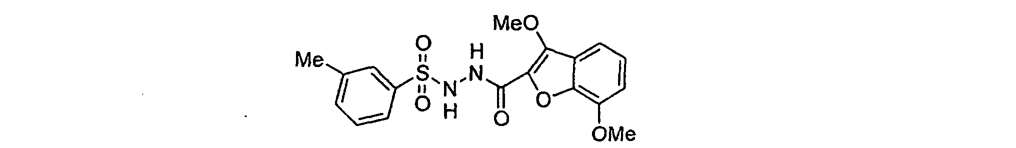

This was run in accordance with Example 61 except 3-methylphenyl sulfonyl chloride was used instead of benzenesulfonyl chloride. The desired product was purified by column chromatography eluting with ethyl acetate/hexanes (70% yield).
MS: 391.0 (M+1 for C₁₈H₁₈N₂O₆S); mp: 179.4-180.9°C. HPLC (C18 column, 1:1 MeCN/H₂O+0.1% TFA) 97.8%, RT = 5.22 min. Micro: (calc) (C₁₈H₁₈N₂O₆S·0.7H₂O) C: 53.55, H: 4.84, N: 6.94, S: 7.94; (found) C: 53.61, H: 4.37, N: 6.88, S: 8.19. ¹H NMR (DMSO-d₆) δ 3.90 (s, 3H), 4.01 (s, 3H), 7.07 (d, 1H, J = 8.06 Hz), 7.21 (t, 1H, J = 8.06 Hz), 7.36 (d, 1H, J = 8.06 Hz), 7.38-7.40 (m, 1H), 7.42 (s, 1H), 7.60 (d, 1H, J = 6.83 Hz), 7.64 (s, 1H), 10.0 (s, 1H), and 10.09 (s, 1H).

### EXAMPLE 65

### 2-Benzofurancarboxylic acid, 3-methoxy-5-phenyl-, 2-(phenylsulfonyl)hydrazide

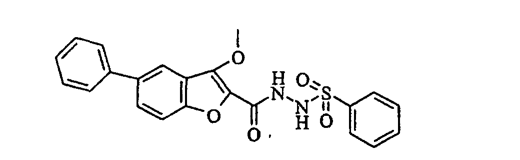

### Step 1. 3-Methoxy-5-phenyl-benzofuran-2-carboxylic acid hydrazide

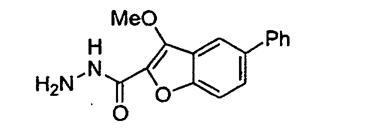

This was run in accordance with Example 1, Step 2 except 3-methoxy-5-phenylbenzofuran-2-carboxylic acid was used instead of dibenzofuran-2-carboxylic acid (yield 94%).
MS: 283.0 (M+1 for C₁₆H₁₄N₂O₃); mp: 175.3-177.3°C. HPLC (C18 column, 1:1 MeCN/H₂O+0.1% TFA) 99.8%, RT = 1.42 min. Micro: (calc) (C₁₆H₁₄N₂O₃·0.1H₂O) C: 67.64, H: 5.04, N: 9.86; (found) C: 67.62, H: 5.05 N: 9.65. ¹H NMR (DMSO-d₆) δ 4.20 (s, 3H), 4.52 (s, 2H), 7.35 (t, 1H, J = 7.33 Hz), 7.45 (t, 2H, J = 7.33 Hz), 7.62 (d, 1H, J = 8.79 Hz), 7.69-7.74 (m, 3H), 8.02 (d, 1H, J = 1.47 Hz), and 9.27 (s, 1H).

### Step 2. 2-Benzofurancarboxylic acid, 3-methoxy-5-phenyl-, 2-(phenylsulfonyl)-hydrazide

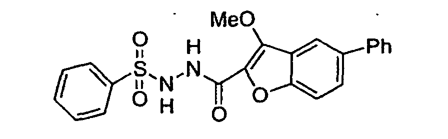

This was run in accordance with Example 35 except 3-methoxy-5-phenyl-benzofuran-2-carboxylic acid hydrazide was used instead of dibenzofuran-2-carboxylic acid hydrazide, and phenyl sulfonyl chloride was used instead of 3-fluorophenyl chloride. The desired product was purified by recrystallization with ethyl acetate/hexanes (53.9% yield).
MS: 423.0 (M+1 for C₂₂H₁₈N₂O₅S); mp: 180.7-181.3°C. HPLC (C18 column, 1:1 MeCN/H₂O+0.1% TFA) 98.4%, RT = 10.39 min. Micro: (calc) (C₂₂H₁₈N₂O₅S) C: 62.55, H: 4.29, N: 6.63, S: 7.59; (found) C: 62.16, H: 4.05, N: 6.57, S: 7.63; ¹H NMR (DMSO-d₆) δ 4.07 (s, 3H), 7.35 (t, 1H, J = 8.06 Hz), 7.45 (dd, 2H, J = 1.71 and 7.33 Hz), 7.52 (dd, 2H, J = 1.22 and 7.82 Hz), 7.62 (t, 2H, J = 6.84 Hz), 7.70 (d, 2H, J = 8.55 Hz), 7.76 (dd, 1H, J = 1.95 and 8.79 Hz), 7.82 (d, 2H, J = 7.08 Hz), 8.01 (d, 1H, J = 1.47 Hz), 10.08 (d, 1H, J = 3.2 Hz), and 10.18 (d, 1H, J = 3.2 Hz).

### EXAMPLE 66

### 2-Benzofurancarboxylic acid, 3-ethoxy-5-phenyl-, 2-(phenylsulfonyl)hydrazide

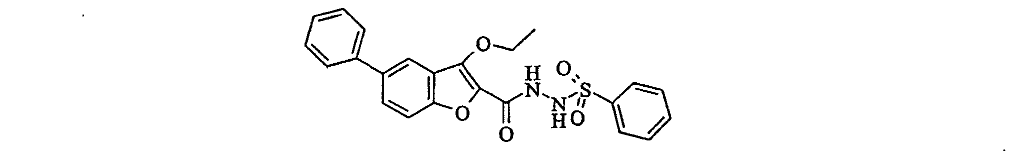

### Step 1. 3-Ethoxy-5-phenyl-benzofuran-2-carboxylic acid hydrazide

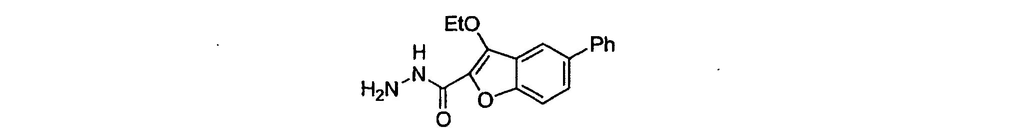

This was run in accordance with Example 1, Step 2 except 3-ethoxy-5-phenylbenzofuran-2-carboxylic acid was used instead of dibenzofuran-2-carboxylic acid (yield 44%).
MS: 297.3 (M+1 for C₁₇H₁₆N₂O₃); mp: 94.8-96.8°C. HPLC (C18 column, 1:1 MeCN/H₂O+0.1% TFA) 97.3%, RT = 2.44 min. ¹H NMR (DMSO-d₆) δ 1.34 (t, 3H, J = 7.08 Hz), 4.53 (s+q, 4H, J = 6.84 Hz), 7.36 (t, 1H, J = 6.11 Hz), 7.45 (t, 2H, J = 7.33 Hz), 7.62 (d, 1H, J = 8.79 Hz), 7.70-7.73 (m, 3H), 7.95 (d, 1H, J = 1.47 Hz), and 9.22 (s, 1H).

### Step 2. 2-Benzofurancarboxylic acid, 3-ethoxy-5-phenyl-, 2-(phenylsulfonyl)hydrazide

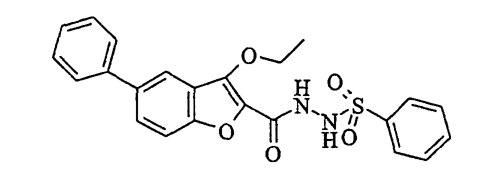

This was run in accordance with Example 35 except 3-ethoxy-5-phenyl-benzofuran-2-carboxylic acid hydrazide was used instead of dibenzofuran-2-carboxylic acid hydrazide, and phenyl sulfonyl chloride was used instead of 3-fluorophenyl chloride. The desired product was purified by column chromatography with ethyl acetate/hexanes (42.5% yield).
MS: 437.1 (M+1 for C₂₃H₂₀N₂O₅S); mp: 184.3-184.6°C; HPLC (C18 column, 1:1 MeCN/H₂O+0.1% TFA) 99.9%, RT = 13.67 min. Micro: (calc) (C₂₃H₂₀N₂O₅S·0.2H₂O) C: 62.62, H: 4.66, N: 6.35; (found) C: 62.72, H: 4.52, N: 6.25. ¹H NMR (DMSO-d₆) δ 1.34 (t, 3H, J = 7.09 Hz), 4.45 (q, 2H, J = 6.83 Hz), 7.45 (t, 1H, J = 7.33 Hz), 7.55 (t, 2H, J = 7.33 Hz), 7.64 (t, 2H, J = 7.81 Hz), 7.72 (t, 2H, J = 5.63 Hz), 7.80 (d, 2H, J = 8.55 Hz), 7.85 (dd, 1H, J = 1.71 and 8.79 Hz), 7.92 (d, 2H, J = 7.08 Hz), 8.05 (d, 1H, J = 1.71 Hz), 10.18 (s, 1H), and 10.18 (s, 1H).

### EXAMPLE 67

### 2-Benzofurancarboxylic acid, 3-methoxy-7-phenyl-, 2-(phenylsulfonyl)hydrazide

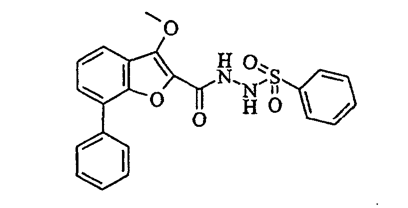

### Step 1. 3-Methoxy-7-phenyl-benzofuran-2-carboxylic acid hydrazide

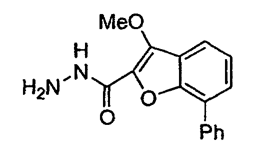

The hydrazide was prepared in accordance with Example 1, Step 2 except that except 3-methoxy-7-phenylbenzofuran-2-carboxylic acid was used instead of dibenzofuran-2-carboxylic acid (yield 44%).
MS: 283.2 (M+1 for C₁₆H₁₄N₂O₃); mp: 177.8-179.13°C. HPLC (C18 column, 1:1 MeCN/H₂O+0.1% TFA) 99.0%, RT = 1.42 min. Micro: (calc) (C₁₆H₁₄N₂O₃·0.5H₂O) C: 65.97, H: 5.19, N: 9.62; (found) C: 66.06, H: 4.93, N: 9.16. ¹H NMR (DMSO-d₆) δ 4.15 (s, 3H), 4.54 (s, 2H), 7.37-7.52 (m, 2H), 7.50 (dt, 2H, J = 1.71 and 7.33 Hz), 7.64 (dd, 1H, J = 0.98 and 7.33 Hz), 7.78 (dd, 1H, J = 0.98 and 7.33 Hz), 7.90 (dd, 2H, J = 1.47 and 8.55 Hz), and 9.30 (s, 1H).

### Step 2. 2-Benzofurancarboxylic acid, 3-methoxy-7-phenyl-, 2-(phenylsulfonyl)-hydrazide

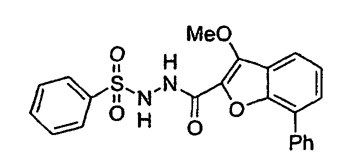

. This was run in accordance with Example 35 except 3-methoxy-7-phenyl benzofuran-2-carboxylic acid hydrazide was used instead of dibenzofuran-2-carboxycylic acid hydrazide, and phenyl sulfonyl chloride was used instead of 3-fluorophenyl chloride. The desired product was purified by recrystallization with ethyl acetate/hexanes (53.9% yield).
MS: 423.0 (M+1 for C₂₂H₁₈N₂O₅S); mp: 186.7-187.3°C. HPLC (C18 column, 1:1 MeCN/H₂O+0.1% TFA) 97.7%, RT = 10.04 min. Micro: (calc) (C₂₂H₁₈N₂O₅S) C: 62.55, H: 4.29, N: 6.63, S: 7.59; (found) C: 62.09, H: 4.34, N: 6.58, S: 7.72. ¹H NMR (DMSO-d₆) δ 4.00 (s, 3H), 7.38-7.41 (m, 2H), 7.41-7.57 (m, 4H), 7.60 (d, 1H, J = 7.57 Hz), 7.67 (dd, 1H, J = 1.23 and 7.58 Hz), 7.77-7.83 (m, 3H), 7.87 (dd, 1H, J = 1.22 and 7.08 Hz), 10.08 (d, 1H, J = 2.8 Hz), and 10.17 (d, 1H, J = 2.8 Hz).

### EXAMPLE 68

### Naptho[2,3-b]furan-2-carboxylic acid, 3-methoxy-, 2-(phenylsulfonyl)hydrazide

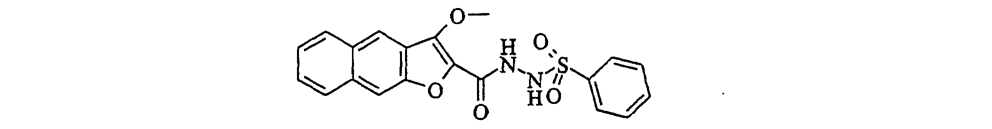

### Step 1. 3-Methoxy-naptho[2,3-b]furan-2-carboxylic acid hydrazide

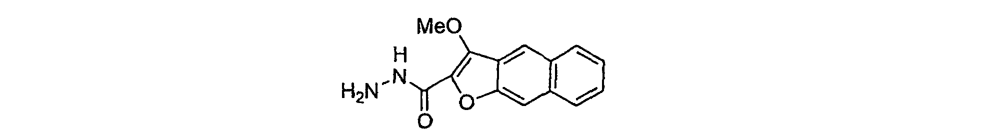

The hydrazide was prepared in accordance with Example 1, Step 2 except that 3-methoxy-naptho[2,3-*b*]furan-2-carboxylic acid, which was prepared in accordance with the methods of Einhorn et al., *J. Het. Chem*., 1985:1243, 1247, was used instead of dibenzofuran-2-carboxylic acid (yield 90%).
MS: 257.1 (M+1 for C₁₄H₁₂N₂O₃). ¹H NMR (CDCl₃) δ 3.4 (t, 1H, J = 3.66 Hz), 3.59 (s, 1H), 4.35 (s, 3H), 5.90 (br., 1H), 7.37-7.46 (m, 2H), 7.85 (s, 2H), 7.88 (d, 1H,
J = 9.59 Hz), and 8.21 (s, 1H).

### Step 2. Naptho[2,3-b]furan-2-carboxylic acid, 3-methoxy-, 2-(phenylsulfonyl)-hydrazide

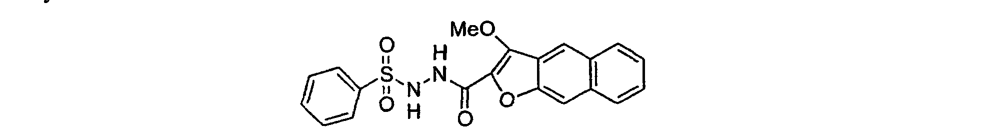

This was run in accordance with Example 35 except 3-methoxynapthofuran-2-carboxylic acid hydrazide was used instead of dibenzofuran-2-carboxycylic acid hydrazide, and phenyl sulfonyl chloride was used instead of 3-fluorophenyl chloride. The desired product was purified by chromatography with ethyl acetate/hexanes (38% yield).
MS: 397.0 (M+1 for C₂₀H₁₆N₂O₅S); mp: 205.7-207.1°C. HPLC (C18 column, 1:1 MeCN/H₂O+0.1% TFA) 98.7%, RT = 7.61 min. ¹H NMR (DMSO-d₆) δ 4.09 (s, 3H), 7.43-7.49 (m, 1H), 7.53 (t, 3H, J = 7.32 Hz), 7.61 (t, 1H, J = 7.81 Hz), 7.83 (d, 2H, J = 7.33 Hz), 8.0-8.07 (m, 3H), 8.45 (s, 1H), 10.12 (s, 1H), and 10.34 (s, 1H).

### EXAMPLE 69

### 2-Benzofurancarboxylic acid, 5,7-dichloro 2-(phenylsulfonyl) sulfonyl hydrazide

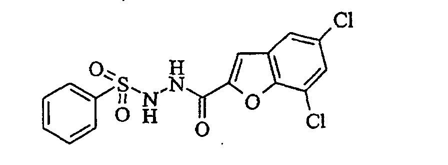

### Step 1. 5,7-Dichloro-benzofuran-2-carboxylic acid hydrazide

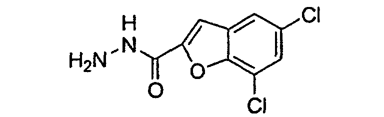

The hydrazide was prepared in accordance with Example 1, Step 2 using 5,7-dichloro-benzofuran-2-carboxylic acid that was made according to the procedure in Kurdakar R., *Proc.-Indian Acad. Sci.* Sect A, 1963;58:336, 339 instead of dibenzofuran-2-carboxylic acid (yield 71 %).
MS: 244.9 (M+1 for C₉H₆N₂O₂Cl₂); mp: 237.8-240.0°C. HPLC (C18 column, 1:1 MeCN/H₂O+0.1% TFA) 98.0%, RT = 2.2 min. Micro: (calc) (C₉H₆N₂O₂Cl₂·0.65H₂O) C: 42.10, H: 2.87, N: 10.91, Cl: 27.61; (found) C: 42.09, H: 2.50, N: 10.641, Cl: 27.21. ¹H NMR (DMSO-d₆) δ 4.61 (s, 2H), 7.54 (s, 1H), 7.68 (d, 1H, J = 1.95 Hz), 7.85 (d, 1H, J = 1.95 Hz), and 10.15 (s, 1H).

### Step 2. 2-Benzofurancarboxylic acid, 5,7-dichloro 2-(phenylsulfonyl)sulfonyl hydrazide

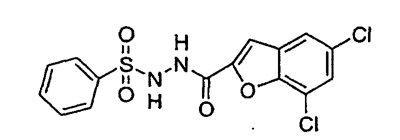

This was run in accordance with Example 35 except that 5,7-dichloro-benzofuran-2-carboxylic acid hydrazide was used instead of dibenzofuran-2-carboxylic acid hydrazide, and phenyl sulfonyl chloride was used instead of 3-fluorophenyl chloride. The desired product was purified by recrystallization in ethyl acetate (52.1 % yield).
MS: 384.8 (M+1 for C₁₅H₁₀N₂O₄SCl₂); mp: 216.4-218.1°C; Micro: (calc) for (C₁₅H₁₀N₂O₄SCl₂) C: 46.77, H: 2.62, N: 7.27, S: 8.32, Cl: 18.41; (found) C: 46.69, H: 2.95, N: 7.14, S: 8.12, Cl: 18.45; HPLC (C18 column, 1:1 MeCN/H₂O+0.1% TFA) 94.97%, RT = 8.10 min. ¹H NMR (DMSO-d₆) δ 7.53 (t, 2H, J = 7.81 Hz), 7.51-7.61 (m, 1H), 7.66 (s, 1H), 7.72 (d, 1H, J = 1.96 Hz), 7.81 (d, 2H, J = 7.33 Hz), 7.72 (d, 1H, J = 1.96 Hz), 10.27 (d, 1H, J = 2.40 Hz), and 11.10 (d, 1H, J = 2.40 Hz).

### EXAMPLE 70

### 2-Benzofurancarboxylic acid, 5,7-dichloro 2-[[2-(trifluoromethyl)phenyl)]-sulfonyl]hydrazide

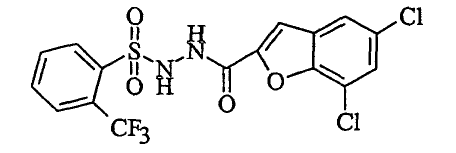

This was run in accordance with Example 69 except that 2-trifluoromethyl phenyl sulfonyl chloride was used instead of benzene sulfonyl chloride. The desired product was purified by column chromatography eluting with ethyl acetate/hexanes (61.7% yield).
MS: 452.9 (M+1 for C₁₆H₉N₂O₄SF₃Cl₂); mp: 245.6-247.6°C; Micro: (calc) (C₁₆H₉N₂O₄SF₃Cl₂) C: 42.40, H: 2.00, N: 6.18, S: 7.07, F: 12.58; (found) C: 42.79, H: 1.99, N: 6.04, S: 6.87, F: 12.31; HPLC (C18 column, 1:1 MeCN/H₂O+0.1% TFA) 99.5%, RT = 13.50 min. ¹H NMR (DMSO-d₆) δ 7.65 (s, 1H), 7.72 (d, 1H, J = 1.96 Hz), 7.78-7.84 (m, 2H), 7.88 (d, 1H, J = 1.96 Hz), 7.96 (d, 1H, J = 6.60 Hz), 8.16 (d, 1H, J = 6.35 Hz), 10.27 (s, 1H), and 11.18 (s, 1H).

### EXAMPLE 71

### 2-Benzofurancarboxylic acid, 5,7-dichloro 2-[(2-methylphenyl)sulfonyl]hydrazide

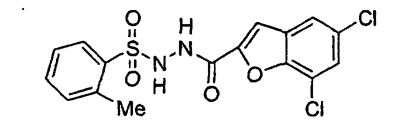

This was run in accordance with Example 69 except that 2-methyl phenyl sulfonyl chloride was used instead of benzene sulfonyl chloride. The desired product was purified by recrystallizing in ethyl acetate/hexanes and then chromatographing in the same solvent system (28.7% yield).
MS: 398.9 (M+1 for C₁₆H₁₂N₂O₄SCl₂); mp: 212-214°C; Micro: (calc) (C₁₆H₁₂N₂O₄SCl₂) C: 48.13, H: 3.03, N:7.02, S: 8.03; (found) C: 48.08, H: 2.78, N: 6.87, S: 8.14; HPLC (C 18 column, 1:1 MeCN/H₂O+0.1% TFA) 97.9%, RT = 11.86 min. ¹H NMR (DMSO-d₆) δ 2.68 (s, 3H), 7.26 (t, 1H, J = 7.58 Hz), 7.37 (d, 1H, J = 7.33 Hz), 7.48 (t, 1H, J = 7.33 Hz), 7.63 (s, 1H), 7.71 (d, 1H, J = 1.95 Hz), 7.80 (d, 1H, J = 7.09 Hz), 7.86 (d, 1H, J = 1.71 Hz), 10.19 (s, 1H), and 11.03 (s, 1H).

### EXAMPLE 72

### 2-Benzofurancarboxylic acid, 5,7-dichloro 2-[(3-methylphenyl)sulfonyl]-hydrazide

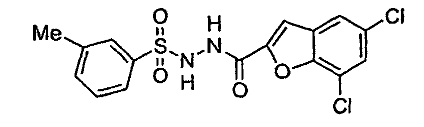

This was run in accordance with Example 69, except that 3-methyl phenyl sulfonyl chloride was used instead of benzene sulfonyl chloride. The desired product was purified by recrystallizing in ethyl alcohol (34.1% yield).
MS: 398.9 (M+1 for C₁₆H₁₂N₂O₄SCl₂); mp: 230.4-231.1°C; Micro: (calc) (C₁₆H₁₂N₂O₄SCl₂) C: 48.13, H: 3.03, N: 7.02, S:8.03; (found) C: 48.10, H: 2.87, N: 6.96, S: 8.15; HPLC (C18 column, 1:1 MeCN/H₂O+0.1% TFA) 99.6%,
RT = 10.45 min. ¹H NMR (DMSO-d₆) δ 2.32 (s, 3H), 7.38-7.45 (m, 2H), 7.59 (d, 1H, J = 7.57 Hz), 7.63 (s, 1H), 7.66 (s, 1H), 7.72 (d, 1H, J = 1.96 Hz), 7.80 (d, 1H, J = 1.71 Hz), 10.23 (s, 1H), and 11.07 (s, 1H).

### EXAMPLE 73

### 2-Benzofurancarboxylic acid, 5,7-dichloro 2-[(2-bromophenyl)sulfonyl]-hydrazide

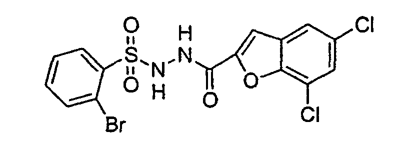

This was run in accordance with Example 69 except that 2-bromophenyl sulfonyl chloride was used instead of benzene sulfonyl chloride. The desired product was purified by column chromatography eluting with ethyl acetate/hexanes 1:2 (26.9% yield).
MS: 462.8 (M+1 for C₁₅H₉N₂O₄SCl₂Br); mp: 196.2-196.6°C. HPLC (C18 column, 1:1 MeCN/H₂O+0.1% TFA) 99.0%, RT = 14.32 min. ¹H NMR (DMSO-d₆) δ 7.56-7.64 (m, 2H), 7.75 (s, 1H), 7.80 (d, 1H, J = 1.96 Hz), 7.91 (d, 1H, J = 6.84 Hz), 7.96 (d, 1H, J = 1.71 Hz), 8.09 (dd, 1H, J = 6.10 and 1.95 Hz), 10.41 (s, 1H), and 11.16 (s, 1H).

### EXAMPLE 74

### 2-Benzofurancarboxylic acid, 5,7-dichloro 2-[(3-bromophenyl)sulfonyl]-hydrazide

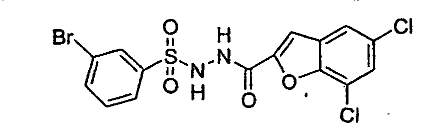

This was run in accordance with Example 69 except that 3-bromophenyl sulfonyl chloride was used instead of benzene sulfonyl chloride. The desired product was purified by recrystallizing in ethyl acetate (35.2% yield).
MS: 462.8 (M+1 for C₁₅H₉N₂O₄SCl₂Br); mp: 248.5-248.8°C; Micro: (calc) (C₁₅H₉N₂O₄SCl₂Br) C: 48.13, H: 3.03, N: 7.02, S: 8.03; (found) C: 48.10, H: 2.87, N: 6.96, S: 8.15; HPLC (C18 column, 1:1 MeCN/H₂O+0.1% TFA) 97.5%,
RT = 14.78 min. ¹H NMR (DMSO-d₆) δ 7.49 (t, 1H, J = 8.06 Hz), 7.67 (s, 1H), 7.73 (d, 1H, J = 1.95 Hz), 7.78 (dd, 1H, J = 6.83 and 0.98 Hz), 7.84 (dd, 1H, J = 0.97 and 1.95 Hz), 7.89 (d, 1H, J = 1.96 Hz), 7.94 (s, 1H), 10.50 (d, 1H, J = 2.40 Hz), and 11.17 (d, 1H, J = 2.40 Hz).

### EXAMPLE 75

### 2-Benzofurancarboxylic acid, 5,7-dichloro 2-[(3-chlorophenyl)sulfonyl]-hydrazide

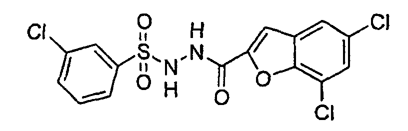

This was run in accordance with Example 69 except that 3-chlorophenyl sulfonyl chloride was used instead of benzene sulfonyl chloride. The desired product was purified by recrystallizing in ethyl acetate (62.2% yield).
MS: 420.9 (M+1 for C₁₅H₉N₂O₄SCl₃); mp: 245.6-245.7°C; Micro: (calc) (C₁₅H₉N₂O₄SCl₃) C: 42.93, H: 2.16, N: 6.68, S: 7.64, Cl: 25.34; (found) C: 42.96, H: 2.10, N: 6.75, S: 7.73, Cl: 25.08; HPLC (C18 column, 1:1 MeCN/H₂O+0.1% TFA) 92.7%, RT = 13.64 min. ¹H NMR (DMSO-d₆) δ 7.56, (t, 1H, J = 8.06 Hz), 7.67 (s, 1H), 7.71-7.77 (m, 3H), 7.82 (d, 1H, J = 1.71 Hz), 7.89 (d, 1H, J = 1.95 Hz), 10.51 (d, 1H, J = 2.40 Hz), and 11.17 (d, 1H, J = 2.40 Hz).

### EXAMPLE 76

### 2-Benzofurancarboxylic acid, 5,7-dichloro 2-[[2-(trifluoromethoxy)phenyl]sulfonyl]hydrazide

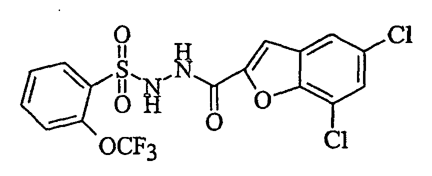

This was run in accordance with Example 69 except that 2-trifluoromethoxyphenyl sulfonyl chloride was used instead of benzene sulfonyl chloride. The desired product was purified by column chromatography eluting with 1:2 ethyl acetate/hexanes (33.5% yield).
MS: 468.9 (M+1 for C₁₆H₉N₂O₅SF₃Cl₂); mp: 234.5-236.1°C; Micro: (calc) (C₁₆H₉N₂O₅SF₃Cl₂) C: 40.99, H: 1.93, N: 5.97, S: 6.83; (found) C: 41.05, H: 1.88, N: 5.81, S: 6.38; HPLC (C18 column, 1:1 MeCN/H₂O+0.1% TFA) 97.1%,
RT = 14.82 min. ¹H NMR (DMSO-d₆) δ 7.47 (t, 1H, J = 7.81 Hz), 7.53 (d, 1H, J = 8.31 Hz), 7.65 (s, 1H), 7.72 (d, 1H, J = 1.95 Hz), 7.76 (dd, 1H, J = 1.46 and 8.06 Hz), 7.89 (d, 1H, J = 1.71 Hz), 7.93 (dd, 1H, J = 1.71 and 7.81 Hz), 10.42 (s, 1H), and 11.15 (s, 1H).

### EXAMPLE 77

### Benzofurancarboxylic acid, 5-methyl-, 2-(phenylsulfonyl)hydrazide

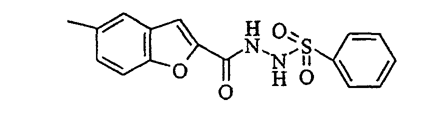

### Step 1. 5-Methyl-benzofuran-2-carboxylic acid, hydrazide

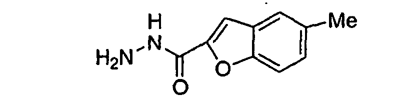

This was run in accordance with Example 1, Step 2 using 5-methyl-benzofuran-2-carboxylic acid that was prepared in accordance to Pappalardo, *Boll. Sci. Fac. Ind. Bologna* 1952:168, instead of dibenzofuran-2-carboxylic acid (95.5% yield). MS: 190.9 (M+1 for C₁₀H₁₀N₂O₂); mp: 148.3-150.3°C. HPLC (C18 column, 1:1 MeCN/H₂O+0.1% TFA) 98.4%, RT = 1.77 min. Micro: (calc) (C₁₀H₁₀N₂O₂·0.15H₂O) C: 62.26, H: 5.38, N: 14.52; (found) C: 62.26, H: 5.38, N: 14.52. ¹H NMR (DMSO-d₆) δ 2.37 (s, 3H), 4.41 (d, 2H, J = 3.66 Hz), 7.21 (dd, 1H, J = 8.55 and 1.47 Hz), 7.39 (d, 1H, J = 0.73 Hz), 7.46 (d, 1H, J = 9.28 Hz), 7.49 (s, 1H), and 9.95 (s, 1H).

### Step 2. Benzofurancarboxylic acid, 5-methyl-, 2-(phenylsulfonyl)hydrazide

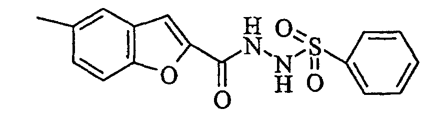

This was run in accordance with Example 35 using 5-methylbenzofuran-2-carboxylic acid hydrazide instead of dibenzofuran-2-carboxycylic acid hydrazide was used, and benzene sulfonylchloride instead of 3-fluorophenyl sulfonyl chloride was used. The desired product was purified by column chromatography eluting with ethyl acetate/hexanes (63.5% yield).
MS: 331.0 (M+1 for C₁₆H₁₄N₂O₄S); mp: 183.3-183.6°C. HPLC (C18 column, 1:1 MeCN/H₂O+0.1% TFA) 99.0%, RT = 5.10 min. Micro: (calc) (C₁₆H₁₄N₂O₄S) C: 58.17, H: 4.27, N: 8.48, S: 9.71; (found) C: 58.09, H: 4.30, N: 8.21, S: 9.65. ¹H NMR (DMSO-d₆) δ 2.36 (s, 3H), 7.25 (dd, 1H, J = 8.55 and 1.71 Hz), 7.47-7.60 (m, 5H), 7.61 (t, 1H, J = 2.20 Hz), 7.80 (dd, 2H, J = 7.08 and 1.47 Hz), 10.16 (s, 1H), and 10.88 (s, 1H).

### EXAMPLE 78

### 2-Benzofurancarboxylic acid, 5-chloro-3-methoxy, 2-(phenylsulfonyl)hydrazide

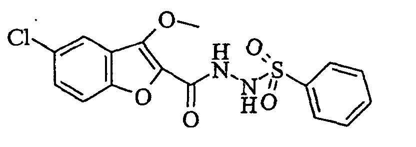

### Step 1. 5-Chloro-3-methoxy-2-carboxylic acid hydrazide

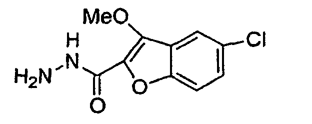

This was run in accordance with Example 1, Step 2 except 5-chloro-3-methoxy-benzofuran-2-carboxylic acid, which was prepared in accordance with the methods of Perkin, *J. Chem. Soc.,* 1951:101-103, was used instead of dibenzofuran-2-carboxylic acid (76% yield).
MS: 241.0.9 (M+1 for C₁₀H₉N₂O₃Cl); mp: 205.6-206.3°C. HPLC (C18 column, 1:1 MeCN/H₂O+0.1% TFA) 94.7%, RT = 2.12 min. ¹H NMR (DMSO-d₆) δ 4.12 (s, 3H), 4.52 (s, 2H), 7.46 (dd, 1H, J = 8.79 and 2.19 Hz), 7.58 (d, 1H, J = 8.79 Hz), 7.91 (d, 1H, J = 2.20 Hz), and 9.31 (s, 1H).

### Step 2. 2-Benzofurancarboxylic acid, 5-chloro-3-methoxy-, 2-(phenylsulfonyl)-hydrazide

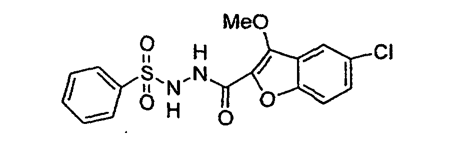

This was run in accordance with Example 35 except 5-chloro-3-methoxy-benzofuran-2-carboxylic acid hydrazide was used instead of dibenzofuran-2-carboxycylic acid hydrazide was used, and benzene sulfonylchloride instead of 3-fluorophenyl sulfonyl chloride was used (28.5% yield).
MS: 381.0 (M+1 for C₁₆H₁₃N₂O₅SCl); mp: 242.2-244.2°C. HPLC (C18 column, 1:1 MeCN/H₂O+0.1% TFA) 98.0%, RT = 6.38 min. Micro: (calc) (C₁₆H₁₃N₂O₅SCl·0.3H₂O) C: 49.64, H: 3.54, N: 7.24, S: 8.28; (found) C: 49.78, H: 3.23, N: 7.23, S: 8.56. ¹H NMR (DMSO-d₆) δ 4.01 (s, 3H), 7.48-7.54 (m, 3H), 7.58-7.64 (m, 2H), 7.81 (d, 2H, J = 7.57 Hz), 7.91 (d, 1H, J = 1.96 Hz), 10.09 (s, 1H), and 10.23 (s, 1H).

### EXAMPLE 79

### 2-Benzofurancarboxylic acid, 5-methyl-, 2-[[2-(trifluoromethyl)phenyl]sulfonyl]-hydrazide

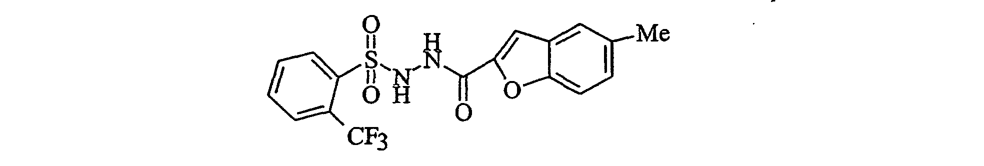

This was run in accordance with Example 77 except 2-trifluoromethyl phenyl sulfonyl chloride was used instead of benzene sulfonyl chloride. The desired product was purified by column chromatography eluting with ethyl acetate/hexanes (62.5% yield).
MS: 398.9 (M+1 for C₁₇H₁₃N₂O₄SF₃); mp: 194.0-195.4°C. HPLC (C18 column, 1:1 MeCN/H₂O+0.1% TFA) 98.8%, RT = 8.34 min. Micro: (calc) (C₁₇H₁₃N₂O₄SF₃) C: 51.26, H: 3.29 N: 7.03, S: 8.05, F: 14.31; (found) C: 51.24, H: 3.40, N: 6.87, S: 7.93, F: 13.87. ¹H NMR (DMSO-d₆) δ 2.36 (s, 3H), 7.25 (dd, 1H, J = 8.30 and 1.71 Hz), 7.47-7.51 (m, 3H), 7.76-7.83 (m, 2H), 7.95 (dd, 2H, J = 7.08 and 1.96 Hz), 8.15 (dd, 2H, J = 6.84 and 2.20 Hz), 10.15 (s, 1H), and 10.96 (s, 1H).

### EXAMPLE 80

### 2-Benzofurancarboxylic acid, 5-methyl-, 2-([3-methylphenyl)sulfonyl]hydrazide

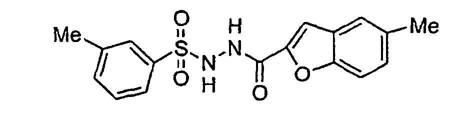

This was run in accordance with Example 77 except 3-methylphenyl sulfonyl chloride was used instead of benzene sulfonyl chloride. The desired product was purified by column chromatography eluting with ethyl acetate/hexanes (64.6% yield). MS: 345.0 (M+1 for C₁₇H₁₆N₂O₄S); mp: 184.1-186.0°C. HPLC (C18 column, 1:1 MeCN/H₂O+0.1% TFA) 99.28%, RT = 5.91 min.; Micro: (calc) (C₁₇H₁₆N₂O₄S) C: 59.29, H: 4.69, N: 8.14, S: 9.29; (found) C: 59.15, H: 4.67, N: 8.13, S: 9.56. ¹H NMR (DMSO-d₆) δ 2.31 (s, 3H), 2.36 (s, 3H), 7.25 (dd, 1H, J = 8.31 and 1.95 Hz), 7.37-7.43 (m, 2H), 7.48-7.51 (m, 3H), 7.59 (d, 1H, J = 7.33 Hz), 7.62 (s, 1H), 10.11 (s, 1H), and 10.84 (s, 1H).

### EXAMPLE 81

### 2-Benzofurancarboxylic acid, 5-methyl-, 2-[2-(methylphenyl)sulfonyl]hydrazide

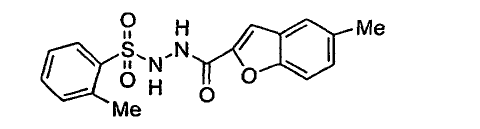

This was run in accordance with Example 77 except 2-methylphenyl sulfonyl chloride was used instead of benzene sulfonyl chloride. The desired product was purified by column chromatography eluting with ethyl acetate/hexanes (57.0% yield). MS: 345.0 (M+1 for C₁₇H₁₆N₂O₄S); mp: 174.5-174.7°C. HPLC (C18 column, 1:1 MeCN/H₂O+0.1% TFA) 99.6%, RT = 6.68 min. Micro: (calc) (C₁₇H₁₆N₂O₄S) C: 59.29, H: 4.69, N: 8.14, S: 9.29; (found) C: 59.21, H: 4.51, N: 8.05, S: 9.38. ¹H NMR (DMSO-d₆) δ 2.36 (s, 3H), 2.67 (s, 3H), 7.25 (t, 2H, J = 7.57 Hz), 7.35 (d, 1H, J = 7.32 Hz), 7.45-7.49 (m, 4H), 7.79 (dd, 1H, J = 7.82 and 1.22 Hz), 10.07 (s, 1H), and 10.79 (s, 1H).

### EXAMPLE 82

### 2-Benzofurancarboxylic acid, 5-methyl-, 2-[(3-chloro-2-methylphenyl)sulfonyl]hydrazide

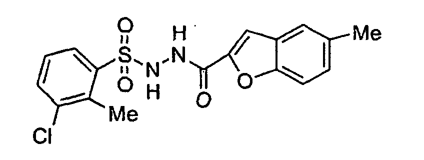

This was run in accordance with Example 77 except 3-chloro-2-methylphenyl sulfonyl chloride was used instead of benzene sulfonyl chloride. The desired product was purified by column chromatography eluting with ethyl acetate/hexanes (40.2% yield).
MS: 378.9 (M+1 for C₁₇H₁₅N₂O₄SCl); mp: 182.4-184.0°C. HPLC (C18 column, 1:1
MeCN/H₂O+0.1% TFA) 99.2%, RT = 11.05 min. Micro: (calc) (C₁₇H₁₅N₂O₄SCl) C: 53.90, H: 3.99, N: 7.39, S: 8.46, Cl: 9.36; (found) C: 54.13, H: 4.03, N: 7.27, S: 8.41, Cl: 9.31. ¹H NMR (DMSO-d₆) δ 2.36 (s, 3H), 2.71 (s, 3H), 7.25 (dd, 1H, J = 8.55 and 1.71 Hz), 7.29 (t, 1H, J = 8.06 Hz), 7.46-7.50 (m, 3H), 7.69 (d,1H, J = 8.06 Hz), 7.81 (dd, 1H, J = 8.06 and 0.98 Hz), 10.34 (s, 1H), and 10.85 (s, 1H).

### EXAMPLE 83

### 2-Benzofurancarboxylic acid, 5-methyl-, 2-[3-(bromophenyl)sulfonyl]hydrazide

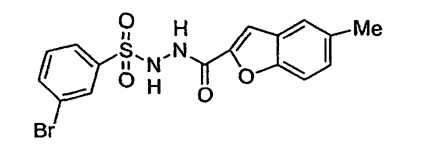

This was run in accordance with Example 77 except 3-bromophenyl sulfonyl chloride was used instead of benzene sulfonyl chloride. The desired product was purified by recrystallizing in ethyl acetate/hexanes (58.7% yield).
MS: 408.9 (M+1 for C₁₆H₁₃N₂O₄SBr); mp: 207.2-209.9°C. HPLC (C18 column, 1:1 MeCN/H₂O+0.1% TFA) 98.8%, RT = 8.77 min. Micro: (calc) (C₁₆H₁₃N₂O₄SBr) C: 46.96, H: 3.20, N: 6.84, S: 7.83, Br: 19.52; (found) C: 46.99, H: 3.05, N: 6.77, S: 8.30, Br: 20.24. ¹H NMR (DMSO-d₆) δ 2.36 (s, 3H), 7.25 (dd, 1H, J = 8.79 and 1.71 Hz), 7.44 (d, 1H, J = 7.58 Hz), 7.46-7.51 (m, 4H), 7.74 (dq, 1H, J = 7.57 and 1.71 Hz), 7.92 (dd, 1H, J = 7.81 and 1.71 Hz), 10.30 (s, 1H), and 10.92 (s, 1H).

### EXAMPLE 84

### 2-Benzofurancarboxylic acid, 5-methyl-, 2-[(3-chlorophenyl)sulfonyl]hydrazide

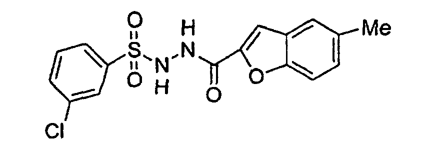

This was run in accordance with Example 77 except 3-chlorophenyl sulfonyl chloride was used instead of benzene sulfonyl chloride. The desired product was purified by chromatographing with ethyl acetate/hexanes (75.3% yield). MS: 365.0 (M+1 for C₁₆H₁₃N₂O₄SCl); mp: 186.1-186.3°C. HPLC (C18 column, 1:1 MeCN/H₂O+0.1% TFA) 98.7%, RT = 8.08 min. Micro: (calc) (C₁₆H₁₃N₂O₄SCl) C: 52.68, H: 3.59, N: 7.68, S: 8.79, Cl: 9.72; (found) C: 52.64, H: 3.60, N: 7.59, S: 8.71, Cl: 9.89. ¹H NMR (DMSO-d₆) δ 2.36 (s, 3H), 7.25 (dd, 1H, J = 8.54 and 1.22 Hz), 7.48-7.57 (m, 3H), 7.70-7.75 (m, 2H), 7.81 (s, 1H), 10.40 (s, 1H), and 10.96 (s, 1H).

### EXAMPLE 85

### 2-Benzofurancarboxylic acid, 5-methyl-, 2-[[2-(trifluoromethoxy)phenyl]sulfonyl]hydrazide

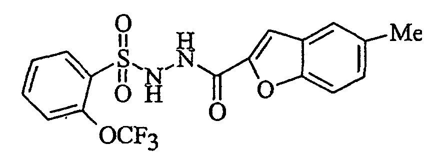

This was run in accordance with Example 77 except 2-trifluoromethoxy phenyl sulfonyl chloride was used instead of benzene sulfonyl chloride. The desired product was purified by chromatographing with ethyl acetate/hexanes (72.5% yield).
MS: 415.0 (M+1 for C₁₇H₁₇₃N₂O₅SF₃); mp: 226.7-227.0°C. HPLC (C18 column, 1:1 MeCN/H₂O+0.1% TFA) 98.8%, RT = 8.83 min. Micro: (calc) (C₁₇H₁₇₃N₂O₅SF₃) C: 49.28, H: 3.16, N: 6.76, S: 7.74, F: 13.75; (found) C: 49.18, H: 3.10, N: 6.67, S: 7.84, F: 14.00. ¹H NMR (DMSO-d₆) δ 2.36 (s, 3H), 7.25 (dd, 1H, J = 8.55 and 1.71 Hz), 7.44 (dd, 1H, J = 8.55 and 0.97 Hz), 7.47-7.51 (m, 4H), 7.74 (dt, 1H, J = 8.30 and 1.71 Hz), 7.92 (dd, 1H, J = 7.82 and 1.47 Hz), 10.30 (s, 1H), and 10.92 (s, 1H).

### EXAMPLE 86

### 2-Benzofurancarboxylic acid, 7-methyl-, 2-(phenylsulfonyl)hydrazide

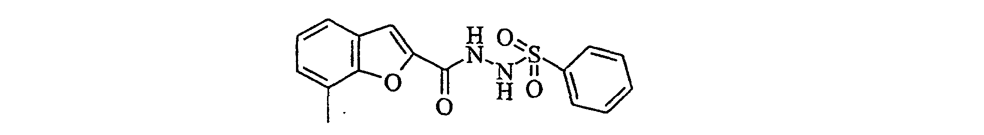

### Step 1. 7-Methylbenzofuran-2-carboxylic acid hydrazide

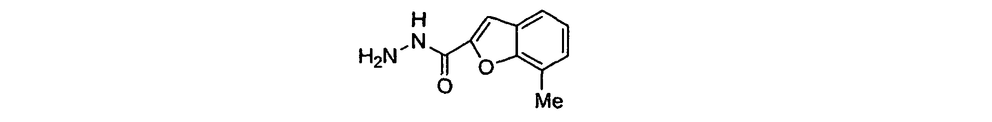

This was run in accordance with Example 1, Step 2 except 7-methyl-benzofuran-2-carboxylic acid that was prepared in accordance to Pappalardo, *Boll. Sci. Fac. Ind. Bologna,* 1952:168, was used instead of dibenzofuran-2-carboxylic acid (60% yield).
MS: 190.9 (M+1 for C₁₀H₁₀N₂O₂); mp: 171.6-173.4°C. HPLC (C18 column, 1:1 MeCN/H₂O+0.1% TFA) 98.2%, RT = 1.79 min. ¹H NMR (DMSO-d₆) δ 2.48, (s, 3H), 4.55 (s, 2H), 7.15-7.22 (m, 2H), 7.44 (s, 1H), 7.51 (dd, 1H, J = 6.84 and 0.98 Hz), and 9.98 (s, 1H).

### Step 2. 2-Benzofurancarboxylic acid, 7-methyl-, 2-(phenylsulfonyl)hydrazide

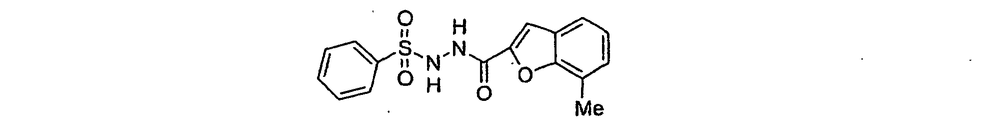

This was run in accordance with Example 35 except 7-methyl-benzofuran-2-carboxylic acid hydrazide was used instead of dibenzofuran-2-carboxylic acid hydrazide, and phenyl sulfonyl chloride was used instead of 3-fluorophenyl chloride.

The desired product was purified by recrystallizing in ethyl acetate/hexanes (71% yield).
MS: 331.0 (M+1 for C₁₆H₁₄N₂O₄S); mp: 217.5-218.0°C. HPLC (C18 column, 1:1 MeCN/H₂O+0.1% TFA) 97.8%, RT = 5.14 min. Micro: (calc) (C₁₆H₁₄N₂O₄S) C: 58.17, H: 4.27, N: 8.48, S: 9.71; (found) C: 57.87, H: 4.26, N: 8.37, S: 9.70. ¹H NMR (DMSO-d₆) δ 2.48 (s, 3H), 7.18 (t, 1H, J = 7.57 Hz), 7.24 (d, 1H, J = 7.09 Hz), 7.50-7.54 (m, 4H), 7.62 (t, 1H, J = 7.57 Hz), 7.82 (d, 2H, J = 7.33 Hz), 10.19 (d, 1H, J = 2.4 Hz), and 10.91 (d, 1H, J = 2.4 Hz).

### EXAMPLE 87

### 2-Benzofurancarboxylic acid, 4-methyl-, 2-(phenylsulfonyl)hydrazide

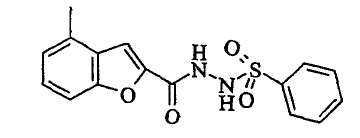

### Step 1. 4-Methylbenzofuran-2-carboxylic acid hydrazide

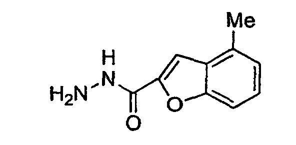

This was run in accordance with Example 1, Step 2 except 4-methyl-benzofuran-2-carboxylic acid that was prepared in accordance to Pappalardo, *Boll. Sci. Fac. Ind. Bologna,* 1952:168, was used instead of dibenzofuran-2-carboxylic acid (60% yield).
MS: 190.9 (M+1 for C₁₀H₁₀N₂O₂); mp: 175.4-177.0°C. HPLC (C18 column, 1:1 MeCN/H₂O+0.1% TFA) 95.4%, RT = 1.76 min. ¹H NMR (DMSO-d₆) δ 2.46 (s, 3H), 4.52 (d, 2H, J = 3.91 Hz), 7.07 (d, 1H, J = 7.33 Hz), 7.29 (t, 1H, J = 8.30 Hz), 7.39 (d, 1H, J = 8.30 Hz), 7.54 (d, 1H, J = 0.97 Hz), and 9.95 (s, 1H).

### Step 2. 2-Benzofurancarboxylic acid, 4-methyl-, 2-(phenylsulfonyl)hydrazide

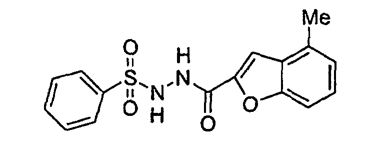

This was run in accordance with Example 35 except 4-methyl-benzofuran-2-carboxylic acid hydrazide was used instead of dibenzofuran-2-carboxylic acid hydrazide, and phenyl sulfonyl chloride was used instead of 3-fluorophenyl chloride (91% yield).
MS: 331.1 (M+1 for C₁₆H₁₄N₂O₄S); mp: 217.0-218.0°C. HPLC (C18 column, 1:1 MeCN/H₂O+0.1% TFA) 98.3%, RT = 4.80 min. Micro: (calc) (C₁₆H₁₄N₂O₄S) C: 58.17, H: 4.27, N: 8.48, S: 9.71; (found) C: 57.73, H: 4.22, N: 8.31, S: 9.71. ¹H NMR (DMSO-d₆) δ 2.46 (s, 3H), 7.10 (d, 1H, J = 7.32 Hz), 7.32 (t, 1H, J = 8.30 Hz), 7.39 (d, 1H, J = 8.30 Hz), 7.49-7.53 (m, 2H), 7.60 (dd, 1H, J = 5.62 and 1.22 Hz), 7.61 (s, 1H), 7.80 (d, 2H, J = 7.32 Hz), 10.16 (s, 1H), and 10.87 (s, 1H).

### EXAMPLE 88

### 2-Benzofurancarboxylic acid, 3-methyl-, 2-(phenylsulfonyl)hydrazide

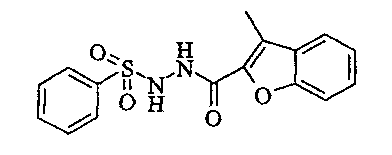

### Step 1. 3-Methylbenzofuran-2-carboxylic acid hydrazide

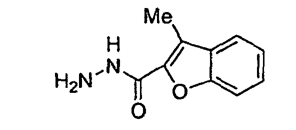

This was run in accordance with Example 1, Step 2 except 3-methyl-benzofuran-2-carboxylic acid was used instead of dibenzofuran-2-carboxylic acid (74% yield).
MS: 191.0 (M+1 for C₁₀H₁₀N₂O₂); mp: 136-137°C. HPLC (C18 column, 1:1 MeCN/H₂O+0.1% TFA) 95.3%, RT = 1.87 min. ¹H NMR (DMSO-d₆) δ 2.52 (s, 3H), 4.48 (s, 2H), 7.29 (dt, 1H, J = 7.82 and 0.74 Hz), 7.39-7.43 (m, 1H), 7.51 (d, 1H, J = 8.31 Hz), 7.68 (d, 1H, J = 7.82 Hz), and 9.79 (s, 1H).

### Step 2. 2-Benzofurancarboxylic acid, 3-methyl-, 2-(phenylsulfonyl)hydrazide

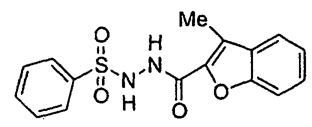

This was run in accordance with Example 35 except 3-methyl-benzofuran-2-carboxylic acid hydrazide was used instead of dibenzofuran-2-carboxylic acid hydrazide, and phenyl sulfonyl chloride was used instead of 3-fluorophenyl chloride (68.5% yield).
MS: 331.1 (M+1 for C₁₆H₁₄N₂O₄S); mp: 221.6-222.2°C. HPLC (C18 column, 1:1 MeCN/H₂O+0.1% TFA) 99.4%, RT = 5.50 min. Micro: (calc) (C₁₆H₁₄N₂O₄S) C: 58.17, H: 4.27, N: 8.48, S: 9.71; (found) C: 58.31, H: 4.22, N: 8.55, S: 9.73. ¹H NMR (DMSO-d₆) δ 2.35 (s, 3H), 7.31 (t, 1H, J = 8.06 Hz), 7.46 (t, 1H, J = 8.30 Hz), 7.50-7.60 (m, 3H), 7.61-7.63 (m, 1H), 7.69 (d, 1H, J = 7.81 Hz), 7.81 (d, 2H, J = 7.32 Hz), 10.07 (s, 1H), and 10.70 (s, 1H).

### EXAMPLE 89

### 2-Benzofurancarboxylic acid, 3,5-dimethyl-, 2-(phenylsulfonyl)hydrazide

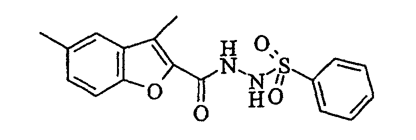

### Step 1. 3,5-di-Methylbenzofuran-2-carboxylic acid hydrazide

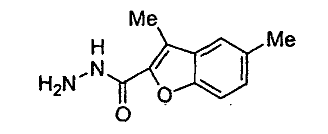

This was run in accordance with Example 1, Step 2 except 3,5-dimethyl-benzofuran-2-carboxylic acid, which was prepared in accordance to Dey, *J. Chem. Soc*., 1945:1646, was used instead of dibenzofuran-2-carboxylic acid (40% yield).
MS: 190.9 (M+1 for C₁₀H₁₀N₂O₂); mp: 142.7-145.1°C. HPLC (C18 column, 1:1 MeCN/H₂O+0.1% TFA) 95.1%, RT = 1.82 min. ¹H NMR (DMSO-d₆) δ 2.37 (s, 3H), 2.47 (s, 3H), 4.47 (s, 2H), 7.21 (dd, 1H, J = 8.54 and 1.46 Hz), 7.38 (d, 1H, J = 8.30 Hz), 7.45 (d, 1H, J = 0.73 Hz), and 9.74 (s, 1H).

### Step 2. 2-Benzofurancarboxylic acid, 3,5-dimethyl-, 2-(phenylsulfonyl)hydrazide

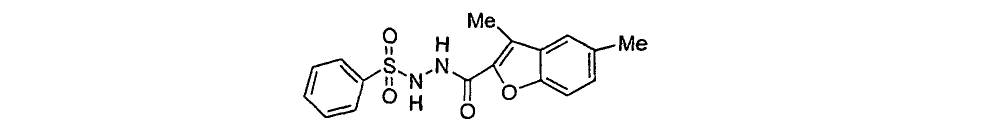

This was run in accordance with Example 35 except 3,5-dimethyl-benzofuran-2-carboxylic acid hydrazide was used instead of dibenzofuran-2-carboxylic acid hydrazide, and phenylsulfonyl chloride was used instead of 3-fluorophenyl chloride (47.3% yield).
MS: 345.1 (M+1 for C₁₇H₁₆N₂O₄S); mp: 225.9-227.1°C. HPLC (C18 column, 1:1 MeCN/H₂O+0.1% TFA) 99.0%, RT = 7.20 min. Micro: (calc) (C₁₇H₁₆N₂O₄S·0.6H₂O) C: 57.34, H: 4.87, N: 7.87, S: 9.00; (found) C: 57.46, H: 4.48, N: 7.90, S: 9.37. ¹H NMR (DMSO-d₆) δ 2.31 (s, 3H), 2.37 (s, 3H), 7.25 (dd, 1H, J = 1.47 and 8.80 Hz), 7.42 (d, 1H, J = 8.55 Hz), 7.45 (s, 1H), 7.51 (t, 2H, J = 7.82 Hz), 7.58-7.62 (m, 1H), 7.80 (d, 2H, J = 7.08 Hz), 10.4 (s, 1H), and 10.64 (s, 1H).

### EXAMPLE 90

### 2-Benzofurancarboxylic acid, 5-chloro-3-methyl, 2-(phenylsulfonyl)hydrazide

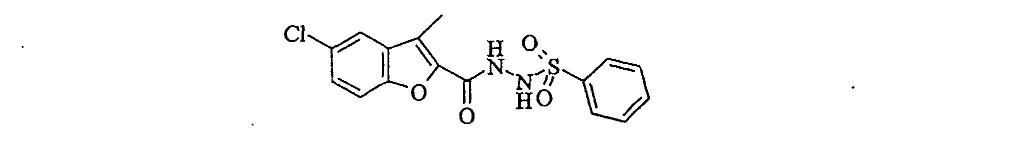

### Step 1. 5-Chloro-3-methyl-benzofuran-2-carboxylic acid hydrazide

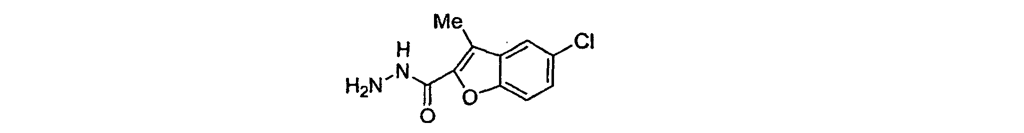

This was run in accordance with Example 1, Step 2 except 5-chloro-3-methyl-benzofuran-2-carboxylic acid, which was prepared in accordance to Suzuki et al., *Bull. Chem. Soc. Jpn*., 1983:2762-2767, was used instead of dibenzofuran-2-carboxylic acid (40% yield).
MS: 225.1 (M+1 for C₁₀H₉ClN₂O₂); mp: 214.3-214.7°C. HPLC (C18 column, 1:1 MeCN/H₂O+0.1% TFA) 94.4%, RT = 1. 98 min.

### Step 2. 2-Benzofurancarboxylic acid, 5-chloro-3-methyl, 2-(phenylsulfonyl)-hydrazide

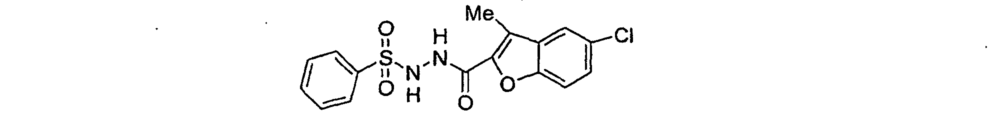

This was run in accordance with Example 35 except 5-chloro-3-methyl-benzofuran-2-carboxylic acid hydrazide was used instead of dibenzofuran-2-carboxylic acid hydrazide, and phenylsulfonyl chloride was used instead of 3-fluorophenyl chloride (43.2% yield).
MS: 365.0 (M+1 for C₁₆H₁₃N₂O₄SCl); mp: 246.8-247.8°C. HPLC (C18 column, 1:1 MeCN/H₂O+0.1% TFA) 98.6%, RT = 8.24 min. Micro: (calc) (C₁₆H₁₃N₂O₄SCl·0.5H₂O) C: 51.31, H: 3.77, N: 7.48, S: 8.56; (found) C: 51.46, H: 3.44, N: 8.16, S: 8.86; ¹H NMR (DMSO-d₆) δ 2.30 (s, 3H), 7.45 (dd, 1H, J = 2.20 and 8.80 Hz), 7.42 (d, 1H, J = 8.55 Hz), 7.51 (t, 2H, J = 7.32 Hz), 7.56-7.61 (m, 2H), 7.78-7.80 (m, 32H), 10.09 (d, 1H, J = 2.8 Hz), and 10.75 (d, 1H, J = 2.8 Hz).

### EXAMPLE 91

### 2-Benzofurancarboxylic acid, 5-amino-, 2-(phenylsulfonyl)hydrazide

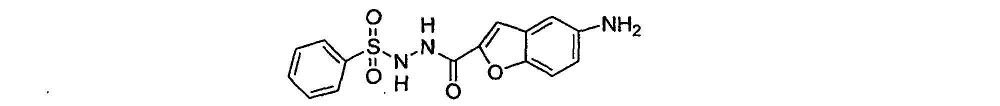

A solution of Example 34 (0.11 g, 0.305 mmol) in THF (20 mL) was treated with H₂ at 40 psi in the presence of Raney Ni (trace) in a Parr shaker. The catalyst was removed by filtration, the solvents removed, and the product was isolated by column chromatography eluting with ethyl acetate (50% yield).
MS: 332.0 (M+1 for C₁₅H₁₃N₃O₄S); mp: 206.9-207.7°C. HPLC (C18 column, 1:1 MeCN/H₂O+0.1% TFA) 99.46%, RT = 1.54 min. Micro: (calc) (C₁₅H₁₃N₃O₄S) C: 54.37, H: 3.95, N: 12.68, S: 9.68; (found) C: 54.18, H: 4.14,.N: 12.44, S: 9.56. ¹H NMR (DMSO-d₆) δ 4.99 (s, 2H), 6.71, (d, 2H, J = 2.20 Hz), 7.25 (d, 1H, J = 9.52 Hz), 7.30 (s, 1H), 7.51 (t, 2H, J = 7.82 Hz), 7.61 (t, 1H, J = 7.33 Hz), 7.79 (d, 2H, J = 7.57 Hz), 10.10 (s, 1H), and 10.73 (s, 1H).

### EXAMPLE 92

### 2-Dibenzofurancarboxylic acid, 2-[(2-fluorophenyl)sulfonyl]hydrazide

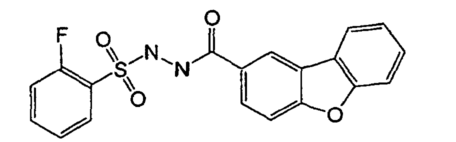

Synthesis of 2-Dibenzofurancarboxylic acid 2-[(2-fluorophenyl)sulfonyl]-hydrazide: A solution of dibenzofuran-2-carboxylic acid hydrazide (0.3 M) in pyridine (0.513 mL) was treated with a solution of the 2-fluorobenzenesulfonyl chloride (1.0 M) in CH₂Cl₂ (0.225 mL). The reaction was placed in a Bohdan Miniblock apparatus and shaken at 45°C for 16 hours. The reaction was cooled to room temperature, treated with polymer-supported polyamine quench resin (Aldrich, 100 mg), and shaken for 16 hours. The solution was filtered and concentrated. The residue was purified by preparative HPLC on a Phenomenex Develofil 28 x 100 mm C18 column eluting with a gradient of 10% to 100% CH₃CN/H₂O + 3% n-propanol over 6.5 minutes.
MS: 385.4 (M+1 for C₁₉H₁₃N₂O₄SF); HPLC (Waters Alliance 2790 column, solvent gradient of 60% to 100% CH₃CN/H₂O + 0.1 % formic acid over 1 minute with 3 minute run time) RT = 3.59 min., 100%.

### EXAMPLE 93

### 2-Dibenzofurancarboxylic acid, 2-(2,6-difluorophenylsulfonyl)hydrazide

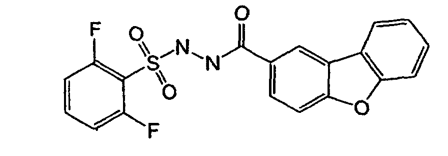

Example 93 was synthesized in accordance with the methods of Example 92 except that 2,6-difluorobenzenesulfonyl chloride was used instead of 2-fluorobenzenesulfonyl chloride.
MS: 403.4 (M+1 for C₁₉H₁₂N₂O₄S₁F₂); HPLC (Waters Alliance 2790 column, solvent gradient of 60% to 100% CH₃CN/H₂O + 0.1% formic acid over I minute with 3 minute run time) RT = 3.78 min., 100%.

### EXAMPLE 94

### 2-Dibenzofurancarboxylic acid, 2-[(2-bromophenyl)sulfonyl]hydrazide

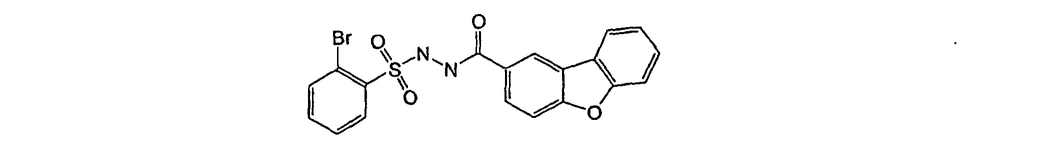

Example 94 was synthesized in accordance with the methods of Example 92 except that 2-bromobenzenesulfonyl chloride was used instead of 2-fluorobenzenesulfonyl chloride.
MS: 446.3 (M+1 for C₁₉H₁₃N₂O₄S₁Br); HPLC (Waters Alliance 2790 column, solvent gradient of 60% to 100% CH₃CN/H₂O + 0.1% formic acid over 1 minute with 3 minute run time) RT = 2.22 min., 76.0%.

### EXAMPLE 95

### 2-Dibenzofurancarboxylic acid, 2-[(3-bromophenyl)sulfonyl]hydrazide

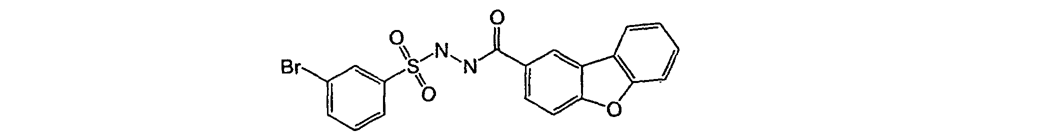

Example 95 was synthesized in accordance with the methods of Example 92 except that 3-bromobenzenesulfonyl chloride was used instead of 2-fluorobenzenesulfonyl chloride.
MS: 445.3 (M + 1 for C₁₉H₁₃N₂O₄S₁Br); HPLC (Waters Alliance 2790 column, solvent gradient of 60% to 100% CH₃CN/H₂O + 0.1% formic acid over 1 minute with 3 minute run time) RT = 3.59 min., 100%.

### EXAMPLE 96

### 2-Dibenzofurancarboxylic acid, 2-[(4-methyl-3-nitrophenyl)sulfonyl]hydrazide

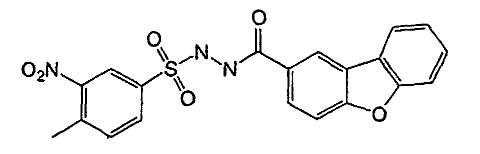

Example 96 was synthesized in accordance with the methods of Example 92 except that 4-methyl-3-nitrobenzenesulfonyl chloride was used instead of 2-fluorobenzenesulfonyl chloride.
MS: 426.4 (M+1 for C₂₀H₁₅N₃O₆S₁), HPLC (Waters Alliance 2790 column, solvent gradient of 60% to 100% CH₃CN/H₂O + 0.1% formic acid over 1 minute with 3 minute run time) RT = 2.21 min., 92.7%.

### EXAMPLE 97

### 2-Dibenzofizrancarboxylic acid, 2-[2-chloro-5-(trifluoromethyl)phenyl]sulfonyl)-hydrazide

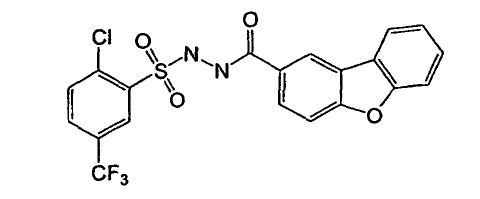

Synthesis of dibenzofuran-2-carboxylic acid 2-(2-chloro-5-trifluoromethylphenylsulfonyl)hydrazide: Example 97 was synthesized in accordance with the methods of Example 92 except that 2-chlorotrifluoromethylbenzenesulfonyl chloride was used instead of 2-fluorobenzenesulfonyl chloride.
MS: 469.8 (M+1 for C₂₀H₁₂N₂O₄S₁Cl F₃); HPLC (Waters Alliance 2790 column, solvent gradient of 60% to 100% CH₃CN/H₂O + 0.1% formic acid over 1 minute with a 3 minute run time) RT = 2.33 min., 87.7%.

### EXAMPLE 98

### 2-Dibenzofurancarboxylic acid, 2-[(2-trifluoromethoxyphenyl)sulfonyl]hydrazide

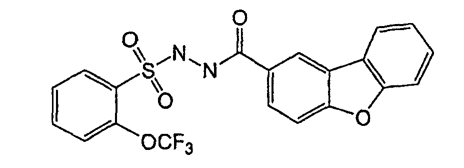

Synthesis of 2-dibenzofurancarboxylic acid 2-[(2-trifluoromethoxyphenyl)sulfonyl]hydrazide: Example 98 was synthesized in accordance with the methods of Example 92 except that 2-trifluoromethoxylbenzenesulfonyl chloride was used instead of 2-fluorobenzenesulfonyl chloride.
MS: 451.4 (M +1 for C₂₀H₁₃F₃N₂O₅S); HPLC (Waters Alliance 2790 column, solvent gradient of 60% to 100% CH₃CN/H₂O + 0.1% formic acid over 1 minute with 3 minute run time) RT = 2.28 min., 94.7%.

### EXAMPLE 99

### 2-Dibenzofurancarboxylic acid, 2-[(4-bromo-2-trifluoromethoxyphenyl)sulfonyl]-hydrazide

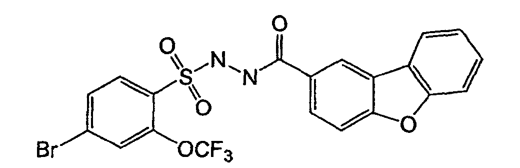

Synthesis of 2-dibenzofurancarboxylic acid 2-[(4-bromo-2-trifluoromethoxylphenyl)sulfony]hydrazide: Example 99 was synthesized in accordance with the methods of Example 92 except that 4-bromo-2-trifluoromethoxylbenzenesulfonyl chloride was used instead of 2-fluorobenzenesulfonyl chloride.
MS: 530.3 (M+1 for C₂₀H₁₂BrF₃N₂O₅S); HPLC (Waters Alliance 2790 column, solvent gradient of 60% to 100% CH₃CN/H₂O + 0.1% formic acid over 1 minute with 3 minute run time) RT = 2.42 min., 95.1 %.

### EXAMPLE 100

### 2-Dibenzofurancarboxylic acid, 2-[(2-nitrophenyl)sulfonyl]hydrazide

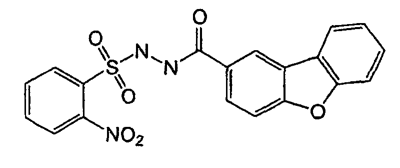

Synthesis of 2-dibenzofurancarboxylic acid 2-[(2-nitrophenyl)-sulfonyl]hydrazide: Example 100 was synthesized in accordance with the methods of Example 92 except that 2-nitrobenzenesulfonyl chloride was used instead of 2-fluorobenzenesulfonyl chloride.
MS: 412.4 (M+1 for C₁₉H₁₃N₃O₆S); HPLC (Waters Alliance 2790 column, solvent gradient of 60% to 100% CH₃CN/H₂O + 0.1% formic acid over 1 minute with 3 minute run time) RT = 3.03 min., 88.3%.

### EXAMPLE 101

### 2-Dibenzofurancarhoxylic acid, 2-[(4-carboxyphenyl)sulfonyl]hydrazide

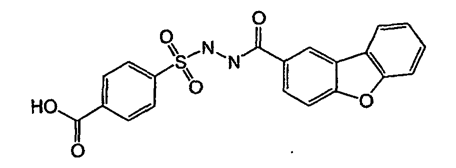

Synthesis of 2-dibenzofurancarboxylic acid 2-[(4-carboxyphenyl)-sulfonyl]hydrazide: Example 101 was synthesized in accordance with the methods of Example 92 except that 4-carboxybenzenesulfonyl chloride was used instead of 2-fluorobenzenesulfonyl chloride.
MS: 411.4 (M+1 for C₂₀H₁₄N₃O₆S); HPLC (Waters Alliance 2790 column, solvent gradient of 60% to 100% CH₃CN/H₂O + 0.1% formic acid over I minute with 3 minute run time) RT = 1.85 min., 89.2%.

### EXAMPLE 102

### 2-Benzofurancarboxylic acid, 7-methoxy-, 2-[(3-chlorophenyl)sulfonyl]hydrazide

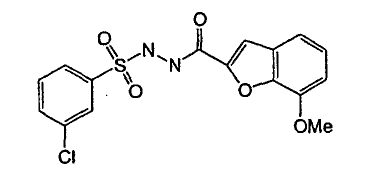

Synthesis of 2-benzofurancarboxylic acid, 7-methoxy-, 2-[(3-chlorophenyl)sulfonyl]hydrazide: A solution of 2-(7-methoxyl)- benzofurancarboxylic acid hydrazide (0.3 M) in pyridine (0.513 mL) was treated with a solution of the 3-chlorobenzenesulfonyl chloride (1.0 M) in CH₂Cl₂ (0.225 mL). The reaction was placed in a Bohdan Miniblock apparatus and shaken at 45°C for 16 hours. The reaction was cooled to room temperature, treated with polymer-supported polyamine quench resin (Aldrich, 100 mg), and shaken for 16 hours. The solution was filtered and concentrated. The residue was purified by preparative HPLC on a Phenomenex Develofil 28 x 100 mm C18 column eluting with a gradient of 10% to 100% CH₃CN /H₂O+3% n-propanol over 6.5 minutes.
MS: 380.8 (M+1 for C₁₆H₁₃ClN₂O₅S); HPLC (Waters Alliance 2790 column, solvent gradient of 60% to 100% CH₃CN/H₂O + 0.1% formic acid over 1 minute with 3 minute run time) RT = 3.67 min., 98.9%.

### EXAMPLE 103

### 2-Benzofurancarboxylic acid, 7-methoxy-, 2-[(3-fluorophenyl)sulfonyl]hydrazide

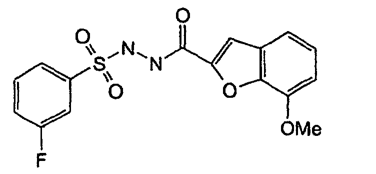

Synthesis of 2-benzofurancarboxylic acid, 7-methoxy-, 2-[(3-chloro-4-fluorophenyl)sulfonyl]hydrazide: Example 103 was synthesized in accordance with the methods of Example 102 except that 3-fluorobenzenesulfonyl chloride was used instead of 3-chlorobenzenesulfonyl chloride.
MS: 364.4 (M+1 for C₁₆H₁₃N₂O₅SF); HPLC (Waters Alliance 2790 column, solvent gradient of 60% to 100% CH₃CN/H₂O + 0.1% formic acid over 1 minute with 3 minute run time) RT = 3.47 min., 100%.

### EXAMPLE 104

### 2-Benzofurancarboxylic acid, 7-methoxy-, 2-[(2,3-dichlorophenyl)sulfonyl]-hydrazide

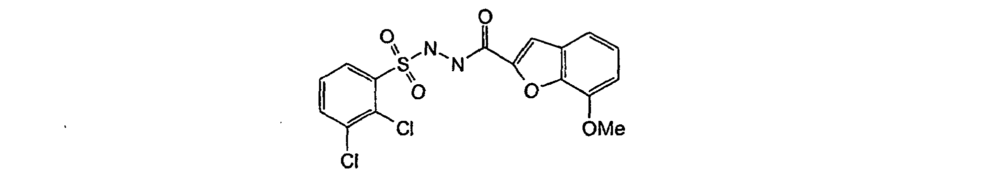

Synthesis of 2-benzofurancarboxylic acid, 7-methoxy-, 2-[(2,3-dichlorophenyl)-sulfonyl]hydrazide. Example 104 was synthesized in accordance with the methods of Example 102 except that 2,3-dichlorobenzenesulfonyl chloride was used instead of 3-chlorobenzenesulfonyl chloride.
MS: 415.3 (M+1 for C₁₆H₁₂N₂O₅SCl₂); HPLC (Waters Alliance 2790 column, solvent gradient of 60% to 100% CH₃CN/H₂O + 0.1% formic acid over 1 minute with 3 minute run time) RT = 3.79 min., 92.7%.

### EXAMPLE 105

### 2-Benzofurancarboxylic acid, 7-methoxy-, 2-[(3-chloro-4-fluorophenyl)sulfonyl]hydrazide

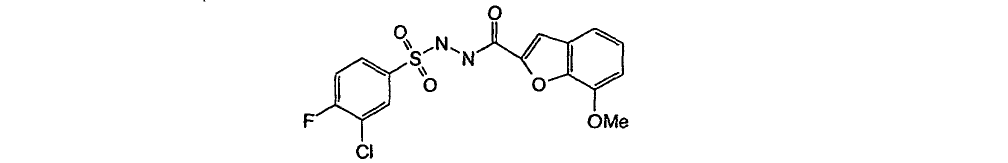

Synthesis of 2-benzofurancarboxylic acid, 7-methoxy-, 2-[(3-chloro-4-fluorophenyl)sulfonyl]hydrazide: Example 105 was synthesized in accordance with the methods of Example 102 except that 3-chloro-4-fluorobenzenesulfonyl chloride was used instead of 3-chlorobenzenesulfonyl chloride.
MS: 398.8 (M+1 for C₁₆H₁₂N₂O₅SClF); HPLC (Waters Alliance 2790 column, solvent gradient of 60% to 100% CH₃CN/H₂O + 0.1% formic acid over 1 minute with 3 minute run time) RT = 3.75 min., 100%.

### EXAMPLE 106

### 2-Benzofurancarboxylic acid, 7-methoxy-, 2-[(5-chloro-2-methoxyphenyl)-sulfonyl]hydrazide

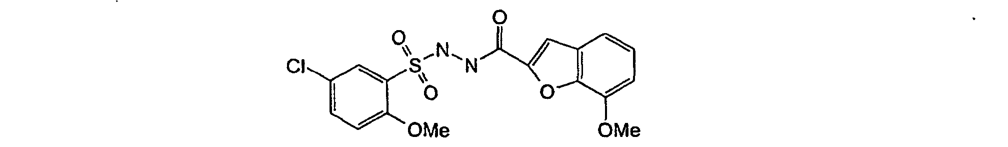

Synthesis of 2-benzofurancarboxylic acid, 7-methoxy-, 2-[(5-chloro-2methoxyphenyl)sulfonyl]hydrazide: Example 106 was synthesized in accordance with the methods of Example 102 except that 5-chloro-2-methoxylbenzenesulfonyl chloride was used instead of 3-chlorobenzenesulfonyl chloride.
MS: 410.8 (M+1 for C₁₇H₁₅N₂O₆SCl); HPLC (Waters Alliance 2790 column, solvent gradient of 60% to 100% CH₃CN/H₂O + 0.1% formic acid over 1 minute with 3 minute run time) RT = 3.69 min., 97.3%.

### EXAMPLE 107

### 2-Benzofurancarboxylic acid, 7-methoxy-, 2-[(5-bromo-2-methoxyphenyl)sulfonyl]hydrazide

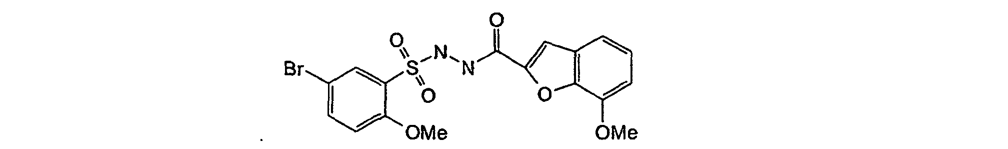

Synthesis of2-benzofurancarboxylic acid, 7-methoxy-, 2-[(5-bromo-2-methoxyphenyl)sulfonyl]hydrazide: Example 107 was synthesized in accordance with the methods of Example 102 except that 5-bromo-2-methoxylbenzenesulfonyl chloride was used instead of 3-chlorobenzenesulfonyl chloride.
MS: 455.3 (M+1 for C₁₇H₁₅N₂O₆SBr); HPLC (Waters Alliance 2790 column, solvent gradient of 60% to 100% CH₃CN/H₂O + 0.1% formic acid over 1 minute with 3 minute run time) RT = 3.74 min., 97.8%.

### EXAMPLE 108

### 2-Benzofurancarboxylic acid, 7-methoxy-, 2-[(4-ethylphenyl)sulfonyl]hydrazide

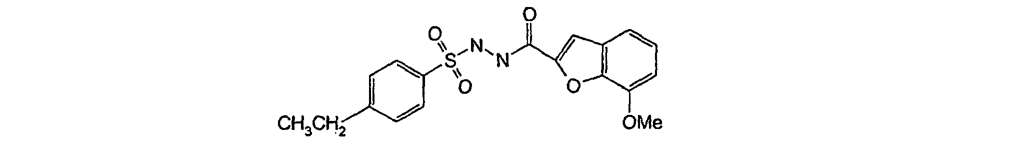

Synthesis of 2-benzofurancarboxylic acid, 7-methoxy-, 2-[(4-ethylphenyl)sulfonyl]hydrazide: Example 108 was synthesized in accordance with the methods of Example 102 except that 4-ethylbenzenesulfonyl chloride was used instead of 3-chlorobenzenesulfonyl chloride.
MS: 374.4 (M+1 for C₁₈H₁₈N₂O₅S); HPLC (Waters Alliance 2790 column, solvent gradient of 60% to 100% CH₃CN/H₂O + 0.1% formic acid over 1 minute with 3 minute run time) RT = 3.75 min., 86.1%.

### EXAMPLE 109

### 2-Benzofurancarboxylic acid, 7-methoxy-, 2-[(3-methoxyphenyl)sulfonyl]-hydrazide

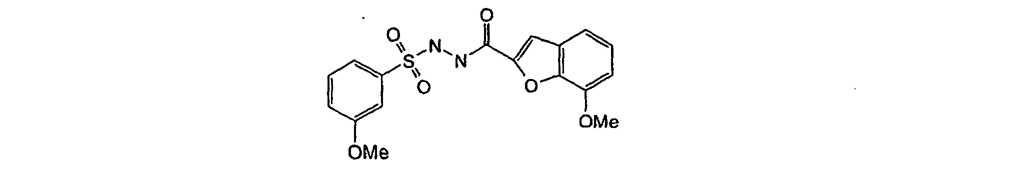

Synthesis of 2-benzofurancarboxylic acid, 7-methoxy-, 2-[(3-methoxyphenyl)sulfonyl]hydrazide: Example 109 was synthesized in accordance with the methods of Example 102 except that 3-methoxylbenzenesulfonyl chloride was used instead of 3-chlorobenzenesulfonyl chloride.
MS: 376.4 (M+1 for C₁₇H₁₆N₂O₆S); HPLC (Waters Alliance 2790 column, solvent gradient of 60% to 100% CH₃CN/H₂O + 0.1% formic acid over 1 minute with 3 minute run time) RT = 3.45 min., 100%.

### EXAMPLE 110

### 2-Dibenzofurancarboxylic acid, 2-[(2,3-dichlorophenyl)sulfonyl]hydrazide

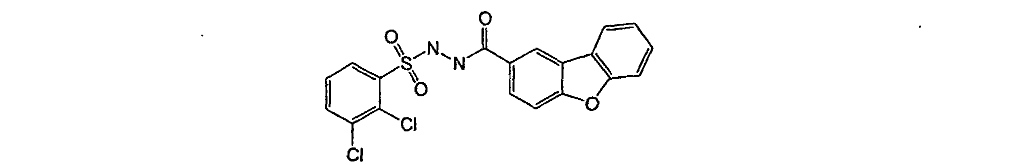

Synthesis of 2-dibenzofurancarboxylic acid, 2-[(2,3-dichlorophenyl)-sulfonyl]hydrazide: Example 110 was synthesized in accordance with the methods of Example 92 except that 2,3-dichlorobenzenesulfonyl chloride was used instead of 2-fluorobenzenesulfonyl chloride.
MS: 436.3 (M+1 for C₁₉H₁₂N₂O₄SCl₂); HPLC (Waters Alliance 2790 column, solvent gradient of 60% to 100% CH₃CN/H₂O + 0.1% formic acid over 1 minute with 3 minute run time) RT = 4.18 min., 94.5%.

### EXAMPLE 111

### 2-Naphthalenecarboxylic acid, 2-[(3-methylphenyl)sulfonyl]hydrazide

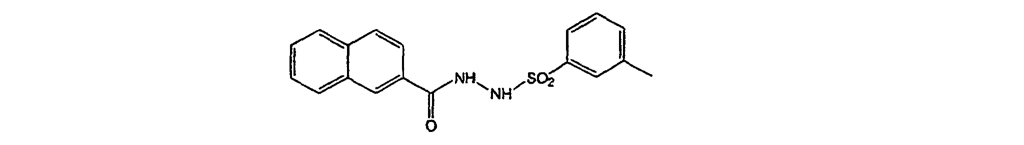

Synthesis of 2-naphthalenecarboxylic acid, 2-[(3-methylphenyl)sulfonyl]-hydrazide: A solution of 2-naphthalenecarboxylic acid, hydrazide (0.3 M) in pyridine (0.513 mL) was treated with a solution of the 3-methylbenzenesulfonyl chloride (1.0 M) in CH₂Cl₂ (0.225 mL). The reaction was placed in a Bohdan Miniblock apparatus and shaken at 45°C for 16 hours. The reaction was cooled to room temperature, treated with polymer-supported polyamine quench resin (Aldrich, 100 mg), and shaken for 16 hours. The solution was filtered and concentrated. The residue was purified by preparative HPLC on a Phenomenex Develofil 28 x 100 mm C18 column eluting with a gradient of 10% to 100% CH₃CN/H₂O+3% n-propanol over 6.5 minutes.
MS: 341.4 (M+1 for C₁₈H₁₆N₂O₃S); HPLC (Waters Alliance 2790 column, solvent gradient of 60% to 100% CH₃CN/H₂O + 0.1% formic acid over 1 minute with 3 minute run time) RT = 2.18 min., 72.0%.

### EXAMPLE 112

### 2-Naphthalenecarboxylic acid, 2-[(4-methylphenyl)sulfonyl]hydrazide

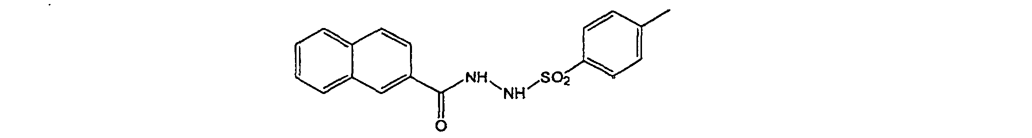

Synthesis of 2-naphthalenecarboxylic acid, 2-[(4-methylphenyl)-sulfonyl]hydrazide: Example 112 was synthesized in accordance with the methods of Example 111 except that 4-methylbenzenesulfonyl chloride was used instead of 3-methylbenzenesulfonyl chloride.
MS: 341.4 (M+1 for C₁₈H₁₆N₂O₃S); HPLC (Waters Alliance 2790 column, solvent gradient of 60% to 100% CH₃CN/H₂O + 0.1% formic acid over 1 minute with 3 minute run time) RT = 1.59 min., 100%.

### EXAMPLE 113

### 2-Naphthalenecarboxylic acid, 2-[[(2-(trifluoromethyl)phenyl]sulfonyl]hydrazide

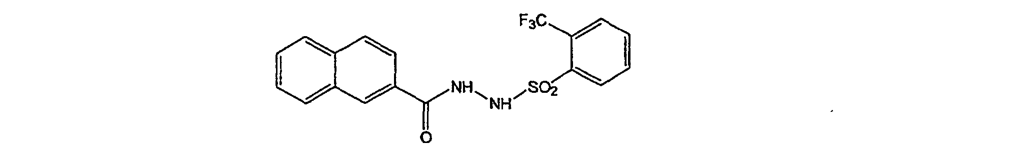

Synthesis of 2-naphthalenecarboxylic acid, 2-[[(2-(trifluoromethyl)-phenyl]sulfonyl]hydrazide: Example 113 was synthesized in accordance with the methods of Example 111 except that 2-trifluoromethylphenylsulfonyl chloride was used instead of 3-methylbenzenesulfonyl chloride.
MS: 395.4 (M+1 for C₁₈H₁₃N₂O₃SF₃); HPLC (Waters Alliance 2790 column, solvent gradient of 60% to 100% CH₃CN/H₂O + 0.1% formic acid over 1 minute with 3 minute run time) RT = 2.16 min., 84.3%.

### EXAMPLE 114

### 2-Naphthalenecarboxylic acid, 2-[[(4-(trifluoromethyl)phenyl]sulfonyl]hydrazide

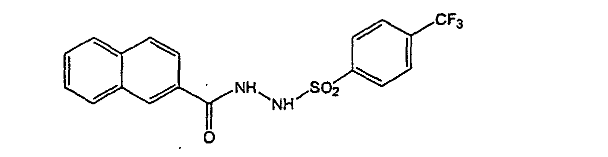

Synthesis of 2-naphthalenecarboxylic acid, 2-[[4-(trifluoromethyl)-phenyl]sulfonyl]hydrazide: Example 114 was synthesized in accordance with the methods of Example 111 except that 4-trifluoromethylphenylsulfonyl chloride was used instead of 3-methylbenzenesulfonyl chloride.
MS: 395.4 (M+1 for C₁₈H₁₃N₂O₃SF₃); HPLC (Waters Alliance 2790 column, solvent gradient of 60% to 100% CH₃CN/H₂O + 0.1% formic acid over 1 minute with 3 minute run time) RT = 2.13 min., 97.0%.

### EXAMPLE 115

### 2-Naphthalenecarboxylic acid, 2-[(3-chloro-4-fluorophenyl)sulfonyl]hydrazide

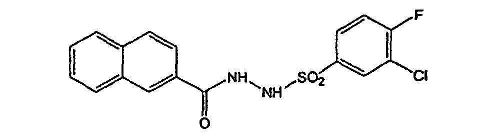

Synthesis of 2-naphthalenecarboxylic acid, 2-[(3-chloro-4-fluorophenyl)-sulfonyl]hydrazide: Example 115 was synthesized in accordance with the methods of Example 111 except that 3-chloro-4-fluorophenylsulfonyl chloride was used instead of 3-methylbenzenesulfonyl chloride.
MS: 379.9 (M+1 for C₁₇H₁₂N₂O₃SClF); HPLC (Waters Alliance 2790 column, solvent gradient of 60% to 100% CH₃CN/H₂O + 0.1 % formic acid over 1 minute with 3 minute run time) RT = 3.90 min., 97.8%.

### EXAMPLE 116

### 2-Naphthalenecarboxylic acid, 2-[(3-methoxyphenyl)sulfonyl]hydrazide

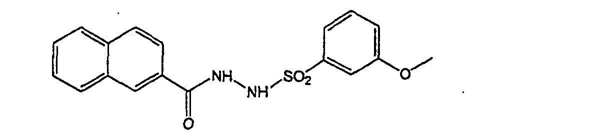

Synthesis of 2-naphthalenecarboxylic acid, 2-[(3-methoxyphenyl)-sulfonyl]hydrazide: Example 116 was synthesized in accordance with the methods of Example 111 except that 3-methoxylphenylsulfonyl chloride was used instead of 3-methylbenzenesulfonyl chloride.
MS: 357.4 (M+1 for C₁₈H₁₆N₂O₄S); HPLC (Waters Alliance 2790 column, solvent gradient of 60% to 100% CH₃CN/H₂O + 0.1% formic acid over 1 minute with 3 minute run time) RT = 3.60 min., 99.9%.

### EXAMPLE 117

### 2-Naphthalenecarboxylic acid, 2-[(3-chlorophenyl)sulfonyl]hydrazide

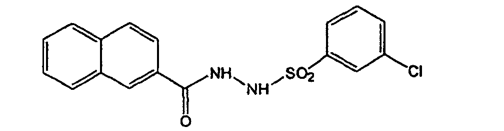

Synthesis of 2-naphthalenecarboxylic acid, 2-[(3-chlorophenyl)-sulfonyl]hydrazide: Example 117 was synthesized in accordance with the methods of Example 111 except that 3-chlorophenylsulfonyl chloride was used instead of 3-methylbenzenesulfonyl chloride.
MS: 361.8 (M+1 for C₁₇H₁₃N₂O₃SCl); HPLC (Waters Alliance 2790 column, solvent gradient of 60% to 100% CH₃CN/H₂O + 0.1% formic acid over 1 minute with 3 minute run time) RT = 3.69 min., 100%.

### EXAMPLE 118

### 2-Naphthalenecarboxylic acid, 2-[(2-chlorophenyl)sulfonyl]hydrazide

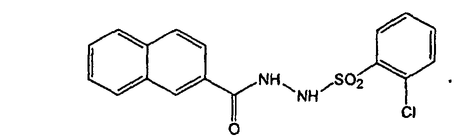

Synthesis of 2-naphthalenecarboxylic acid, 2-[(2-chlorophenyl)sulfonyl]-hydrazide: Example 118 was synthesized in accordance with the methods of Example 111 except that 2-chlorophenylsulfonyl chloride was used instead of 3-methylbenzenesulfonyl chloride.
MS: 361.8 (M+1 for C₁₇H₁₃N₂O₃SCl); HPLC (Waters Alliance 2790 column, solvent gradient of 60% to 100% CH₃CN/H₂O + 0.1% formic acid over 1 minute with 3 minute run time) RT = 3.67 min., 99.9%.

### EXAMPLE 119

### 2-Naphthalenecarboxylic acid, 2-[(3-fluorophenyl)sulfonyl]hydrazide

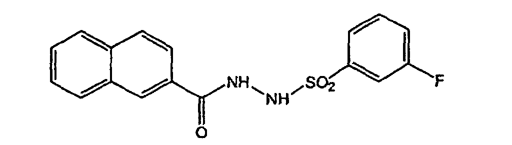

Synthesis of 2-naphthalenecarboxylic acid, 2-[(3-fluorophenyl)sulfonyl]-hydrazide: Example 119 was synthesized in accordance with the methods of Example 111 except that 3-fluorophenylsulfonyl chloride was used instead of 3-methylbenzenesulfonyl chloride.
MS: 345.4 (M+1 for C₁₇H₁₃N₂O₃SF); HPLC (Waters Alliance 2790 column, solvent gradient of 60% to 100% CH₃CN/H₂O + 0.1% formic acid over 1 minute with 3 minute run time) RT = 3.61 min., 99.9%.

### EXAMPLE 120

### 2-Naphthalenecarboxylic acid, 2-[(2,3-dichlorophenyl)sulfonyl]hydrazide

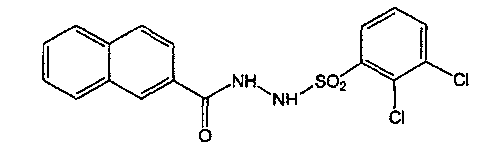

Synthesis of 2-naphthalenecarboxylic acid, 2-[(2,3-dichlorophenyl)-sulfonyl]hydrazide: Example 120 was synthesized in accordance with the methods of Example 111 except that 2,3-dichlorophenylsulfonyl chloride was used instead of 3-methylbenzenesulfonyl chloride.
MS: 396.3 (M+1 for C₁₇H₁₂N₂O₃SCl₂); HPLC (Waters Alliance 2790 column, solvent gradient of 60% to 100% CH₃CN/H₂O + 0.1% formic acid over 1 minute with 3 minute run time) RT = 3.95 min., 99.4%.

### EXAMPLE 121

### 2-Naphthalenecarboxylic acid, 2-[(4-ethylphenyl)sulfonyl]hydrazide

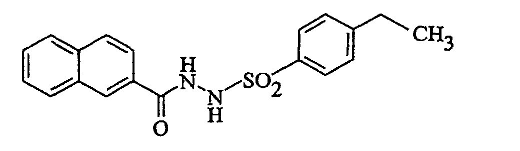

Synthesis of 2-naphthalenecarboxylic acid, 2-[(4-ethylphenyl)sulfonyl]hydrazide: Example 121 was synthesized in accordance with the methods of Example 111 except that 4-ethanylsulfonyl chloride was used instead of 3-methylbenzenesulfonyl chloride.
MS: 355.4 (M+1 for C₁₉H₁₈N₂O₃S); HPLC (Waters Alliance 2790 column, solvent gradient of 60% to 100% CH₃CN/H₂O + 0.1% formic acid over 1 minute with 3 minute run time) RT = 3.90 min., 100%.

### EXAMPLE 122

### 3-Quinolinecarboxylic acid, 2-[(4-pentylphenyl)sulfonyl)hydrazide

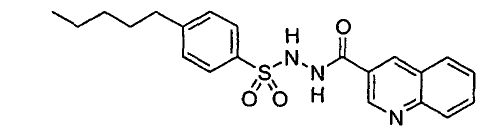

3-Quinolinecarboxylic acid hydrazide (1.0 g, 5.34 mmol) was dissolved in THF (60 mL). N,N-diisopropylethylamine (3.72 mL, 21.36 mmol) was added, and the reaction was cooled to 0°C. 4-Pentylphenylsulfonyl chloride (0.67 mL, 5.34 mmol) was added followed by DMAP (5 mg, 0.04 mmol). The reaction was allowed to warm to room temperature. After 3.5 hours of stirring, the red solution was washed with saturated sodium bicarbonate and brine and dried over Na₂SO₄. The reaction was then concentrated down and recrystallized from dichloromethane. The desired product was isolated (0.155 g, 8.9%).
MS: 398.5 (M+1 for C₁₆H₁₃N₃O₃S); HPLC (Waters Alliance 2790 column, solvent gradient of 60% to 100% CH₃CN/H₂O + 0.1% formic acid over 1 minute with 3 minute run time) RT = 2.17 min., 100%.

### EXAMPLE 123

### 3-Quinolinecarboxylic acid, 2-[[2-(trifluoromethyl)phenyl]sulfonyl]hydrazide

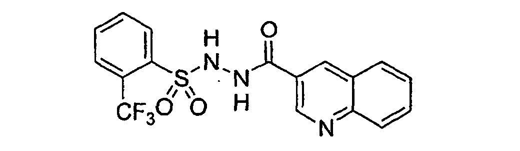

Synthesis of 3-quinolinecarboxylic acid, 2-[[2-(trifluoromethyl)phenyl]-sulfonyl]hydrazide: Example 123 was synthesized in accordance with the methods of Example 122 except that 2-trifluoromethylphenylsulfonyl chloride was used instead of 4-pentylphenylsulfonyl chloride.
MS: 396.4 (M+1 for C₁₇H₁₂N₃O₃SF₃); HPLC (Waters Alliance 2790 column, solvent gradient of 60% to 100% CH₃CN/H₂O + 0.1 % formic acid over 1 minute with 3 minute run time) RT = 1.93 min., 100%.

### EXAMPLE 124

### 3-Quinolinecarboxylic acid, 2-[[2,5-bis(2,2,2-trifluoroethoxyl)phenyl]sulfonyl]hydrazide

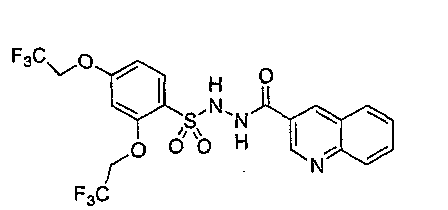

Synthesis of 3-quinolinecarboxylic acid, [[2,5-bis(2,2,2-trifluoroethoxyl)phenyl]sulfonyl]hydrazide: Example 124 was synthesized in accordance with the methods of Example 122 except that [2,5-bis(2,2,2-trifluoroethoxyl)phenyl]sulfonyl chloride was used instead of 4-pentylphenylsulfonyl chloride.
MS: 524.4 (M+1 for C₁₇H₁₂N₃O₃SF₃); HPLC (Waters Alliance 2790 column, solvent gradient of 60% to 100% CH₃CN/H₂O + 0.1% formic acid over 1 minute with 3 minute run time) RT = 2.07 min., 100%.

### EXAMPLE 125

### 3-Quinolinecarboxylic acid, 2-[(3-methylphenyl)sulfonyl]hydrazide

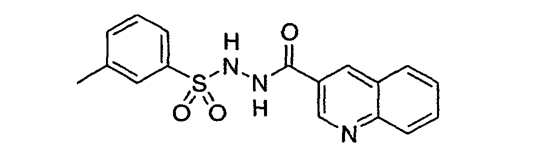

Synthesis of 3-quinolinecarboxylic acid, [(3-methylphenyl)sulfonyl]-hydrazide: Example 125 was synthesized in accordance with the methods of Example 122 except that (3-methylphenylsulfonyl chloride was used instead of 4-pentylphenylsulfonyl chloride.
MS: 342.4 (M+1 for C₁₇H₁₂N₃O₃SF₃); HPLC (Waters Alliance 2790 column, solvent gradient of 60% to 100% CH₃CN/H₂O + 0.1% formic acid over 1 minute with 3 minute run time) RT = 1.83 min., 100%.

### EXAMPLE 126

### 3-Quinolinecarboxylic acid, 2-[[2-(trifluoromethoxy)phenyl]sulfonyl]hydrazide

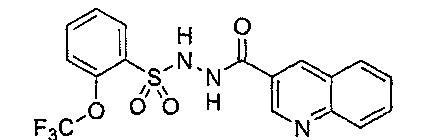

Synthesis of 3-quinolinecarboxylic acid, 2-[[2-(trifluoromethoxy)phenyl]-sulfonyl]hydrazide: Example 126 was synthesized in accordance with the methods of Example 122 except that 2-(2-trifluoromethoxy)-phenylsulfonyl chloride was used instead of 4-pentylphenylsulfonyl chloride.
MS: 412.4 (M+1 for C₁₇H₁₂N₃O₃SF₃); HPLC (Waters Alliance 2790 column, solvent gradient of 60% to 100% CH₃CN/H₂O + 0.1% formic acid over 1 minute with 3 minute run time) RT = 1.94 min., 99.5%.

### EXAMPLE 127

### 3-Quinolinecarboxylic acid, 2-[(4-methylphenyl)sulfonyl]hydrazide

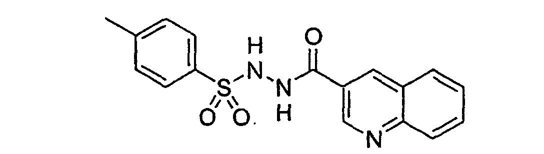

Synthesis of 3-quinolinecarboxylic acid, 2-[(4-methylphenyl)sulfonyl]-hydrazide: Example 127 was synthesized in accordance with the methods of Example 122 except that (4-methylphenyl)sulfonyl chloride was used instead of 4-pentylphenylsulfonyl chloride.
MS: 342.4 (M+1 for C₁₇H₁₂N₃O₃SF₃); HPLC (Waters Alliance 2790 column, solvent gradient of 60% to 100% CH₃CN/H₂O + 0.1% formic acid over 1 minute with 3 minute run time) RT = 1.98 min., 98.3%.

### EXAMPLE 128

### 6-Quinolinecarboxylic acid, 2-[(3-methylphenyl)sulfonyl]hydrazide

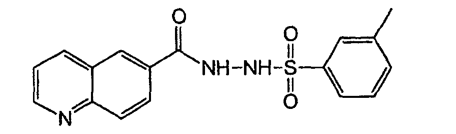

Synthesis of 6-quinolinecarboxylic acid, 2-[(3-methylphenyl)sulfonyl]-hydrazide: To a suspension of 6-quinolinecarboxylic acid hydrazide (0.1 g, 0.53 mmol) in pyridine (7 mL). Then the 3-methylbenzenesulfonyl chloride (0.107 g, 0.55 mmol) was added, and the reaction was stirred at room temperature overnight. The solvent was removed, and the desired product was purified by recrystallization in ethyl acetate.
MS: 342.4 (M+1 for C₁₇H₁₅N₃O₃S); mp: 234.0-234.6°C. HPLC (C18 column, 1:1 MeCN/H₂O+0.1% TFA) 96.6%, RT = 1.77 min., ¹H NMR (400 MHz DMSO) δ 2.29(S, 3H), 7.40(m, 2H), 7.67(m, 2H), 7.86(t, 1H, J=6.5 Hz), 8.12(d, 1H, J=8.5 Hz), 8.22(d, 1H, J=8.8 Hz), 8.53(S, 1H), 8.84(d, 1H, J=8.0 Hz), 9.16(s, 1H), 10.14(s, 1H), 11.01(s, 1H).

### EXAMPLE 129

### 3-Quinolinecarboxylic acid, 2-[(3-methylphenyl)sulfonyl]hydrazide

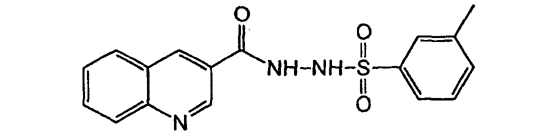

Synthesis of 3-quinolinecarboxylic acid, 2-[(3-methylphenyl)-sulfonyl]hydrazide: Example 129 was synthesized in accordance with the methods of Example 128 except that 3-quinolinecarboxylic acid hydrazide was used instead of 6-quinolinecarboxylic acid hydrazide.
MS: 342.4 (M+1 for C₁₇H₁₅N₃O₃S; mp: 238.6-239.4°C. HPLC (C18 column, 1:1 MeCN/H₂O+0.1% TFA) 95.9%, RT = 2.43 min., ¹H NMR (400 MHz, DMSO) δ 2.30(S, 3H), 7.40(m, 2H), 7.65(m, 2H), 7.79(t, 1H, J=7.7Hz), 7.99(t, 1H, J=7.9 Hz), 8.18(m, 2H), 8.91(s, 1H), 9.22(s, 1H), 10.22(s, 1H), 11.14(s, 1H).

### EXAMPLE 130

### 6-Quinolinecarboxylic acid, 2-[(3-chlorophenyl)sulfonyl]hydrazide

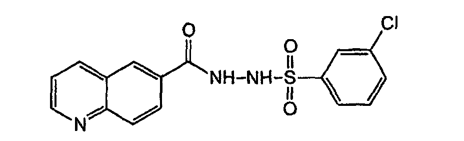

Synthesis of 6-quinolinecarboxylic acid, 2-[(3-chlorophenyl)sulfonyl]-hydrazide: Example 130 was synthesized in accordance with the methods of Example 128 except that 3-chlorophenylsulfonyl chloride was used instead of 3-methylbenzenesulfonyl chloride.
MS: 362.8 (M+1 for C₁₆H₁₂N₃O₃SCl); mp: 238.4-239.1°C. HPLC (C18 column, 1:1 MeCN/H₂O+0.1% TFA) 93.7%, RT = 2.10 min., ¹H NMR (400MHz, DMSO) δ 7.54(t, 1H, J=8.0 Hz), 7.78(d, 1H, J=7.8 Hz), 7.85(m, 3H), 8.11(d, 1H, J=8.3 Hz), 8.19(d, 1H, J=9.0 Hz), 8.53(S, 1H), 8.81(d, 1H, J=8.3 Hz), 9.14(S, 1H), 10.44(S, 1H), 11.09(S, 1H).

### EXAMPLE 131

### 3-Quinolinecarboxylic acid, 2-[(3-chlorophenyl)sulfonyl]hydrazide

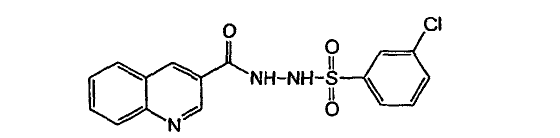

Synthesis of 3-quinolinecarboxylic acid, 2-[(3-chlorophenyl)sulfonyl]-hydrazide: Example 131 was synthesized in accordance with the methods of Example 128 except that 3-quinolinecarboxylic acid hydrazide was used instead of 6-quinolinecarboxylic acid hydrazide.
MS: 362.8 (M+1 for C₁₆H₁₂N₃O₃SCl); mp: 249.5-250.4°C. HPLC (C18 column, 1:1 MeCN/H₂O+0.1% TFA) 94.0%, RT = 2.7 min., ¹H NMR (400 MHz, DMSO) δ 7.56(S, 1H), 7.70-8.00(m, 4H), 8.10-8.20(m, 3H), 9.14(S,1H), 10.48(S,1H), 11.15(S, 1H).

### EXAMPLE 132

### 6-Quinolinecarboxylic acid, 2-[(3-fluorophenyl)sulfonyl]hydrazide

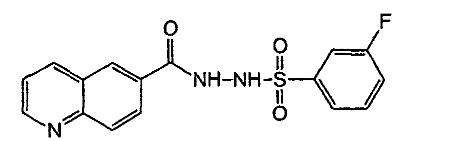

Synthesis of 6-quinolinecarboxylic acid, 2-[(3-fluorophenyl)sulfonyl]-hydrazide: Example 132 was synthesized in accordance with the methods of Example 128 except that 3-fluorophenylsulfonyl chloride was used instead of 3-methylbenzenesulfonyl chloride.
MS: 346.35 (M+1 for C₁₆H₁₂N₃O₃SF); mp: 250.6-251.3°C. HPLC (C18 column, 1:1 MeCN/H₂O + 0.1% TFA) 94.7%, RT = 1.8 min., ¹H NMR (400MHz, DMSO) δ 7.40-7.70(m, 4H), 7.80(S, 1H), 8.09(d,1H, J=7.6 Hz), 8.15(d, 1H, J=9.5 Hz), 8.51(S, 1H), 8.76(d, 1H, J=10.5 Hz), 9.12(S, 1H), 10.40(S, 1H), 11.05(S,1H).

### EXAMPLE 133

### 3-Quinolinecarboxylic acid, 2-[(3-fluorophenyl)sulfonyl]hydrazide

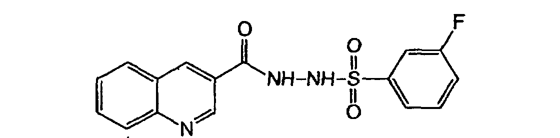

Synthesis of 3-quinolinecarboxylic acid, 2-[(3-fluorophenyl)sulfonyl]-hydrazide: Example 133 was synthesized in accordance with the methods of Example 132 except that 3-quinolinecarboxylic acid hydrazide was used instead of 6-quinolinecarboxylic acid hydrazide.
MS: 346.35 (M+1 for C₁₆H₁₂N₃O₃SF); mp: 247.8-248.4°C. HPLC (C18 column, 1:1 MeCN/H₂O+0.1% TFA) 94.7%, RT = 2.5 min., ¹H NMR (400 MHz, DMSO) δ 7.40-7.80 (m, 5H), 7.89(t, 1H, J=7.^# Hz), 8.00-8.20(m, 2H), 8.78(S, 1H), 9.10(S, 1H), 10.43(S, 1H), 11.10(S, 1H).

### EXAMPLE 134

### 6-Quinolinecarboxylic acid, 2-[(2-trifluoromethylphenyl)sulfonyl]hydrazide

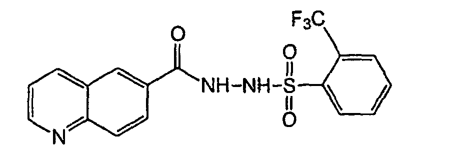

Synthesis of 6-quinolinecarboxylic acid, 2-[(2-trifluoromethylphenyl)-sulfonyl]hydrazide: Example 134 was synthesized in accordance with the methods of Example 128 except that 2-(trifluoromethyl)phenylsulfonyl chloride was used instead of 3-methylbenzenesulfonyl chloride.
MS: 396.4 (M+1 for C₁₇H₁₂N₃O₃SF₃), mp: 236.7-237.4°C; HPLC (C18 column, 1:1 MeCN/H₂O + 0.1% TFA) 99.8%, RT = 1.96 min., ¹H NMR (400 MHz, DMSO) δ 7.60-7.80(m, 3H), 7.94(d, 1H, J=6.9 Hz), 8.01(d, 1H, J=8.3 Hz), 8.10(d, 1H, J=9.1 Hz), 8.19(d, 1H, J=7.1 Hz), 8.45(S, 1H), 8.65(d, 1H, J=8.8 Hz), 9.08(S, 1H), 10.13(S, 1H), 11.06(S, 1H).

### EXAMPLE 135

### 3-Quinolinecarboxylic acid, 2-[(2-trifluoromethylphenyl)sulfonyl]hydrazide

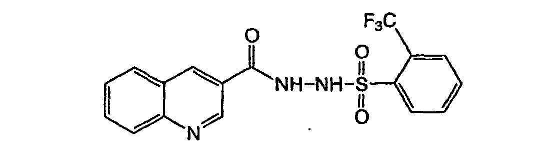

Synthesis of 3-quinolinecarboxylic acid, 2-[(2-trifluoromethylphenyl)-sulfonyl]hydrazide: Example 135 was synthesized in accordance with the methods of Example 134 except that 3-quinolinecarboxylic acid hydrazide was used instead of 6-quinolinecarboxylic acid hydrazide.
MS: 396.4 (M+1 for C₁₇H₁₂N₃O₃SF₃), mp: 203.6-204.4°C; HPLC (C18 column, 1:1 MeCN/H₂O + 0.1% TFA) 93.2%, RT = 2.88 min., ¹H NMR (400 MHz, DMSO) δ 7.40-8.0(m, 3H), 8.23(S, 1H), 8.67(S, 1H), 9.10(S, 1H),10.24(S, 1H), 11.18(S, 1H).

### EXAMPLE 136

### 6-Quinolinecarboxylic acid, 2-[(2-trifluoromethoxyphenyl)sulfonyl]hydrazide

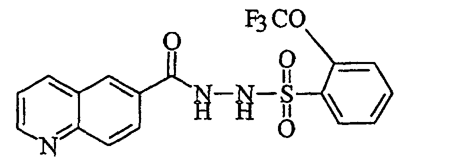

Synthesis of 6-quinolinecarboxylic acid, 2-[(2-trifluoromethoxyphenyl)sulfonyl]hydrazide: Example 136 was synthesized in accordance with the methods of Example 128 except that 2-(trifluoromethoxyl)phenylsulfonyl chloride was used instead of 3-methylbenzenesulfonyl chloride.
MS: 412.4 (M+1 for C₁₇H₁₂N₃O₄SF₃), mp: 243.5-243.9°C; HPLC (C18 column, 1:1 MeCN/H₂O + 0.1% TFA) 92.1%, RT = 2.05 min., ¹H NMR (400 MHz, DMSO) δ 7.40-7.55(m, 2H), 7.60-7.80(m, 2H), δ 7.90-8.20(m, 3H), 8.43(S, 1H), 8.59(d, 1H, J=8.1 Hz), 9.03(S, 1H),10.32(S, 1H), 11.00(S, 1H).

### EXAMPLE 137

### 3-Quinolinecarboxylic acid, 2-[(2-trifluoromethoxyphenyl)sulfonyl]hydrazide

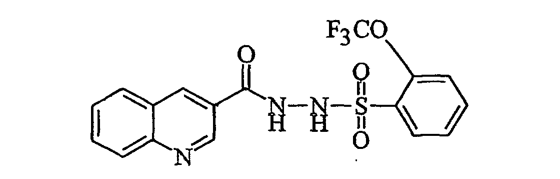

Synthesis of 3-quinolinecarboxylic acid, 2-[(2-trifluoromethoxylphenyl)sulfony]hydrazide: Example 137 was synthesized in accordance with the methods of Example 136 except that 3-quinolinecarboxylic acid hydrazide was used instead of 6-quinolinecarboxylic acid hydrazide.
MS: 412.4 (M+1 for C₁₇H₁₂N₃O₄SF₃), mp: 175.1-175.9°C; HPLC (C18 column, 1:1 MeCN/H₂O + 0.1% TFA) 91.2%, RT = 2.97 min., ¹H NMR (400 MHz, DMSO) δ 7.40-8.10(m, 8H), 8.70(S, 1H), 9.06(S, 1H), 10.39(S, 1H), 11.08(S, 1H).

### EXAMPLE 138

### 6-Quinolinecarboxylic acid, 2-[(3,5-dichlorophenyl)sulfonyl]hydrazide

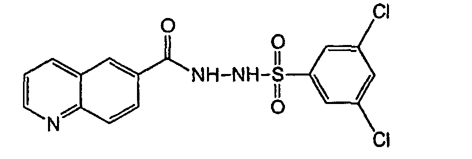

Synthesis of 6-quinolinecarboxylic acid, 2-[(3,5-dichlorophenyl)sulfonyl]hydrazide: Example 138 was synthesized in accordance with the methods of Example 128 except that 3,5-dichlorophenylsulfonyl chloride was used instead of 3-methylbenzenesulfonyl chloride.
MS: 397.3 (M+1 for C₁₆H₁₁N₃O₃SCl₂); mp: 241.6-242.3°C. HPLC (C18 column, 1:1 MeCN/H₂O+0.1% TFA) 91.9%, RT = 2.2 min., ¹H NMR (400 MHz, DMSO) δ 7.81(S, 3H), 7.96(S, 1H), 8.18(d, 1H, J=9.1 Hz), 8.21(d, 1H, J=9.0 Hz), 8.54(S, 1H), 8.80(d, 1H, J=8.1 Hz), 9.13(S, 1H), 10.65(S, 1H), 11.14(S, 1H).

### EXAMPLE 139

### 3-Quinolinecarboxylic acid, 2-[(3,5-dichlorophenyl)sulfonyl]hydrazide

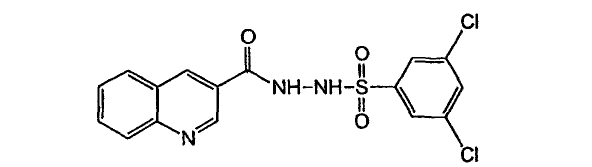

Synthesis of 3-quinolinecarboxylic acid, 2-[(3,5-dichlorophenyl)sulfonyl]hydrazide: Example 139 was synthesized in accordance with the methods of Example 138 except that 3-quinolinecarboxylic acid hydrazide was used instead of 6-quinolinecarboxylic acid hydrazide.
MS: 397.3 (M+1 for C₁₆H₁₁N₃O₃SCl₂); mp: 241.3-241.9°C. HPLC (C18 column, 1:1 MeCN/H₂O+0.1% TFA) 94.5%, RT = 4.0 min., ¹H NMR (400 MHz, DMSO) δ 7.75(t, 2H, J=7.1 Hz), 7.83(S, 2H), 7.96(S, 1H), 8.10-8.30(m, 2H), 8.92(S, 1H), 9.19(S, 1H), 10.67(S, 1H), 11.23(S,1H).

### EXAMPLE 140

### 6-Quinolinecarboxylic acid, 2-[(3-chloro-4-fluorophenyl)sulfonyl]hydrazide

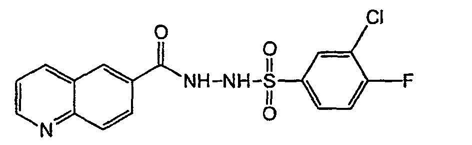

Synthesis of 6-quinolinecarboxylic acid, 2-[(3-chloro-4-fluorophenyl)sulfonyl]hydrazide: Example 140 was synthesized in accordance with the methods of Example 128 except that 3-chloro-4-fluorophenylsulfonyl chloride was used instead of 3-methylbenzenesulfonyl chloride.
MS: 380.8 (M+1 for C₁₆H₁₁N₃O₃SClF). mp: 241.9-242.6°C; HPLC (C18 column, 1:1 MeCN/H₂O + 0.1% TFA) 93.60%, RT = 2.09 min., ¹H NMR (400 MHz, DMSO) δ 7.58(t, 1H, J=9.1 Hz), 7.70-7.90(m, 2H), 8.80-8.20(m, 3H),8.52(S, 1H),8.78(d, 1H, J=8.8 Hz) 9.13(S, 1H),10.48(S, 1H), 11.09(S, 1H).

### EXAMPLE 141

### 3-Quinolinecarboxylic acid, 2-[(3-chloro-4-fluorophenyl)sulfonyl]hydrazide

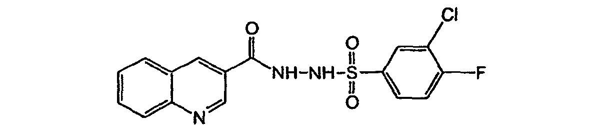

Synthesis of 3-quinolinecarboxylic acid, 2-[(3-chloro-4-fluorophenyl)sulfonyl]-hydrazide: Example 141 was synthesized in accordance with the methods of Example 140 except that 3-quinolinecarboxylic acid hydrazide was used instead of 6-quinolinecarboxylic acid hydrazide.
MS: 380.8 (M+1 for C₁₆H₁₁N₃O₃SClF). mp: 245.0-245.7°C; HPLC (C18 column, 1:1 MeCN/H₂O + 0.1% TFA) 95.69%, RT = 3.09 min., ¹H NMR (400 MHz, DMSO) δ 7.59(t, 1H, J=8.7 Hz), 7.89(t, 1H, J=5.6 Hz), 7.70-7.90(m, 2H), 8.80-8.20(m, 3H), 8.81(d, 1H, J=8.8 Hz) 9.13(S, 1H), 10.51(S, 1H), 11.14(S, 1H).

### EXAMPLE 142

### 6-Quinolinecarboxylic acid, 2-[(3-methoxyphenyl)sulfonyl]hydrazide

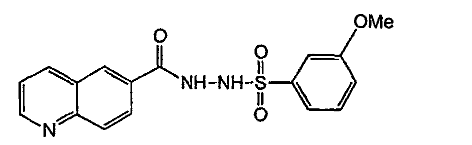

Synthesis of 6-quinolinecarboxylic acid, 2-[(3-methoxyphenyl)sulfonyl]hydrazide: Example 142 was synthesized in accordance with the methods of Example 128 except that 3-methoxylphenylsulfonyl chloride was used instead of 3-methylbenzenesulfonyl chloride.
MS: 358.4 (M+1 for C₁₇H₁₅N₃O₄S). mp: 201.9-202.7°C; HPLC (C18 column, 1:1 MeCN/H₂O + 0.1% TFA) 97.64%, RT = 1.73 min., ¹H NMR (400 MHz, DMSO) δ 3.73(S, 3H), 7.10-7.20(m, 2H), 7.30-7.50(m, 2H), 7.85(t, 1H, J=4.7 Hz), 8.19(d, 1H, J=8.7 Hz), 8.21(d, 1H, J=8.7 Hz), 8.59(S, 1H), 8.84(d, 1H, J=7.3 Hz), 9.17(S, 1H), 10.21(S, 1H), 11.01(S, 1H).

### EXAMPLE 143

### 3-Quinolinecarboxylic acid, 2-[(3-methoxyphenyl)sulfonyl]hydrazide

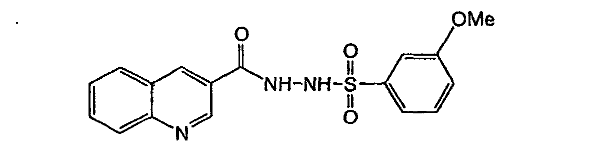

Synthesis of 3-quinolinecarboxylic acid, 2-[(3-methoxyphenyl)sulfonyl]-hydrazide: Example 143 was synthesized in accordance with the methods of Example 142 except that 3-quinolinecarboxylic acid hydrazide was used instead of 6-quinolinecarboxylic acid hydrazide.
MS: 358.4 (M+1 for C₁₇H₁₅N₃O₄S). mp: 226.9-227.2°C; HPLC (C18 column, 1:1 MeCN / H₂O + 0.1 % TFA) 96.70%, RT = 2.39 min., ¹H NMR (400 MHz, DMSO) δ 3.72(S, 3H), 7.10-7.20(m, 1H), 7.30-7.50(m, 2H), 7.70(t, 1H, J=7.3 Hz), 7.89(t, 1H, J=8.0 Hz), 8.00-8.20(m, 3H), 8.77(S, 1H), 9.09(S, 1H), 10.24(S, 1H), 11.03(S, 1H).

### EXAMPLE 144

### 6-Quinolinecarboxylic acid, 2-[(3-bromophenyl)sulfonyl]hydrazide

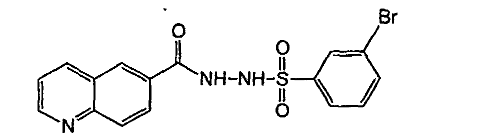

Synthesis of 6-quinolinecarboxylic acid, 2-[(3-bromophenyl)sulfonyl]hydrazide: Example 144 was synthesized in accordance with the methods of Example 128 except that 3-bromophenylsulfonyl chloride was used instead of 3-methylbenzenesulfonyl chloride.
MS: 407.3 (M+1 for C₁₆H₁₂N₃O₃SBr). mp: 216.0-216.8°C; HPLC (C18 column, 1:1 MeCN/H₂O + 0.1% TFA) 96.88%, RT = 2.00 min., ¹H NMR (400 MHz, DMSO) δ 7.47(t, 2H, J=8.1), 7.70-7.90(m, 2H),7.97(S, 1H), 8.00-8.20(m, 2H), 8.49(S, 1H), 8.73(d, 1H, J=8.0 Hz) 9.11(S, 1H), 10.43(S, 1H), 11.06(S, 1H).

### EXAMPLE 145

### 3-Quinolinecarboxylic acid, 2-[(3-bromophenyl)sulfonyl]hydrazide

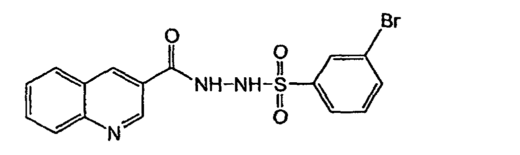

Synthesis of 3-quinolinecarboxylic acid, 2-[(3-bromophenyl)sulfonyl]hydrazide: Example 145 was synthesized in accordance with the methods of Example 144 except that 3-quinolinecarboxylic acid hydrazide was used instead of 6-quinolinecarboxylic acid hydrazide.
MS: 407.3 (M+1 for C₁₆H₁₂N₃O₃SBr). mp: 231.5-232.3°C; HPLC (C18 column, 1:1 MeCN/H₂O +0.1% TFA) 95.54%, RT = 2.95 min. ¹H NMR (400MHz, DMSO) δ 7.48(t, 2H, J=8.1), 7.71(t, 1H, J=8.1 Hz), 7.80-7.90(m, 2H),7.98(S, 1H), 8.00-8.20(m, 2H), 8.80(S, 1H), 9.11(S, 1H), 10.47 (S, 1H), 11.13(S, 1H).

### EXAMPLE 146

### Benzofuran-2-carboxylic acid 2-(phenylsulfonyl)hydrazide

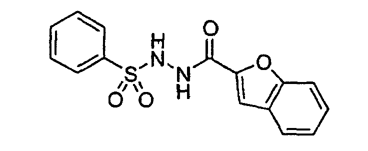

### Step 1. The preparation of Benzofuran-2-carboxylic acid hydrazide

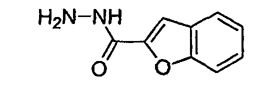

Benzofuran-2-carboxylic acid (3.85 g, 23.74 mmol) was dissolved in THF (144 mL). *n*-Methyl morpholine (5.74 mL, 52.24 mmol) was added, and the reaction was cooled to 0°C. Isobutyl chloroformate (3.39 mL, 26.12 mmol) was added, and the reaction was allowed to stir for 10 minutes. A solution of hydrazine (14.9 mL, 474.8 mmol) in THF (19 mL) was added slowly to the reaction, and the reaction was allowed to warm to room temperature. The reaction was allowed to stir for 1 hour. The solution was then washed with saturated sodium bicarbonate and brine, dried over Na₂SO₄, and concentrated. The precipitate was triturated with acetone to yield the desired product (2.37 g, 56.7%).
MS: 176.9 (M+1 for C₉H₈N₂O₂); mp: 164-167°C. TLC: SiO₂, R_{f}= 0.23 (1:1 hexane/EtOAc). Analysis (C₉H₈N₂O₂) (calc) C: 61.36, H: 4.58, N: 15.90; (found) C: 60.96, H: 4.46, N: 15.57. IR. (KBr, cm⁻¹) 3324, 3182, 2984, 1598.
¹H NMR (400 MHz, DMSO) δ 4.53 (s, 2H), 7.28 (t, 1H, J = 6.8 Hz), 7.40 (t, 1H, J = 6.8 Hz), 7.47 (s, 1H), 7.60 (d, 1H, J = 8.1 Hz), 7.72 (d, 1H, J = 7.6 Hz), and 9.99 (s, 1H).

Step 2. The preparation of Benzofuran-2-carboxylic acid 2-(phenylsulfonyl)hydrazide: Benzofuran-2-carboxylic acid hydrazide (0.4 g, 2.27 mmol) was suspended in THF (25 mL). N,N-diisopropylethyl amine (1.58 mL, 9.08 mmol) was added, and the reaction was cooled to 0°C. Phenylsulfonyl chloride (0.32 mL, 2.50 mmol) was added followed by DMAP (5 mg, 0.04 mmol). The reaction was allowed to warm to room temperature. The solution was orange after it stirred for 1 hour, and DMF (20 mL) was added and the solution became clear. After 4 hours of stirring, the solution was diluted with EtOAc (200 mL). It was then washed with saturated sodium bicarbonate and brine and dried over Na₂SO₄. The reaction was then concentrated down and purified by flash chromatography eluting with 3:1 hexane/EtOAc. The desired product was isolated (0.38 g, 53.0%).
MS: 317.0 (M+1 for C₁₅H₁₂N₂O₄S). TLC: SiO₂, R_{f}= 0.23 (1:1 hexane/EtOAc); mp: 175-180°C; HRMS: 317.0598 (M+1 for C₁₅H₁₂N₂O₄S), HPLC: (30% H₂O/70% CH₃CN/0.1% TFA), Ret. Time: 2.911, Purity: 93.12%. IR (KBr, cm⁻¹) 3338, 3131, 1676, 1581, 1349, 1163. ¹H NMR (400 MHz, CDCl₃) δ 7.23-7.67 (m, 9H), 7.93 (d, 2H, J = 5.6 Hz), and 8.52 (s, 1H).

### EXAMPLE 147

### 7-Ethoxy-2-benzofurancarboxylic acid 2-(phenylsulfonyl)-hydrazide

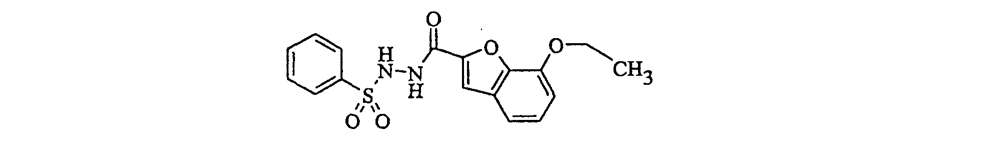

### Step 1. The preparation of 7-ethoxy-benzofuran-2-carboxylic acid hydrazide

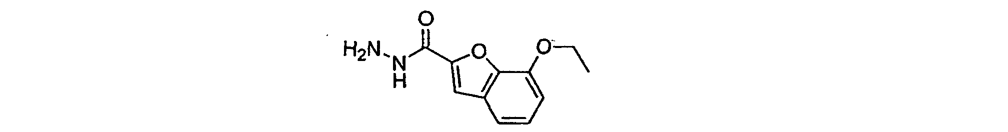

7-Ethoxy-benzofuran-2-carboxylic acid (3.0 g, 14.5 mmol) was dissolved in THF (88 mL). N-Methyl morpholine (3.51 mL, 31.9 mmol) was added, and the reaction was cooled to 0°C. Isobutyl chloroformate (2.07 mL, 15.95 mmol) was added, and the reaction was allowed to stir for 15 minutes. Hydrazine (9.11 mL, 290 mmol) was added slowly to the reaction, and the reaction was allowed to warm to room temperature. The reaction was allowed to stir for 1 hour. The solution was added EtOAc and was then washed with saturated sodium bicarbonate and brine, dried over Na₂SO₄, and concentrated. The precipitate was triturated with CH₂Cl₂ and was then recrystallized in hot methanol (50 mL) and water to yield the desired product (2.63 g, 82.4%).
MS: 221.0 (M+1 for C₁₁H₁₂N₂O₃); mp: 129-130°C. TLC: SiO₂, R_{f} = 0.15 (1:1 hexane/EtOAc); Analysis (C₁₁H₁₂N₂O₃·0.03 H₂O) (calc) C: 59.85, H: 5.51, N: 12.69; (found) C: 59.51, H: 5.46, N: 12.46. IR (KBr, cm⁻¹) 3264, 1587, 1202. ¹H NMR (400 MHz, DMSO) δ 1.38 (t, 3H, J = 6.8 Hz), 4.21 (q, 2H, J = 6.8 Hz), 4.53 (s, 2H), 6.98 (dd, 1H, J = 0.7, 8.0 Hz), 7.16 (t, 1H, 8.0 Hz), 7.24 (dd, 1H, J = 1.0, 7.8 Hz), 7.44 (s, 1H), and 9.94 (s, 1H).

Step 2. The preparation of 7-ethoxy-2-benzofurancarboaylic acid 2-(phenylsulfonyl)hydrazide: 7-Ethoxy-benzofuran-2-carboxylic acid hydrazide (0.5 g, 2.27 mmol) was dissolved in THF (25 mL). N,N-diisopropylethylamine (1.58 mL, 9.08 mmol) was added, and the reaction was cooled to 0°C. Phenylsulfonyl chloride (0.32 mL, 2.50 mmol) was added followed by DMAP (5 mg, 0.04 mmol). The reaction was allowed to warm to room temperature. The solution was orange after it stirred for 1 hour. After 3 hours of stirring, the solution was added with EtOAc and washed with saturated sodium bicarbonate and brine and dried over Na₂SO₄. The reaction was then concentrated down and purified by flash chromatography eluting with 1:1 hexane/EtOAc. The desired product was isolated (0.05 g, 5.6%).
MS: 361.0 (M+1 for C₁₇H₁₆N₂O₅S). TLC: SiO₂, R_{f}= 0.17 (1:1 hexane/EtOAc); mp: 223-224°C. HRMS: (calc) 361.0558, (found) 361.0853 (M+1 for C₁₇H₁₆N₂O₅S) HPLC: (50% H₂O/50% CH₃CN/0.1% TFA) Ret. Time: 2.596, Purity: 98.71%. IR (KBr, cm⁻¹) 3268, 3124, 2982, 1666, 1586, 1344, 1166, 1087, 724. ¹H NMR (400 MHz, DMSO) δ 1.37 (t, 3H, J = 6.8 Hz), 4.19 (q, 2H, J = 7.1 Hz), 7.01 (d, 1H,

J = 8.0 Hz), 7.16-7.26 (m, 2H), 7.49-7.62 (m, 4H), 7.81 (d, 1H, J = 7.3 Hz), 10.17 (s, 1H), and 10.92 (s, 1H).

### EXAMPLE 148

### 7-Chloro-benzofuran-2-carboxylic acid 2-(phenylsulfonyl)hydrazide

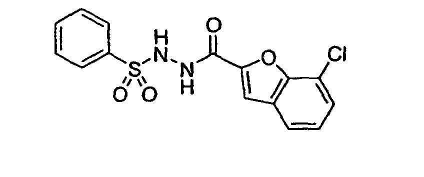

### Step 1. The preparation of 7-Chloro-benzofuran-2-carboxylic acid hydrazide

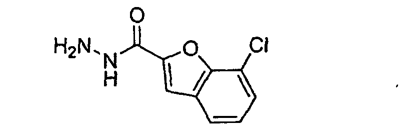

7-Chloro-benzofuran-2-carboxylic acid (1.0 g, 5.1 mmol) was dissolved in THF (31 mL). N-Methyl morpholine (1.2 mL, 11.2 mmol) was added, and the reaction was cooled to 0°C. Isobutyl chloroformate (0.73 mL, 5.6 mmol) was added, and the reaction was allowed to stir for 10 minutes. Hydrazine (3.2 mL, 102 mmol) was added slowly to the reaction, and the reaction was allowed to warm to room temperature. The reaction was allowed to stir for 1 hour. The solution was added acetate and was washed with saturated sodium bicarbonate and brine, dried over Na₂SO₄, and concentrated. The precipitate was triturated with CH₂Cl₂. The light brown precipitate was then recrystallized in hot methanol (25 mL) and water to yield the desired product (0.94 g, 87.8%).
MS: 211.0 (M+1 for C₉H₇N₂O₂Cl); mp: 179-180°C. TLC: SiO₂, R_{f}= 0.11 (1:1 hexane/EtOAc); Analysis (C₉H₇N₂O₂Cl·0.005H₂O) (calc) C: 51.28, H: 3.36, N: 13.29; (found) C: 50.91, H: 3.44, N: 12.99. IR (KBr, cm⁻¹) 3307, 3222, 3029, 1592, 1300, 993. ¹H NMR (400 MHz, DMSO) δ 4.60 (s, 2H), 7.32 (t, 1H, J = 7.9 Hz), 7.53 (d, 1H, J = 7.8 Hz), 7.59 (s, 1H), 7.73 (d, 1H, J = 7.8 Hz), and 10.10 (s, 1H).

### Step 2. The preparation of 7-Chloro-benzofuran-2-carboxylic acid

2-(phenylsulfonyl)hydrazide: 7-Chloro-benzofuran-2-carboxylic acid hydrazide (0.5 g, 2.38 mmol) was suspended in THF (26 mL). N,N-diisopropylethylamine (1.66 mL, 9.52 mmol) was added, and the reaction was cooled to 0°C. Phenylsulfonyl chloride (0.33 mL, 2.62 mmol) was added followed by DMAP (5 mg, 0.04 mmol). The reaction was allowed to warm to room temperature. The solution was yellow after it stirred for 2 hours, and DMF (10 mL) was added. After another hour of stirring, the solution was diluted with EtOAc (150 mL), and washed with saturated sodium bicarbonate and brine and dried over Na₂SO₄. The reaction was then concentrated down and purified by flash chromatography eluting with 3:1 hexane/EtOAc. The desired product was isolated (0.04 g, 4.6%). MS: 351.0 (M+1 for C₁₅H₁₁N₂O₄SCl). TLC: SiO₂, R_{f} = 0.57 (1:1 hexane/EtOAc); mp: 223-224°C. HRMS: (calc) 351.0206, (found) 351.0215 (M+1 for C₁₅H₁₁N₂O₄SCl) HPLC: Ret. Time: 9.862, Purity: 98.26%. IR (KBr, cm⁻¹) 3291, 3033, 1670, 1344, 1164. ¹H NMR (400 MHz, CDCl₃) δ 7.24-7.64 (m, 7H), 7.94 (d, 2H, J = 7.1 Hz), 8.62 (s, 1H), and 9.12 (s, 1H).

### EXAMPLE 149

### 2,3-Dihydro-benzofuran-5-carboxylic acid 2-(phenylsulfonyl)hydrazide

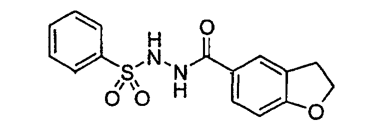

### Step 1. The preparation of 2,3-Dihydro-benzofuran-5-carboxylic acid hydrazide

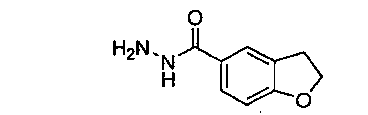

2,3-Dihydro-benzofuran-5-carboxylic acid (0.5 g, 3.05 mmol) was dissolved in THF (19 mL). N-Methyl morpholine (0.74 mL, 6.71 mmol) was added, and the reaction was cooled to 0°C. Isobutyl chloroformate (0.43 mL, 3.35 mmol) was added, and the reaction was allowed to stir for 10 minutes. Hydrazine (1.91 mL, 61 mmol) was added slowly to the reaction, and the reaction was allowed to warm to room temperature. The reaction was allowed to stir for 1 hour. The solution was added EtOAc and was washed with saturated sodium bicarbonate and brine, dried over Na₂SO₄, and concentrated. The crude desired product was collected (0.14 g, 17.1%) and carried on to the next reaction.
MS: 179.1 (M+1 for C₉H₁₀N₂O₂). TLC: SiO₂, R_{f} = 0.36 (1:1 hexane/EtOAc). ¹H NMR (400 MHz, DMSO) δ 3.16 (t, 2H, J = 8.8 Hz), 4.39 (s, 2H), 4.54 (t, 2H, J = 8.8 Hz), 6.74 (d, 1H, J = 8.3 Hz), 7.58 (d, 1H, J = 7.8 Hz), 7.67 (s, 1H), and 9.50 (s, 1H).

### Step 2. The preparation of 2,3-Dihydro-benzofuran-5-carboxylic acid

2-(phenylsulfonyl)hydrazide: 2,3-Dihydro-benzofuran-5-carboxylic acid hydrazide (0.25 g, 1.40 mmol) was dissolved in THF (16 mL). N,N-diisopropylethylamine (0.98 mL, 5.6 mmol) was added, and the reaction was cooled to 0°C. Phenylsulfonyl chloride (0.20 mL, 1.54 mmol) was added followed by DMAP (5 mg, 0.04 mmol). The reaction was allowed to warm to room temperature. After 2 hours of stirring, the solution was diluted with EtOAc (200 mL), and washed with saturated sodium bicarbonate and brine and dried over Na₂SO₄. The reaction was then concentrated down and purified by flash chromatography eluting with 1:1 hexane/EtOAc. The desired product was isolated (0.09 g, 20.0%).
MS: 319.0 (M+1 for C₁₅H₁₄N₂O₄S); mp: 181-183°C; TLC: SiO₂, R_{f}= 0.46 (1:1 hexane/EtOAc); HRMS: (calc) 319.0752, (found) 319.0761 (M+1 for C₁₅H₁₄N₂O₄S) HPLC: (50% H₂O/50% CH₃CN/0.1% TFA) Ret. Time: 9.862, Purity: 95.04%. ¹H NMR (400 MHz, DMSO) δ 3.13 (t, 2H, J = 8.8 Hz), 4.54 (t, 2H, J = 8.8 Hz), 6.74 (d, 1H, J = 8.3 Hz), 7.46-7.57 (m, 5H), 7.77 (d, 2H, J = 7.0 Hz), 9.85 (s, 1H), and 10.42 (s, 1H).

### EXAMPLE 150

### 2-Indolecarboxylic acid, 5-amino-2-phenylsulfonyl hydrazide

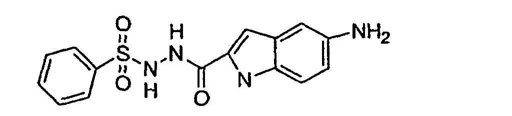

Example 150 was synthesized in accordance with the methods of Example 157, Step 1, except that 2-indolecarboxylic acid, 5-nitro-2-phenylsulfonyl hydrazide was used instead of 2-benzofurancarboxylic acid, 5-nitro-, 2-(phenylsulfonyl)hydrazide (yield 98%).
MS: 331.1 (M+1 for C₁₅H₁₄N₄O₃S); mp: 240.2-241.1; HPLC (C18 column, 1:1 MeCN/H₂O = 0.1% TFA) 95.1%, RT = 1.6 min.; ¹HNMR (DMSO-d₆) 6.60 (d, 1H, J = 6.6 Hz), 6.64 (S, 1H), 6.87 (S, 1H), 7.05 (d, 1H, J = 8.4 Hz), 7.40-7.50 (m, 4H), 7.60 (d, 1H, J = 7.3 Hz), 7.80 (d, 2H, J = 7.0 Hz), 10.47 (s, 1H), 11.08 (s, 1H), and 11.09 (s, 1H).

### EXAMPLE 151

### 5-Chloro-benzofuran-2-carboxylic acid 2-(phenylsulfonyl)hydrazide

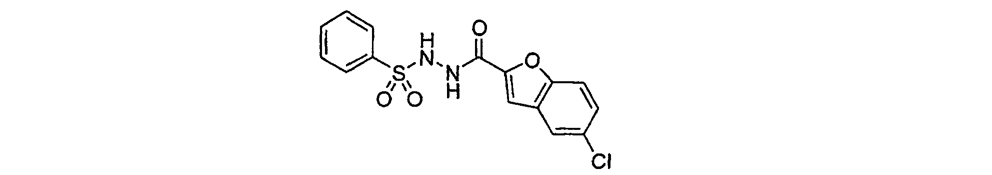

### Step 1. The preparation of 5-Chloro-benzofuran-2-carboxylic acid hydrazide

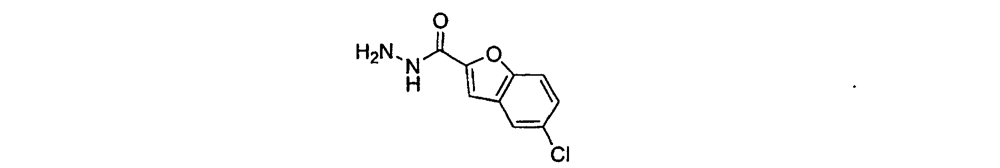

5-Chloro-benzofuran-2-carboxylic acid (1.0 g, 5.1 mmol) was dissolved in THF (31 mL). N-Methyl morpholine (1.2 mL, 11.2 mmol) was added, and the reaction was cooled to 0°C. Isobutyl chloroformate (0.73 mL, 5.6 mmol) was added, and the reaction was allowed to stir for 20 minutes. Hydrazine (3.2 mL, 102 mmol) was added slowly to the reaction, and the reaction was allowed to warm to room temperature. The reaction was allowed to stir for 1.5 hours. The solution was added EtOAc and washed with saturated sodium bicarbonate and brine, dried over Na₂SO₄, and concentrated. The precipitate was then recrystallized in hot methanol (25 mL) and water to yield the desired product (1.07 g, 99.8%).
MS: 211.1 (M+1 for C₉H₇N₂O₂Cl). TLC: SiO₂, R_{f}= 0.09 (1:1 hexane/EtOAc); mp: 186-187°C; Analysis (C₉H₇N₂O₂Cl) (calc) C: 51.32, H: 3.35, N: 13.30; (found) C: 51.25, H: 3.44, N: 12.90. IR (KBr, cm⁻¹) 3321, 3186, 3020, 1666, 1599, 1442, 1315, 1183, 985, 804, 695. ¹H NMR (400 MHz, DMSO) δ 4.58 (s, 2H), 7.46 (d, 2H, J = 8.4 Hz), 7.67 (d, 1H, J = 8.8 Hz), 7.84 (s, 1H), and 10.08 (s, 1H).

### Step 2. The preparation of 5-chloro-benzofuran-2-carboxylic acid

2-(phenylsulfonyl)hydrazide: 5-Chloro-benzofuran-2-carboxylic acid hydrazide (0.4 g, 1.90 mmol) was suspended in THF (21 mL). N,N-diisopropylethylamine (1.32 mL, 7.6 mmol) was added, and the reaction was cooled to 0°C. Phenylsulfonyl chloride (0.27 mL, 2.09 mmol) was added followed by DMAP (5 mg, 0.04 mmol). The reaction was allowed to warm to room temperature. The solution was orange after it stirred for 2 hours, and DMF (10 mL) was added and the solution became clear. After 15 minutes of stirring, the solution was diluted with EtOAc (200 mL). It was then washed with saturated sodium bicarbonate and brine and dried over Na₂SO₄. The reaction was then concentrated down and purified by flash chromotagraphy eluting with 2:1 hexane/EtOAc. The desired product was isolated (0.03 g, 3.8%).
MS: 350.9 (M+1 for C₁₅H₁₁N₂O₄SCl). TLC: SiO₂, R_{f}= 0.55 (1:1 hexane/EtOAc); mp: 181-183°C; HRMS: (calc) 351.0206, (found) 351.0206 (M+1 for C₁₅H₁₁N₂O₄SCl) HPLC: (50% H₂O/50% CH₃CN/0.1% TFA) Ret. Time: 8.755,
Purity: 97.13%. IR (KBr, cm⁻¹) 3291, 3066, 1670, 1344, 1164. ¹H NMR (400 MHz, CDCl₃) δ 4.79 (br, 1H), 7.24-7.69 (m, 7H), 7.92 (d, 2H, J = 7.6 Hz), and 8.51 (br, 1H).

### EXAMPLE 152

### Isoquinoline-1-carboxylic acid 2-(phenylsulfonyl)hydrazide

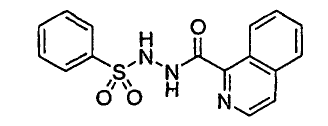

### Step 1. The preparation of Isoquinoline-1-carboxylic acid hydrazide

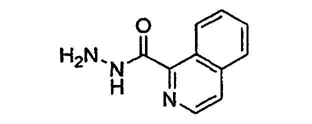

Isoquinoline-1-carboxylic acid (2.0 g, 11.55 mmol) was dissolved in THF (70 mL). N-Methyl morpholine (2.79 mL, 25.41 mmol) was added, and the reaction was cooled to 0°C. Isobutyl chloroformate (1.65 mL, 12.7 mmol) was added, and the reaction was allowed to stir for 20 minutes. Hydrazine (7.25 mL, 231 mmol) was added slowly to the reaction, and the reaction was allowed to warm to room temperature. The reaction was allowed to stir for 1.5 hours. The solution was diluted with EtOAc (200 mL) and then washed with saturated sodium bicarbonate and brine, dried over Na₂SO₄, and concentrated and carried on to the next reaction.
MS: 187.9 (M+1 for C₁₀H₉N₃O). TLC: SiO₂, R_{f}=0.03 (1:1 hexane/EtOAc).
Step 2. The preparation of isoquinoline-1-carboxylic acid 2-(phenylsulfonyl)-hydrazide: Isoquinoline-1-carboxylic acid hydrazide (2.0 g, 10.68 mmol) was dissolved in THF (119 mL). N,N-diisopropylethylamine (7.44 mL, 42.7 mmol) was added, and the reaction was cooled to 0°C. Phenylsulfonyl chloride (1.50 mL, 11.80 mmol) was added followed by DMAP (5 mg, 0.04 mmol). The reaction was allowed to warm to room temperature. After 3 hours of stirring, the solution was diluted with EtOAc (200 mL). It was then washed with saturated sodium bicarbonate and brine and dried over Na₂SO₄. The reaction was then concentrated down and purified by flash chromotagraphy eluting with 2:1 hexane/EtOAc. The desired product was isolated (0.09 g, 2.5%).
MS: 328.1 (M+1 for C₁₆H₁₃N₃O₃S). TLC: SiO₂, R_{f}= 0.48 (1:1 hexane/EtOAc); mp: 198-200°C. HPLC: (50% H₂O/50% CH₃CN/0.1% TFA) Ret. Time: 5.616, Purity: 98.81%. Analysis (C₁₆H₁₃N₃O₃S) (calc) C: 58.70, H: 4.00, N: 12.84; (found) C: 58.49, H: 3.99, N: 12.59. IR (KBr, cm⁻¹) 3192, 1669, 1341, 1164. ¹H NMR (400 MHz, DMSO) δ 7.53-7.68 (m, 4H), 7.75-7.79 (m, 1H), 7.88 (d, 2H, J = 7.0 Hz), 7.94-8.00 (m, 3H), 8.48 (d, 1H, J = 5.6 Hz), 10.26 (s, 1H), and 10.82 (s, 1H).

### EXAMPLE 153

### 1H-Indole-5-carboxylic acid 2-(phenylsulfonyl)hydrazide

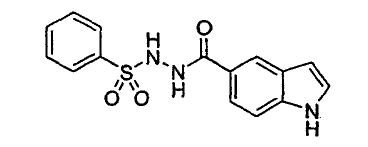

### Step 1. The preparation of 1H-Indole-5-carboxylic acid hydrazide

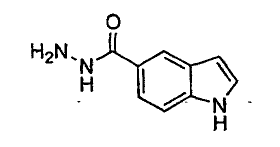

1*H*-Indole-5-carboxylic acid (2.0 g, 12.4 mmol) was dissolved in THF (75 mL). N-Methyl morpholine (3.00 mL, 27.3 mmol) was added, and the reaction was cooled to 0°C. Isobutyl chloroformate (1.77 mL, 13.7 mmol) was added, and the reaction was allowed to stir for 20 minutes. Hydrazine (7.78 mL, 248 mmol) was added slowly to the reaction, and the reaction was allowed to warm to room temperature. The reaction was allowed to stir for 1.5 hours. The solution was diluted with EtOAc (200 mL) and then washed with saturated sodium bicarbonate and brine, dried over Na₂SO₄, and concentrated. The precipitate was recrystallized from hot MeOH (25 mL) and hexane (1 mL). The desired product was collected (0.52 g, 23.8%).
MS: 175.9 (M+1 for C₉H₉N₃O); mp: 198-200°C; TLC: SiO₂, R_{f}= 0.06 (1:1 hexane/EtOAc); Analysis (C₉H₉N₃O) (calc) C: 61.70, H: 5.18, N: 23.99; (found) C: 61.90, H: 5.29, N: 24.09. IR (KBr, cm⁻¹) 3271, 1585, 1521. ¹H NMR (400 MHz, DMSO) δ 4.37 (s, 2H), 6.47 (s, 1H), 7.35-7.38 (m, 2H), 7.55 (d, 1H, J = 8.5 Hz), 8.04 (s, 1H), 9.53 (s, 1H), and 11.27 (s, 1H).

### Step 2. The preparation of 1H-Indole-5-carboxylic acid 2-(phenylsulfonyl)-hydrazide:

1*H*-Indole-5-carboxylic acid hydrazide (0.5 g, 2.85 mmol) was suspended in THF (32 mL). N,N-diisopropylethylamine (1.99 mL, 11.40 mmol) was added and the reaction was cooled to 0°C. Phenylsulfonyl chloride (0.36 mL, 2.85 mmol) was added followed by DMAP (5 mg 0.04 mmol). The reaction was allowed to warm to room temperature, and DMF (3 mL) was added. After 3 hours of stirring, the solution was diluted with EtOAc (200 mL). It was then washed with saturated sodium bicarbonate and brine and dried over Na₂SO₄. The reaction was then concentrated down and purified by flash chromotagraphy eluting with 2:1 hexane/EtOAc. The desired product was isolated (0.41 g, 23.8%).
MS: 316.0 (M+1 for C₁₅H₁₃N₃O₃S). TLC: SiO₂, R_{f}= 0.38 (1:1 hexane/EtOAc); mp: 209-211°C. HPLC: (50% H₂O/50% CH₃CN/0.1% TFA) Ret. Time: 4.102, Purity: 96.75%. Analysis (C₁₅H₁₃N₃O₃S) (calc) C: 57.13, H: 4.16, N: 13.32; (found) C: 57.28, H: 4.08, N: 13.06. IR (KBr, cm⁻¹) 3293, 3168, 1649, 1312, 1168. ¹H NMR (400 MHz, DMSO) δ 6.48 (s, 1H), 7.33-7.41 (m, 3H), 7.47 (t, 2H, J = 7.3 Hz), 7.57 (t, 1H, J = 7.4 Hz), 7.80 (d, 2H, J = 7.3 Hz), 7.94 (s, 1H), 9.84 (s, 1H), 10.45 (s, 1H), and 11.33 (s, 1H).

### EXAMPLE 154

### Quinoline-3-carboxylic acid 2-(phenylsulfonyl)hydrazide

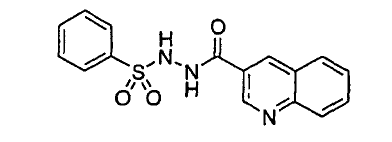

Quinoline-3-carboxylic acid hydrazide (0.34 g, 1.82 mmol) was dissolved in THF (20 mL). N,N-diisopropylethylamine (1.27 mL, 7.28 mmol) was added, and the reaction was cooled to 0°C. Phenylsulfonyl chloride (0.23 mL, 1.82 mmol) was added followed by DMAP (5 mg, 0.04 mmol). The reaction was allowed to warm to room temperature. After 2 hours of stirring, the red solution was diluted with EtOAc (100 mL). It was then washed with saturated sodium bicarbonate and brine and dried over Na₂SO₄. The reaction was then concentrated down and purified by flash chromotagraphy eluting with 2:1 hexane/EtOAc. The desired product was isolated (0.13 g, 21.8%).
MS: 328.1 (M+1 for C₁₆H₁₃N₃O₃S). TLC: SiO₂, R_{f}= 0.21 (1:1 hexane/EtOAc); mp: 249-250°C. HPLC: (50% H₂O/50% CH₃CN/0.1% TFA) Ret. Time: 2.701, Purity: 97.64%. Analysis (C₁₆H₁₃N₃O₃S·0.08H₂O) (calc) C: 58.45, H: 4.03, N: 12.78; (found) C: 58.52, H: 4.25, N: 12.38. IR (KBr, cm⁻¹) 3282, 3064, 2823, 1691, 1294, 1164. ¹H NMR (400 MHz, DMSO) δ 7.51 (t, 2H, J = 7.8 Hz), 7.58-7.68 (m, 2H), 7.83-7.86 (m, 3H), 8.02-8.08 (m, 2H), 8.68 (s, 1H), 9.02 (s, 1H), 10.18 (s, 1H), and 11.00 (s, 1H).

### EXAMPLE 155

### Quinoline-4-carboxylic acid 2-(phenylsulfonyl)hydrazide

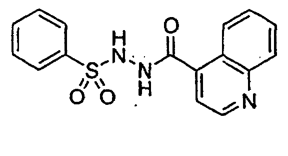

### Step 1. The preparation of quinoline-4-carboxylic acid hydrazide

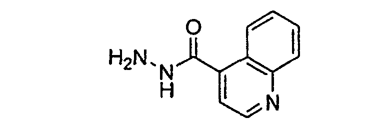

Quinoline-4-carboxylic acid (0.75 g, 4.33 mmol) was dissolved in THF (18 mL). N-Methyl morpholine (1.05 mL, 9.52 mmol) was added, and the reaction was cooled to 0°C. Isobutyl chloroformate (0.62 mL, 4.76 mmol) was added, and the reaction was allowed to stir for 20 minutes. Hydrazine (2.72 mL, 86.6 mmol) was added slowly to the reaction, and the reaction was allowed to warm to room temperature. The reaction stirred for 1.5 hours. The solution was diluted with EtOAc (100 mL) and then washed with saturated sodium bicarbonate and brine, dried over Na₂SO₄, and concentrated. The precipitate was carried on to the next reaction.
MS: 187.9 (M+1 for C₁₀H₉N₃O). ¹H NMR (400 MHz, DMSO) δ 4.64 (s, 2H), 7.45-7.48 (m, 1H), 7.60-7.65 (m, 1H), 7.75-7.79 (m, 1H), 8.01-8.15 (m, 2H), 8.90-8.93 (m, 1H), and 9.89 (s, 1H).

### Step 2. The preparation of quinoline-4-carboxylic acid 2-(phenylsulfonyl)-hydrazide:

Quinoline-4-carboxylic acid hydrazide (0.6 g, 3.25 mmol) was dissolved in THF (36 mL). N,N-diisopropylethylamine (2.26 mL, 13.0 mmol) was added, and the reaction was cooled to 0°C. Phenylsulfonyl chloride (0.41 mL, 3.25 mmol) was added followed by DMAP (5 mg, 0.04 mmol). The reaction was allowed to warm to room temperature. After 2.5 hours of stirring, the red solution was diluted with EtOAc (150 mL). It was then washed with saturated sodium bicarbonate and brine and dried over Na₂SO₄. The reaction was then concentrated down and purified by flash chromotagraphy eluting with 1:1 hexane/EtOAc. The desired product was isolated (0.05 g, 18.8%).
MS: 328.0 (M+1 for C₁₆H₁₃N₃O₃S). TLC: SiO₂, R_{f}= 0.10 (1:1 hexane/EtOAc); mp: 153-156°C; HRMS: (calc) 328.0756, (found) 328.0753 (M+1 for C₁₆H₁₃N₃O₃S) HPLC: (50% H₂O/50% CH₃CN/0.1% TFA) Ret. Time: 2.284, Purity: 100.0%. IR (KBr, cm⁻¹) 3199, 2963, 1745, 1665, 1168. ¹H NMR (400 MHz, DMSO) δ 7.43 (d, 1H, J = 4.2 Hz), 7.49-7.80 (m, 8H), 7.91 (d, 1H, J = 7.1 Hz), 8.01-8.07 (m, 1H), 8.94 (d, 1H, J = 3.9 Hz), 10.36 (s, 1H), 10.93 (s, 1H).

Additional compounds that can be prepared according to the scheme and examples set forth above:
1. Dibenzofuran-2-carboxylic acid 2-( 4-fluoro-3-methylphenylsulfonyl)-hydrazide
2. 7-Methoxy-1*H*-indole-2-carboxylic acid, 2-(phenylsulfonyl)hydrazide
3. 7-Chloro-1*H*-indole-2-carboxylic acid, 2-(phenylsulfonyl)hydrazide
4. 5-Methoxy-1*H*-indole-2-carboxylic acid, 2-(phenylsulfonyl)hydrazide
5. 5-Chloro-1*H*-indole-2-carboxylic acid, 2-(phenylsulfonyl)hydrazide
6. 5-Chloro-1*H*-indole-2-carboxylic acid, 2-[(2-trifluoromethyl)-phenylsulfonyl]hydrazide
7. 5-Methyl-1*H*-indole-2-carboxylic acid, 2-(phenylsulfonyl)ohydrazide
8. 5-Methyl-1*H*-indole-2-carboxylic acid, 2-[(2-trifluoromethyl)-phenylsulfonyl]hydrazide

### EXAMPLE 156

### 2-Indolecarboxylic acid, [(5-N, N-dimethylamino)-, 2-phenylsulfonyl] hydrazide hydrochloride salt

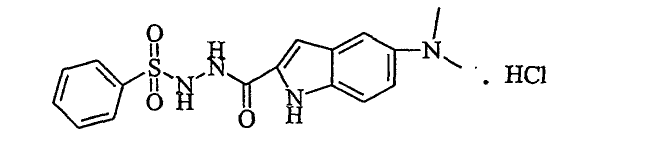

### Synthesis of 2-indolecarboxylic acid, [(5-N,N-dimethylamino),

2-phenylsulfonyl] hydrazide hydrochloride salt: Example 156 was synthesized in accordance with the methods of example 167 except that 2-indolecarboxylic acid, 5-amino-,2-phenylsulfonyl hydrazide was used instead of dibenzofurancarboxylic acid, 2-amino-, 2-(phenylsulfonyl)hydrazide (yield 94%).
MS: 359.1 ( M+1 for C₁₇H₁₈N₄O₃S); mp: 251.6-252.7°C; HPLC (C18 column, 1:1 MeCN / H₂O = 0.1%TFA) 82.4%, RT=2.2 min; ¹HNMR(DMSO-D₆) δ 2.46(S, 6H), 7.51(t, 2H, J=7.8Hz), 7.62(t, 1H, J=7.2Hz), 7.68(S, 1H), 7.70-7.90(m, 4H), 8.09(S, 1H), 10.23(S, 1H), 11.03(S, 1H)

### EXAMPLE 157

### 2-Benzofurancarboxylic acid, 5-(phenylsulfonylamino)-, 2-(phenylsulfonyl) hydrazide Step 1. 2-Benzofurancarboxylic acid, 5-amino-, 2-(phenylsulfonyl)hydrazide

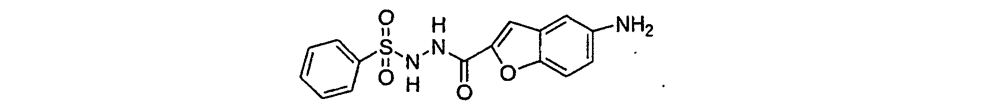

This was prepared in accordance with the following method.
2-Benzofurancarboxylic acid, 5-nitro-, 2-(phenylsulfonyl)hydrazide (0.82 g) was dissolved in 50 mL of THF:EtOH (1:1), treated with wet Raney Ni (0.6 g) and hydrogenated at 50 Psi for 15 hours. The solvent was removed to give 0.73 g (97%) of the desired product as a white solid.

### Step 2. 2-Benzofurancarboxylic acid, 5-(phenylsulfonylamino)-, 2-(phenylsulfonyl)hydrazide

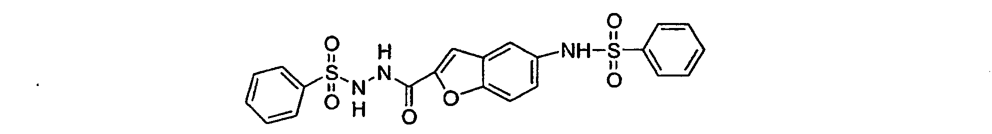

2-Benzofurancarboxylic acid, 5-amino-, 2-(phenylsulfonyl)hydrazide (0.117 g, 0.35 mmol) was dissolved in 7 mL of dry pyridine, then benzenesulfonyl chloride (0.03 mL, 0.39 mmol) was added, the reaction was stirred at RT overnight. The solvent was removed and recrystallized from ethyl acetate and hexane to give 0.155 g (94%) of the desired product as a white solid.
MS: 472.0 (M+1 for C₂₁H₁₇N₃O₆S₂); mp: 261.5-261.8°C; HPLC (C18 column, 1:1 MeCN/H₂O = 0.1%TFA) 96.5%, RT = 6.0 min.; ¹HNMR(DMSO-D₆) δ 7.10 (d, 1H, J = 6.8Hz), 7.41(S, 1H), 7.40-7.60 (m, 8H), 7.67 (d, 2H, J = 7.5 Hz), 7.79 (d, 2H, J=7.3Hz), 10.15(s, 1H), 10.25(s, 1H), 10.89(s, 1H).

### EXAMPLE 158

### 2-Benzofurancarboxylic acid, 5-(methylsulfonylamino)-, 2-(phenylsulfonyl) hydrazide

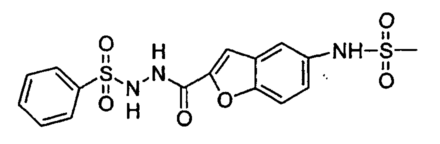

Synthesis of 2-benzofurancarboxylic acid, 5-(methylsulfonylamino)-, 2-(phenylsulfonyl)hydrazide: Example 158 was synthesized in accordance with the methods of Example 157 except that methylsulfonyl chloride was used instead of benzenesulfonyl chloride(yield 92%).
MS: 410.0 (M+1 for C₁₆H₁₅N₃O₆S₂); mp: 255.4-256.1°C; HPLC (C18 column, 1:1 MeCN / H₂O = 0.1%TFA) 90.0%, RT = 3.3 min.; ¹HNMR(DMSO-D₆) δ 2.92 (S, 3H), 7.26 (d, 1H, J = 6.8Hz), 7.50-7.60 (m, 6H), 7.81 (d, 1H, J = 7.6 Hz), 9.60 (s, 1H), 9.70 (s, 1H), 10.18 (s, 1H), 10.93 (s, 1H).

### EXAMPLE 159

### 2-Dibenzofurancarboxylic acid, 2-(4-nitrophenyl)sulfonyl hydrazide

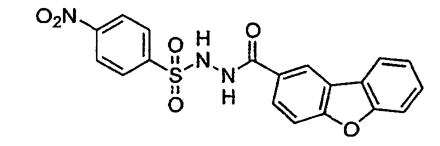

Synthesis of 2-dibenzofurancarboxylic acid, 2-(4-nitrophenyl)sulfonyl hydrazide: Example 159 was synthesized in accordance with the methods of Example 92 except that 4-nitrobenzenesulfonyl chloride was used instead of 2-fluorobenzenesulfonyl chloride(yield 93%).
MS: 412.0 (M+1 for C₁₉H₁₃N₃O₆S): mp: 239.3-239.9°C; HPLC (C18 column, 1:1 MeCN/H₂O + 0.1% TFA) 98.5%, RT = 14.52 min; ¹HNMR(DMSO-D₆) δ 7.40 (t, 1H, J = 8.30 Hz), 7.53 (t, 1H, J = 7.33 Hz), 7.73 (m, 2H), 7.85 (dd, 1H, J = 8.55 and 1.71 Hz), 8.11-8.17 (m, 3H) 8.36 (d, 2H, J = 5.86 Hz), 8.50 (s, 1H), 10.55 (s, 1H), 10.89 (s, 1H).

### EXAMPLE 160

### 2-Benzofurancarboxylic acid, 5-(4-methoxyphenylsulfonylamino)-, 2-(phenylsulfonyl)hydrazide

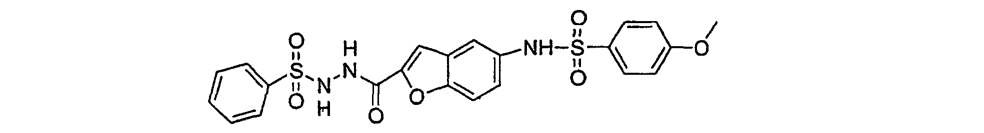

Synthesis of 2-benzofurancarboxylic acid, 5-(4-methoxyphenylsulfonylaminoamino)-, 2-(phenylsulfonyl)hydrazide: Example 160 was synthesized in accordance with the methods of Example 157 except that 4-methoxybenzenesulfonyl chloride was used instead of benzenesulfonyl chloride(yield 93%).
MS: 502.0 (M+1 for C₂₂H₁₉N₃O₇S₂); mp: 213-214°C; HPLC (C18 column, 1:1 MeCN/H₂O = 0.1% TFA) 96.0%, RT = 4.8 min., ¹HNMR (DMSO-D₆) δ 3.72 (s, 3H), 6.96 (d, 2H, J = 8.8 Hz), 7.14 (d, 1H, J = 7.1 Hz), 7.40-7.7 (m, 4H), 7.78 (d, 2H, J = 7.5 Hz), 7.98 (t, 2H, J = 6.8 Hz), 8.48 (t, 1H, J = 7.8 Hz), 8.88 (d, 1H, J = 5.1 Hz), 10.18 (s, 1H), 10.21 (s, 1H), 10.92 (s, 1H).

### EXAMPLE 161

### 2-Benzofurancarboxylic acid, 5-(4-methylphenylsulfonylamino)-, 2-(phenylsulfonyl) hydrazide

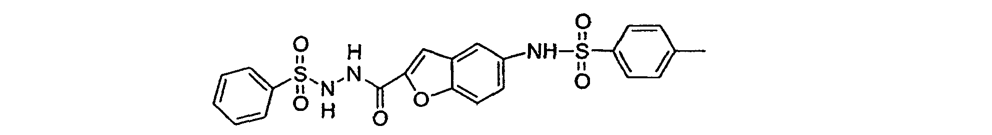

Synthesis of 2-benzofurancarboxylic acid, 5-(4-methylphenylsulfonylamino)-, 2-(phenylsulfonyl) hydrazide: Example 161 was synthesized in accordance with the methods of Example 157 except that 4-methylbenzenesulfonyl chloride was used instead of benzenesulfonyl chloride(yield 91%).
MS: 486.0 (M+1 for C₂₂H₁₉N₃O₆S₂); mp: 181-183°C; HPLC (C18 column, 1:1 MeCN/H₂O = 0.1%TFA) 93.0%, RT = 5.5 min.; ¹HNMR(DMSO-D₆) δ 2.26 (S, 3H), 7.07 (d, 1H, J = 7.8 Hz), 7.25 (d, 2H, J = 8.1 Hz), 7.40-7.60 (m, 4H), 7.80 (d, 2H, J = 7.6 Hz), 7.99 (t, 2H, J = 6.8 Hz), 8.48 (t, 1H, J = 7.8 Hz), 8.88 (d, 1H, J = 5.1 Hz), 10.18 (s, 1H), 10.27 (s, 1H), 10.91 (s, 1H).

### EXAMPLE 162

### 2-Dibenzofurancarboxylic acid, 2-[(2-nitrophenyl)sulfonyl] hydrazide

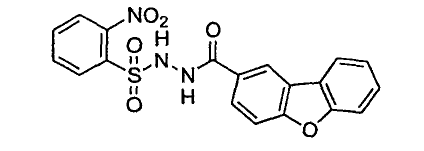

Synthesis of 2-dibenzofurancarboxylic acid, 2-[(2-nitrophenyl) sulfonyl]hydrazide: Example 162 was synthesized in accordance with the methods of Example 92 except that 2-nitrobenzenesulfonyl chloride was used instead of 2-fluorobenzenesulfonyl chloride(yield 93%).
MS: 412.0 (M+1 for C₁₉H₁₃N₃O₆S): mp: 223.4-224.1°C; HPLC (C18 column, 1:1 MeCN/H₂O + 0.1% TFA) 93.2%, RT = 9.8 min; ¹HNMR (CDCl₃) δ 7.42 (t, 1H,

J = 8.30 Hz), 7.48 (t, 1H, J = 7.33 Hz), 7.65-7.95 (m, 6H), 8.10 (d, 1H, J = 5.86 Hz), 8.19 (d, 1H, J = 1.47 Hz), 10.38 (s, 1H), 10.94 (s, 1H).

### EXAMPLE 163

### 2-Benzofurancarboxylic acid, 5-(4-pentylphenylsulfonylamino)-, 2-(phenylsulfonyl)hydrazide

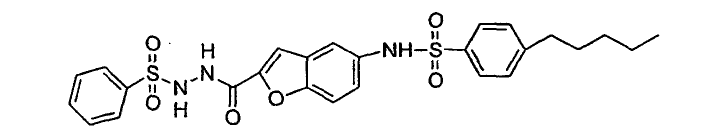

Synthesis of 2-benzofurancarboxylic acid, 5-(4-pentylphenylsulfonyl amino)-, 2-(phenylsulfonyl)hydrazide: Example 163 was synthesized in accordance with the methods of Example 157 except that 4-pentylbenzenesulfonyl chloride was used instead of benzenesulfonyl chloride(yield 97%).
MS: 541.0 (M+1 for C₂₆H₂₇N₃O₆S₂); mp: 169.0-169.8°C; HPLC (C18 column, 1:1 MeCN/H₂O = 0.1% TFA) 93.2%, RT = 16.9 min.; ¹HNMR (DMSO-D₆) δ 0.78 (t, 3H, J = 6.9 Hz), 1.18 (m, 4H), 1.42 (m, 2H), 2.50 (m, 2H), 7.13 (d, 1H, J = 8.3 Hz), 7.27 (d, 2H, J = 8.1 Hz), 7.40-7.60 (m, 4H), 7.80 (d, 2H, J = 7.6 Hz), 8.01 (t, 2H, J = 6.6 Hz), 8.52 (t, 1H, J = 7.8 Hz), 8.89 (d, 1H, J = 5.1 Hz), 10.17 (s, 1H), 10.27 (s, 1H), 10.91 (s, 1H).

### EXAMPLE 164

### 2-Benzofurancarboxylic acid, 5-(N-isopropylamino)-, 2-(phenylsulfonyl) hydrazide

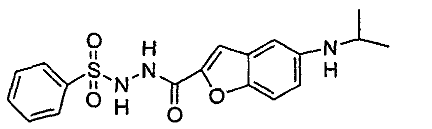

2-Benzofurancarboxylic acid, 5-(N-isopropylamino)-, 2-(phenylsulfonyl) hydrazide was prepared in accordance with the following method: 2-Benzofurancarboxylic acid, 5-amino-, 2-(phenylsulfonyl)hydrazide (0.47 g) was dissolved in 20 mL of EtOH, treated with 20% Pd/C (50 mg), 3 mL of acetone and hydrogenated at 50-38 Psi for 15 hours. The solvent was removed to give 0.49 g (93%) of the desired product as a white solid.
MS: 374.1 (M+1 for C₁₈H₁₉N₃O₄S); mp: 94.7-95.5°C; HPLC (C18 column, 1:1 MeCN/H₂O = 0.1% TFA) 99.9%, RT = 1.7 min.; ¹HNMR (DMSO-D₆) δ 1.09 (d, 6H, J = 6.4 Hz), 3.45 (m, 1H), 6.65 (S, 1H), 6.77 (d, 1H, J = 6.8 Hz), 7.29 (d, 1H, J = 8.7 Hz), 7.33 (S, 1H), 7.53 (t, 2H, J = 7.6 Hz), 7.61 (t, 1H, J = 7.4 Hz), 7.81 (d, 2H, J = 7.6 Hz), 10.08 (s, 1H), 10.09 (s, 1H), 10.75 (s, 1H).

### EXAMPLE 165

### 2-Benzofurancarboxylic acid, 5-(4-propyllphenylsulfonylamino)-, 2-(phenylsulfonyl) hydrazide

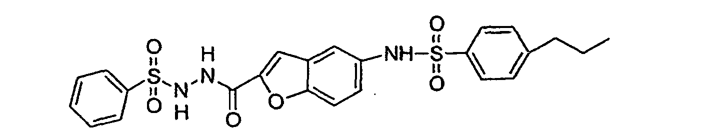

Synthesis of 2-benzofurancarboxylic acid, 5-(4-propylphenylsulfonylamino)-, 2-(phenylsulfonyl) hydrazide: Example 165 was synthesized in accordance with the methods of Example 157 except that 4-propylbenzenesulfonyl chloride was used instead of benzenesulfonyl chloride(yield 97%).
MS: 514.1 (M+1 for C₂₄H₂₃N₃O₆S₂); mp: 160.4-161.1°C; HPLC (C18 column, 1:1 MeCN/H₂O = 0.1% TFA) 93.5%, RT = 9.3 min.; ¹HNMR (DMSO-D₆) δ 0.80 (m, 3H), 1.56 (m, 2H), 2.50 (m, 2H), 7.12 (m, 1H), 7.27 (d, 2H, J = 8.1 Hz), 7.40-7.60 (m, 4H), 7.80 (d, 2H, J = 7.6 Hz), 8.01 (t, 2H, J = 6.6 Hz), 8.52 (t, 1H, J = 7.8 Hz), 8.89 (d, 1H, J = 5.1 Hz), 10.17 (s, 1H), 10.25 (s, 1H), 10.90 (s, 1H).

### EXAMPLE 166

### 2-Dibenzofurancarboxylic acid, 2-[(2-aminophenyl)sulfonyl]hydrazide

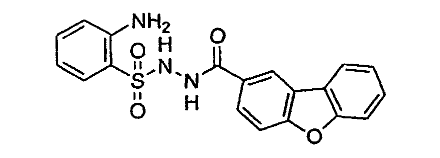

Synthesis of 2-dibenzofurancarboxylic acid, 2-[(2-aminophenyl)sulfonyl] hydrazide: Example 166 was synthesized in accordance with the methods of Example 157, Step 1, except that 2-dibenzofurancarboxylic acid, 2-[(2-nitrophenyl)sulfonyl]hydrazide was used instead of 2-benzofurancarboxylic acid, 5-nitro-, 2-(phenylsulfonyl)hydrazide(yield 94%).
MS: 382.0 (M+1 for C₁₉H₁₅N₃O₄S); mp: 217.9-218.4°C; HPLC (C18 column, 1:1 MeCN/H₂O = 0.1% TFA) 98.9%, RT = 6.7 min.; ¹HNMR (DMSO-d₆) δ 6.15 (S, 2H), 6.50 (t, 1H, J = 8.20 Hz), 6.76 (t, 1H, J = 9.0 Hz), 7.21 (t, 1H, J = 8.30 Hz), 7.40-7.60 (m, 3H), 7.72 (t, 1H, J = 8.30 Hz), 7.85 (t, 1H, J = 7.1 Hz), 8.10 (d, 1H, J = 7.4 Hz), 8.52 (s, 1H), 9.75 (s, 1H), 10.68 (s, 1H).

### EXAMPLE 167

### 2-Dibenzofurancarboxylic acid, 2-[(2-N,N-dimethylaminophenyl)sulfonyl]hydrazide hydrochloride salt

### Step 1: 2-Dibenzofurancarboxylic acid, 2-[(N,N-dimethyl-2-aminophenyl)sulfonyl] hydrazide

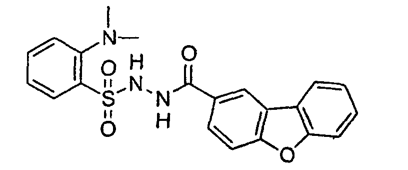

Synthesis of 2-dibenzofurancarboxylic acid, 2-[(2-N,N-dimethylaminophenyl)-sulfonyl]hydrazide: This was prepared in accordance with the following method: 2-Benzofurancarboxylic acid, 2-amino-, 2-(phenylsulfonyl)hydrazide (0.1 g) was dissolved in 4 mL of EtOH, treated with 20% Pd/C (20 mg), 1 mL of formaldehyde and hydrogenated at 50-38 Psi for 16 hours. The solvent was removed to give 0.096 g (89%) of the desired product as a oily liquid.

### Step 2: 2-Dibenzofurancarboxylic acid, 2-[(2-N,N-dimethylaminophenyl)sulfonyl] hydrazide hydrochloride salt.

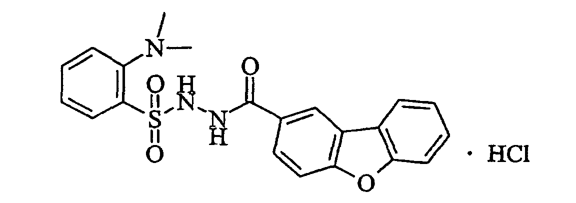

This was prepared in accordance with the following method: 2-Dibenzofurancarboxylic acid, 2-[(2-N,N-dimethylaminophenyl)sulfonyl]hydrazide was dissolved in 5 mL of MeOH, then 2 mL of 1 M HCl (ether) was added slowly and the mixture was stirred at RT for 10 minutes, the solvent was removed to yield a white solid as the desired product.
MS: 410.1 (M+1 for C₂₁H₁₉N₃O₄S); mp: 103.2-103.9; HPLC (C18 column, 1:1 MeCN/H₂O = 0.1% TFA) 81.2%, RT = 8.1 min.; ¹HNMR (DMSO-d₆) δ 2.97 (S, 6H), 6.68 (d, 1H, J = 8.8 Hz), 7.40 (t, 1H, J = 6.42 Hz), 7.50-7.80 (m, 6H), 7.84 (d, 1H, J = 8.30 Hz), 8.17 (d, 1H, J = 6.1 Hz), 8.49 (S, 1H), 10.41 (S, 1H), 10.68 (S, 1H).

### EXAMPLE 168

### 2-Benzofurancarboxylic acid, 5-(N,N-dimethylamino)-, 2-(phenylsulfonyl) hydrazide . hydrochloride salt

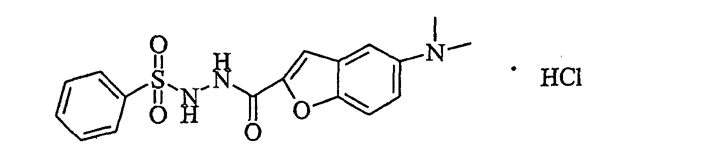

2-Benzofurancarboxylic acid, 5-(N,N-dimethylamino)-, 2-(phenylsulfonyl) hydrazide hydrochloride salt: Example 168 was synthesized in accordance with the methods of Example 167 except that 2-benzofurancarboxylic acid, 5-amino-, 2-(phenylsulfonyl)hydrazide was used instead of 2-dibenzofurancarboxylic acid, 2-[(2-N,N-dimethylaminophenyl) sulfonyl] hydrazide (yield 89%).
MS: 360.1 (M+1 for C₁₇H₁₇N₃O₄S); mp: 218.3-218.9°C; HPLC (C18 column, 1:1 MeCN/H₂O = 0.1% TFA) 99.7%, RT = 1.6 min.; ¹HNMR (DMSO-D₆) δ 2.46 (S, 6H), 7.51 (t, 2H, J = 7.8 Hz), 7.62 (t, 1H, J = 7.2 Hz), 7.68 (S, 1H), 7.70-7.90 (m, 4H), 8.09 (s, 1H), 10.23 (s, 1H), 11.03 (s, 1H).

### EXAMPLE 169

### 2-Indolecarboxylic acid, 5-chloro-2-[(2-(trifluoromethyl)phenyl]sulfonyl hydrazide

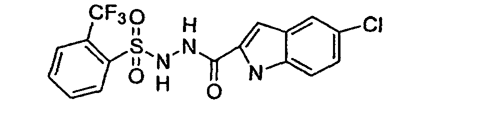

### Step 1. The preparation of 5-chloroindole-2-carboxylic acid hydrazide

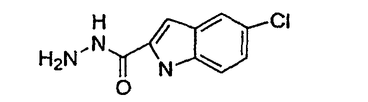

This was synthesized in accordance with the methods of Example 152 Step 1 except that 5-chloroindole-2-carboxylic acid was used instead of benzofuran-2-carboxylic acid (yield 94%).
Step 2. Synthesis of 2-Indolecarboxylic acid, 5-chloro-2-[(2-(trifluoromethyl)-phenyl]sulfonyl hydrazide was run in accordance with Example 51 except that 5-chloroindole-2-carboxylic acid hydrazide was used instead of 5-chloro-2-benzofurancarboxylic acid hydrazide. (86% yield).
MS: 418.0 (M+1 for C₁₆H₁₁N₃O₃SF₃Cl): mp: 66.8-67.9°C; HPLC (C18 column, 1:1 MeCN/H₂O + 0.1% TFA) 91.2%, RT = 1.49 min; ¹HNMR (DMSO-d₆), 7.16 (S, 1H), 7.60-7.90 (m, 3H), 8.03 (t, 2H, J = 6.7 Hz), 8.56 (t, 1H, J = 7.8 Hz), 8.90 (d, 1H, J = 5.2 Hz), 10.08 (s, 1H), 10.89 (s, 1H) ,11.83 (s, 1H).

### EXAMPLE 170

### 2-Indolecarboxylic acid, 5-nitro-2-phenylsulfonyl hydrazide

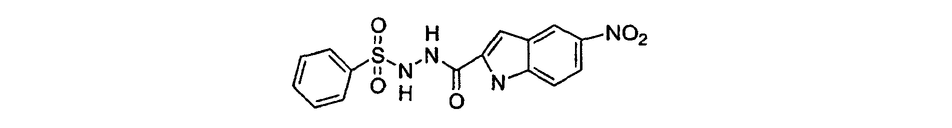

### Step 1. The preparation of 5-nitroindole-2-carboxylic acid hydrazide

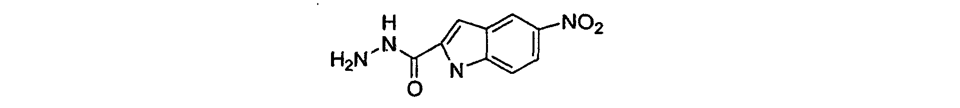

This was synthesized in accordance with the methods of Example 152, Step 1 except that 5-nitroindole-2-carboxylic acid was used instead of benzofuran-2-carboxylic acid (yield 92%).
Step 2. Synthesis of 2-indolecarboxylic acid, 5-nitro-2-phenylsulfonyl hydrazide was run in accordance with Example 51 except that 5-nitroindole-2-carboxylic acid hydrazide was used instead of 5-chloro-2-benzofurancarboxylic acid hydrazide and phenylsulfonyl chloride was used instead of 2-trifluoromethyl phenylsulfonyl chloride (86% yield).
MS: 361.1 (M+1 for C₁₅H₁₂N₄O₅S1): mp: 268.2-269.1°C; HPLC (C18 column, 1:1 MeCN/H₂O + 0.1 %TFA) 89.2%, RT = 4.6 min; ¹HNMR (DMSO-d₆), 7.41 (S, 1H), 7.50-7.70 (m, 4H), 7.95 (d, 2H, J = 7.1 Hz), 8.12 (dd, 1H, J = 2.6 and 6.8 Hz), 8.09 (s, 1H), 10.18 (s, 1H), 10.96 (s, 1H), and 12.29 (s, 1H).

### EXAMPLE 171

### 2-Indolecarboxylic acid, 5-chloro-2-phenylsulfonyl hydrazide

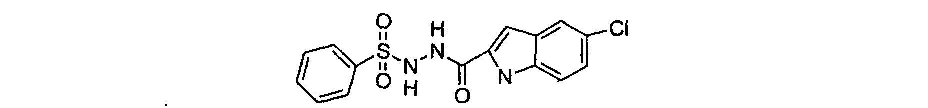

### Step 1. The preparation of 5-chloroindole-2-carboxylic acid hydrazide

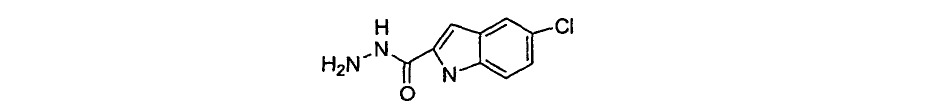

This was synthesized in accordance with the methods of Example 152, Step 1 except that 5-chloroindole-2-carboxylic acid was used instead of benzofuran-2-carboxylic acid (yield 90%).
Step 2. Synthesis of 2-Indolecarboxylic acid, 5-chloro-2-phenylsulfonyl hydrazide was run in accordance with Example 51 except that 5-chloroindole-2-carboxylic acid hydrazide was used instead of 5-chloro-2-benzofurancarboxylic acid hydrazide and phenylsulfonyl chloride was used instead of 2-trifluoromethyl- phenylsulfonyl chloride (89% yield).
MS: 350.1 (M+1 for C₁₅H₁₂ClN₃O₃S): mp: 79.1-79.9°C; HPLC (C18 column, 1:1 MeCN/H₂O + 0.1% TFA) 93.8%, RT = 6.1 min; ¹HNMR (DMSO-d₆) 7.16 (S, 1H), 7.40-7.60 (m, 3H), 7.80 (d, 1H, J = 7.6 Hz), 8.01 (t, 2H, J = 5.1 Hz), 10.18 (s, 1H), 8.90 (d, 1H, J = 5.2 Hz), 10.79 (s, 1H), 10.14 (s, 1H) and 11.82 (s, 1H).

### EXAMPLE 172

*In vitro enzyme assay* - human Branched Chain Amino Acid Aminotransferase cytosolic form (hBCATc) and mitochondrial form (hBCATm) as well as rat BCAT cytosolic form were assayed at 37°C in 25 mM phosphate buffer pH 7.8. In addition, 2 mM DTT and 12.5 mM EDTA were added to the assay mixture. A coupling enzyme assay was used to monitor the production of glutamate. The formation of NADH from NAD+ at 340 nM was followed on a 96-well plate Molecular Devices plate reader. The following components were used in this coupled assay: 4 mM ADP, 1 mM NAD+, 750 µM L-Leucine, 500 µM α-ketoglutarate, 10 µM pyridoxal phosphate, 1 unit glutamate dehydrogenase, and 1.25 µg of the appropriate BCAT enzyme. Inhibitions by compounds were assayed by adding various concentrations to this coupled assay procedure as a DMSO stock up to a 5% v/v DMSO/buffer ratio.

*Enzyme Preparation-* humanBCATc and ratBCATc were expressed in BL21(DE3) cells. A growing culture of cells was induced with 1 mM IPTG at room temperature overnight. These cells were harvested by centrifugation at 10,000 g for 20 minutes. The supernatant was discarded, and the cells were stored at -80°C until needed. The protein was purified by the following method. The cell paste was resuspended in extraction buffer (0.1 M phosphate, pH 8.0, with 0.01 M Tris-HCL and 5 mM TCEP and then lysed on a French Press at 1000 psi. The lysate was centrifuged at 10,000 g for 15 minutes, and the pellet was discarded. The supernatant was loaded onto a Hitrap Chelating column previously charged with 0.1 M NiSO₄ and washed with 3 to 4 column volumes of extraction buffer. The column was then washed with 0.01 Tris, pH 7.5, 10% glycerol, 150 mM NaCl, 5 mM TCEP for two column volumes. The column was then further washed with two column volumes of 0.1 M phosphate, pH 6.0, 0.01 M Tris, 10% glycerol and 750 mM NaCl. Finally, the column was washed with two column volumes of the same buffer plus 50 mM imidazole. The BCAT enzyme was then eluted with the same buffer but with 350 mM imidazole. The eluant was then dialyzed overnight against 10 mM phosphate buffer pH 8.0, 10 % glycerol, and 5 mM TCEP. The dialyzed protein was then loaded onto a Q-sepharose column and eluted using a salt gradient. Active fractions were collected and further purified on a Superose-12 column to yield pure BCAT protein.

**TABLE 1**

| In-Vitro Data | | |
|---|---|---|
| Example | RrBCATc (µM) | RhBCATc (µM) |
| 1 | 0.18 | 2.9, 1.4 |
| 2 | 0.13 | 2.6 |
| 3 | 2.5 | 17.8 |
| 4 | 1.22 | 12.9 |
| 5 | 0.86, 0.46, 0.53 | 5.76, 3.0 |
| 6 | 0.66 | 12.9 |
| 7 | 0.59 | 11.5 |
| 8 | 0.3 | 3.0 |
| 9 | 2.18, 3.3 | 17.9, 29 |
| 10 | 2.9 | 65.6 |
| 11 | -- | -- |
| 12 | -- | 90.5 |
| 13 | 0.39 | 15.7 |
| 14 | 0.37,0.14 | 2.2, 1.4 |
| 15 | 0.65,0.22 | 3.6, 3.3 |
| 16 | 0.39,0.20 | 3.0,3.1 |
| 17 | 1.1, 0.54 | 11.4 |
| 18 | 0.53,0.4 | 7.9 |
| 19 | 0.22 | 6.7 |
| 20 | 0.39 | 15.7 |
| 21 | - | >100 |
| 22 | 4.1 | 24.6 |
| 23 | 5.4 | 17.80 |
| 24 | 1.20 | 24 |
| 25 | 1.8 | 21.9 |
| 26 | 15.20,6.90 | 138.0 |
| 27 | 45.2 | 171 |
| 28 | -- | >100 |
| 29 | 0.29 | 10.1 |
| 30 | 5.7 | 25 |
| 31 | 0.81 | 2.60,4.30 |
| 32 | >100 | >100 |
| 33 | 3.3 | 12.2 |
| 34 | 0.33 | 5.1 |
| 35 | 0.31 | 3.57 |
| 36 | 1.1 | 17.5, 18.20 |
| 37 | 3.5 | 41.3 |
| 38 | 0.77,0.61 | 29.9 |
| 39 | 0.21 | 2.29 |
| 40 | 1.00 | 4.86, 8.80 |
| 41 | 14.5 | 28.8 |
| 42 | 1.3 | 5.3, 10.40 |
| 43 | 0.47 | 1.8 |
| 44 | - | 3.44 |
| 45 | 0.21 | 2.29 |
| 46 | - | 1.08,0.7 |
| 47 | 0.15, 0.2 | 0.84, 1.47 |
| 48 | 0.91 | 4.21, 7.60 |
| 49 | 3.1,4.0 | 1.75, 3.60 |
| 50 | 1.3 | 5.18, 9.10 |
| 51 | 0.072,0.14 | 0.79,0.82 |
| 52 | 0.3 | 2.19 |
| 53 | | |
| 54 | - | 3.76 |
| 55 | - | 3.53,3.3 |
| 56 | <0.50 | 7.26 |
| 57 | 0.14 | 1.14 |
| 58 | 0.22 | 3.57 |
| 59 | 0.12 | 3.42 |
| 60 | 0.11 | 21.10, 12.8 |
| 61 | 0.1 | 1.3 |
| 62 | 0.33 | 4.77 |
| 63 | 5.29 | 66.3 |
| 64 | 0.19 | 3.31 |
| 65 | 0.1 | 3.23 |
| 66 | 0.15 | 1.38 |
| 67 | 2.4 | 17.3 |
| 68 | 0.31 | 2.78 |
| 69 | 0.05 | 3.86 |
| 70 | 0.43 | 11.62 |
| 71 | 0.88 | 6.57 |
| 72 | 1.86 | >10, 5.35 |
| 73 | 1.89 | 14.42 |
| 74 | 3.71 | 64.5 |
| 75 | 0.88 | >10, 5.4 |
| 76 | 1.51 | 11.24 |
| 77 | 0.35 | 6.15,3.0 |
| 78 | | 3.70 |
| 79 | 0.22 | 16.9, 1.81 |
| 80 | 0.25 | 2.62 |
| 81 | 0.31 | 1.74 |
| 82 | 0.51 | 3.28 |
| 83 | 0.87 | 9.66 |
| 84 | 0.97 | 6.13 |
| 85 | 1.39 | 21.18 |
| 86 | 0.24 | 15.2 |
| 87 | 1.16 | 41.07 |
| 88 | 4 | 40 |
| 89 | >100 | >100 |
| 90 | >100 | >100 |
| 91 | 0.11 | 19.6 |
| 92 | 1.1 | 12.1 |
| 93 | 5.9 | 63.5 |
| 94 | 0.72 | 5.5 |
| 95 | 0.3 | 2.9 |
| 96 | 36.3 | 51.6 |
| 97 | 36.4 | 67.5 |
| 98 | 0.65 | 2.8 |
| 99 | 35.1 | 46.3 |
| 100 | 4.0 | 28.1 |
| 101 | 22 | 48.1 |
| 102 | 0.37 | 6.0 |
| 103 | 0.85 | 12 |
| 104 | 1.4 | 23 |
| 105 | 3.3 | 54 |
| 106 | 5.6 | 42 |
| 107 | 7.5 | 41 |
| 108 | 5.6 | 57 |
| 109 | 0.46 | 9.8 |
| 110 | 0.72 | 11.1 |
| 111 | 0.47 | 0.47, 2.8 |
| 112 | 6.2 | 14.6 |
| 113 | 0.53 | 4.1 |
| 114 | 26.5 | 59.7 |
| 115 | 5.2 | 59.9 |
| 116 | 1.2 | 8.2 |
| 117 | 0.82 | 5.7 |
| 118 | 1.8 | 25.0 |
| 119 | 2.2 | 12.7 |
| 120 | 2.0 | 18.1 |
| 121 | 11.6 | 55.0 |
| 122 | 56.7 | 58.0 |
| 123 | 2.2 | 11.6 |
| 124 | 100 | 53.1 |
| 125 | 2.4 | 18.4 |
| 126 | 12.5 | 36.2 |
| 127 | | 16.8 |
| 128 | | 12 |
| 129 | | 13 |
| 130 | | 22 |
| 131 | | 61 |
| 132 | | 40 |
| 133 | | >100 |
| 134 | | 17 |
| 135 | 2.8 | 13 |
| 136 | | 40 |
| 137 | 14 | 41 |
| 138 | | >100 |
| 139 | | 41 |
| 140 | | >100 |
| 141 | | >100 |
| 142 | | 46 |
| 143 | | 80 |
| 144 | | 29 |
| 145 | | 80 |
| 146 | 2.5 | 17.8 |
| 147 | - | 12.9 |
| 148 | 0.59 | 11.5 |
| 149 | 3.3 | 17.9 |
| 150 | | |
| 151 | 0.3 | 3 |
| 152 | - | >100 |
| 153 | 1.9 | 15.3 |
| 154 | 3.6 | 24.2 |
| 155 | -- | >100 |
| 156 | | |
| 157 | | 8.7 |
| 158 | | 26 |
| 159 | 4 | 28 |
| 160 | | |
| 161 | | |
| 162 | | |
| 163 | | |
| 164 | | |
| 165 | | |
| 166 | | |
| 167 | | |
| 168 | | |
| 169 | | |
| 170 | | |
| 171 | | |

## Claims

1. A compound of Formula I 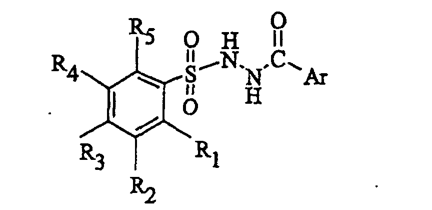 wherein:
R₃ is H, or F, Br, alkyl, carboxy, alkoxy, substituted alkoxy,
R₁, R₂, R₄, and R₅ are independently, H, halogen, alkyl substituted alkyl, alkoxy, substituted alkoxy, cyano, nitro, amino, alkylamine, and thioalkyl, Ar is bicyclic heteroaryl, tricyclic heteroaryl, substituted bicyclic heteroaryl, except (6-methoxybenzofuran)-2-nyl, 3-quinolinyl;
Ar being selected from the group consisting of: 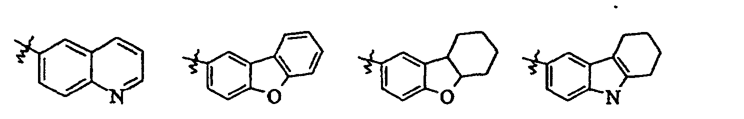 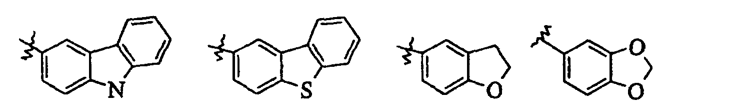 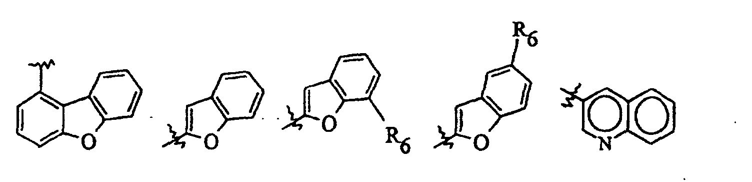 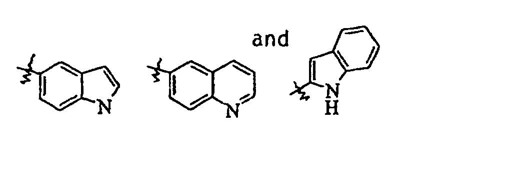
wherein R₆ is alkoxy, substituted alkoxy, halogen, aryloxy, substituted aryloxy, alkylamino, substituted alkylamino, arylalkylamino, substituted arylalkylamino, amide or substituted amide;
or a pharmaceutically acceptable salt, ester, or amide thereof.

2. A compound according to Claim 1 wherein R₁, R₂, R₃, R₄, and R₅ are hydrogen.

3. A compound according to Claim 1 wherein Ar is a tricyclic heteroaryl.

4. A compound according to Claim 1 wherein Ar is a bicyclic heteroaryl.

5. A compound according Claim 1 wherein the compound is:
Dibenzofuran-2-carboxylic acid, 2-(phenylsulfonyl)hydrazide;
2,3,4,9-Tetrahydro-1H-carbazole-6-carboxylic acid, 2-(phenylsulfonyl)-hydrazide;
Benzofuran-2-carboxylic acid, 2-(phenylsulfonyl)hydrazide;
5-Methoxy-2-benzofurancarboylic acid, 2-(phenylsulfonyl)-hydrazide;
7-Methoxy-2-benzofurancarboxylic acid, 2-(phenylsulfonyl)-hydrazide;
7-Ethoxy-2-benzofurancarboxylic acid, 2-(phenylsulfonyl)-hydrazide;
7-Chloro-benzofuran-2-carboxylic acid, 2-(phenylsulfonyl)-hydrazide;
5-Chloro-benzofuran-2-carboxylic acid, 2-(phenylsulfonyl)-hydrazide;
2,3-Dihydro-benzofuran 5-carboxylic acid, 2-(phenylsulfonyl)-hydrazide;
Benzo[1,3] dioxole-5-carboxylic acid, 2-(phenylsulfonyl)hydrazide;
1H-Indole-5-carboxylic acid, 2-(phenylsulfonyl)hydrazide;
1-Dibenzofurancarboxylic acid, 2-(phenylsulfonyl)hydrazide;
Quinoline-6-carboxylic acid, 2-(phenylsulfonyl)hydrazide;
2-Dibenzofurancarboxylic acid, 2-[(2-methylphenyl)sulfonyl]hydrazide;
2-Dibenzofurancarboxylic acid, 2-[(3-methylphenyl)sulfonyl]hydrazide;
2-Dibenofurancarboxylic acid, 2-[(3-chlorophenyl)sulfonyl]-hydrazide;
2-Dibenzofurancarboxylic acid, 2-[(2-chlorophenyl)sulfonyl]hydrazide;
Dibenzofuran-2-carboxylic acid, 2-(3,5-dichlorophenylsulfonyl)hydrazide;
Quinoline-6-carboxylic acid, 2-(phenylsulfonyl)hydrazide;
Quinoline-8-carboxylic acid, 2-(phenylsulfonyl)hydrazide;
2-Benzofurancarboxylic acid, 5-chloro-2-[(4-fluorophenyl)]-sulfonyl hydrazide;
2-Benzofurancarboxylic acid, 7-methoxy-, 2-[(4-fluorophenyl)sulfonyl]hydrazide;
2-Dibenzofurancarboxylic acid, 2-[(4-fluorophenyl)sulfonyl]hydrazide;
2-Dibenzofurancarboxylic acid, 2-[(2-chloro-4-fluorophenyl)sulfonyl]hydrazide;
2-Dibenzofurancarboxylic acid, 2-[(3-cyanophenyl)sulfonyl]-hydrazide;
2-Benzofurancarhoxylic acid, 5-chloro-, 2-[(3-cyanophenyl)sulfonyl]hydrazide;
2-Dibenzofurancarboxylic acid, 2-[(2,6-dichlorophenyl)sulfonyl]hydrazide;
2-Benzofurancarboxylic acid, 5-bromo-, 2-(phenylsulfonyl)-hydrazide;
2-Henzofurancarboxylic acid, 5-chloro-, 2-[(2-chloro4-fluorophenyl)sulfonyl]hydrazide;
2-Benzofurancarboxylic acid, 5-chloro-, 2-[(3,5-dichlorophenyl)sulfonyl]hydrazide;
2-Dibenzofurancarboxylic acid, 2-[[3,5-bis(trifluoromethyl)-phenyl]sulfonyl]hydrazide;
2-Benzofurancarboxylic acid, 5-chloro, 2-[(3-chloro4-fluorophenyl)sulfonyl]hydrazide;
2-Benzofurancarboxylic acid, 5-nitro-, 2-(phenylsulfonyl)-hydrazide;
2-Dibenzofurancarboxylic acid, 2-[(3-fluorophenyl)sulfonyl]hydrazide;
2-Dibenzofurancarboxylic acid, 2-[(5-fluoro-2-methylphenyl)sulfonyl]hydrazide;
2-Dibenzofurancarboxylic acid, 2-[(3,4-difluorophenyl)sulfonyl]hydrazide;
2-Dibenzofurancarboxylic acid, 2-[(3-methoxyphenyl)sulfonyl]hydrazide;
2-Dibenzofurancarboxylic acid, 2-[(3-chloro-4-fluorophenyl)sulfonyl]hydrazide;
2-Benzofurancarboxylic acid, 5-chloro-2-[(3-fluorophenyl)sulfonyl]hydrazide;
2-Benzofurancarboxylic acid, 5-chloro-2-[(3,4-difluorophenyl)sulfonyl]hydrazide;
2-Benzofurancarboxylic acid, 5-chloro-2-[(2-chlorophenyl)sulfonyl]hydrazide;
2-Benzofurancarboxylic acid, 5-chloro-2-[(3-chlorophenyl)sulfonyl]hydrazide;
2-Benzofurancarboxylic acid, 5-chloro-2-[(2-bromophenyl)sulfonyl]hydrazide;
2-Benzofurancarboxylic acid, 5-chloro-2-[(3-bromophenyl)sulfonyl]hydrazide;
2-Benzofurancarboxylic acid, 5-chloro-2-[(2-methylphenyl)sulfonyl]hydrazide;
2-Benzofurancarboxylic acid, 5-chloro-2-((3-methylphenyl)sulfonyl]hydrazide;
2-Benzofurancarboxylic acid, 5-chloro-2-[(3-methoxyphenyl)sulfonyl]hydrazide;
2-Benzofurancarboxylic acid, 5-chloro-2-[(3-chloro2-methylphenyl)sulfonyl]hydrazide;
2-Benzofurancarboxylic acid, 5-chloro-2-[(5-fluoro2-methylphenyl)sulfonyl]hydrazide;
2-Benzofurancarboxylic acid, 5-chloro-2-[[(2-(trifluoromethyl)phenyl]sulfonyl]hydrazide;
2-Benzofurancarboxylic acid, 5-chloro-2-[[2-(trifluoromethoxy)phenyl]sulfonyl]hydrazide;
2-Benzofurancarboxylic acid, 7-methoxy-, 2-[[2-(trifluoromethyl)phenyl]sulfonyl]hydrazide;
2-Benzofurancarboxylic acid, 7-methoxy-, 2-[(3-methylphenyl)sulfonyl]hydrazide;
2-Benzofurancarboxylic acid, 5-methoxy-3-(1-methylethoxy), 2-(phenylsulfonyl)hydrazide;
2-Benzofurancarboxylic acid, 3,5-dimethoxy-, 2-(phenylsulfonyl)hydrazide;
2-Benzofurancarboxylic acid, 3,6-dimethoxy-, 2-(phenylsulfonyl)hydrazide;
2-Benzofurancarboxylic acid, 4,6-dimethoxy-, 2-(phenylsulfonyl)hydrazide;
2-Benzofurancarboxylic acid, 3,7-dimeihoxy-, 2-(phenylsulfonyl)hydrazide;
2-Benzofurancarboxylic acid, 3,7-dimethoxy-, 2-[(2-trifluoromethylphenyl)sulfonyl]hydrazide;
2-Benzofuranearboxylic acid, 3,7-dimethoxy-, 2-[(4-fluorophenyl)sulfonyl]hydrazide;
2-Benzofurancarboxylic acid, 3,7-dimethoxy-, 2-[(3-methylphenyl)sulfonyl]hydrazide;
2-Benzofurancarboxylic acid, 3-methoxy-5-phenyl-, 2-(phenylsulfonyl)hydrazide;
2-Benzofuraacarboxylic acid, 3-etboxy-5-phenyl-, 2-(phenylsulfonyl)hydrazide;
2-Benzofurancarboxylic acid, 3-methoxy-7-phenyl-, 2-(phenylsulfonyl)hydrazide;
Naptho[2,3-b]furan-2-carboxylic acid, 3-methoxy-, 2-(phenyisulfonyl)hydrazide;
2-Benzofurancarboxylic acid, 5,7-dichloro 2-(phenylsulfonyl)-sulfonyl hydrazide;
2-Benzofurancarboxylic acid, 5,7-dichloro 2-[[2-(trifluoromethyl)phenyl)]sulfonyl]hydrazide;
2-Benzofurancarboxylic acid, 5,7-dichloro 2-[(2-methylphenyl)sulfonyl]hydrazide;
2-Benzofurancarboxylic acid, 5,7-dichloro 2-[(3-methylphenyl) sulfonyl]hydrazide;
2-Benzofurancarboxylic acid, 5,7-dichloro 2-[(2-bromophenyl)sulfonyl]hydrazide;
2-Benzofurancarboxylic acid, 5,7-dichloro 2-[(3-bromophenyl)sulfonyl]hydrazide;
2-Benzofurancarboxylic acid, 5,7-dichloro 2-[(3-chlorophenyl)sulfonyl]hydrazide;
2-Benzofurancarboxylic acid, 5,7-dichloro 2-[[2-(trifluoromethoxy)phenyl]sulfonyl]hydrazide;
Benzofurancarboxylic acid, 5-methyl-, 2-(phenylsulfonyl)-hydrazide;
2-Benzofurancarboxylic acid, 5-chloro-3-methoxy, 2-(phenylsulfonyl)hydrazide;
2-Benzofurancarboxylic acid, 5-methyl-, 2-[[2-(trifluoromethyl)phenyl]sulfonyl]hydrazide;
2-Benzofurancarboxylic acid, 5-methyl-, 2-([3-methylphenyl)sulfonyl]hydrazide;
2-Benzofurancarboxylic acid, 5-methyl-, 2-[2-(methylphenyl)sulfonyl]hydrazide;
2-Benzofurancarboxylic acid, 5-methyl-, 2-[(3-chloro2-methylphenyl)sulfonyl]hydrazide;
2-Benzofurancarboxylic acid, 5-methyl-, 2-[3-(bromophenyl)sulfonyl]hydrazide;
2-Benzofurancarboxylic acid, 5-methyl-, 2-[(3-chlorophenyl)sulfonyl]hydrazide;
2-Benzofurancarboxylic acid, 5-methyl-, 2-[[2-(trifluoromethoxy)phenyl]sulfonyl]hydrazide;
2-Beozofurancarboxylic acid, 7-methyl-, 2-(phenylsulfonyl)-hydrazide;
2-Benzofurancarboxylic acid, 4-methyl-, 2-(phenylsulfonyl)-hydrazide;
2-Benzofurancarboxylic acid, 3-methyl-, 2-(phenylsulfonyl)-hydrazide;
2-Benzofurancarboxylic acid, 3,5-dimethyl-, 2-(phenylsulfonyl)hydrazide;
2-Benzofurancarboxylic acid, 5-chloro-3-methyl, 2-(phenylsulfonyl)hydrazide;
2-Benzofurancarboxylic acid, 5-amino-, 2-(phenylsulfonyl)-hydrazide;
2-Dibenzofurancarboxylic acid, 2-[(2-fluorophenyl)sulfonyl]hydrazide;
2-Dibenzofurancarboxylic acid, 2-(2,6-difluorophenylsulfonyl)hydrazide;
2-Dibenzofurancarboxylic acid, 2-[(2-bromophenyl)sulfonyl]hydrazide;
2-Dibenzofurancarboxylic acid, 2-[(3-bromophenyl)sulfonyl]hydrazide;
2-Dibenzofurancarboxylic acid, 2-[(4-methyl-3-nitrophenyl)sulfonyl]hydrazide;
2-Dibenzofurancarboxylic acid, 2-[2-chloro-5-(trifluoromethyl)phenyl]sulfonyl)hydrazide;
2-Dibenzofurancarboxylic acid, 2-[(2-trifluoromethoxyphenyl)sulfonyl]hydrazide;
2-Dibenzofurancarboxylic acid, 2-[(4-bromo-2trifluoromethoxyphenyl)sulfonyl]hydrazide;
2-Dibenzofurancarboxylic acid, 2-[(2-nitrophenyl)sulfonyl]-hydrazide;
2-Dibenzofurancarboxylic acid, 2-[(4-carboxyphenyl)sulfonyl]hydrazide;
2-Dibenzofurancarboxylic acid, 2-[(4,5-dichloro-2-thienyl)sulfonyl]hydrazide;
2-Benzofurancarboxylic acid, 7-methoxy-, 2-[(3-chlorophenyl)sulfonyl]hydrazide;
2-Benzofurancarboxylic acid, 7-methoxy-, 2-[(3-fluorophenyl)sulfonyl]hydrazide;
2-Benzofurancarboxylic acid, 7-methoxy-, 2-[(2,3-dichlorophenyl)sulfonyl]hydrazide;
2-Benzofurancarboxylic acid, 7-methoxy-, 2-[(3-chloro-4fluorophenyl)sulfonyl]hydrazide;
2-Benzofurancarboxylic acid, 7-methoxy-, 2-[(5-chloro-2methoxyphenyl)sulfonyl]hydrazide;
2-Benzofurancarboxylic acid, 7-methoxy-, 2-[(5-bromo-2methoxyphenyl)sulfonyl]hydrazide;
2-Benzofurancarboxylic acid, 7-methoxy-, 2-[(4-ethylphenyl)sulfonyl]hydrazide;
2-Benzofurancarboxylic acid, 7-methoxy-, 2-[(3-methoxyphenyl)sulfonyl]hydrazide;
2-Dibenzofurancarboxylic acid, 2-[(2,3-dichlorophenyl)sulfonyl]hydrazide;
2-Naphthalenecarboxylic acid, 2-[(3-methylphenyl)sulfonyl]-hydrazide;
2-Naphthalenecarboxylic acid, 2-[[(2-(trifluoromethyl)phenyl]sulfonyl]hydrazide;
2-Naphthalenecarboxylic acid, 2-[[(4-(trifluoromethyl)phenyl]sulfonyl]hydrazide;
2-Naphthalenecarboxylic acid, 2-[(3-chloro-4-fluorophenyl)sulfonyl]hydrazide;
2-Naphthalenecarboxylic acid, 2-[(3-methoxyphenyl)sulfonyl]hydrazide;
2-Naphthalenecarboxylic acid, 2-[(3-chlorophenyl)sulfonyl]-hydrazide;
2-Naphthalenecarboxylic acid, 2-[(2-chlorophenyl)sulfonyl]-hydrazide;
2-Naphthalenecarboxylic acid, 2-[(3-fluorophenyl)sulfonyl]-hydrazide;
2-Naphthalenecarboxylic acid, 2-[(2,3-dichlorophenyl)sulfonyl]hydrazide;
2-Naphthalenecarboxylic acid, 2-[(4-ethylphenyl)sulfonyl]-hydrazide;
3-Quinolinecarboxylic acid, 2-[(4-pentylphenyl)sulfonyl)-hydrazide;
3-Quinolinecarboxylic acid, 2-[[2(trifluoromethyl)phenyl]sulfonyl]hydrazide;
3-Quinolinecarboxylic acid, 2-[[2,5-bis(2,2,2-trifluoroethoxyl)phenyl]sulfonyl]hydrazide;
3-Quinolinecarboxylic acid, 2-[(3-methylphenyl)sulfonyl]-hydrazide;
3-Quinolinecarboxylic acid, 2-[[2-(trifluoromethoxy)phenyl]sulfonyl]hydrazide;
3-Quinolinecarboxylic acid, 2-[(4-methylphenyl)sulfonyl]-hydrazide;
6-Quinolinecarboxylic acid, 2-[(3-methylphenyl)sulfonyl]-hydrazide;
3-Quinolinecarboxylic acid, 2-[(3-methylphenyl)sulfonyl]-hydrazide;
6-Quinolinecarboxylic acid, 2-[(3-chlorophenyl)sulfonyl]-hydrazide;
3-Quinolinecarboxylic acid, 2-[(3-chlorophenyl)sulfonyl]-hydrazide;
6-Quinolinecarboxylic acid, 2-[(3-fluorophenyl)sulfonyl]-hydrazide;
3-Quinolinecarboxylic acid, 2-[(3-fluorophenyl)sulfonyl]-hydrazide;
6-Quinolinecarboxylic acid, 2-[(2-trifluoromethylphenyl)sulfonyl]hydrazide;
3-Quinolinecarboxylic acid, 2-[(2-trifluoromethylphenyl)sulfonyl]hydrazide;
6-Quinolinecarboxylic acid, 2-[(2-trifluoromethoxyphenyl)sulfonyl]hydrazide;
3-Quinolinecarboxylic acid, 2-[(2-trifluoromethoxyphenyl)sulfonyl]hydrazide;
6-Quinolinecarboxylic acid, 2-[(3,5-dichlorophenyl)sulfonyl]hydrazide;
3-Quinolinecarboxylic acid, 2-[(3,5-dichlorophenyl)sulfonyl]hydrazide;
6-Quinolinecarboxylic acid, 2-[(3-chloro-4-fluorophenyl)sulfonyl]hydrazide;
3-Quinolinecarboxylic acid, 2-[(3-chloro-4-fluorophenyl)sulfonyl]hydrazide;
6-Quinolinecarboxylic acid, 2-[(3-methoxyphenyl)sulfonyl]-hydrazide;
3-Quinolinecarboxylic acid, 2-[(3-methoxyphenyl)sulfonyl]-hydrazide;
6-Quinolinecarboxylic acid, 2-[(3-bromophenyl)sulfonyl]-hydrazide;
3-Quinolinecarboxylic acid, 2-[(3-bromophenyl)sulfonyl]-hydrazide;
Benzofuran-2-carboxylic acid 2-(phenylsulfonyl)hydrazide;
7-Ethoxy-2-benzofurancarboxylic acid 2-(phenylsulfonyl)-hydrazide;
7-Chloro-benzofuran-2-carboxylic acid 2-(phenylsulfonyl)-hydrazide;
2,3-Dihydro-benzofuran-5-carboxylic acid 2-(phenylsulfonyl)hydrazide;
5-Chloro-benzofuran-2-carboxylic acid 2-(phenylsulfonyl)-hydrazide;
Isoquinoline-1-carboxylic acid 2-(phenylsulfonyl)hydrazide;
1H-Indole-5-carboxylic acid 2-(phenylsulfonyl)hydrazide;
Quinoline-3-carboxylic acid 2-(phenylsulfonyl)hydrazide;
Quinoline-4-carboxylic acid 2-(phenylsulfonyl)hydrazide;
1H-Indole-5-carboxylic acid, 2-[(3-chlorophenyl)sulfonyl]-hydrazide;
1H-Indole-5-carboxylic acid, 2-[(2-chlorophenyl)sulfonyl]-hydrazide;
1H-Indole-5-carboxylic acid, 2-[(3-fluorophenyl)sulfonyl]-hydrazide;
1H-Indole-5-carboxylic acid, 2-[(3-trifluoromethylphenyl)sulfonyl]hydrazide;
1H-Indole-5-carboxylic acid, 2-[(2-trifluoromethylphenyl)sulfonyl]hydrazide;
1H-Indole-5-carboxylic acid, 2-[(2-trifluoromethoxyphenyl)sulfonyl]hydrazide;
1H-Indole-5-carboxylic acid, 2-[(3trifluoromethoxyphenyl)sulfonyl]hydrazide;
1H-Indole-5-carboxylic acid, 2-[(3-bromophenyl)sulfonyl]-hydrazide;
1H-Indole-5-carboxylic acid, 2-[(3-methylphenyl)sulfonyl]-hydrazide;
1H-Indole-5-carboxylic acid, 2-[(2-methylphenyl)sulfonyl]-hydrazide;
6-Quinolinecarboxylic acid, 2-[(2-chlorophenyl)sulfonyl]-hydrazide;
6-Quinolinecarboxylic acid, 2-[(3-trifluoromethylphenyl)sulfonyl]hydrazide;
6-Quinolinecarboxylic acid, 2-[(3-trifluoromethoxyphenyl)sulfonyl]hydrazide;
6-Quinolinecarboxylic acid, 2-[(2-methylpheuyl)sulfonyl]-hydzazide;
Dibenzofuran-2-carboxylic acid, 2-(4-fluoro-3methylphenylsulfonyl)hydrazide;

6. A pharmaceutical composition comprising a compound of Claim 1 and a pharmaceutically acceptable carrier.

7. Use of a compound of Formula I as claimed in claim1 for preparation of a medicament for treatment or preventing neuronal loss associated with stroke, ischemia, CNS trauma, hypoglycemia or surgery, or treating a neurodegenerative disease, or treating or preventing the adverse consequences of the overstimulation of the excitatory amino acids, or treating anxiety, psychosis, glaucoma, CMV retinitis, diabetic retinopathy, urinary incontinence, migraine headache, convulsions, aminoglycoside antibiotics-induced hearing loss, Parkinson's disease, chronic pain, neuropathic pain, or inducing anesthesia, opioid tolerance or withdrawal, or enhancing cognition.

8. The use of Claim 7 wherein Ar is a bicyclic heteroaryl.

9. The use of Claim 7 wherein Ar is a tricyclic heteroaryl.

10. The use of Claim 7 wherein R₁, R₂, R₃, R₄, and R₅ are hydrogen.

11. The use of Claim 7 wherein the compound is:
Dibenzofuran-2-carboxylic acid, 2-(phenylsulfonyl)hydrazide;
2,3,4,9-Tetrahydro-1H-carbazole-6-carboxylic acid, 2-(phenylsulfonyl)-hydrazide;
Benzofuran-2-carboxylic acid, 2-(phenylsulfonyl)hydrazide;
5-Methoxy-2-benzofurancarboylic acid, 2-(phenylsulfonyl)-hydrazide;
7-Methoxy-2-benzofurancarboxylic acid, 2-(phenylsulfonyl)-hydrazide;
7-Ethoxy-2-benzofurancarboxylic acid, 2-(phenylsulfonyl)-hydrazide;
7-Chloro-benzofuran-2-carboxylic acid, 2-(phenylsulfonyl)-hydraxide;
5-Chloro-benzofuran-2-carboxylic acid, 2-(phenylsulfonyl)-hydrazide;
2,3-Dihydro-benzofuran-5-carboxylic acid, 2-(phenylsulfonyl)-hydrazide;
Benzo[1,3]dioxole-5-carboxylic acid, 2-(phenylsulfonyl)hydrazide;
1H-Indole-5-carboxylic acid, 2-(phenylsulfonyl)hydrazide;
1-Dibenzofurancarboxylic acid, 2-(phenylsulfonyl)hydrazide;
Quinoline-6-carboxylic acid, 2-(phenylsulfonyl)hydrazide;
2-Dibenzofurancarboxylic acid, 2-[(2-methylphenyl)sulfonyl]hydrazide;
2-Dibenzofurancarboxylic acid, 2-[(3-methylphenyi)sulfonyl]hydrazide;
2-Dibenofurancarboxylic acid, 2-[(3-chlorophenyl)sulfonyl]-hydrazide;
2-Dibenzofurancarboxylic acid, 2-[(2-chlorophenyl)sulfonyl]hydrazide;
Dibenzofuran-2-carboxylic acid, 2-(3,5-dichloraphenylsulfonyl)hydrazide;
Dibenzofuran-2-carboxylic acid, 2-(3-chloro-2-methylphenylsulfonyl)hydrazide;
Quinoline-6-carboxylic acid, 2-(phenylsulfonyl)hydrazide;
Quinoline-8-carboxylic acid, 2-(phenylsulfonyl)hydrazide;
2-Benzofurancarboxylic acid, 5-chloro-2-[(4-fluorophenyl)]-sulfonyl hydrazide;
2-Benzofurancatboxylic acid, 7-methoxy-, 2-[(4-fluorophenyl)sulfonyl]hydrazide;
2-Dibenzofurancarboxylic acid, 2-[(4-fluorophenyl)sulfonyl]hydrazide;
2-Dibenzofurancarboxylic acid, 2-[(2-chloro-4-fluorophenyl)sulfonyl]hydrazide;
2-Dibenzofurancarboxylic acid, 2-[(3-cyanophenyl)sulfonyl]-hydrazide;
2-Benzofurancarboxylic acid, 5-chloro-, 2-[(3-cyanophenyl)sulfonyl]hydrazide;
2-Dibenzofurancarboxylic acid, 2-[(2,6-dichlorophenyl)sulfonyl]hydrazide;
2-Benzofurancarboxylic acid, 5-bromo-, 2-(phenylsulfonyl)-hydrazide;
2-Benzofurancarboxylic acid, 5-chloro-, 2-[(2-chloro4-fluorophenyl)sulfonyl]hydrazide;
2-Benzofurancarboxylic acid, 5-chloro-, 2-[(3,5-dichlorophenyl)sulfonyl]hydrazide;
2-Dibenzofurancarboxylic acid, 2-[[3,5-bis(trifluoromethyl)-phenyl]sulfonyl]hydrazide;
2-Beazofurancarboxylic acid, 5-chloro, 2-[(3-chloro4-fluorophenyl)sulfonyl]hydrazide;
2-Benzofurancarboxylic acid, 5-nitro-, 2-(phenylsulfonyl)-hydrazide;
2-Dibenzofurancarboxylic acid, 2-[(3-fluorophenyl)sulfonyl]hydrazide;
2-Dibenzofurancarboxylic acid, 2-[(5-fluoro-2-methylphenyl)sulfonyl]hydrazide;
2-Dibenzofurancarboxylic acid, 2-[(3,4-difluorophenyl)sulfonyl]hydrazide;
2-Dibenzofurancarboxylic acid, 2-[(3-chloro-4-fluorophenyl)sulfonyl]hydrazide;
2-Dibenzofurancarboxylic acid, 2-[(3-methoxyphenyl)sulfonyl]hydrazide;
2-Benzofurancarboxylic acid, 5-chloro-2-[(3-fluorophenyl)sulfonyl]hydrazide;
2-Benzofurancarboxylic acid, 5-chloro-2-[(3,4-difluorophenyl)sulfonyl]hydrazide;
2-Benzofurancarboxylic acid, 5-chloro-2-[(2-chlorophenyl)sulfonyl]hydrazide;
2-Benzofurancarboxylic acid, 5-chloro-2-[(3-chlorophenyl)sulfonyl]hydrazide;
2-Benzofurancarboxylic acid, 5-chloro-2-[(2-bromophenyl)sulfonyl]hydrazide;
2-Benzofurancarboxylic acid, 5-chloro-2-[(3-bromophenyl)sulfonyl]hydrazide;
2-Benzofurancarboxylic acid, 5-chloro-2-[(2-methylphenyl)sulfonyl]hydrazide;
2-Benzofurancarboxylic acid, 5-chloro-2-[(3-methylphenyl)sulfonyl]hydrazide;
2-Benzofurancarboxylic acid, 5-chloro-2-[(3-methoxyphenyl)sulfonyl]hydrazide;
2-Benzofurancarboxylic acid, 5-chloro-2-[(3-chloro2-methylphenyl)sulfonyl]hydrazide;
2-Benzofurancarboxylic acid, 5-chloro-2-[(5-fluoro2-methylphenyl)sulfonyl]hydrazide;
2-Benzofurancarboxylic acid, 5-chloro-2-[[(2-(trifluoromethyl)phenyl]sulfonyl]hydrazide;
2-Benzofurancarboxylic acid, 5-chloro-2-[[2-(trifluoromethoxy)phenyl]sulfonyl]hydrazide;
2-Benzofurancarboxylic acid, 7-methoxy-, 2-[(2-(trifluoromethyl)phenyl]sulfonyl]hydrazide;
2-Benzofurancarboxylic acid, 7-methoxy-, 2-[(3-methylphenyl)sulfonyl]hydrazide;
2-Benzofurancarboxylic acid, 6-methoxy-, 2-(phenylsulfonyl)hydrazide;
2-Benzofurancarboxylic acid, 5-methoxy-3-(1-methylethoxy), 2-(phenylsulfonyl)hydrazide;
2-Benzofurancarboxylic acid, 3,5-dimethoxy-, 2-(phenylsulfonyl)hydrazide;
2-Benzofurancarboxylic acid, 3,6-dimethoxy-, 2-(phenylsulfonyl)hydrazide;
2-Benzofurancarboxylic acid, 4,6-dimethoxy-, 2-(phenylsulfonyl)hydrazide;
2-Benzofurancarboxylic acid, 3,7-dimethoxy-, 2-(phenylsulfonyl)hydrazide;
2-Benzofurancarboxylic acid, 3,7-dimethoxy-, 2-[(2-trifluoromethylphenyl)sulfonyl]hydrazide;
2-Benzofurancarboxylic acid, 3,7-dimethoxy-, 2-[(4-fluorophenyl)sulfonyl]hydrazide;
2-Benzofurancarboxylic acid, 3,7-dimethoxy-, 2-[(3-methylphenyl)sulfonyl]hydrazide;
2-Benzofurancarboxylic acid, 3-methoxy-5-phenyl-, 2-(phenylsulfonyl)hydrazide;
2-Benzofurancarboxylic acid, 3-ethoxy-5-phenyl-, 2-(phenylsulfonyl)hydrazide;
2-Benzofurancarboxylic acid, 3-methoxy-7-phenyl-, 2-(phenylsulfonyl)hydrazide;
Naptho[2,3-b]furan-2-carboxylic acid, 3-methoxy-, 2-(phenylsulfonyl)hydrazide;
2-Benzofurancarboxylic acid, 5,7-dichloro 2-(phenylsulfonyl)-sulfonyl hydrazide;
2-Benzofurancarboxylic acid, 5,7-dichloro 2-[[2-(trifluoromethyl)phenyl)]sulfonyl]hydrazide;
2-Benzofurancarboxylic acid, 5,7-dichloro 2-[(2-methylphenyl)sulfonyl]hydrazide;
2-Benzofurancarboxylic acid, 5,7-dichloro, 2-[(3-methylphenyl) sulfonyl]hydrazide;
2-Benzofurancarboxylic acid, 5,7-dichloro, 2-[(2-bromophenyl)sulfonyl]hydrazide;
2-Benzofurancarboxylic acid, 5,7-dichloro, 2-[(3-bromophenyl)sulfonyl]hydrazide;
2-Benzofurancarboxylic acid, 5,7-dichloro, 2-[(3-chlorophenyl)sulfonyl]hydrazide;
2-Benzofurancarboxylic acid, 5,7-dichloro, 2-[[2-(trifluoromethoxy)phenyl]sulfonyl]hydrazide;
Benzofurancarboxylic acid, 5-methyl-, 2-(phenylsulfonyl)-hydrazide;
2-Benzofurancarboxylic acid, 5-chloro-3-methoxy, 2-(phenylsulfonyl)hydrazide;
2-Benzofurancarboxylic acid, 5-methyl-, 2-[[2-(trifluoromethyl)phenyl]sulfonyl]hydrazide;
2-Benzofurancarboxylic acid, 5-methyl-, 2-([3-methylphenyl)sulfonyl]hydrazide;
2-Benzofurancarboxylic acid, 5-methyl-, 2-[2-(methylphenyl)sulfonyl]hydrazide;
2-Benzofurancarboxylic acid, 5-methyl-, 2-[(3-chloro2-methylphenyl)sulfonyl]hydrazide;
2-Benzofurancarboxylic acid, 5-methyl-, 2-[3-(bromophenyl)sulfonyl]hydrazide;
2-Benzofurancarboxylic acid, 5-methyl-, 2-[(3-chlorophenyl)sulfonyl]hydrazide;
2-Benzofurancarboxylic acid, 5-methyl-, 2-[[2-(trifluoromethoxy)phenyl]sulfonyl]hydrazide;
2-Benzofurancarboxylic acid, 7-methyl-, 2-(phenylsulfonyl)-hydrazide;
2-Benzofurancarboxylic acid, 4-methyl-, 2-(phenylsulfonyl)-hydrazide;
2-Benzofurancarboxylic acid, 3-methyl-, 2-(phenylsulfonyl)-hydrazide;
2-Benzofurancarboxylic acid, 3,5-dimethyl-, 2-(phenylsulfonyl)hydrazide;
2-Benzofurancarboxylic acid, 5-chloro-3-methyl, 2-(phenylsulfonyl)hydrazide;
2-Benzofurancarboxylic acid, 5-amino-, 2-(phenylsulfonyl)-hydrazide;
2-Dibenzofurancarboxylic acid, 2-[(2-fluorophenyl)sulfonyl]hydrazide;
2-Dibenzofurancarboxylic acid, 2-(2,6-difluorophenylsulfonyl)hydrazide;
2-Dibenzofurancarboxylic acid, 2-[(2-bromophenyl)sulfonyl]hydrazide;
2-Dibenzofurancarboxylic acid, 2-[(3-bromophenyl)sulfonyl]hydrazide;
2-Dibenzofurancarboxylic acid, 2-[(4-methyl-3-nitrophenyl)sulfonyl]hydrazide;
2-Dibenzofurancarboxylic acid, 2-[2-chloro-5-(trifluoromethyl)phenyl]sulfonyl)hydrazide;
2-Dibenzofurancarboxylic acid, 2-[(2-trifluoromethoxyphenyl)sulfonyl]hydrazide;
2-Dibenzofurancarboxylic acid, 2-[(4-bromo-2trifluoromethoxyphenyl)sulfonyl]hydrazide;
2-Dibenzofurancarboxylic acid, 2-[(2-nitrophenyl)sulfonyl]-hydrazide;
2-Dibenzofurancarboxylic acid, 2-[(4-carboxyphenyl)sulfonyl]hydrazide;
2-Dibenzofurancarboxylic acid, 2-[(4,5-dichloro-2-thienyl)sulfonyl]hydrazide;
2-Benzofurancarboxylic acid, 7-methoxy-, 2-[(3-chlorophenyl)sulfonyl]hydrazide;
2-Benzofurancarboxylic acid, 7-methoxy-, 2-[(3-fluorophenyl)sulfonyl]hydrazide;
2-Benzofurancarboxylic acid, 7-methoxy-, 2-[(2,3-dichlorophenyl)sulfonyl]hydrazide;
2-Benzofurancarboxylic acid, 7-methoxy-, 2-[(3-chloro-4fluorophenyl)sulfonyl]hydrazide;
2-Benzofurancarboxylic acid, 7-methoxy-, 2-[(5-chloro-2methoxyphenyl)sulfonyl]hydrazide;
2-Benzofurancarboxylic acid, 7-methoxy-, 2-[(5-bromo-2methoxyphenyl)sulfonyl]hydrazide;
2-Benzofurancarboxylic acid, 7-methoxy-, 2-[(4-ethylphenyl)sulfonyl]hydrazide;
2-Benzofurancarboxylic acid, 7-methoxy-, 2-[(3-methoxyphenyl)sulfonyl]hydrazide;
2-Dibenzofurancarboxylic acid, 2-[(2,3-dichlorophenyl)sulfonyl]hydrazide;
2-Naphthalenecarboxylic acid, 2-[(3-methylphenyl)sulfonyl]-hydrazide;
2-Naphthalenecarboxylic acid, 2-[(4-methylphenyl)sulfonyl]-hydrazide;
2-Naphthalenecarboxylic acid, 2-[[(2-(trifluoromethyl)phenyl]sulfonyl]hydrazide;
2-Naphthalenecarboxylic acid, 2-[[(4-(trifluoromethyl)phenyl]sulfonyl]hydrazide;
2-Naphthalenecarboxylic acid, 2-[(3-chloro-4-fluorophenyl)sulfonyl]hydrazide;
2-Naphthalenecarboxylic acid, 2-[(3-methoxyphenyl)sulfonyl]hydrazide;
2-Naphthalenecarboxylic acid, 2-[(3-chlorophenyl)sulfonyl]-hydrazide;
2-Naphthalenecarboxylic acid, 2-[(2-chlorophenyl)sulfonyl]-hydrazide;
2-Naphthalenecarboxylic acid, 2-[(3-fluorophenyl)sulfonyl]-hydrazide;
2-Naphthalenecarboxylic acid, 2-(2-thienylsulfonyl)hydrazide;
2-Naphthalenecarboxylic acid, 2-[(2,3-dichlorophenyl)sulfonyl]hydrazide;
2-Naphthalenecarboxylic acid, 2-[(4-ethylphenyl)sulfonyl]-hydrazide;
3-Quinolinecarboxylic acid, 2-[(4-pentylphenyl)sulfonyl)-hydrazide;
3-Quinolinecarboxylic acid, 2-[[2(trifluoromethyl)phenyl]sulfonyl]hydrazide;
3-Quinolinecarboxylic acid, 2-[[2,5-bis(2,2,2-trifluoroethoxyl)phenyl]sulfonyl]hydrazide;
3-Quinolinecarboxylic acid, 2-[(3-methylphenyl)sulfonyl]-hydrazide;
3-Quinolinecarboxylic acid, 2-[[2-(trifluoromethoxy)phenyl]sulfonyl]hydrazide;
3-Quinolinecarboxylic acid, 2-[(4-methylphenyl)sulfonyl]-hydrazide;
6-Quinolinecarboxylic acid, 2-[(3-methylphenyl)sulfonyl]-hydrazide;
3-Quinolinecarboxylic acid, 2-[(3-methylphenyl)sulfonyl]-hydrazide;
6-Quinolinecarboxylic acid, 2-[(3-chlorophenyl)sulfonyl]-hydrazide;
3-Quinolinecarboxylic acid, 2-[(3-chlorophenyl)sulfonyl]-hydrazide;
6-Quinolinecarboxylic acid, 2-[(3-fluorophenyl)sulfonyl]-hydrazide;
3-Quinolinecarboxylic acid, 2-[(3-fluorophenyl)sulfonyl]-hydrazide;
6-Quinolinecarboxylic acid, 2-[(2-trifluoromethylphenyl)sulfonyl]hydrazide;
3-Quinolinecarboxylic acid, 2-[(2-trifluoromethylphenyl)sulfonyl]hydrazide;
6-Quinolinecarboxylic acid, 2-[(2-trifluoromethoxyphenyl)sulfonyl]hydrazide;
3-Quinolinecarboxylic acid, 2-[(2-trifluoromethoxyphenyl)sulfonyl]hydrazide;
6-Quinolinecarboxylic acid, 2-[(3,5-dichlomphenynsulfonyl]hydrazide;
3-Quinolinecarboxylic acid, 2-[(3,5-dichlorophenyl)sulfonyl)hydrazide;
6-Quinolinecarboxylic acid, 2-[(3-chloro-4-fluorophenyl)sulfonyl]hydrazide;
3-Quinolinecarboxylic acid, 2-[(3-chloro-4-fluorophenyl)sulfonyl]hydrazide;
6-Quinolinecarboxylic acid, 2-[(3-methoxyphenyl)sulfonyl]-hydrazide;
3-Quinolinecarboxylic acid, 2-[(3-methoxyphenyl)sulfonyl]-hydrazide;
6-Quinolinecarboxylic acid, 2-[(3-bromaphenyl)sulfonyl]-hydrazide;
3-Quinolinecarboxylic acid, 2-[(3-bromophenyl)sulfonyl]-hydrazide;
Benzofuran-2-carboxylic acid, 2-(phenylsulfonyl)hydrazide;
7-Ethoxy-2-benzofurancarboxylic acid, 2-(phenylsulfonyl)-hydrazide; 7-Chloro-benzofuran-2-carboxylic acid, 2-(phenylsulfonyl)-hydrazide;
2,3-Dihydro-benzofuran-5-carboxylic acid, 2-(phenylsulfonyl)hydrazide;
5-Chloro-benzofuran-2-carboxylic acid, 2-(phenylsulfonyl)-hydrazide;
Isoquinoline-1-carboxylic acid, 2-(phenylsulfonyl)hydrazide;
1H-Indole-5-carboxylic acid, 2-(phenylsulfonyl)hydrazide;
Quinoline-3-carboxylic acid, 2-(phenylsulfonyl)hydiazide;
Quinoline-4-carboxylic acid, 2-(phenylsulfonyl)hydrazide;
1H-Indole-5-carboxylic acid, 2-[(3-chlorophenyl)sulfonyl]-hydrazide;
1H-Indole-5-carboxylic acid, 2-[(2-chiorophenyl)sulfonyl]-hydrazide;
1H-Indole-5-carboxylic acid, 2-[(3-fluorophenyl)sulfonyl]-hydrazide;
1H-Indole-5-carboxylic acid, 2-[(3-trifluoromethylphenyl)sulfonyl]hydrazide;
1H-Indole-5-carboxylic acid, 2-[(2-trifluoromethylphenyl)sulfonyl]hydrazide;
1H-Indole-5-carboxylic acid, 2-[(2-trifluoromethoxyphenyl)sulfonyl]hydrazide;
1H-Indole-5-carboxylic acid, 2-[(3trifluoromethoxyphenyl)sulfonyl]hydrazide;
1H-Indolo-5-carboxylic acid, 2-[(3-bromophenyl)sulfonyl]-hydrazide;
1H-Indale-5-carboxylic acid, 2-[(3-methylphenyl)sulfonyl]-hydrazide;
1H-Indole-5-carboxylic acid, 2-[(2-methylphenyl)sulfonyl]-hydrazide;
6-Quinolinecarboxylic acid, 2-[(2-chlorophenyl)sulfonyl]-hydrazide;
6-Quinolinecarboxylic acid, 2-[(3-trifluoromethylphenyl)sulfonyl]-hydrazide;
6-Quinolinecarboxylic acid, 2-[(3-trifluoromethoxyphenyl)-sulfonyl]hydrazide;
6-Quinolinecarboxylic acid, 2-[(2-methylphenyl)sulfonyl]-hydrazide;
Dibenzofuran-2-mboxylic acid, 2-(4-fluoro-3-methylphenylsulfonyl)hydrazide;
2-Naphthalenecarboxylic acid, 2-(phenylsulfonyl)hydrazide.

12. Use of a compound of Claim 1 for manufacturing a medicament for inhibiting branched chain amino acid-dependent aminotransferases in a patient, by administration to the patient of a therapeutically effective amount.

13. The use of Claim 12, wherein Ar is a bicyclic heteroaryl.

14. The use of Claim 12, wherein Ar is a tricyclic heteroaryl.

15. The use of Claim 12, wherein R₁, R₂, R₃, R₄, and R₅ are hydrogen.

16. The use of Claim 12, wherein the compound is:
Dibenzofuran-2-carboxylic acid, 2-(phenylsulfonyl)hydrazide;
2,3,4,9-Tetrahydro-1H-carbazole-6-carboxylic acid, 2-(phenylsulfonyl)hydrazide;
Benzofuran-2-carboxylic acid, 2-(phenylsulfonyl)hydrazide;
5-Methoxy-2-benzofizrancarboylic acid, 2-(phenylsulfonyl)-hydrazide;
7-Methoxy-2-benzofurancarboxylic acid, 2-(phenylsulfonyl)-hydrazide;
7-Ethoxy-2-beazofurancarboxylic acid, 2-(phenylsulfonyl)-hydrazide;
7-Chloro-benzofuran-2-carboxylic acid, 2-(phenylsulfonyl)-hydrazide;
5-Chloro-beazofuran-2-carboxylic acid, 2-(phenylsulfonyl)-hydrazide;
2,3-Dihydro-benzofuran-5-carboxylic acid, 2-(phenylsulfonyl)hydrazide;
Benzo[1,3]dioxole-5-carboxylic acid, 2-(phenylsulfonyl)hydrazide;
1H-Indole-5-carboxylic acid, 2-(phenylsulfonyl)hydrazide;
1-Dibenzofurancarboxylic acid, 2-(phenylsulfonyl)hydrazide;
Quinoline-6-carboxylic acid, 2-(phenylsulfonyl)hydrazide;
2-Dibenzofurancarboxylic acid, 2-[(2-methylphenyl)sulfonyl]hydrazide;
2-Dibenzofurancarboxylic acid, 2-[(3-methylphenyl)sulfonyl)hydrazide;
2-Dibenofurancarboxylic acid, 2-[(3-chlorophenyl)sulfonyl]-hydrazide;
2-Dibenzofurancarboxylic acid, 2-[(2-chlorophenyl)sulfonyl]hydrazide;
Dibenzofuran-2-carboxylic acid, 2-(3,5-dichlorophenylsulfonyl)hydrazide;
Dibenzofuran-2-carboxylic acid, 2-(3-chloro-2-methylphenylsulfonyl)hydrazide;
Quinoline-6-carboxylic acid, 2-(phenylsulfonyl)hydrazide;
Quinoline-8-carboxylic acid, 2-(phenylsulfonyl)hydrazide;
2-Benzofurancarboxylic acid, 5-chloro-2-[(4-fluorophenyl)]-sulfonyl hydrazide;
2-Benzofurancarboxylic acid, 7-methoxy-, 2-[(4-fluorophenyl)sulfonyl]hydrazide;
2-Dibenzofurancarboxylic acid, 2-[(4-fluorophenyl)sulfonyl]hydrazide;
2-Dibenzofurancarboxylic acid, 2-[(2-chloro-4-fluorophenyl)sulfonyl]hydrazide;
2-Dibenzofurancarboxylic acid, 2-[(3-cyanophenyl)sulfonyl]-hydrazide;
2-Benzofurancarboxylic acid, 5-chloro-, 2-[(3-cyanophenyl)sulfonyl]hydrazide;
2-Dibenzofurancarboxylic acid, 2-[(2,6-dichlorophenyl)sulfonyl]hydrazide;
2-Benzofurancarboxylic acid, 5-bromo-, 2-(phenylsulfonyl)-hydrazide;
2-Benzofurancarboxylic acid, 5-chloro-, 2-[(2-chloro4-fluorophenyl)sulfonyl]hydrazide;
2-Benzofurancarboxylic acid, 5-chloro-, 2-[(3,5-dichlorophenyl)sulfonyl]hydrazide;
2-Dibenzofurancarboxylic acid, 2-[[3,5-bis(trifluoromethyl)-phenyl]sulfonyl]hydrazide;
2-Benzofurancarboxylic acid, 5-chloro, 2-[(3-chloro4-fluorophenyl)sulfonyl]hydrazide;
2-Benzofurancarboxylic acid, 5-nitro-, 2-(phenylsulfonyl)-hydrazide;
2-Dibenzofurancarboxylic acid, 2-[(3-fluorophenyl)sulfonyl]hydrazide;
2-Dibenzofurancarboxylic acid, 2-[(5-fluoro-2-methylphenyl)sulfonyl]hydrazide;
2-Dibenzofurancarboxylic acid, 2-[(3,4-difluorophenyl)sulfonyl]hydrazide;
2-Dibenzofurancarboxylic acid, 2-[(3-chloro-4-fluorophenyl)sulfonyl]hydrazide;
2-Dibenzofurancarboxylic acid, 2-[(3-methoxyphenyl)sulfonyl]hydrazide;
2-Benzofurancarboxylic acid, 5-chloro-2-[(3-fluorophenyl)sulfonyl]hydrazide;
2-Benzofurancarboxylic acid, 5-chloro-2-[(3,4-difluorophenyl)sulfonyl]hydrazide;
2-Benzofurancarboxylic acid, 5-chloro-2-[(2-chlorophenyl)sulfonyl]hydrazide;
2-Benzofurancarboxylic acid, 5-chloro-2-[(3-chlorophenyl)sulfonyl]hydrazide;
2-Benzofurancarboxylic acid, 5-chloro-2-[(2-bromophenyl)sulfonyl]hydrazide;
2-Benzofurancarboxylic acid, 5-chloro-2-[(3-bromophenyl)sulfonyl]hydrazide;
2-Benzofurancarboxylic acid, 5-chloro-2-[(2-methylphenyl)sulfonyl]hydrazide;
2-Benzofurancarboxylic acid, 5-chloro-2-[(3-methylphenyl)sulfonyl]hydrazide;
2-Benzofurancarboxylic acid, 5-chloro-2-[(3-methoxyphenyl)sulfonyl]hydrazide;
2-Benzofurancarboxylic acid, 5-chloro-2-[(3-chloro2-methylphenyl)sulfonyl]hydrazsde;
2-Benzofurancarboxylic acid, 5-chloro-2-[(5-fluoro2-methylphenyl)sulfonyl]hydrazide;
2-Benzofurancarboxylic acid, 5-chloro-2-[[(2-(trifluoromethyl)phenyl]sulfonyl]hydrazide;
2-Benzofurancarboxylic acid, 5-chloro-2-[[2-(trifluoromethoxy)phenyl]sulfonyl]hydrazide;
2-Benzofurancarboxylic acid, 7-methoxy-, 2-[[2-(trifluoromethyl)phenyl]sulfonyl]hydrazide;
2-Benzofurancarboxylic acid, 7-methoxy-, 2-[(3-methylphenyl)sulfonyl]hydrazide;
2-Benzofurancarboxylic acid, 6-methoxy-, 2-(phenylsulfonyl)hydrazide;
2-Benzofurancarboxylic acid, 5-methoxy-3-(1-methylethoxy), 2-(phenylsulfonyl)hydrazide;
2-Benzofurancarboxylic acid, 3,5-dimethoxy-, 2-(phenylsulfonyl)hydrazide;
2-Benzofurancarboxylic acid, 3,6-dimethoxy-, 2-(phenylsulfonyl)hydrazide;
2-Benzofurancarboxylic acid, 4,6-dimethoxy-, 2-(phenylsulfonyl)hydrazide;
2-Benzofurancarboxylic acid, 3,7-dimethoxy-, 2-(phenylsulfonyl)hydrazide;
2-Benzofurancarboxylic acid, 3,7-dimethoxy-, 2-[(2-trifluoromethylphenyl)sulfonyl]hydrazide;
2-Benzofurancarboxylic acid, 3,7-dimethoxy-, 2-[(4-fluorophenyl)sulfonyl]hydrazide;
2-Beazofurancarboxylic acid, 3,7-dimethoxy-, 2-[(3-methylphenyl)sulfonyl]hydrazide;
2-Benzofurancarboxylic acid, 3-methoxy-5-phenyl-, 2-(phenylsulfonyl)hydrazide;
2-Benzofurancarboxylic acid, 3-ethoxy-5-phenyl-, 2-(phenylsulfonyl)hydrazide;
2-Benzofurancarboxylic acid, 3-methoxy-7-phenyl-, 2-(phenylsulfonyl)hydrazide;
Naptho[2,3-b]furan-2-carboxylic acid, 3-methoxy-, 2-(phenylsulfonyl)hydrazide;
2-Benzofurancarboxylic acid, 5,7-dichloro 2-(phenylsulfonyl)-sulfonyl hydrazide;
2-Benzofurancarboxylic acid, 5,7-dichloro 2-[[2-(trifluoromethyl)phenyl)]sulfonyl]hydrazide;
2-Benzofurancarboxylic acid, 5,7-dichloro 2-[(2-methylphenyl)sulfonyl]hydrazide;
2-Benzofurancarboxylic acid, 5,7-dichloro 2-[(3-methylphenyl) sulfonyl]hydrazide;
2-Beozofurancarboxylic acid, 5,7-dichloro 2-[(2-bromophenyl)sulfonyl]hydrazide;
2-Benzofurancarboxylic acid, 5,7-dichloro 2-[(3-bromophenyl)sulfonyl]hydrazide;
2-Benzofurancarboxylic acid, 5,7-dichloro 2-[(3-chlorophenyl)sulfonyl]hydrazide;
2-Benzofurancarboxylic acid, 5,7-dichloro 2-[[2-(trifluoromethoxy)phenyl]sulfonyl]hydrazide;
Benzofurancarboxylic acid, 5-methyl-, 2-(phenylsulfonyl)-hydrazide;
2-Benzofurancarboxylic acid, 5-chloro-3-methoxy, 2-(phenylsulfonyl)hydrazide;
2-Benzofurancarboxylic acid, 5-methyl-, 2-[[2-(trifluoromethyl)phenyl]sulfonyl]hydrazide;
2-Benzofurancarboxylic acid, 5-methyl-, 2-([3-methylphenyl)sulfonyl]hydrazide;
2-Benzofurancarboxylic acid, 5-methyl-, 2-[2-(methylphenyl)sulfonyl]hydrazide;
2-Benzofurancarboxylic acid, 5-methyl-, 2-[(3-chloro2-methylphenyl)sulfonyl]hydrazide;
2-Benzofurancarboxylic acid, 5-methyl-, 2-[3-(bromophenyl)sulfonyl]hydrazide;
2-Benzofurancarboxylic acid, 5-methyl-, 2-[(3-chlorophenyl)sulfonyl]hydrazide;
2-Benzofurancarboxylic acid, 5-methyl-, 2-[[2-(trifluoromethoxy)phenyl]sulfonyl]hydrazide;
2-Benzofurancarboxylic acid, 7-methyl-, 2-(phenylsulfonyl)-hydrazide;
2-Benzofurancarboxylic acid, 4-methyl-, 2-(phenylsulfonyl)-hydrazide;
2-Benzofurancarboxylic acid, 3-methyl-, 2-(phenylsulfonyl)-hydrazide;
2-Benzofurancarboxylic acid, 3,5-dimethyl-, 2-(phenylsulfonyl)hydrazide;
2-Benzofurancarboxylic acid, 5-chloro-3-methyl, 2-(phenylsulfonyl)hydrazide;
2-Benzofurancarboxylic acid, 5-amino-, 2-(phenylsulfonyl)-hydrazide;
2-Dibenzofurancarboxylic acid, 2-[(2-fluorophenyl)sulfonyl]hydrazide;
2-Dibenzofurancarboxylic acid, 2-(2,6-difluorophenylsulfonyl)hydrazide;
2-Dibenzofurancarboxylic acid, 2-[(2-bromophenyl)sulfonyl]hydrazide;
2-Dibenzofurancarboxylic acid, 2-[(3-bromophenyl)sulfonyl]hydrazide;
2-Dibenzofurancarboxylic acid, 2-[(4-methyl-3-nitrophenyl)sulfonyl]hydrazide;
2-Dibenzofurancarboxylic acid, 2-[2-chloro-5-(trifluoromethyl)phenyl]sulfonyl)hydrazide;
2-Dibenzofurancarboxylic acid, 2-[(2-trifluoromethoxyphenyl)sulfonyl]hydrazide;
2-Dibenzofurancarboxylic acid, 2-[(4-bromo-2trifluoromethoxyphenyl)sulfonyl]hydrazide;
2-Dibenzofurancarboxylic acid, 2-[(2-nitrophenyl)sulfonyl]-hydrazide;
2-Dibenzofurancarboxylic acid, 2-[(4-carboxyphenyl)sulfonyl]hydrazide;
2-Dibenzofurancarboxylic acid, 2-[(4,5-dichloro-2-thienyl)sulfonyl]hydrazide;
2-Benzofurancarboxylic acid, 7-methoxy-, 2-[(3-chlorophenyl)sulfonyl]hydrazide;
2-Benzofurancarboxylic acid, 7-methoxy-, 2-[(3-fluorophenyl)sulfonyl]hydrazide;
2-Benzofurancarboxylic acid, 7-methoxy-, 2-[(2,3-dichlorophenyl)sulfonyl]hydrazide;
2-Benzofurancarboxylic acid, 7-methoxy-, 2-[(3-chloro-4fluorophenyl)sulfonyl]hydrazide;
2-Benzofurancarboxylic acid, 7-methoxy-, 2-[(5-chloro-2methoxyphenyl)sulfonyl]hydrazide;
2-Benzofurancarboxylic acid, 7-methoxy-, 2-[(5-bromo-2methoxyphenyl)sulfonyl]hydrazide;
2-Benzofurancarboxylic acid, 7-methoxy-, 2-[(4-ethylphenyl)sulfonyl]hydrazide;
2-Benzofurancarboxylic acid, 7-methoxy-, 2-[(3-methoxyphenyl)sulfonyl]hydrazide;
2-Dibenzofurancarboxylic acid, 2-[(2,3-dichlorophenyl)sulfonyl]hydrazide;
2-Naphthalenecarboxylic acid, 2-[(3-methylphenyl)sulfonyl]-hydrazide;
2-Naphthalenecarboxylic acid, 2-[(4-methylphenyl)sulfonyl]-hydrazide;
2-Naphthalenecarboxylic acid, 2-[[(2-(trifluoromethyl)phenyl]sulfonyl]hydrazide;
2-Naphthalenecarboxylic acid, 2-[[(4-(trifluoromethyl)phenyl]sulfonyl]hydrazide;
2-Naphthalenecarboxylic acid, 2-[(3-chloro-4-fluorophenyl)sulfonyl]hydrazide;
2-Naphthalenecarboxylic acid, 2-[(3-methoxyphenyl)sulfonyl]hydrazide;
2-Naphthalenecarboxylic acid, 2-[(3-chlorophenyl)sulfonyl]-hydrazide;
2-Naphthalenecarboxylic acid, 2-[(2-chlorophenyl)sulfonyl]-hydrazide;
2-Naphthalenecarboxylic acid, 2-[(3-fluorophenyl)sulfonyl]-hydrazide;
2-Naphthalenecarboxylic acid, 2-[(2,3-dichlorophenyl)sulfonyl]hydrazide;
2-Naphthalenecarboxylic acid, 2-[(4-ethylphenyl)sulfonyl]-hydrazide;
3-Quinolinecarboxylic acid, 2-[(4-pentylphenyl)sulfonyl)-hydrazide;
3-Quinolinecarboxylic acid, 2-[[2(trifluoromethyl)phenyl]sulfonyl]hydrazide;
3-Quinolinecarboxylic acid, 2-[[2,5-bis(2,2,2-trifluoroethoxyl)phenyl]sulfonyl]hydrazide;
3-Quinolinecarboxylic acid, 2-[(3-methylphenyl)sulfonyl]-hydrazide;
3-Quinolinecarboxylic acid, 2-[[2-(trifluoromethoxy)phenyl]sulfonyl]hydrazide;
3-Quinolinecarboxylic acid, 2-[(4-methylphenyl)sulfonyl]-hydrazide;
6-Quinolinecarboxylic acid, 2-[(3-methylphenyl)sulfonyl]-hydrazide;
3-Quinolinecarboxylic acid, 2-[(3-methylphenyl)sulfonyl]-hydrazide;
6-Quinolinecarboxylic acid, 2-[(3-chlorophenyl)sulfonyl]-hydrazide;
3-Quinolinecarboxylic acid, 2-[(3-chlorophenyl)sulfonyl]-hydrazide;
6-Quinolinecarboxylic acid, 2-[(3-fluorophenyl)sulfonyl]-hydrazide;
3-Quinolinecarboxylic acid, 2-[(3-fluorophenyl)sulfonyl]-hydrazide;
6-Quinolinecarboxylic acid, 2-[(2-trifluoromethylphenyl)sulfonyl]hydrazide;
3-Quinolinecarboxylic acid, 2-[(2-trifluoromethylphenyl)sulfonyl]hydrazide;
6-Quinolinecarboxylic acid, 2-[(2-trifluoromethoxyphenyl)sulfonyl]hydrazide;
3-Quinolinecarboxylic acid, 2-[(2-trifluoromethoxyphenyl)sulfonyl]hydrazide;
6-Quinolinecarboxylic acid, 2-[(3,5-dichlorophenyl)sulfonyl]hydrazide;
3-Quinolinecarboxylic acid, 2-[(3,5-dichlorophenyl)sulfonyl]hydrazide;
6-Quinolinecarboxylic acid, 2-[(3-chloro-4-fluorophenyl)sulfonyl]hydrazide;
3-Quinolinecarboxylic acid, 2-[(3-chloro-4-fluorophenyl)sulfonyl]hydrazide;
6-Quinolinecarboxylic acid, 2-[(3-methoxyphenyl)sulfonyl]-hydrazide;
3-Quinolinecarboxylic acid, 2-[(3-methoxyphenyl)sulfonyl]-hydrazide;
6-Quinolinecarboxylic acid, 2-[(3-bromophenyl)sulfonyl]-hydrazide;
3-Quinolinecarboxylic acid, 2-[(3-bromophenyl)sulfonyl]-hydrazide;
Benzofuran-2-carboxylic acid 2-(phenylsulfonyl)hydrazide;
7-Ethoxy-2-benzofurancarboxylic acid 2-(phenylsulfonyl)-hydrazide;
7-Chloro-benzofuran-2-carboxylic acid 2-(phenylsulfonyl)-hydrazide;
2,3-Dihydro-beazofuran-5-carboxylic acid 2-(phenylsulfonyl)hydrazide;
5-Chloro-benzofuran-2-carboxylic acid 2-(phenylsulfonyl)-hydrazide;
Isoquinoline-1-carboxylic acid, 2-(phenylsulfonyl)hydrazide;
1H-Indole-5-carboxylic acid, 2-(phenylsulfonyl)hydrazide;
Quinoline-3-carboxylic acid, 2-(phenylsulfonyl)hydrazide;
Quinoline-4-carboxylic acid, 2-(phenylsulfonyl)hydrazide;
1H-Indole-5-carboxylic acid, 2-[(3-chlorophenyl)sulfonyl]-hydrazide;
1H-Indole-5-carboxylic acid, 2-[(2-chlorophenyl)sulfonyl]-hydrazide;
1H-Indole-5-carboxylic acid, 2-[(3-fluorophenyl)sulfonyl]-hydrazide;
1H-Indole-5-carboxylic acid, 2-[(3-trifluoromethylphenyl)sulfonyl]hydrazide;
1H-Indole-5-carboxylic acid, 2-[(2-trifluoromethylphenyl)sulfonyl]hydrazide;
1H-Indole-5-carboxylic acid, 2-[(2-trifluoromethoxyphenyl)sulfonyl]hydrazide;
1H-Indole-5-carboxylic acid, 2-[(3trifluoromethoxyphenyl)sulfonyl]hydrazide;
1H-Indole-5-carboxylic acid, 2-[(3-bromophenyl)sulfonyl]-hydrazide;
1H-Indole-5-carboxylic acid, 2-[(3-methylphenyl)sulfonyl]-hydrazide;
1H-Indole-5-carboxylic acid, 2-[(2-methylphenyl)sulfonyl]-hydrazide;
6-Quinolinecarboxylic acid, 2-[(2-chlorophenyl)sulfonyl]-hydrazide;
6-Quinolinecarboxylic acid, 2-[(3-trifluoromethylphenyl)sulfonyl]hydrazide;
6-Quinolinecarboxylic acid, 2-[(3-trifluoromethoxyphenyl)sulfonyl]hydrazide;
6-Quinolinecarboxylic acid, 2-[(2-methylphenyl)sulfonyl]-hydrazide;
Dibenzofuran-2-carboxylic acid, 2-(4-fluoro-3methylphenylsulfonyl)hydrazide;
2-Naphthalenecarboxylic acid, 2-(phenylsulfonyl)hydrazide.

17. A method for preparing a compound of Formula I 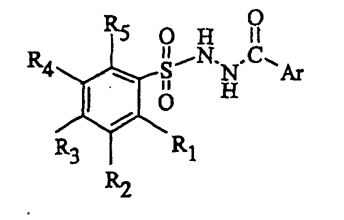 wherein:
R₃ is H, or F, Br, alkyl, carboxy, alkoxy, substituted alkoxy,
R₁, R₂, R₄, and R₅ are independently, H, halogen, alkyl, substituted alkyl alkoxy, substituted alkoxy, cyano, nitro, amino, alkylamine, and thioalkyl Ar is bicyclic heteroaryl, tricyclic heteroaryl, substituted bicyclic heteroaryl, except (6-methoxybenzofuran)-2-nyl, 3-quinolinyl which comprises reacting a hydrazide of the formula ArC(O)NH₂NH₂ with a sulfonyl halide of the formula: 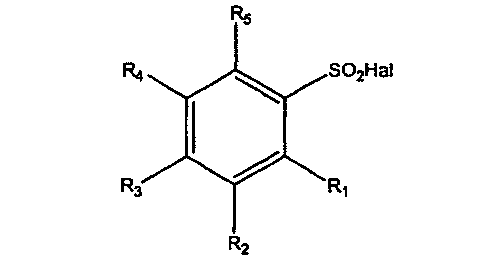
where R₁, R₂, R₃, R₄, R₅ and Ar are as defined above and Hal is halogen.

18. A medicine for treatment or or preventing neuronal loss associated with stroke, ischemia, CNS trauma, hypoglycemia or surgery, or treating a neurodegenerative disease, or treating or preventing the adverse consequences of the overstimulation of the excitatory amino acids, or treating anxiety, psychosis, glaucoma, CMV retinitis, diabetic retinopathy, urinary incontinence, migraine headache, convulsions, aminoglycoside antibiotics-induced hearing loss, Parkinson's disease, chronic pain, neuropathic pain, or inducing anesthesia, opioid tolerance or withdrawal, or enhancing cognition comprising a thereapeutically effective amount of a compound as claimed in claim 1.

19. A compound of Formula I as defined in claim 1 or a pharmaceutically acceptable salt, ester or amide thereof for use as a medicament.

## Patentansprüche

1. Eine Verbindung von Formel I 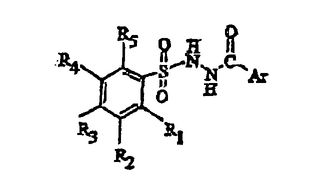 worin:
R₃ H oder F, Br, Alkyl, Carboxy, Alkoxy, substituiertes Alkoxy ist,
R₁, R₂, R₄ und R₅ sind unabhängig H, Halogen, Alkyl, substituiertes Alkyl, Alkoxy, substituiertes Alkoxy, Cyano, Nitro, Amino, Alkylamin und Thioalkyl, Ar ist bicyclisches Heteroaryl, tricyclisches Heteroaryl, substituiertes bicyclisches Heteroaryl, ausgenommen (6-Methoxybenzofuran)-2-nyl, 3-Chinolinyl;
wobei Ar gewählt wird aus der Gruppe bestehend aus: 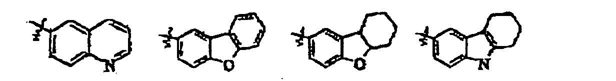 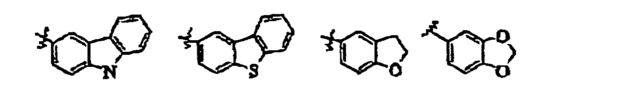 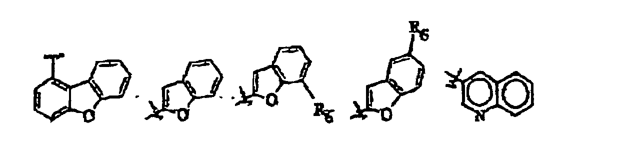 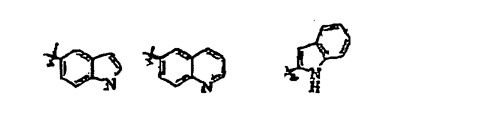 worin R₆ Alkoxy, substituiertes Alkoxy, Halogen, Aryloxy, substituiertes Aryloxy, Alkylamino, substituiertes Alkylamino, Arylalkylamino, substituiertes Arylalkylamino, Amid oder substituiertes Amid ist; oder ein pharmazeutisch verträgliches Salz, Ester oder Amid davon.

2. Eine Verbindung gemäß Anspruch 1, worin R₁, R₂, R₃, R₄ und R₅ Wasserstoff sind.

3. Eine Verbindung gemäß Anspruch 1, worin Ar ein tricyclisches Heteroaryl ist.

4. Eine Verbindung gemäß Anspruch 1, worin Ar ein bicyclisches Heteroaryl ist.

5. Eine Verbindung gemäß Anspruch 1, worin die Verbindung folgendes ist:
Dibenzofuran-2-carbonsäure, 2-(Phenylsulfonyl)hydrazid;
2,3,4,9-Tetrahydro-1H-carbazol-6-carbonsäure, 2(Phenylsulfonyl)-hydrazid;
Benzofuran-2-carbonsäure, 2-(Phenylsulfonyl)hydrazid;
5-Methoxy-2-benzofurancarbonsäure, 2-(Phenylsulfonyl)hydrazid;
7-Methoxy-2-benzofurancarbonsäure, 2-(Phenylsulfonyl)hydrazid;
7-Ethoxy-2-benzofurancarbonsäure, 2-(Phenylsulfonyl)hydrazid;
7-Chlor-benzofuran-2-carbonsäure, 2-(Phenylsulfonyl)hydrazid;
5-Chlor-benzofuran-2-carbonsäure, 2-(Phenylsulfonyl)hydrazid;
2,3-Dihydro-benzofuran-5-carbonsäure, 2-(Phenylsulfonyl)hydrazid;
Benzo[1,3] Dioxol-5-carbonsäure, 2(Phenylsulfonyl)hydrazid;
1H-Indol-5-carbonsäure, 2-(Phenylsulfonyl)hydrazid;
1-Dibenzofurancazbonsäure, 2-(Phenylsulfonyl)hydrazid;
Chinolin-6-carbonsäure, 2-(Phenylsulfonyl)hydrazid;
2-Dibenzofurancarbonsäure, 2-[(2-Methylphenyl)sulfonyl]hydrazid;
2-Dibenzofurancarbonsäure, 2-[(3-Methylphenyl)sulfonyl]hydrazid;
2-Dibenzofurancarbonsäure, 2-[(3-Chlorphenyl)sulfonyl]hydrazid;
2-Dibenzofurancarbonsäure, 2-[(2-Chlorphenyl)sulfonyl]hydrazid;
Dibenzofuran-2-carbonsäure, 2-(3,5-Dichlorphenylsulfonyl)hydrazid;
Chinolin-6-carbonsäure, 2-(Phenylsulfonyl)hydrazid;
Chinolin-8-carbonsäure, 2-(Phenylsulfonyl)hydrazid;
2-Benzofurancarbonsäure, 5-Chlor-2-[(4-fluorphenyl)]sulfonylhydrazid;
2-Benzofurancarbonsäure, 7-Methoxy, 2-[(4-Fluorphenyl)sulfonyl]hydrazid;
2-Dibenzofurancarbonsäure, 2-[(4-Fluorphenyl)sulfonyl]hydrazid;
2-Dibenzofurancarbonsäure, 2-[(2-Chlor-4-fluorphenyl)sulfonyl]hydrazid;
2-Dibenzofurancarbonsäure, 2-[(3-Cyanophenyl)sulfonyl]hydrazid;
2-Benzofurancarbonsäure, 5-Chlor-, 2-[(3-Cyanophenyl)sulfonyl)hydrazid;
2-Dibenzofurancarbonsäure; 2-[(2,6-Dichlorphenyl)sulfonyl]hydrazid;
2-Benzofurancarbonsäure, 5-Brom-, 2-(Phenylsulfonyl)hydrazid;
2-Benzofurancarbonsäure, 5-Chlor-, 2-[(2-Chlor-4fluorphenyl)sulfonyl]hydrazid;
2-Benzofurancarbonsäure, 5-Chlor-, 2-[(3,5-Dichlorphenyl)sulfonyl]hydraaid:
2-Dibenzofurancarbonsäure, 2-[[3,5-bis(trifluormethyl)phenyl]-sulfonyl]hydrazid;
2-Benzofurancarbonsäure, 5-Chlor, 2-[(3-Chlor-4fluorphenyl)sulfonyl]hydrazid;
2-Benzofurancarbonsäure, 5-Nitro-, 2-(Phenylsulfonyl)hydrazid;
2-Dibenzofurancarbonsäure, 2-[(3-fluorphenyl)sulfonyl]hydrazid;
2-Dibenzofurancarbonsäure, 2-[(5-Fluor-2-methylphenyl)sulfonyl]hydrazid;
2-Dibenzofurancarbonsäure, 2-[(3,4-Difluorphenyl)sulfonyl)hydrazid;
2-Dibenzofurancarbonsäure, 2-[(3-Methoxyphenyl)sulfonyl]hydrazid;
2-Dibenzofurancarbonsäure, 2-[(3-Chlor-4-fluorphenyl)sulfonyl]hydrazid;
2-Benzofurancarbonsäure, 5-Chlor-2-[(3-fluorphenyl)sulfonyl]hydrazid;
2-Benzofurancarbonsäure, 5-Chlor-2-[(3,4-difluorphenyl)sulfonyl]hydrazid;
2-Benzofurancarbonsäure, 5-Chlor-2-[(2-chlorphenyl)sulfonyl]hydrazid;
2-Benzofurancarbonsäure, 5-Chlor-2-[(3-chlorphenyl)sulfonyl]hydrazid;
2-Benzofurancarbonsäure, 5-Chlor-2-[(2-bromphenyl)sulfonyl]hydrazid;
2-Benzofurancarbonsäure, 5-Chlor-2-[(3-bromphenyl)sulfonyl]hydrazid;
2-Benzofurancarbonsäure, 5-Chlor-2-[(2-methylphenyl)sulfonyl]hydrazid;
2-Benzofurancarbonsäure, 5-Chlor-2-[(3-methylphenyl)sulfonyl]hydrazid;
2-Benzofurancarbonsäure, 5-Chlor-2-[(3-methoxyphenyl)sulfonyl]hydrazid;
2-Benzofurancarbonsäure, 5-Chlor-2-[(3-chlor-2methylphenyl)sulfonyl]hydrazid;
2-Benzofurancarbonsäure, 5-Chlor-2-[(5-fluor-2methylphenyl)sulfonyl]hydrazid;
2-Benzofurancarbonsäure, 5-Chlor-2-[[(2-(trifluormethyl)phenyl]sulfonyl]hydrazid;
2-Benzofurancarbonsäure, 5-Chlor-2-[[2-(trifluormethoxy)phenyl]sulfonyl]hydrazid;
2-Benzofurancarbonsäure, 7-Methoxy-, 2-[[2(Trifluormethyl)-phenyl]sulfonyl]hydrazid;
2-Benzofurancarbonsäure, 7-Methoxy-, 2-[(3-Methylphenyl)sulfonyl]hydrazid;
2-Benzofurancarbonsäure, 5-Methoxy-3-(1-methylethoxy), 2-(Phenylsulfonyl)hydrazid;
2-Benzofurancarbonsäure, 3,5-Dimethoxy-, 2-(Phenylsulfonyl)-hydrazid;
2-Benzofurancarbonsäure, 3,6-Dimethoxy-, 2-(Phenylsulfonyl)-hydrazid;
2-Benzofurancarbonsäure, 4,6-Dimethoxy-, 2-(Phenylsulfonyl)-hydrazid;
2-Benzofurancarbonsäure, 3,7-Dimethoxy-, 2-(Phenylsulfonyl)-hydrazid;
2-Benzofurancarbonsäure, 3,7-Dimethoxy-, 2-[(2-Trifluormethylphenyl)sulfonyl)hydrazid;
2-Benzofurancarbonsäure, 3,7-Dimethoxy-, 2-[(4-Fluorphenyl)-sulfonyl]hydrazid;
2-Benzofurancarbonsäure, 3,7-Dimethoxy-, 2-[(3-Methylphenyl)sulfonyl]hydrazid;
2-Benzofurancarbonsäure, 3-Methoxy-5-phenyl-, 2-(Phenylsulfonyl)hydrazid;
2-Benzofurancarbonsäure, 3-Ethoxy-5-phenyl-, 2-(Phenylsulfonyl)hydrazid;
2-Benzofurancarbonsäure, 3-Methoxy-7-phenyl-, 2-(Phenylsulfonyl)hydrazid;
Naphto[2,3-b]furan-2-carbonsäure, 3-Methoxy-, 2-(Phenylsulfonyl)hydrazid;
2-Benzofurancarbonsäure, 5,7-Dichlor 2-(Phenylsulfonyl)sulfonylhydrazid;
2-Benzofurancarbonsäure, 5,7-Dichlor 2-[[2-(Trifluormethyl)-phenyl)]sulfonyl]hydrazid;
2-Benzofurancarbonsäure, 5,7-Dichlor 2-[(2-Methylphenyl)sulfonyl]hydrazid;
2-Benzofurancarbonsäure, 5,7-Dichlor 2-[(3-Methylphenyl)sulfonyl]hydrazid;
2-Benzofurancarbonsäure, 5,7-Dichlor 2-[(2-Bromphenyl)sulfonyl]hydrazid;
2-Benzofurancarbonsäure, 5,7-Dichlor 2-[(3-Bromphenyl)sulfonyl]hydrazid;
2-Benzofurancarbonsäure, 5,7-Dichlor 2-[(3-Chlorphenyl)sulfonyl]hydrazid;
2-Benzofurancarbonsäure, 5,7-Dichlor 2-[[2(Trifluormethoxy)phenyl]sulfonyl]hydrazid;
Benzofurancarbonsäure, 5-Methyl-, 2-(Phenylsulfonyl)hydrazid;
2-Benzofurancarbonsäure, 5-Chlor-3-methoxy, 2-(Phenylsulfonyl)hydrazid;
2-Benzofurancarbonsäure, 5-Methyl-, 2-[[2-(Trifluormethyl)phenyl]sulfonyl]hydrazid;
2-Benzofurancarbonsäure, 5-Methyl-, 2-([3-Methylphenyl)sulfonyl]hydrazid;
2-Benzofurancarbonsäure, 5-Methyl-, 2-[2-(Methylphenyl)sulfonyl]hydrazid;
2-Benzofurancarbonsäure, 5-Methyl-, 2-[(3-Chlor-2-methylphenyl)sulfonyl]hydrazid;
2-Benzofurancarbonsäure, 5-Methyl-, 2-[3-(Bromphenyl)sulfonyl)hydrazid;
2-Benzofurancarbonsäure, 5-Methyl-, 2-[(3-Chlorphenyl)sulfonyl]hydrazid;
2-Benzofurancarbonsäure, 5-Methyl-, 2-[[2-(Trifluormethoxy)-phenyl]sulfonyl]hydrazid;
2-Benzofurancarbonsäure, 7-Methyl-, 2-(Phenylsulfonyl)hydrazid;
2-Benzofurancarbonsäure, 4-Methyl-, 2-(Phenylsulfonyl)hydrazid;
2-Benzofurancarbonsäure, 3-Methyl-, 2-(Phenylsulfonyl)hydrazid;
2-Benzofurancarbonsäure, 3,5-Dimethyl-, 2-(Phenylsulfonyl)hydrazid;
2-Benzofurancarbonsäure, 5-Chlor-3-methyl, 2-(Phenylsulfonyl)hydrazid;
2-Benzofurancarbonsäure, 5-Amino-, 2-(Phenylsulfonyl)hydrazid;
2-Dibenzofurancarbonsäure, 2-[(2-Fluorphenyl)sulfonyl]hydrazid;
2-Dibenzofurancarbonsäure, 2-(2,6-Difluorphenylsulfonyl)hydrazid; .
2-Dibenzofurancarbonsäure, 2-[(2-Bromphenyl)sulfonyl]hydrazid;
2-Dibenzofurancarbonsäure, 2-[(3-Bromphenyl)sulfonyl]hydrazid;
2-Dibenzofurancarbonsäure, 2-[(4-Methyl-3-nitrophenyl)sulfonyl]hydrazid;
2-Dibenzofurancarbonsäure, 2-[2-Chlor-5-(trifluormethyl)phenyl]sulfonyl)hydrazid;
2-Dibenzofurancarbonsäure, 2-[(2-Trifluormethoxyphenyl)sulfonyl]hydrazid;
2-Dibenzofurancarbonsäure, 2-[(4-Brom-2-trifluormethoxyphenyl)sulfonyl]hydrazid;
2-Dibenzofurancarbonsäure, 2-[(2-Nitrophenyl)sulfonyl]hydrazid;
2-Dibenzofurancarbonsäure, 2-[(4-Carboxyphenyl)sulfonyl]hydrazid;
2-Dibenzofurancarbonsäure, 2-[(4,5-Dichlor-2-thienyl)sulfonyl]hydrazid;
2-Benzofurancarbonsäure, 7-Methoxy-, 2-[(3-Chlorphenyl)sulfonyl]hydrazid;
2-Benzofurancarbonsäure, 7-Methoxy-, 2-[(3-Fluorphenyl)sulfonyl]hydrazid;
2-Benzofurancarbonsäure, 7-Methoxy, 2-[(2,3-Dichlorphenyl)sulfonyl]hydrazid;
2-Benzofurancarbonsäure, 7-Methoxy-, 2-[(3-Chlor-4-fluorphenyl)sulfonyl]hydrazid;
2-Benzofurancarbonsäure, 7-Methoxy-, 2-[(5-Chlor-2-methoxyphenyl)sulfonyl]hydrazid;
2-Benzofurancarbonsäure, 7-Methoxy-, 2-[(5-Brom-2-methoxyphenyl)sulfonyl]hydrazid;
2-Benzofurancarbonsäure, 7-Methoxy-, 2-[(4-Ethylphenyl)sulfonyl]hydrazid;
2-Benzofurancarbonsäure, 7-Methoxy-, 2-[(3-Methoxyphenyl)sulfonyl]hydrazid;
2-Dibenzofurancarbonsäure, 2-[(2,3-Dichlorphenyl)sulfonyl]hydrazid;
2-Naphthalencarbonsäure, 2-[(3-Methylphenyl)sulfonyl]hydrazid;
2-Naphthalencarbonsäure, 2-[[(2(Trifluormethyl)phenyl]sulfonyl]hydrazid;
2-Naphthalencarbonsäure, 2-[[(4-(Trifluormethyl)phenyl]sulfonyl]hydrazid;
2-Naphthalencarbonsäure, 2-[(3-Chlor-4-fluorphenyl)sulfonyl]hydrazid;
2-Naphthalencarbonsäure, 2-[(3-Methoxyphenyl)sulfonyl]hydrazid;
2-Naphthalencarbonsäure, 2-[(3-Chlorphenyl)sulfonyl]hydrazid;
2-Naphthalencarbonsäure, 2-[(2-Chlorphenyl)sulfonyl]hydrazid;
2-Naphthalencarbonsäure, 2-[(3-Fluorphenyl)sulfonyl]hydrazid;
2-Naphthalencarbonsäure, 2-[(2,3-Dichlorphenyl)sulfonyl]hydrazid;
2-Naphthalencarbonsäure, 2-[(4-Ethylphenyl)sulfonyl]hydrazid;
3-Chinolincarbonsäure, 2-[(4-Pentylphenyl)sulfonyl)hydrazid;
3-Chinolincarbonsäure, 2-[[2(Trifluormethyl)phenyl]sulfonyl]hydrazid;
3-Chinolincarbonsäure, 2-[[2,5-bis(2,2,2-Trifluorethoxyl)phenyl]sulfonyl]hydrazid;
3-Chinolincarbonsäure, 2-[(3-Methylphenyl)sulfonyl]hydrazid;
3-Chinolincarbonsäure, 2-[[2-(Trifluormethoxy)phenyl]sulfonyl]hydrazid;
3-Chinolincarbonsäure, 2-[(4-Methylphenyl)sulfonyl]hydrazid;
6-Chinolincarbonsäure, 2-[(3-Methylphenyl)sulfonyl)hydrazid;
3-Chinolincarbonsäure, 2-[(3-Methylphenyl)sulfonyl)hydrazid;
6-Chinolincarbonsäure, 2-[(3-Chlorphenyl)sulfonyl)hydrazid;
3-Chinolincarbonsäure, 2-[(3-Chlorphenyl)sulfonyl)hydrazid;
6-Chinolincarbonsäure, 2-[(3-Fluorphenyl)sulfonyl)hydrazid;
3-Chinolincarbonsäure, 2-[(3-Fluorphenyl)sulfonyl)hydrazid;
6-Chinolincarbonsäure, 2-[(2-Trifluormethylphenyl)sulfonyl)-hydrazid;
3-Chinolincarbonsäure, 2-[(2-Trifluormethylphenyl)sulfonyl)-hydrazid;
6-Chinolincarbonsäure, 2-[(2-Trifluormethoxyphenyl)sulfonyl)-hydrazid;
3-Chinolincarbonsäure, 2-[(2-Trifluormethoxyphenyl)sulfonyl)-hydrazid;
6-Chinolincarbonsäure, 2-[(3,5-Dichlorphenyl)sulfonyl]hydrazid;
3-Chinolincarbonsäure, 2-[(3,5-Dichlorphenyl)sulfonyl]hydrazid;
6-Chinolincarbonsäure, 2-[(3-Chlor-4-fluorphenyl)sulfonyl]hydrazid;
3-Chinolincarbonsäure, 2-[(3-Chlor-4-fluorphenyl)sulfonyl]hydrazid;
6-Chinolincarbonsäure, 2-[(3-Methoxyphenyl)sulfonyl]hydrazid;
3-Chinolincarbonsäure, 2-[(3-Methoxyphenyl)sulfonyl]hydrazid;
6-Chinolincarbonsäure, 2-[(3-Bromphenyl)sulfonyl]-hydrazid;
3-Chinolincarbonsäure, 2-[(3-Bromphenyl)sulfonyl]-hydrazid;
Benzofuran-2-carbonsäure 2-(Phenylsulfonyl)hydrazid;
7-Ethoxy-2-benzofurancarbonsäure 2-(Phenylsulfonyl)hydrazid;
7-Chlor-benzofuran-2-carbonsäure 2-(Phenylsulfonyl)hydrazid;
2,3-Dihydro-benzofuran-5-carbonsäure 2-(Phenylsulfonyl)hydrazid;
5-Chlor-benzofuran-2-carbonsäure 2-(Phenylsulfonyl)hydrazid;
Isochinolin-1-carbonsäure 2-(Phenylsulfonyl)hydrazid;
1H-Indol-5-carbonsäure 2-(Phenylsulfonyl)hydrazid;
Chinolin-3-carbonsäure 2-(Phenylsulfonyl)hydrazid;
Chinolin-4-carbonsäure 2-(Phenylsulfonyl)hydrazid;
1H-Indol-5-carbonsäure, 2-[(3-Chlorphenyl)sulfonyl]hydrazid;
1H-Indol-5-carbonsäure, 2-[(2-Chlorphenyl)sulfonyl]hydrazid;
1H-Indol-5-carbonsäure, 2-[(3-Fluorphenyl)sulfonyl]hydrazid;
1H-Indol-5-carbonsäure, 2-[(3-Trifluormethylphenyl)sulfonyl]hydrazid;
1H-Indol-5-carbonsäure, 2-[(2-Trifluormethylphenyl)sulfonyl]hydrazid;
1H-Indol-5-carbonsäure, 2-[(2-Trifluormethoxyphenyl)sulfonyl]hydrazid;
1H-Indol-5-carbonsäure, 2-[(3-Trifluormethoxyphenyl)sulfonyl]hydrazid;
1H-Indol-5-carbonsäure, 2-[(3-Bromphenyl)sulfonyl]hydrazid;
1H-Indol-5-carbonsäure, 2-[(3-Methylphenyl)sulfonyl]hydrazid;
1H-Indol-5-carbonsäure, 2-[(2-Methylphenyl)sulfonyl]hydrazid;
6-Chinolincarbonsäure, 2-[(2-Chlorphenyl)sulfonyl]hydrazid;
6-Chinolincarbonsäure, 2-[(3-Trifluormethylphenyl)sulfonyl]-hydrazid;
6-Chinolincarbonsäure, 2-[(3-Trifluormethoxyphenyl)sulfonyl]hydrazid;
6-Chinolincarbonsäure, 2-[(2-Methylphenyl)sulfonyl]hydrazid;
Dibenzofuran-2-carbonsäure, 2-(4-Fluor-3-methylphenylsulfonyl)hydrazid.

6. Eine pharmazeutische Zusammensetzung, die eine Verbindung von Anspruch 1 und einen pharmazeutisch verträglichen Träger umfaßt.

7. Verwendung einer Verbindung von Formel I wie in Anspruch 1 beansprucht, zur Herstellung eines Medikaments zur Behandlung oder Vorbeugung von Nervenschwund im Zusammenhang mit Schlaganfall, Ischämie, ZNS-Trauma, Hypoglykämie oder Operation, oder zur Behandlung einer neurodegenerativen Krankheit, oder zur Behandlung oder Vorbeugung der nachteiligen Folgen der Überstimulation der exzitatorischen Aminosäuren, oder zur Behandlung von Angst, Psychose, Glaukom, CMV-Retinitis, diabetischer Retinopathie, Harninkontinenz, Migränekopfschmerz, Krämpfen, Aminoglykosid-Antibiotika-induziertem Gehörverlust, Parkinson-Krankheit, chronischem Schmerz, neuropathischem Schmerz, oder zur Induzierung von Anästhesie, Opioidtoleranz oder -entzug, oder zur Verbesserung der Wahrnehmung.

8. Die Verwendung von Anspruch 7, worin Ar ein bicyclisches Heteroaryl ist.

9. Die Verwendung von Anspruch 7, worin Ar ein tricyclisches Heteroaryl ist.

10. Die Verwendung von Anspruch 7, worin R₁, R₂, R₃, R₄ und R₅ Wasserstoff sind.

11. Die Verwendung von Anspruch 7, worin die Verbindung folgendes ist:
Dibenzofuran-2-carbonsäure, 2-(Phenylsulfonyl)hydrazid;
2,3,4,9-Tetrahydro-1H-carbazol-6-carbonsäure, 2-(Phenylsulfonyl)-hydrazid;
Benzofuran-2-carbonsäure, 2-(Phenylsulfonyl)hydrazid;
5-Methoxy-2-benzofurancarbonsäure, 2-(Phenylsulfonyl)hydrazid;
7-Methoxy-2-benzofurancarbonsäure, 2-(Phenylsulfonyl)hydrazid;
7-Ethoxy-2-benzofurancarbonsäure, 2-(Phenylsulfonyl)hydrazid;
7-Chlor-benzofuran-2-carbonsäure, 2-(Phenylsulfonyl)hydrazid;
5-Chlor-benzofuran-2-carbonsäure, 2-(Phenylsulfonyl)hydrazid;
2,3-Dihydro-benzofuran-5-carbonsäure, 2-(Phenylsulfonyl)hydrazid;
Benzo[1,3] Dioxol-5-carbonsäure, 2-(Phenylsulfonyl)hydrazid;
1H-Indol-5-carbonsäure, 2-(Phenylsulfonyl)hydrazid;
1-Dibenzofurancarbonsäure, 2-(Phenylsulfonyl)hydrazid;
Chinolin-6-carbonsäure, 2-(Phenylsulfonyl)hydrazid;
2-Dibenzofurancarbonsäure, 2-[(2-Methylphenyl)sulfonyl]hydrazid;
2-Dibenzofurancarbonsäure, 2-[(3-Methylphenyl)sulfonyl]hydrazid;
2-Dibenzofurancarbonsäure, 2-[(3-Chlorphenyl)sulfonyl]hydrazid;
2-Dibenzofurancarbonsäure, 2-[(2-Chlorphenyl)sulfonyl]hydrazid;
Dibenzofuran-2-carbonsäure, 2-(3,5-Dichlorphenylsulfonyl)hydrazid;
Dibenzofuran-2-carbonsäure, 2-(3-Chlor-2-methylphenylsulfonyl)hydrazid;
Chinolin-6-carbonsäure, 2-(Phenylsulfonyl)hydrazid;
Chinolin-8-carbonsäure, 2-(Phenylsulfonyl)hydrazid;
2-Benzofurancarbonsäure, 5-Chlor-2-[(4-fluorphenyl)]sulfonylhydrazid;
2-Benzofurancarbonsäure, 7-Methoxy-, 2-[(4-Fluorphenyl)sulfonyl]hydrazid;
2-Dibenzofurancarbonsäure, 2-[(4-Fluorphenyl)sulfonyl]hydrazid;
2-Dibenzofurancarbonsäure, 2-[(2-Chlor-4-fluorphenyl)sulfonyl]hydrazid;
2-Dibenzofurancarbonsäure, 2-[(3-Cyanophenyl)sulfonyl]hydrazid;
2-Benzofurancarbonsäure, 5-Chlor-, 2-[(3-Cyanophenyl)sulfonyl]hydrazid;
2-Dibenzofurancarbonsäure, 2-[(2,6-Dichlorphenyl)sulfonyl]hydrazid;
2-Benzofurancarbonsäure, 5-Brom-, 2-(Phenylsulfonyl)hydrazid;
2-Benzofurancarbonsäure, 5-Chlor-, 2-[(2-Chlor-4-fluorphenyl)sulfonyl]hydrazid;
2-Benzofurancarbonsäure, 5-Chlor-, 2-[(3,5-Dichlorphenyl)sulfonyl]hydrazid;
2-Dibenzofurancarbonsäure, 2-[[3,5-bis(Trifluormethyl)phenyl]-sulfonyl]hydrazid;
2-Benzofurancarbonsäure, 5-Chlor, 2-[(3-Chlor-4-fluorphenyl)sulfonyl]hydrazid;
2-Benzofurancarbonsäure, 5-Nitro-, 2-(Phenylsulfonyl)hydrazid;
2-Dibenzofurancarbonsäure, 2-[(3-Fluorphenyl)sulfonyl]hydrazid;
2-Dibenzofurancarbonsäure, 2-[(5-Fluor-2-methylphenyl)sulfonyl]hydrazid;
2-Dibenzofurancarbonsäure, 2-[(3,4-Difluorphenyl)sulfonyl]hydrazid;
2-Dibenzofurancarbonsäure, 2-[(3-Chlor-4-fluorphenyl)sulfonyl]hydrazid;
2-Dibenzofurancarbonsäure, 2-[(3-Methoxyphenyl)sulfonyl]hydrazid;
2-Benzofurancarbonsäure, 5-Chlor-2-[(3-fluorphenyl)sulfonyl]hydrazid;
2-Benzofurancarbonsäure, 5-Chlor-2-[(3,4-Difluorphenyl)sulfonyl]hydrazid;
2-Benzofurancarbonsäure, 5-Chlor-2-[(2-chlorphenyl)sulfonyl]hydrazid;
2-Benzofurancarbonsäure, 5-Chlor-2-[(3-chlorphenyl)sulfonyl]hydrazid;
2-Benzofurancarbonsäure, 5-Chlor-2-[(2-bromphenyl)sulfonyl]hydrazid;
2-Benzofurancarbonsäure, 5-Chlor-2-[[3-bromphenyl)sulfonyl]hydrazid;
2-Benzofurancarbunsäure, 5-Chlor-2-[(2-methylphenyl)sulfonyl]hydrazid;
2-Benzofurancarbonsäure, 5-Chlor-2-[(3-methylphenyl)sulfonyl]hydrazid;
2-Benzofurancarbonsäure, 5-Chlor-2-[(3-methoxyphenyl)sulfonyl]hydrazid;
2-Benzofurancarbonsäure, 5-Chlor-2-[(3-chlor-2-methylphenyl)-sulfonyl]hydrazid;
2-Benzofurancarbonsäure, 5-Chlor-2-[(5-fluor-2-methylphenyl)-sulfonyl]hydrazid;
2-Benzofurancarbonsäure, 5-Chlor-2-[[(2-(trifluormethyl)phenyl)-sulfonyl]hydrazid;
2-Benzofurancarbonsäure, 5-Chlor-2-[[(2-(trifluormethoxy)phenyl)-sulfonyl]hydrazid;
2-Benzofurancarbonsäure, 7-Methoxy-, 2-[[2-(trifluormethyl)-phenyl)-sulfonyl]hydrazid;
2-Benzofurancarbonsäure, 7-Methoxy-, 2-[(3-Methylphenyl)sulfonyl]hydrazid;
2-Benzofurancarbonsäure, 6-Methoxy, 2-(Phenylsulfonyl)hydrazid;
2-Benzofurancarbonsäure, 5-Methoxy-3-(1-methylethoxy), 2-(Phenylsulfonyl)hydrazid;
2-Benzofurancarbonsäure, 3,5-Dimethoxy-, 2-(Phenylsulfonyl)-hydrazid;
2-Benzofurancarbonsäure, 3,6-Dimethoxy-, 2-(Phenylsulfonyl)-hydrazid;
2-Benzofurancarbonsäure, 4,6-Dimethoxy-, 2-(Phenylsulfonyl)-hydrazid;
2-Benzofurancarbonsäure, 3,7-Dimethoxy-, 2-(Phenylsulfonyl)-hydrazid;
2-Benzofurancarbonsäure, 3,7-Dimethoxy-, 2-[(2-Trifluormethylphenyl)sulfonyl]hydrazid;
2-Benzofurancarbonsäure, 3,7-Dimethoxy-, 2-[(4-(Fluorphenyl)-sulfonyl]hydrazid;
2-Benzofurancarbonsäure, 3,7-Dimethoxy-, 2-[(3-Methylphenyl)sulfonyl]hydrazid;
2-Benzofurancarbonsäure, 3-Methoxy-5-phenyl-, 2-(Phenylsulfonyl)hydrazid;
2-Benzofurancarbonsäure, 3-Ethoxy-5-phenyl-, 2-(Phenylsulfonyl)hydrazid;
2-Benzofurancarbonsäure, 3-Methoxy-7-phenyl-, 2-(Phenylsulfonyl)hydrazid;
Naptho[2,3-b]furan-2-carbonsäure, 3-Methoxy-, 2-(Phenylsulfonyl)hydrazid;
2-Benzofurancarbonsäure, 5,7-Dichlor 2-(Phenylsulfonyl)sulfonylhydrazid;
2-Benzofurancarbonsäure, 5,7-Dichlor 2-[[2-(Trifluormethyl)-phenyl]sulfonyl]hydrazid;
2-Benzofurancarbonsäure, 5,7-Dichlor 2-[(2-Methylphenyl)sulfonyl]hydrazid;
2-Benzofurancarbonsäure, 5,7-Dichlor, 2-[(3-Methylphenyl)sulfonyl]hydrazid;
2-Benzofurancarbonsäure, 5,7-Dichlor, 2-[(2-Bromphenyl)sulfonyl]hydrazid;
2-Benzofurancarbonsäure, 5,7-Dichlor, 2-[(3-Bromphenyl)sulfonyl]hydrazid;
2-Benzofurancarbonsäure, 5,7-Dichlor, 2-[(3-Chlorphenyl)sulfonyl]hydrazid;
2-Benzofurancarbonsäure, 5,7-Dichlor, 2-[[2-(Trifluormethoxy)phenyl]sulfonyl]hydrazid;
Benzofurancarbonsäure, 5-Methyl-, 2-(Phenylsulfonyl)hydrazid;
2-Benzofurancarbonsäure, 5-Chlor-3-methoxy, 2-(Phenylsulfonyl)hydrazid;
2-Benzofurancarbonsäure, 5-Methyl-, 2-[[2-(Trifluormethyl)phenyl]sulfonyl]hydrazid;
2-Benzofurancarbonsäure, 5-Methyl-, 2-([3-Methylphenyl)sulfonyl]hydrazid;
2-Benzofurancarbonsäure, 5-Methyl-, 2-[2-(Methylphenyl)sulfonyl]hydrazid;
2-Benzofurancarbonsäure, 5-Methyl-, 2-[(3-Chlor-2-methylphenyl)sulfonyl]hydrazid;
2-Benzofurancarbonsäure, 5-Methyl-, 2-[3-(Bromphenyl)sulfonyl]hydrazid;
2-Benzofurancarbonsäure, 5-Methyl-, 2-[(3-Chlorphenyl)sulfonyl]hydrazid;
2-Benzofurancarbonsäure, 5-Methyl-, 2-[[2-(Trifluormethoxy)-phenyl]sulfonyl]hydrazid;
2-Benzofurancarbonsäure, 7-Methyl-, 2-(Phenylsulfonyl)hydrazid;
2-Benzofurancarbonsäure, 4-Methyl-, 2-(Phenylsulfonyl)hydrazid;
2-Benzofurancarbonsäure, 3-Methyl-, 2-(Phenylsulfonyl)hydrazid;
2-Benzofurancarbonsäure, 3,5-Dimethyl-, 2-(Phenylsulfonyl)hydrazid;
2-Benzofurancarbonsäure, 5-Chlor-3-methyl, 2-(Phenylsulfonyl)hydrazid;
2-Benzofurancarbonsäure, 5-Amino-, 2-(Phenylsulfonyl)hydrazid;
2-Dibenzofurancarbonsäure, 2-[(2-Fluorphenyl)sulfonyl]hydrazid;
2-Dibenzofurancarbonsäure, 2-(2,6-Difluorphenylsulfonyl)hydrazid;
2-Dibenzofurancarbonsäure, 2-[(2-Bromphenyl)sulfonyl]hydrazid;
2-Dibenzofurancarbonsäure, 2-[(3-Bromphenyl)sulfonyl]hydrazid;
2-Dibenzofurancarbonsäure, 2-[(4-Methyl-3-nitrophenyl)sulfonyl]hydrazid;
2-Dibenzofurancarbonsäure, 2-[2-chlor-5-(trifluormethyl)phenyl]sulfonyl)hydrazid;
2-Dibenzofurancarbonsäure, 2-[(2-Trifluormethoxyphenyl)sulfonyl]hydrazid;
2-Dibenzofurancarbonsäure, 2-[(4-Brom-2-trifluormethoxyphenyl)sulfonyl]hydrazid;
2-Dibenzofurancarbonsäure, 2-[(2-Nitrophenyl)sulfonyl]hydrazid;
2-Dibenzofurancarbonsäure, 2-[(4-Carboxyphenyl)sulfonyl]hydrazid;
2-Dibenzofurancarbonsäure, 2-[(4,5-Dichlor-2-thienyl)sulfonyl]hydrazid;
2-Benzofurancarbonsäure, 7-Methoxy-, 2-[(3-Chlorphenyl)sulfonyl]hydrazid;
2-Benzofurancarbonsäure, 7-Methoxy-, 2-[(3-Fluorphenyl)sulfonyl]hydrazid;
2-Benzofurancarbonsäure, 7-Methoxy-, 2-[(2,3-Dichlorphenyl)-sulfonyl]hydrazid;
2-Benzofurancarbonsäure, 7-Methoxy-, 2-[(3-Chlor-4-fluorphenyl)sulfonyl]hydrazid;
2-Benzofurancarbonsäure, 7-Methoxy-, 2-[(5-Chlor-2-methoxyphenyl)sulfonyl]hydrazid;
2-Benzofurancarbonsäure, 7-Methoxy-, 2-[(5-Brom-2-methoxyphenyl)sulfonyl]hydrazid;
2-Benzofurancarbonsäure, 7-Methoxy-, 2-[(4-Ethylphenyl)sulfonyl]hydrazid;
2-Benzofurancarbonsäure, 7-Methoxy-, 2-[(3-Methoxyphenyl)sulfonyl]hydrazid;
2-Dibenzofurancarbonsäure, 2-[(2,3-Dichlorphenyl)sulfonyl]hydrazid;
2-Naphthalencarbonsäure, 2-[(3-Methylphenyl)sulfonyl]hydrazid;
2-Naphthalencarbonsäure, 2-[(4-Methylphenyl)sulfonyl]hydrazid;
2-Naphthalencarbonsäure, 2-[[(2-Trifluormethyl)phenyl]sulfonyl]hydrazid;
2-Naphthalencarbonsäure, 2-[[(4-Trifluormethyl)phenyl]sulfonyl]hydrazid;
2-Naphthalencarbonsäure, 2-[(3-Chlor-4-fluorphenyl)sulfonyl]hydrazid;
2-Naphthalencarbonsäure, 2-[(3-Methoxyphenyl)sulfonyl]hydrazid;
2-Naphthalencarbonsäure, 2-[(3-Chlorphenyl)sulfonyl]hydrazid;
2-Naphthalencarbonsäure, 2-[(2-Chlorphenyl)sulfonyl]hydrazid;
2-Naphthalencarbonsäure, 2-[(3-Fluorphenyl)sulfonyl]hydrazid;
2-Naphthalencarbonsäure, 2-(2-Thienylsulfonyl)hdrazid;
2-Naphthalencarbonsäure, 2-[(2,3-Dichlorphenyl)sulfonyl]hydrazid;
2-Naphthalencarbonsäure, 2-[(4-Ethylphenyl)sulfonyl]hydrazid;
3-Chinolincarbonsäure, 2-[(4-Pentylphenyl)sulfonyl)hydrazid;
3-Chinolincarbonsäure, 2-[[2-(Trifluormethyl)phenyl]sulfonyl]hydrazid;
3-Chinolincarbonsäure, 2-[[2,5-bis(2,2,2-Trifluorethoxyl)phenyl]sulfonyl]hydrazid;
3-Chinolincarbonsäure, 2-[(3-Methylphenyl)sulfonyl]hydrazid;
3-Chinolincarbonsäure, 2-[[2-(Trifluormethoxy)phenyl]sulfonyl]hydrazid;
3-Chinolincarbonsäure, 2-[(4-Methylphenyl)sulfonyl]hydrazid;
6-Chinolincarbonsäure, 2-[(3-Methylphenyl)sulfonyl]hydrazid;
3-Chinolincarbonsäure, 2-[(3-Methylphenyl)sulfonyl]hydrazid;
6-Chinolincarbonsäure, 2-[(3-Chlorphenyl)sulfonyl]hydrazid;
3-Chinolincarbonsäure, 2-[(3-Chlorphenyl)sulfonyl]hydrazid;
6-Chinolincarbonsäure, 2-[(3-Fluorphenyl)sulfonyl)hydrazid;
3-Chinolincarbonsäure, 2-[(3-Fluorphenyl)sulfonyl]hydrazid;
6-Chinolincarbonsäure, 2-[(2-Trifluormethylphenyl)sulfonyl]-hydrazid;
3-Chinolincarbonsäure, 2-[(2-(Trifluormethylphenyl)sulfonyl]-hydrazid;
6-Chinolincarbonsäure, 2-[(2-Trifluormethoxyphenyl)sulfonyl]hydrazid;
3-Chinolincarbonsäure, 2-[(2-Trifluormethoxyphenyl)sulfonyl]hydrazid;
6-Chinolincarbonsäure, 2-[(3,5-Dichlorphenyl)sulfonyl]hydrazid;
3-Chinolincarbonsäure, 2-[(3,5-Dichlorphenyl)sulfonyl]hydrazid;
6-Chinolincarbonsäure, 2-[(3-Chlor-4-fluorphenyl)sulfonyl]hydrazid;
3-Chinolincarbonsäure, 2-[(3-Chlor-4-fluorphenyl)sulfonyl]hydrazid;
6-Chinolincarbonsäure, 2-[(3-Methoxyphenyl)sulfonyl]hydrazid;
3-Chinolincarbonsäure, 2-[(3-Methoxyphenyl)sulfonyl]hydrazid;
6-Chinolincarbonsäure, 2-[(3-Bromphenyl)sulfonyl]-hydrazid;
3-Chinolincarbonsäure, 2-[(3-Bromphenyl)sulfonyl]-hydrazid;
Benzofuran-2-carbonsäure, 2-(Phenylsulfonyl)hydrazid;
7-Ethoxy-2-benzofurancarbonsäure, 2-(Phenylsulfonyl)hydrazid;
7-Chlor-benzofuran-2-carbonsäure, 2-(Phenylsulfonyl)hydrazid;
2,3-Dihydro-benzofuran-5-carbonsäure, 2-(Phenylsulfonyl)hydrazid;
5-Chlor-benzofuran-2-carbonsäure, 2-(Phenylsulfonyl)hydrazid;
Isochinolin-1-carbonsäure, 2-(Phenylsulfonyl)hydrazid;
1H-Indol-5-carbonsäure, 2-(Phenylsulfonyl)hydrazid,
Chinolin-3-carbonsäure, 2-(Phenylsulfonyl)hydrazid;
Chinolin-4-carbonsäure, 2-(Phenylsulfonyl)hydrazid;
1H-Indol-5-carbonsäure, 2-[(3-Chlorphenyl)sulfonyl]hydrazid;
1H-Indol-5-carbonsäure, 2-[(2-Chlorphenyl)sulfonyl]hydrazid;
1H-Indol-5-carbonsäure, 2-[(3-Fluorphenyl)sulfonyl]hydrazid;
1H-Indol-5-carbonsäure, 2-[(3-Trifluormethylphenyl)sulfonyl]hydrazid;
1H-Indol-5-carbonsäure, 2-[(2-Trifluormethylphenyl)sulfonyl]hydrazid;
1H-Indol-5-carbonsäure, 2-[(2-Trifluormethoxyphenyl)sulfonyl]hydrazid;
1H-Indol-5-carbonsäure, 2-[(3-Trifluormethoxyphenyl)sulfonyl]hydrazid;
1H-Indol-5-carbonsäure, 2-[(3-Bromphenyl)sulfonyl]hydrazid;
1H-Indol-5-carbonsäure, 2-[(3-Methylphenyl)sulfonyl]hydrazid;
1H-Indol-5-carbonsäure, 2-[(2-Methylphenyl)sulfonyl]hydrazid;
6-Chinolincarbonsäure, 2-[(2-Chlorphenyl)sulfonyl]hydrazid;
6-Chinolincarbonsäure, 2-[(3-Trifluormethylphenyl)sulfonyl]-hydrazid;
6-Chinolincarbonsäure, 2-[(3-Trifluormethoxyphenyl)sulfonyl]hydrazid;
6-Chinolincarbonsäure, 2-[2-Methylphenyl)sulfonyl]hydrazid;
Dibenzofuran-2-carbonsäure, 2-(4-Fluor-3-methylphenylsulfonyl)hydrazid;
2-Naphthalencarbonsäure, 2-(Phenylsulfonyl)hydrazid.

12. Verwendung einer Verbindung von Anspruch 1 zur Herstellung eines Medikaments zur Hemmung von verzweigtkettigten Aminosäureabhängigen Aminotransferasen in einem Patienten durch Verabreichen einer therapeutisch wirksamen Menge an den - Patienten.

13. Die Verwendung von Anspruch 12, worin Ar ein bicyclisches Heteroaryl ist.

14. Die Verwendung von Anspruch 12, worin Ar ein tricyclisches Heteroaryl ist.

15. Die Verwendung von Anspruch 12, worin R₁, R₂, R₃, R₄ und R₅ Wasserstoff sind.

16. Die Verwendung von Anspruch 12, worin die Verbindung folgendes ist:
Dibenzofuran-2-carbonsäure, 2-(Phenylsulfonyl)hydrazid;
2,3,4,9-Tetrahydro-1H-carbazol-6-carbonsäure, 2-(Phenylsulfonyl)-hydrazid;
Benzofuran-2-carbonsäure, 2-(Phenylsulfonyl)hydrazid;
5-Methoxy-2-benzofurancarbonsäure, 2-(Phenylsulfonyl)hydrazid;
7-Methoxy-2-benzofurancarbonsäure, 2-(Phenylsulfonyl)hydrazid;
7-Ethoxy-2-benzofurancarbonsäure, 2-(Phenylsulfonyl)hydrazid;
7-Chlor-benzofuran-2-carbonsäure, 2-(Phenylsulfonyl)hydrazid;
5-Chlor-benzofuran-2-carbonsäure, 2-(Phenylsulfonyl)hydrazid;
2,3-Dihydro-benzofuran-5-carbonsäure, 2-(Phenylsulfonyl)hydrazid;
Benzo[1,3]Dioxol-5-carbonsäure, 2-(Phenylsulfonyl)hydrazid;
1H-Indol-5-carbonsäure, 2-(Phenylsulfonyl)hydrazid;
1-Dibenzofurancarbonsäure, 2-(Phenylsulfonyl)hyrazid;
Chinolin-6-carbonsäure, 2-(Phenylsulfonyl)hydrazid;
2-Dibenzofurancarbonsäure, 2-[(2-Methylphenyl)sulfonyl]hydrazid;
2-Dibenzofurancarbonsäure, 2-[(3-Methylphenyl)sulfonyl]hydrazid;
2-Dibenzofurancarbonsäure, 2-[(3-Chlorphenyl)sulfonyl]hydrazid;
2-Dibenzofurancarbonsäure, 2-[(2-Chlorphenyl)sulfonyl]hydrazid;
Dibenzofuran-2-carbonsäure, 2-(3,5-Dichlorphenylsulfonyl)hydrazid;
Dibenzofuran-2-carbonsäure, 2-(3-Chlor-2-methylphenylsulfonyl)hydrazid;
Chinolin-6-carbonsäure, 2-(Phenylsulfonyl)hydrazid;
Chinolin-8-carbonsäure, 2-(Phenylsulfonyl)hydrazid;
2-Benzofurancarbonsäure, 5-Chlor-2-[(4-fluorphenyl)]sulfonylhydrazid;
2-Benzofurancarbonsäure, 7-Methoxy-, 2-[(4-Fluorphenyl)sulfonyl]hydrazid;
2-Dibenzofurancarbonsäure, 2-[(4-Fluorphenyl)sulfonyl]hydrazid;
2-Dibenzofurancarbonsäure, 2-[(2-Chlor-4-fluorphenyl)sulfonyl]hydrazid;
2-Dibenzofurancarbonsäure, 2-[(3-Cyanophenyl)sulfonyl]hydrazid;
2-Benzofurancarbonsäure, 5-Chlor-, 2-[(3-Cyanophenyl)sulfonyl]hydrazid;
2-Dibenzofurancarbonsäure, 2-[(2,6-Dichlorphenyl)sulfonyl]hydrazid;
2-Benzofurancarbonsäure, 5-Brom-, 2-(Phenylsulfonyl)hydrazid;
2-Benzofurancarbonsäure, 5-Chlor-, 2-[(2-Chlor-4-fluorphenyl)sulfonyl]hydrazid;
2-Benzofurancarbonsäure, 5-Chlor-, 2-[(3,5-Dichlorphenyl)sulfonyl]hydrazid;
2-Dibenzofurancarbonsäure, 2-[[3,5-bis(Trifluormethyl)phenyl]-sulfonyl]hydrazid;
2-Benzofurancarbonsäure, 5-Chlor, 2-[(3-Chlor-4-fluorphenyl)sulfonyl]hydrazid;
2-Benzofurancarbonsäure, 5-Nitro-, 2-(Phenylsulfonyl)hydrazid;
2-Dibenzvfurancarbonsäure, 2-[(3-Fluorphenyl)sulfonyl]hydrazid;
2-Dibenzofurancarbonsäure, 2-[(5-Fluor-2-methylphenyl)sulfonyl]hydrazid;
2-Dibenzofurancarbonsäure, 2-[(3,4-Difluorphenyl)sulfonyl]hydrazid;
2-Dibenzofurancarbonsäure, 2-[(3-Chlor-4-fluorphenyl)sulfonyl]hydrazid;
2-Dibenzofurancarbonsäure, 2-[(3-Methoxyphenyl)sulfonyl]hydrazid;
2-Benzofurancarbonsäure, 5-Chlor-2-[(3-fluorphenyl)sulfonyl]hydrazid;
2-Benzofurancarbonsäure, 5-Chlor-2-[(3,4-difluorphenyl)sulfonyl]hydrazid;
2-Benzofurancarbonsäure, 5-Chlor-2-[(2-chlorphenyl)sulfonyl]hydrazid;
2-Benzofurancarbonsäure, 5-Chlor-2-[(3-chlorphenyl)sulfonyl]hydrazid;
2-Benzofurancarbonsäure, 5-Chlor-2-[(2-bromphenyl)sulfonyl]hydrazid;
2-Benzofurancarbonsäure, 5-Chlor-2-[(3-bromphenyl)sulfonyl]hydrazid;
2-Benzofurancarbonsäure, 5-Chlor-2-[(2-methylphenyl)sulfonyl]hydrazid;
2-Benzofurancarbonsäure, 5-Chlor-2-[(3-methylphenyl)sulfonyl]hydrazid;
2-Benzofurancarbonsäure, 5-Chlor-2-[(3-methoxyphenyl)sulfonyl]hydrazid;
2-Benzofurancarbonsäure, 5-Chlor-2-[(3-chlor-2-methylphenyl)-sulfonyl]hydrazid;
2-Benzofurancarbonsäure, 5-Chlor-2-[(5-fluor-2-methylphenyl)-sulfonyl]hydrazid;
2-Benzofurancarbonsäure, 5-Chlor-2-[[(2-(trifluormethyl)phenyl)sulfonyl]hydrazid;
2-Benzofurancarbonsäure, 5-Chlor-2-[[2-(trifluormethoxy)phenyl]sulfonyl]hydrazid;
2-Benzofurancarbonsäure, 7-Methoxy-, 2-[[2-(Trifluormethyl)-phenyl]sulfonyl]hydrazid;
2-Benzofurancarbonsäure, 7-Methoxy-, 2-[(3-Methylphenyl)sulfonyl]hydrazid;
2-Benzofurancarbonsäure, 6-Methoxy-, 2-(Phenylsulfonyl)hydrazid;
2-Benzofurancarbonsäure, 5-Methoxy-3-(1-methylethoxy), 2-(Phenylsulfonyl)hydrazid;
2-Benzofurancarbonsäure, 3,5-Dimethoxy-, 2-(Phenylsulfonyl)-hydrazid;
2-Benzofurancarbonsäure, 3,6-Dimethoxy-, 2-(Phenylsulfonyl)-hydrazid;
2-Benzofurancarbonsäure, 4,6-Dimethoxy-, 2-(Phenylsulfonyl)-hydrazid;
2-Benzofurancarbonsäure, 3,7-Dimethoxy-, 2-(Phenylsulfonyl)-hydrazid;
2-Benzofurancarbonsäure, 3,7-Dimethoxy-, 2-[(2-Trifluormethylphenyl)sulfonyl]hydrazid;
2-Benzofurancarbonsäure, 3,7-Dimethoxy-, 2-[(4-Fluorphenyl)sulfonyl)hydrazid;
2-Benzofurancarbonsäure, 3,7-Dimethoxy-, 2-[(3-Methylphenyl)sulfonyl]hydrazid;
2-Benzofurancarbonsäure, 3-Methoxy-5-phenyl-, 2-(Phenylsulfonyl)hydrazid;
2-Benzofurancarbonsäure, 3-Ethoxy-5-phenyl-, 2-(Phenylsulfonyl)hydrazid;
2-Benzofurancarbonsäure, 3-Methoxy-7-phenyl-, 2-(Phenylsulfonyl)hydrazid;
Naptho[2,3-b]furan-2-carbonsäure, 3-Methoxy-, 2-(Phenylsulfonyl)hydrazid;
2-Benzofurancarbonsäure, 5,7-Dichlor 2-(Phenylsulfonyl)sulfonylhydrazid;
2-Benzofurancarbonsäure, 5,7-Dichlor 2-[[2-(Trifluormethyl)-phenyl]sulfonyl)hydrazid;
2-Benzofurancarbonsäure, 5,7-Dichlor 2-[(2-Methylphenyl)sulfonyl]hydrazid;
2-Eenzvfurancarbonsäure, 5,7-Dichlor 2-[(3-Methylphenyl)sulfonyl]hydrazid;
2-Benzofurancarbonsäure, 5,7-Dichlor 2-[(2-Bromphenyl)sulfonyl]hydrazid;
2-Benzofurancarbonsäure, 5,7-Dichlor 2-[(3-Bromphenyl)sulfonyl]hydrazid;
2-Benzofurancarbonsäure, 5,7-Dichlor 2-[(3-Chlorphenyl)sulfonyl]hydrazid;
2-Benzofurancarbonsäure, 5,7-Dichlor 2-[[2-(Trifluormethoxy)phenyl]sulfonyl]hydrazid;
Benzofurancarbonsäure, 5-Methyl-, 2-(Phenylsulfonyl)hydrazid;
2-Benzofurancarbonsäure, 5-Chlor-3-methoxy, 2(Phenylsulfonyl)hydrazid;
2-Benzofurancarbonsäure, 5-Methyl-, 2-[[2-(Trifluormethyl)phenyl]sulfonyl]hydrazid;
2-Benzofurancarbonsäure, 5-Methyl-, 2-([3-Methylphenyl)sulfonyl]hydrazid;
2-Benzofurancarbonsäure, 5-Methyl-, 2-[2-(Methylphenyl)sulfonyl]hydrazid;
2-Benzofurancarbonsäure, 5-Methyl-, 2-[(3-Chlor-2-methylphenyl)sulfonyl]hydrazid;
2-Benzofurancarbonsäure, 5-Methyl-, 2-[3-(Bromphenyl)sulfonyl]hydrazid;
2-Benzofurancarbonsäure, 5-Methyl-, 2-[(3-Chlorphenyl)sulfonyl]hydrazid;
2-Benzofurancarbonsäure, 5-Methyl-, 2-[[2-(Trifluormethoxy)-phenyl]sulfonyl]hydrazid;
2-Benzofurancarbonsäure, 7-Methyl-, 2-(Phenylsulfonyl)hydrazid;
2-Benzofurancarbonsäure, 4-Methyl-, 2-(Phenylsulfonyl)hydrazid;
2-Benzofurancarbonsäure, 3-Methyl-, 2-(Phenylsulfonyl)hydrazid;
2-Benzofurancarbonsäure, 3,5-Dimethyl-, 2-(Phenylsulfonyl)hydrazid;
2-Benzofurancarbonsäure, 5-Chlor-3-methyl, 2-(Phenylsulfonyl)hydrazid;
2-Benzofurancarbonsäure, 5-Amino-, 2-(Phenylsulfonyl)hydrazid;
2-Dibenzofurancarbonsäure, 2-[(2-Fluorphenyl)sulfonyl]hydrazid;
2-Dibenzofurancarbonsäure, 2-(2,6-Difluorphenylsulfonyl)hydrazid;
2-Dibenzofurancarbonsäure, 2-[(2-Bromphenyl)sulfonyl]hydrazid;
2-Dibenzofurancarbonsäure, 2-[(3-Bromphenyl)sulfonyl]hydrazid;
2-Dibenzofurancarbonsäure, 2-[(4-Methyl-3-nitrophenyl)sulfonyl]hydrazid;
2-Dibenzofurancarbonsäure, 2-[2-Chlor-5-(trifluormethyl)phenyl]sulfonyl]hydrazid;
2-Dibenzofurancarbonsäure, 2-[(2-Trifluormethoxyphenyl)sulfonyl]hydrazid;
2-Dibenzofurancarbonsäure, 2-[(4-Brom-2-trifluormethoxyphenyl)sulfonyl]hydrazid;
2-Dibenzofurancarbonsäure, 2-[(2-Nitrophenyl)sulfonyl]hydrazid;
2-Dibenzofurancarbonsäure, 2-[(4-Carboxyphenyl)sulfonyl]hydrazid;
2-Dibenzofurancarbonsäure, 2-[(4,5-Dichlor-2-thienyl)sulfonyl]hydrazid;
2-Benzofurancarbonsäure, 7-Methoxy-, 2-[(3-Chlorphenyl)sulfonyl]hydrazid;
2-Benzofurancarbonsäure, 7-Methoxy-, 2-[(3-Fluorphenyl)sulfonyl]hydrazid;
2-Benzofurancarbonsäure, 7-Methoxy-, 2-[(2,3-Dichlorphenyl)-sulfonyl]hydrazid;
2-Benzofurancarbonsäure, 7-Methoxy-, 2-[(3-Chlor-4-fluorphenyl)sulfonyl]hydrazid;
2-Benzofurancarbonsäure, 7-Methoxy-, 2-[(5-Chlor-2-methoxyphenyl)sulfonyl]hydrazid;
2-Benzofurancarbonsäure, 7-Methoxy-, 2-[(5-Brom-2-methoxyphenyl)sulfonyl]hydrazid;
2-Benzofurancarbonsäure, 7-Methoxy-, 2-[(4-Ethylphenyl)sulfonyl]hydrazid;
2-Benzofurancarbonsäure, 7-Methoxy-, 2-[(3-Methoxyphenyl)sulfonyl]hydrazid;
2-Dibenzofurancarbonsäure, 2-[(2,3-Dichlorphenyl)sulfonyl]hydrazid;
2-Naphthalencarbonsäure, 2-[(3-Methylphenyl)sulfonyl]hydrazid;
2-Naphthalencarbonsäure, 2-[(4-Methylphenyl)sulfonyl]hydrazid;
2-Naphthalencarbonsäure, 2-[[2-(Trifluormethyl)phenyl)sulfonyl]hydrazid;
2-Naphthalencarbonsäure, 2-[[(4-Trifluormethyl)phenyl]sulfonyl]hydrazid;
2-Naphthalencarbonsäure, 2-[(3-Chlor-4-fluorphenyl)sulfonyl]hydrazid;
2-Naphthalencarbonsäure, 2-[(3-Methoxyphenyl)sulfonyl]hydrazid;
2-Naphthalencarbonsäure, 2-[(3-Chlorphenyl)sulfonyl]hydrazid;
2-Naphthalencarbonsäure, 2-[(2-chlorphenyl)sulfonyl]hydrazid;
2-Naphthalencarbonsäure, 2-[(3-Fluorphenyl)sulfonyl]hydrazid;
2-Naphthalencarbonsäure, 2-[(2,3-Dichlorphenyl)sulfonyl]hydrazid;
2-Naphthalencarbonsäure, 2-[(4-Ethylphenyl)sulfonyl]hydrazid;
3-Chinolincarbonsäure, 2-[(4-Pentylphenyl)sulfonyl)hydrazid;
3-Chinolincarbonsäure, 2-[[2(Trifluormethyl)phenyl]sulfonyl]hydrazid;
3-Chinolincarbonsäure, 2-[[2,5-bis(2,2,2-Trifluorethoxyl)phenyl]sulfonyl]hydrazid;
3-Chinolincarbonsäure, 2-[(3-Methylphenyl)sulfonyl]hydrazid;
3-Chinolincarbonsäure, 2-[[2-(Trifluormethoxy)phenyl]sulfonyl]hydrazid;
3-Chinolincarbonsäure, 2-[(4-Methylphenyl)sulfonyl]hydrazid;
6-Chinolincarbonsäure, 2-[(3-Methylphenyl)sulfonyl]hydrazid;
3-Chinolincarbonsäure, 2-[(3-Methylphenyl)sulfonyl]hydrazid;
6-Chinolincarbonsäure, 2-[(3-Chlorphenyl)sulfonyl]hydrazid;
3-Chinolincarbonsäure, 2-[(3-Chlorphenyl)sulfonyl]hydrazid;
6-Chinolincarbonsäure, 2-[(3-Fluorphenyl)sulfonyl]hydrazid;
3-Chinolincarbonsäure, 2-[(3-Fluorphenyl)sulfonyl]hydrazid;
6-Chinolincarbonsäure, 2-[(2-Trifluormethylphenyl)sulfonyl]-hydrazid;
3-Chinolincarbonsäure, 2-[(2-Trifluormethylphenyl)sulfonyl)-hydrazid;
6-Chinolincarbonsäure, 2-[(2-(Trifluormethoxyphenyl)sulfonyl]hydrazid;
3-Chinolincarbonsäure, 2-[(2-Trifluormethoxyphenyl)sulfonyl]hydrazid;
6-Chinolincarbonsäure, 2-[(3,5-Dichlorphenyl)sulfonyl]hydrazid;
3-Chinolincarbonsäure, 2-[(3,5-Dichlorphenyl)sulfonyl]hydrazid;
6-Chinolincarbonsäure, 2-[(3-Chlor-4-fluorphenyl)sulfonyl]hydrazid;
3-Chinolincarbonsäure, 2-[(3-Chlor-4-fluorphenyl)sulfonyl]hydrazid;
6-Chinolincarbonsäure, 2-[(3-Methoxyphenyl)sulfonyl]hydrazid;
3-Chinolincarbonsäure, 2-[(3-Methoxyphenyl)sulfonyl]hydrazid;
6-Chinolincarbonsäure, 2-[(3-Bromphenyl)sulfonyl]-hydrazid;
3-Chinolincarbonsäure, 2-[(3-Bromphenyl)sulfonyl]-hydrazid;
Benzofuran-2-carbonsäure 2-(Phenylsulfonyl)hydrazid;
7-Ethoxy-2-benzofurancarbonsäure 2-(Phenylsulfonyl)hydrazid;
7-Chlor-benzofuran-2-carbonsäure 2-(Phenylsulfonyl)hydrazid;
2,3-Dihydro-benzofuran-5-carbonsäure 2-(Phenylsulfonyl)hydrazid;
5-Chlor-benzofuran-2-carbonsäure 2-(Phenylsulfonyl)hydrazid;
Isochinolin-1-carbonsäure, 2-(Phenylsulfonyl)hydrazid;
1H-Indol-5-carbonsäure, 2-(Phenylsulfonyl)hydrazid;
Chinolin-3-carbonsäure, 2-(Phenylsulfonyl)hydrazid;
Chinolin-4-carbonsäure, 2-(Phenylsulfonyl)hydrazid;
1H-Indol-5-carbonsäure, 2-[(3-Chlorphenyl)sulfonyl]hydrazid;
1H-Indol-5-carbonsäure, 2-[(2-Chlorphenyl)sulfonyl]hydrazid;
1H-Indol-5-carbonsäure, 2-[(3-Fluorphenyl)sulfonyl]hydrazid;
1H-Indol-5-carbonsäure, 2-[(3-Trifluormethylphenyl)sulfonyl]hydrazid;
1H-Indol-5-carbonsäure, 2-[(2-Trifluormethylphenyl)sulfonyl]hydrazid;
1H-Indol-5-carbonsäure, 2-[(2-Trifluormethoxyphenyl)sulfonyl]hydrazid;
1H-Indol-5-carbonsäure, 2-[(3-Trifluormethoxyphenyl)sulfonyl]hydrazid;
1H-Indol-5-carbonsäure, 2-[(3-Bromphenyl)sulfonyl]hydrazid;
1H-Indol-5-carbonsäure, 2-[(3-Methylphenyl)sulfonyl]hydrazid;
1H-Indol-5-carbonsäure, 2-[(2-Methylphenyl)sulfonyl]hydrazid;
6-Chinolincarbonsäure, 2-[(2-Chlorphenyl)sulfonyl]hydrazid;
6-Chinolincarbonsäure, 2-[(3-Trifluormethylphenyl)sulfonyl]-hydrazid;
6-Chinolincarbonsäure, 2-[(3-Triflüormethoxyphenyl)sulfonyl]hyrazid;
6-Chinolincarbonsäure, 2-[(2-Methylphenyl)sulfonyl]hydrazid;
Dibenzofuran-2-carbonsäure, 2-(4-Fluor-3-methylphenylsulfonyl)hydrazid;
2-Naphthalencarbonsäure, 2-(Phenylsulfonyl)hydrazid.

17. Ein Verfahren zur Herstellung einer Verbindung von Formel I 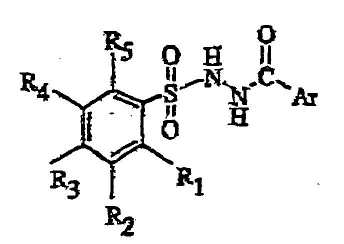 worin:
R₃ H oder F, Br, Alkyl, Carboxy, Alkoxy, substituiertes Alkoxy ist,
R₁, R₂, R₄ und R₅ sind unabhängig H, Halogen, Alkyl, substituiertes Alkyl, Alkoxy, substituiertes Alkoxy, Cyano, Nitro, Amino, Alkylamin und Thioalkyl, Ar ist bicyclisches Heteroaryl, tricyclisches Heteroaryl, substituiertes bicyclisches Heteroaryl, ausgenommen (6-Methoxybenzofuran)-2-nyl, 3-Chinolinyl welches das Reagieren eines Hydrazids von Formel ArC(O)NH₂NH₂ mit einem Sulfonylhalogenid der folgenden Formel umfaßt: 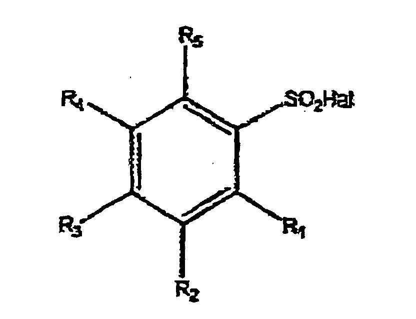
worin R₁, R₂, R₃, R₄, R₅ und Ar wie oben definiert sind und Hal ist Halogen.

18. Ein Medikament zur Behandlung oder Vorbeugung von Nervenschwund im Zusammenhang mit Schlaganfall, Ischämie, ZNS-Trauma, Hypoglykämie oder Operation, oder zur Behandlung einer neurodegenerativen Krankheit, oder zur Behandlung oder Vorbeugung der nachteiligen Folgen der Überstimulation der exzitatorischen Aminosäuren, oder zur Behandlung von Angst, Psychose, Glaukom, CMV-Retinitis, diabetischer Retinopathie, Harninkontinenz, Migränekopfschmerz, Krämpfen, Aminoglykosid-Antibiotika-induziertem Gehörverlust, Parkinson-Krankheit, chronischem Schmerz, neuropathischem Schmerz, oder zur Induzierung von Anästhesie, Opioidtoleranz oder -entzug, oder zur Verbesserung der Wahrnehmung, das eine therapeutisch wirksame Menge einer Verbindung wie in Anspruch 1 beansprucht umfasst.

19. Eine Verbindung von Formel I, wie in Anspruch 1 definiert oder ein pharmazeutisch verträgliches Salz, Ester oder Amid davon, für die Verwendung als ein Medikament.

## Revendications

1. Composé de formule I 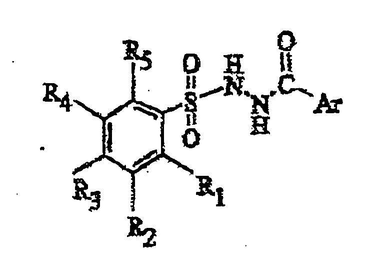 dans laquelle :
R₃ représente H ou un groupe F, Br, alkyle, carboxy, alkoxy ou alkoxy substitué,
R₁, R₂, R₁ et R₅ représentent indépendamment H, un atome d'halogène, un groupe alkyle, alkyle substitué, alkoxy, alkoxy substitué, cyano, nitro, amino, alkylamine ou thioalkyle, Ar représente un groupe hétéroaryle bicyclique, hétéroaryle tricyclique, hétéroaryle bicyclique substitué, à l'exception de (6-méthoxybenzofuranne)-2-nyle, 3-quinolinyle ;
Ar étant choisi dans le groupe consistant en : 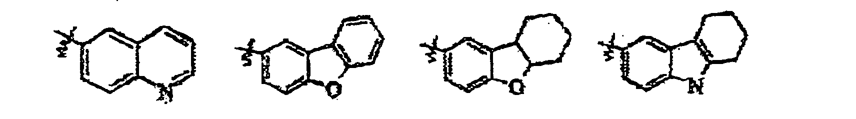 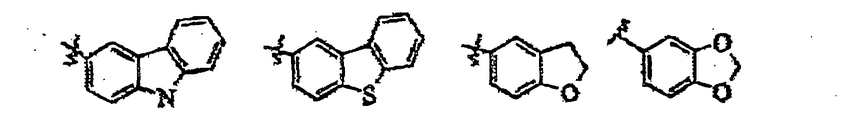 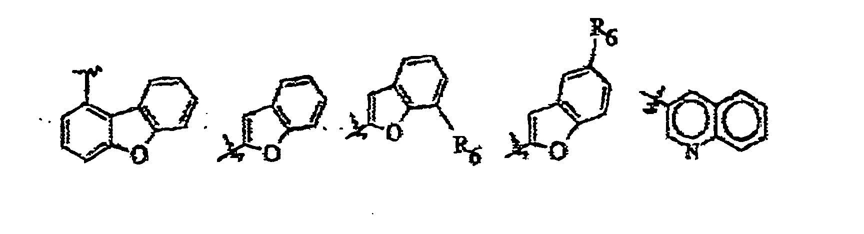 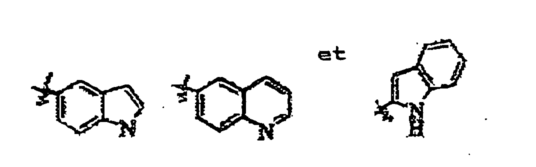
dans lesquelles R₆ représente un groupe alkoxy, alkoxy substitué, un atome d'halogène, un groupe aryloxy, aryloxy substitué, alkylamino, alkylamino substitué, arylalkylamino, arylalkylamino substitué, amide ou amide substitué ;
ou un de ses sels, esters ou amides pharmaceutiquement acceptables.

2. Composé suivant la revendication 1, dans lequel R₁, R₂, R₃, R₄ et R₅ représentent des atomes d'hydrogène.

3. Composé suivant la revendication 1, dans lequel Ar représente un groupe hétéroaryle tricyclique.

4. Composé suivant la revendication 1, dans lequel Ar représente un groupe hétéroaryle bicyclique.

5. Composé suivant la revendication 1, ledit composé étant :
le 2-(phénylsulfonyl)hydrazide d'acide dibenzofuranne-2-carboxylique ;
le 2-(phénylsulfonyl)hydrazide d'acide 2,3,4,9-tétrahydro-1H-carbazole-6-carboxylique ;
le 2-(phénylsulfonyl)hydrazide d'acide benzofuranne-2-carboxylique ;
le 2-(phénylsulfonyl)-hydrazide d'acide 5-méthoxy-2-benzofurannecarboylique ;
le 2-(phénylsulfonyl)-hydrazide d'acide 7-méthoxy-2-benzofurannecarboxylique ;
le 2-(phénylsulfonyl)-hydrazide d'acide 7-éthoxy-2-benzafurannecarboxylique ;
le 2-(phénylsulfonyl)-hydrazide d'acide 7-chlorobenzofuranne-2-carboxylique ;
le 2-(phénylsulfonyl)-hydrazide d'acide 5-chlorobenzofuranne-2-carboxylique ;
le 2-(phénylsulfonyl)-hydrazide d'acide 2,3-dihydrobenzofuranne-5-carboxylique ;
le 2-(phénylsulfonyl)hydrazide d'acide benzo[1,3]-dioxole-5-carboxylique ;
le 2-(phénylsulfonyl)hydrazide d'acide 1H-indole-5-carboxylique ;
le 2-(phénylsulfonyl)hydrazide d'acide 1-dibenzofurannecarboxylique ;
le 2-(phénylsulfonyl)hydrazide d'acide quinoléine-6-carboxylique ;
le 2-[(2-méthylphényl)sulfonyl]-hydrazide d'acide 2-dibenzofurannecarboxylique ;
le 2-[(3-méthylphényl)sulfonyl]-hydrazide d'acide 2-dibenzofurannecarboxylique ;
le 2-[(3-chlorophényl)sulfonyl]-hydrazide d'acide 2-dibenzofurannecarboxylique ;
le 2-[(2-chlorophényl)sulfonyl]-hydrazide d'acide 2-dibenzofurannecarboxylique ;
le 2-(3,5-dichlorophénylsulfonyl)-hydrazide d'acide dibenzofuranne-2-carboxylique ;
le 2-(phénylsulfonyl)hydrazide d'acide quinoléine-6-carboxylique ;
le 2-(phénylsulfonyl)hydrazide d'acide quinoléine-8-carboxylique ;
le 5-chloro-2-[(4-fluorophényl)]-sulfonylhydrazide d'acide 2-benzofurannecarboxylique ;
le 7-méthoxy-,2-[(4-fluorophényl)sulfonyl]hydrazide d'acide 2-benzofurannecarboxylique ;
le 2-[(4-fluorophényl)sulfonyl]hydrazide d'acide 2-dibenzofurannecarboxylique ;
le 2-[(2-chloro-4-fluorophényl)sulfonyl]hydrazide d'acide 2-dibenzofurannecarboxylique ;
le 2-[(3-cyanophényl)sulfonyl]-hydrazide d'acide 2-dibenzofurannecarboxylique ;
le 5-chloro-,2-[(3-cyanophényl)-sulfonyl]hydrazide d'acide 2-benzofurannecarboxylique ;
le 2-[(2,6-dichlorophényl)sulfonyl]-hydrazide d'acide 2-dibenzofurannecarboxylique ;
le 5-bromo-,2-(phénylsulfonyl)-hydrazide d'acide 2-benzofurannecarboxylique ;
le 5-chloro-,2-[(2-chloro-4-fluorophényl)sulfonyl]hydrazide d'acide 2-benzofurannecarboxylique ;
le 5-chloro-,2-[(3,5-dichlorophényl)-sulfonyl]hydrazide d'acide 2-benzofurannecarboxylique ;
le 2- [[3,5-bis(trifluorométhyl)-phényl]sulfonyl]-hydrazide d'acide 2-dibenzofurannecarboxylique ;
le 5-chloro, 2- [ (3-chloro-4-fluorophényl)sulfonyl]hydrazide d'acide 2-benzofurannecarboxylique ;
le 5-nitro,2-(phénylsulfonyl)-hydrazide d'acide 2-benzofurannecarboxylique ;
le 2-[(3-fluorophényl)sulfanyl]-hydrazide d'acide 2-dibenzofurannecarboxylique ;
le 2-[(5-fluoro-2-méthylphényl)sulfonyl]hydrazide d'acide 2-dibenzofurannecarboxylique ;
le 2-[(3,4-difluorophényl)sulfonyl]-hydrazide d'acide 2-dibenzofurannecarboxylique ;
le 2-[(3-méthoxyphényl)sulfonyl]hydrazide d'acide 2-dibenzofurannecarboxylique ;
le 2-[(3-chloro-4-fluorophényl)sulfonyl]hydrazide d'acide 2-dibenzofurannecarboxylique ;
le 5-chloro-2-[(3-fluorophényl)sulfonyl]hydrazide d'acide 2-benzofurannecarboxylique ;
le 5-chloro-2-[(3,4-difluorophényl)sulfonyl]hydrazide d'acide 2-benzofurannecarboxylique ;
le 5-chloro-2- [(2-chlorophényl)sulfonyl]hydrazide d'acide 2-benzofurannecarboxylique ;
le 5-chloro-2-[(3-chlorophényl)sulfonyl]hydrazide d'acide 2-benzofurannecarboxylique ;
le 5-chloro-2-[(2-bromophényl)sulfonyl]hydrazide d'acide 2-benzofurannecarboxylique ;
le 5-chloro-2-[(3-bromophényl)sulfonyl]hydrazide d'acide 2-benzofurannecarboxylique ;
le 5-chloro-2-[(2-méthylphényl)sulfonyl]hydrazide d'acide 2-benzofurannecarboxylique ;
le 5-chloro-2-[(3-méthylphényl)sulfonyl]hydrazide d'acide 2-benzofurannecarboxylique ;
le 5-chloro-2-[(3-méthoxyphényl)sulfonyl]hydrazide d'acide 2-benzofurannecarboxylique ;
le 5-chloro-2-[(3-chloro-2-méthylphényl)sulfonyl]hydrazide d'acide 2-benzofurannecarboxylique ;
le 5-chloro-2-[(5-fluoro-2-méthylphényl)sulfonyl]hydrazide d'acide 2-benzofurannecarboxylique ;
le 5-chloro-2-[[(2-(trifluorométhyl)phényl]sulfonyl]hydrazide d'acide 2-benzofurannecarboxylique ;
le 5-chloro-2-[[(2-(trifluorométhoxy)phényl]sulfonyl]hydrazide d'acide 2-benzofurannecarboxylique ;
le 7-méthoxy-,2-[[(2-(trifluorométhyl)phényl]sulfonyl]hydrazide d'acide 2-benzofurannecarboxylique ;
le 7-méthoxy-,2-[(3-méthylphényl)sulfonyl]hydrazide d'acide 2-benzofurannecarboxylique ;
le 5-méthoxy-3-(1-méthyléthoxy),2-(phénylsulfonyl)hydrazide d'acide 2-benzofurannecarboxylique ;
le 3,5-diméthoxy-,2-(phénylsulfonyl)hydrazide d'acide 2-benzofurannecarboxylique ;
le 3,6-diméthoxy-,2-(phénylsulfonyl)hydrazide d'acide 2-benzofurannecarboxylique ;
le 4,6-diméthoxy-,2-(phénylsulfonyl)hydrazide d'acide 2-benzofurannecarboxylique ;
le 3,7-diméthoxy-,2-(phénylsulfonyl)hydrazide d'acide 2-benzofurannecarboxylique ;
le 3,7-diméthoxy-,2-[(2-trifluorométhylphényl)sulfonyl]hydrazide d'acide 2-benzofurannecarboxylique ;
le 3,7-diméthoxy-,2-[(4-fluorophényl)sulfonyl]hydrazide d'acide 2-benzofurannecarboxylique ;
le 3,7-diméthoxy-,2-[(3-méthylphényl)sulfonyl]hydrazide d'acide 2-benzofurannecarboxylique ;
le 3-méthoxy-5-phényl-,2-(phénylsulfonyl)hydrazide d'acide 2-benzofurannecarboxylique ;
le 3-éthaxy-5-phényl-,2-(phénylsulfonyl)hydrazide d'acide 2-benzofurannecarboxylique ;
le 3-méthoxy-7-phényl-,2-(phénylsulfonyl)hydrazide d'acide 2-benzofurannecarboxylique ;
le 3-méthoxy-,2-(phénylsulfonyl)hydrazide d'acide naphto[2,3-b]furanne-2-carboxylique ;
le 5,7-dichloro-2-(phénylsulfonyl)-sulfonylhydrazide d'acide 2-benzofurannecarboxylique ;
le 5,7-dichloro-2-[[2-(trifluorométhyl)phényl)]sulfonyl]hydrazide d'acide 2-benzofurannecarboxylique ;
le 5,7-dichloro-2-[(2-méthylphényl)sulfonyl]hydrazide d'acide 2-benzofurannecarboxylique ;
le 5,7-dichloro-2-[(3-méthylphényl)sulfonyl]hydrazide d'acide 2-benzofurannecarboxylique ;
le 5,7-dichloro-2-[(2-bromophényl)sulfonyl]hydrazide d'acide 2-benzofurannecarboxylique ;
le 5,7-dichloro-2-[(3-bromophényl)sulfonyl]hydrazide d'acide 2-benzofurannecarboxylique ;
le 5,7-dichloro-2-[(3-chlorophényl)sulfonyl]hydrazide d'acide 2-benzofurannecarboxylique ;
le 5,7-dichloro-2-[[(2-(trifluorométhoxy)phényl]-sulfonyl]hydrazide d'acide 2-benzofurannecarboxylique ;
le 5-méthyl-,2-(phénylsulfonyl)-hydrazide d'acide benzofurannecarboxylique ;
le 5-chloro-3-méthoxy,2-(phénylsulfonyl)hydrazide d'acide 2-benzofurannecarboxylique ;
le 5-méthyl-,2-[[2-(trifluorométhyl)phényl]sulfonyl]hydrazide d'acide 2-benzofurannecarboxylique ;
le 5-méthyl-, 2- ( [3-méthylphényl]sulfonyl]hydrazide d'acide 2-benzofurannecarboxylique ;
le 5-méthyl-,2-[2-(méthylphényl)sulfonyl]hydrazide d'acide 2-benzofurannecarboxylique ;
le 5-méthyl-,2-[(3-chloro-2-méthylphényl)sulfonyl]hydrazide d'acide 2-benzofurannecarboxylique ;
le 5-méthyl-,2-[3-(bromophényl)sulfonyl]hydrazide d'acide 2-benzofurannecarboxylique ;
le 5-méthyl-,2-[(3-chlorophényl)sulfonyl]hydrazide d'acide 2-benzofurannecarboxylique ;
le 5-méthyl-,2-[[2-(trifluorométhoxy)phényl]sulfonyl]hydrazide d'acide 2-benzofurannecarboxylique ;
le 7-méthyl-,2-(phénylsulfonyl)-hydrazide d'acide 2-benzofurannecarboxylique ;
le 4-méthyl-,2-(phénylsulfonyl)-hydrazide d'acide 2-benzofurannecarboxylique ;
le 3-méthyl-,2-(phénylsulfonyl)-hydrazide d'acide 2-benzofurannecarboxylique ;
le 3,5-diméthyl-,2-(phénylsulfonyl)-hydrazide d'acide 2-benzofurannecarboxylique ;
le 5-chloro-3-méthyl,2-(phénylsulfonyl)hydrazide d'acide 2-benzofurannecarboxylique ;
le 5-amino,2-(phénylsulfonyl)-hydrazide d'acide 2-benzofurannecarboxylique ;
le 2-[(2-fluorophényl)sulfonyl]hydrazide d'acide 2-dibenzofurannecarboxylique ;
le 2-(2,6-difluorophénylsulfonyl)hydrazide d'acide 2-dibenzofurannecarboxylique ;
le 2-[(2-bromophényl)sulfonyl]hydrazide d'acide 2-dibenzofurannecarboxylique ;
le 2-[(3-bromophényl)sulfonyl]hydrazide d'acide 2-dibenzofurannecarboxylique ;
le 2-[(4-méthyl-3-nitrophényl)sulfonyl]hydrazide d'acide 2-dibenzofurannecarboxylique ;
le 2-[2-chloro-5-(trifluorométhyl)phényl]sulfonyl)-hydrazide d'acide 2-dibenzofurannecarboxylique ;
le 2-[(2-trifluorométhoxyphényl)sulfonyl)hydrazide d'acide 2-dibenzofurannecarboxylique ;
le 2-[(4-bromo-2-trifluorométhoxyphényl)sulfonyl]-hydrazide d'acide 2-dibenzofurannecarboxylique ;
le 2-[(2-nitrophényl)sulfonyl]-hydrazide d'acide 2-dibenzofurannecarboxylique ;
le 2-[(4-carboxyphényl)sulfonyl]hydrazide d'acide 2-dibenzofurannecarboxylique ;
le 2-[(4,5-dichloro-2-thiényl)sulfonyl]hydrazide d'acide 2-dibenzofurannecarboxylique ;
le 7-méthoxy-,2-[(3-chlorophényl)sulfonyl]hydrazide d'acide 2-benzofurannecarboxylique ;
le 7-méthoxy-,2-[(3-fluorophényl)sulfonyl]hydrazide d'acide 2-benzofurannecarboxylique ;
le 7-méthoxy-,2-[(2,3-dichlororophényl)sulfonyl]-hydrazide d'acide 2-benzofurannecarboxylique ;
le 7-méthoxy-,2-[(3-chloro-4-fluorophényl)sulfonyl]-hydrazide d'acide 2-benzofurannecarboxylique ;
le 7-méthoxy-,2-[(5-chloro-2-méthoxyphényl)sulfonyl]-hydrazide d'acide 2-benzofurannecarboxylique ;
le 7-méthoxy-,2-[(5-bromo-2-méthoxyphényl)sulfonyl]-hydrazide d'acide 2-benzofurannecarboxylique ;
le 7-méthoxy-,2-[(4-éthylphényl)sulfonyl]hydrazide d'acide 2-benzofurannecarboxylique ;
le 7-méthoxy-,2-[(3-méthoxyphényl)sulfonyl]hydrazide d'acide 2-benzofurannecarboxylique ;
le 2-[(2,3-dichlorophényl)sulfonyl]hydrazide d'acide 2-dibenzofurannecarboxylique ;
le 2-[(3-méthylphényl)sulfonyl]-hydrazide d'acide 2-naphtalènecarboxylique ;
le 2-[[(2-(trifluorométhyl)phényl]sulfonyl]hydrazide d'acide 2-naphtalènecarboxylique ;
le 2-[[(4-(trifluorométhyl)phényl]sulfonyl]hydrazide d'acide 2-naphtalènecarboxylique ;
le 2-[(3-chloro-4-fluorophényl)sulfonyl]hydrazide d'acide 2-naphtalènecarboxylique ;
le 2-[(3-méthoxyphényl)sulfonyl]hydrazide d'acide 2-naphtalènecarboxylique ;
le 2-[(3-chlorophényl)sulfonyl]-hydrazide d'acide 2-naphtalènecarboxylique ;
le 2-[(2-chlorophényl)sulfonyl]-hydrazide d'acide 2-naphtalènecarboxylique ;
le 2-[(3-fluorophényl)sulfonyl]-hydrazide d'acide 2-naphtalènecarboxylique ;
le 2-[(2,3-dichlorophényl)sulfonyl]-hydrazzde d'acide 2-naphtalènecarboxylique ;
le 2-[(4-éthylphényl)sulfonyl]-hydrazide d'acide 2-naphtalènecarboxylique ;
le 2-[(4-pentylphényl)sulfonyl]-hydrazide d'acide 3-quinoléinecarboxylique ;
le 2-[[2-(trifluorométhyl)phényl]sulfonyl]hydrazide d'acide 3-quinoléinecarboxylique ;
le 2-[[2,5-bis(2,2,2-trifluoroéthoxyl)phényl]sulfonyl]hydrazide d'acide 3-quinoléinecarboxylique ;
le 2-[(3-méthylphényl)sulfonyl]-hydrazide d'acide 3-quinoléinecarboxylique ;
le 2-[[2-(trifluorométhoxy)phényl]sulfonyl]hydrazide d'acide 3-quinoléinecarboxylique ;
le 2-[(4-méthylphényl)sulfonyl]-hydrazide d'acide 3-quinoléinecarboxylique ;
le 2-[(3-méthylphényl)sulfonyl]-hydrazide d'acide 6-quinoléinecarboxylique ;
le 2-[(3-méthylphényl)sulfonyl]-hydrazide d'acide 3-quinoléinecarboxylique ;
le 2-[(3-chlorophényl)sulfonyl]-hydrazide d'acide 6-quinoléinecarboxylique ;
le 2-[(3-chlorophényl)sulfonyl]-hydrazide d'acide 3-quinoléinecarboxylique ;
le 2-[(3-fluorophényl)sulfonyl]-hydrazide d'acide 6-quinoléinecarboxylique ;
le 2-[(3-fluorophényl)sulfonyl]-hydrazide d'acide 3-quinoléinecarboxylique ;
le 2-[(2-trifluorométhylphényl)sulfonyl]-hydrazide d'acide 6-quinoléinecarboxylique ;
le 2-[(2-trifluorométhylphényl)sulfonyl]-hydrazide d'acide 3-quinoléinecarboxylique ;
le 2-[(2-trifluorométhoxyphényl)sulfonyl]-hydrazide d'acide 6-quinoléinecarboxylique ;
le 2- [(2-trifluorométhoxyphényl)sulfonyl]-hydrazide d'acide 3-quinoléinecarboxylique ;
le 2-[(3,5-dichlorophényl)sulfonyl]-hydrazide d'acide 6-quinoléinecarboxylique ;
le 2-[(3,5-dichlorophényl)sulfonyl]-hydrazide d'acide 3-quinoléinecarboxylique ;
le 2-[(3-chloro-4-fluorophényl)sulfonyl]-hydrazide d'acide 6-quinoléinecarboxylique ;
le 2-[(3-chloro-4-fluorophényl)sulfonyl]-hydrazide d'acide 3-quinoléinecarboxylique ;
le 2-[(3-méthoxyphényl)sulfonyl]-hydrazide d'acide 6-quinoléinecarboxylique ;
le 2-[(3-méthoxyphényl)sulfonyl]-hydrazide d'acide 3-quinoléinecarboxylique ;
le 2-[(3-bromophényl)sulfonyl]-hydrazide d'acide 6-quinoléinecarboxylique ;
le 2-[(3-bromophényl)sulfonyl]-hydrazide d'acide 3-quinoléinecarboxylique ;
le 2-(phénylsulfonyl)hydrazide d'acide benzofuranne-2-carboxylique ;
le 2-(phénylsulfonyl)-hydrazide d'acide 7-éthoxy-2-benzofurannecarboxylique ;
le 2-(phénylsulfonyl)-hydrazide d'acide 7-chlorobenzofuranne-2-carboxylique ;
le 2-(phénylsulfonyl)-hydrazide d'acide 2,3-dihydrobenzofuranne-5-carboxylique ;
le 2-(phénylsulfonyl)-hydrazide d'acide 5-chlorobenzofuranne-2-carboxylique ;
le 2-(phénylsulfonyl)hydrazide d'acide isoquinoléine-1-carboxylique ;
le 2-(phénylsulfonyl)hydrazide d'acide 1H-indole-5-carboxylique ;
le 2-(phénylsulfonyl)hydrazide d'acide quinoléine-3-carboxylique ;
le 2-(phénylsulfonyl)hydrazide d'acide quinoléine-4-carboxylique ;
le 2-[(3-chlorophényl)sulfonyl]-hydrazide d'acide 1H-indole-5-carboxylique ;
le 2-[(2-chlorophényl)sulfonyl]-hydrazide d'acide 1H-indole-5-carboxylique ;
le 2-[(3-fluororophényl)sulfonyl]-hydrazide d'acide 1H-indole-5-carboxylique ;
le 2-[(3-trifluorométhylphényl)sulfonyl]hydrazide d'acide 1H-indole-5-carboxylique ;
le 2-[(2-trifluorométhylphényl)sulfonyl]hydrazide d'acide 1H-indole-5-carboxylique ;
le 2-[(2-trifluorométhoxyphényl)sulfonyl]hydrazide d'acide 1H-indole-5-carboxylique ;
le 2- [(3-trifluorométhoxyphényl)sulfonyl]hydrazide d'acide 1H-indole-5-carboxylique ;
le 2-[(3-bromophényl)sulfonyl]-hydrazide d'acide 1H-indole-5-carboxylique ;
le 2-[(3-méthylphényl)sulfonyl]-hydrazide d'acide 1H-indole-5-carboxylique ;
le 2-[(2-méthylphényl)sulfonyl]-hydrazide d'acide 1H-indole-5-carboxylique ;
le 2-[(2-chlorophényl)sulfonyl]-hydrazide d'acide 6-quinoléinecarboxylique ;
le 2-[(3-trifluorométhylphényl)sulfonyl]hydrazide d'acide 6-quinoléinecarboxylique ;
le 2-[(3-trifluorométhoxyphényl)sulfonyl]hydrazide d'acide 6-quinoléinecarboxylique ;
le 2-[(2-méthylphényl)sulfonyl]-hydrazide d' acide 6-quinoléinecarboxylique ;
le 2-(4-fluoro-3-méthylphénylsulfonyl)hydrazide d'acide dibenzofuranne-2-carboxylique.

6. Composition pharmaceutique comprenant un composé )de la revendication 1 et un support pharmaceutiquement acceptable.

7. Utilisation d'un composé de formule I suivant la revendication 1 pour la préparation d'un médicament destiné au traitement ou à la prévention de la perte neuronale associée à un ictus, une ischémie, un traumatisme du SNC, l'hypoglycémie ou une intervention chirurgicale, ou pour le traitement d'une maladie neurodégénérative, ou pour le traitement ou la prévention des conséquences néfastes de la stimulation excessive des aminoacides excitateurs, ou pour )le traitement de l'anxiété, de la psychose, du glaucome, de la rétinite provoquée par le CMV, de rétinopathie diabétique, de l'incontinence urinaire, des céphalées de la migraine, des convulsions, de la perte d'audition induite par les antibiotiques aminoglycosidiques, de la maladie de Parkinson, de la douleur chronique, de la douleur neuropathique, ou pour induire une anesthésie, une tolérance ou un sevrage des opioïdes, ou pour améliorer la cognition.

8. Utilisation suivant la revendication 7, dans laquelle Ar représente un groupe hétéroaryle bicyclique.

9. Utilisation suivant la revendication 7, dans laquelle Ar représente un groupe hétéroaryle tricyclique.

10. Utilisation suivant la revendication 7, dans laquelle R₁, R₂, R₃, R₄ et R₅ représentent des atomes d'hydrogène.

11. Utilisation suivant la revendication 7, dans laquelle le composé est :
le 2-(phénylsulfonyl)hydrazide d'acide dibenzofuranne-2-carboxylique ;
le 2-(phénylsulfonyl)hydrazide d'acide 2,3,4,9-tétrahydro-1H-carbazole-6-carboxylique ;
le 2-(phénylsulfonyl)hydrazide d'acide benzofuranne-2-carboxylique ;
le 2-(phénylsulfonyl)-hydrazide d'acide 5-méthoxy-2-benzofurannecarboxylique ;
le 2-(phénylsulfonyl)-hydrazide d'acide 7-méthoxy-2-benzofurannecarboxylique ;
le 2-(phénylsulfonyl)-hydrazide d'acide 7-éthoxy-2-benzofurannecarboxylique ;
le 2-(phénylsulfonyl)-hydrazide d'acide 7-chlorobenzofuranne-2-carboxylique ;
le 2-(phénylsulfonyl)-hydrazide d'acide 5-chlorobenzofuranne-2-carboxylique ;
le 2-(phénylsulfonyl)-hydrazide d'acide 2,3-dihydrobenzofuranne-5-carboxylique ;
le 2-(phénylsulfonyl)hydrazide d'acide benzo[1,3]-dioxole-5-carboxylique ;
le 2-(phénylsulfonyl)hydrazide d'acide 1H-indole-5-carboxylique ;
le 2-(phénylsulfonyl)hydrazide d'acide 1-dibenzofurannecarboxylique ;
le 2-(phénylsulfonyl)hydrazide d'acide quinoléine-6-carboxylique ;
le 2-[(2-méthylphényl)sulfonyl]-hydrazide d'acide 2-dibenzofurannecarboxylique ;
le 2-[(3-méthylphényl)sulfonyl]-hydrazide d'acide 2-dibenzofurannecarboxylique ;
le 2-[(3-chlorophényl)sulfonyl]-hydrazide d'acide 2-dibenzofurannecarboxylique ;
le 2-[(2-chlorophényl)sulfonyl]-hydrazide d'acide 2-dibenzofurannecarboxylique ;
le 2-(3,5-dichlorophénylsulfonyl)-hydrazide d'acide dibenzofuranne-2-carboxylique ;
le 2-(3-chloro-2-méthylphénylsulfonyl)hydrazide d'acide dibenzofuranne-2-carboxylique ;
le 2-(phénylsulfonyl)hydrazide d'acide quinoléine-6-carboxylique ;
le 2-(phénylsulfonyl)hydrazide d'acide quinoléine-8-carboxylique ;
le 5-chloro-2-[(4-fluorophényl)]-sulfonylhydrazide d'acide 2-benzofurannecarboxylique ;
le 7-méthoxy-,2-[(4-fluorophényl)sulfonyl]hydrazide d'acide 2-benzofurannecarboxylique ;
le 2-[(4-fluorophényl)sulfonyl]hydrazide d'acide 2-dibenzofurannecarboxylique ;
le 2-[(2-chloro-4-fluorophényl)sulfonyl]hydrazide d'acide 2-dibenzofurannecarboxylique ;
le 2-[(3-cyanophényl)sulfonyl]-hydrazide d'acide 2-benzofurannecarboxylique ;
le 5-chloro-,2-[(3-cyanophényl)-sulfonyl]hydrazide d'acide 2-benzofurannecarboxylique ;
le 2-[(2,6-dichlorophényl)sulfonyl]-hydrazide d'acide 2-dibenzofurannecarboxylique ;
le 5-bromo-,2-(phénylsulfonyl)-hydrazide d'acide 2-benzofurannecarboxylique ;
le 5-chloro-,2-[(2-chloro-4-fluorophényl)sulfonyl]-hydrazide d'acide 2-benzofurannecarboxylique ;
le 5-chloro-, 2- [ (3,5-dichlorophényl)sulfonyl]hydrazide d'acide 2-benzofurannecarboxylique ;
le 2-[[3,5-bis(trifluorométhyl)-phényl]sulfonyl]-hydrazide d'acide 2-dibenzofurannecarboxylique ;
le 5-chloro,2-[(3-chloro-4-fluorophényl)sulfonyl]hydrazide d'acide 2-benzofurannecarboxylique ;
le 5-nitro,2-(phénylsulfonyl)-hydrazide d'acide 2-benzofurannecarboxylique ;
le 2-[(3-fluorophényl)sulfonyl]-hydrazide d'acide 2-dibenzofurannecarboxylique ;
le 2-[(5-fluoro-2-méthylphényl)sulfonyl]hydrazide d'acide 2-dibenzofurannecarboxylique ;
le 2-[(3,4-difluorophényl)sulfonyl]-hydrazide d'acide 2-dibenzofurannecarboxylique ;
le 2-[(3-chloro-4-fluorophényl)sulfanyl]-hydrazide d'acide 2-dibenzofurannecarboxylique : le 2-[(3-méthoxyphényl)sulfonyl]hydrazide d'acide 2-dibenzofurannecarboxylique ;
le 5-chloro-2-[(3-fluorophényl)sulfonyl]hydrazide d'acide 2-benzofurannecarboxylique ;
le 5-chloro-2-[(3,4-difluorophényl)sulfonyl]hydrazide d'acide 2-benzofurannecarboxylique ;
le 5-chloro-2-[(2-chlorophényl)sulfonyl]hydrazide d'acide 2-benzofurannecarboxylique ;
le 5-chloro-2-[(3-chlorophényl)sulfonyl]hydrazide d'acide 2-benzofurannecarboxylique ;
le 5-chloro-2-[ (2-bromophényl) sulfonyl]hydrazide d'acide 2-benzofurannecarboxylique ;
le 5-chloro-2-[(3-bromophényl)sulfonyl]hydrazide d'acide 2-benzofurannecarboxylique ;
le 5-chloro-2-[(2-méthylphényl)sulfonyl]hydrazide d'acide 2-benzofurannecarboxylique ;
le 5-chloro-2-[(3-méthylphényl)sulfonyl]hydrazide d'acide 2-benzofurannecarboxylique ;
le 5-chloro-2-[(3-méthoxyphényl)sulfonyl]hydrazide d'acide 2-benzofurannecarboxylique ;
le 5-chloro-2-[(3-chloro-2-méthylphényl)sulfonyl]hydrazide d'acide 2-benzofurannecarboxylique ;
le 5-chloro-2-[(5-fluoro-2-méthylphényl)sulfonyl]hydrazide d'acide 2-benzofurannecarboxylique ;
le 5-chloro-2-[[(2-(trifluorométhyl)phényl]sulfonyl]hydrazide d'acide 2-benzofurannecarboxylique ;
le 5-chloro-2-[[(2-(trifluorométhoxy)phényl]sulfonyl]hydrazide d'acide 2-benzofurannecarboxylique ;
le 7-méthoxy-,2-[[(2-(trifluorométhyl)phényl]sulfonyl]hydrazide d'acide 2-benzofurannecarboxylique ;
le 7-méthoxy-,2-[(3-méthylphényl)sulfonyl]hydrazide d'acide 2-benzofurannecarboxylique ;
le 6-méthoxy-,2-(phénylsulfonyl)hydrazide d'acide 2-benzofurannecarboxylique ;
le 5-méthoxy-3-(1-méthyléthoxy),2-(phénylsulfonyl)hydrazide d'acide 2-benzofurannecarboxylique ;
le 3,5-diméthoxy-,2-(phénylsulfonyl)hydrazide d'acide 2-benzofurannecarboxylique ;
le 3,6-diméthoxy-,2-(phénylsulfonyl)hydrazide d'acide 2-benzofurannecarboxylique ;
le 4,6-diméthoxy-,2-(phénylsulfonyl)hydrazide d'acide 2-benzofurannecarboxylique ;
le 3,7-diméthoxy-,2-(phénylsulfonyl)hydrazide d'acide 2-benzofurannecarboxylique ;
le 3,7-diméthoxy-,2-[(2-trifluorométhylphényl)sulfonyl]hydrazide d'acide 2-benzofurannecarboxylique ;
le 3,7-diméthoxy-,2-[(4-fluorophényl)sulfonyl]hydrazide d'acide 2-benzofurannecarboxylique ;
le 3,7-diméthoxy-,2-[(3-méthylphényl)sulfonyl]hydrazide d'acide 2-benzofurannecarboxylique ;
le 3-méthoxy-5-phényl-,2-(phénylsulfonyl)hydrazide d'acide 2-benzofurannecarboxylique ;
le 3-éthoxy-5-phényl-,2- (phénylsulfonyl) hydrazide d'acide 2-benzofurannecarboxylique ;
le 3-méthoxy-7-phényl-,2- (phénylsulfonyl)hydrazide d'acide 2-benzofurannecarboxylique ;
le 3-méthoxy-,2-(phénylsulfonyl)hydrazide d'acide naphto[2,3-b]furanne-2-carboxylique ;
le 5,7-dichloro-2-(phénylsulfonyl)-sulfonylhydrazide d'acide 2-benzofurannecarboxylique ;
le 5,7-dichloro-2-[[2-(trifluorométhyl)phényl)]sulfonyl]hydrazide d'acide 2-benzofurannecarboxylique ;
le 5,7-dichloro-2-[(2-méthylphényl)sulfonyl]hydrazide d'acide 2-benzofurannecarboxylique ;
le 5,7-dichloro,2-[(3-méthylphényl)sulfonyl]hydrazide d'acide 2-benzofurannecarboxylique ;
le 5,7-dichloro,2-[(2-bromophényl)sulfonyl]hydrazide d'acide 2-benzofurannecarboxylique ;
le 5,7-dichloro-2,[(3-bromophényl)sulfonyl]hydrazide d'acide 2-benzofurannecarboxylique ;
le 5,7-dichloro-2,[(3-chlorophényl)sulfonyl]hydrazide d'acide 2-benzofurannecarboxylique ;
le 5,7-dichloro,2-[[2-(trifluorométhoxy)phényl]sulfonyl]hydrazide d'acide 2-benzofurannecarboxylique ;
le 5-méthyl-,2-(phénylsulfonyl)-hydrazide d'acide benzofurannecarboxylique ;
le 5-chloro-3-méthoxy,2-(phénylsulfonyl)hydrazide d'acide 2-benzofurannecarboxylique ;
le 5-méthyl-,2-[[2-(trifluorométhyl)phényl]sulfonyl]-hydrazide d'acide 2-benzofurannecarboxylique ;
le 5-méthyl-,2-([3-méthylphényl]sulfonyl]hydrazide d'acide 2-benzofurannecarboxylique ;
le 5-méthyl-,2-[2-(méthylphényl)sulfonyl]hydrazide d'acide 2-benzofurannecarboxylique ;
le 5-méthyl-,2-[(3-chloro-2-méthylphényl)sulfonyl]hydrazide d'acide 2-benzofurannecarboxylique ;
le 5-méthyl-,2-[3-(bromophényl)sulfonyl]hydrazide d'acide 2-benzofurannecarboxylique ;
le 5-méthyl-,2-[(3-chlorophényl)sulfonyl]hydrazide d'acide 2-benzofurannecarboxylique ;
le 5-méthyl-,2-[[2-(trifluorométhoxy)phényl]-sulfonyl]hydrazide d'acide 2-benzofurannecarboxylique ;
le 7-méthyl-,2-(phénylsulfonyl)-hydrazide d'acide 2-benzofurannecarboxylique ;
le 4-méthyl-,2-(phénylsulfonyl)-hydrazide d'acide 2-benzofurannecarboxylique ;
le 3-méthyl-,2-(phénylsulfonyl)-hydrazide d'acide 2-benzofurannecarboxylique ;
le 3,5-diméthyl-,2-(phénylsulfonyl)-hydrazide d'acide 2-benzofurannecarboxylique ;
le 5-chloro-3-méthyl,2-(phénylsulfonyl)hydrazide d'acide 2-benzofurannecarboxylique ;
le 5-amino,2-(phénylsulfonyl)-hydrazide d'acide 2-benzofurannecarboxylique ;
le 2-[(2-fluorophényl)sulfonyl]hydrazide d'acide 2-dibenzofurannecarboxylique ;
le 2-(2,6-difluorophénylsulfonyl)hydrazide d'acide 2-dibenzofurannecarboxylique ;
le 2-[(2-bromophényl)sulfonyl]hydrazide d'acide 2-dibenzofurannecarboxylique ;
le 2-[(3-bromophényl)sulfonyl]hydrazide d'acide 2-dibenzofurannecarboxylique ;
le 2-[(4-méthyl-3-nitrophényl)sulfonyl]hydrazide d'acide 2-dibenzofurannecarboxylique ;
le 2-[2-chloro-5-(trifluorométhyl)phényl]sulfonyl)hydrazide d'acide 2-dibenzofurannecarboxylique ;
le 2-[(2-trifluorométhoxyphényl)sulfonyl]hydrazide d'acide 2-dibenzofurannecarboxylique ;
le 2- [(4-bromo-2-trifluorométhoxyphényl)sulfonyl]hydrazide d'acide 2-dibenzofurannecarboxylique ;
le 2-[(2-nitrophényl)sulfonyl]-hydrazide d'acide 2-dibenzofurannecarboxylique ;
le 2-[[4-carboxyphényl)sulfonyl]hydrazide d'acide 2-dibenzofurannecarboxylique ;
le 2-[(4,5-dichloro-2-thiényl)sulfonyl]hydrazide d'acide 2-dibenzofurannecarboxylique ;
le 7-méthoxy-,2-[(3-chlorophényl)sulfonyl]hydrazide d'acide 2-benzofurannecarboxylique ;
le 7-méthoxy-,2-[(3-fluorophényl)sulfonyl]hydrazide d'acide 2-benzofurannecarboxylique ;
le 7-méthoxy-,2-[(2,3-dichlororophényl)sulfonyl]hydrazide d'acide 2-benzofurannecarboxylique ;
le 7-méthoxy-,2-[(3-chloro-4-fluorophényl)sulfonyl]hydrazide d'acide 2-benzofurannecarboxylique ;
le 7-méthoxy-,2-[(5-chloro-2-méthoxyphényl)sulfonyl]hydrazide d'acide 2-benzofurannecarboxylique ;
le 7-méthoxy-,2-[(5-bromo-2-méthoxyphényl)sulfonyl]hydrazide d'acide 2-benzofurannecarboxylique ;
le 7-méthoxy-,2-[(4-éthylphényl)sulfonyl]hydrazide d'acide 2-benzofurannecarboxylique ;
le 7-méthoxy-,2-[(3-méthoxyphényl)sulfonyl]hydrazide d'acide 2-benzofurannecarboxylique ;
le 2-[(2,3-dichlorophényl)sulfonyl]hydrazide d'acide 2-dibenzofurannecarboxylique ;
le 2-[(3-méthylphényl)sulfonyl]-hydrazide d'acide 2-naphtalènecarboxylique ;
le 2-[(4-méthylphényl)sulfonyl]-hydrazide d'acide 2-naphtalènecarboxylique ;
le 2-[[(2-(trifluorométhyl)phényl]sulfonyl]hydrazide d'acide 2-naphtalènecarboxylique ;
le 2-[[(4-(trifluorométhyl)phényl]sulfonyl]hydrazide d'acide 2-naphtalènecarboxylique ;
le 2-[(3-chloro-4-fluorophényl)sulfonyl]hydrazide d'acide 2-naphtalènecarboxylique ;
le 2-[(3-méthoxyphényl)sulfonyl]hydrazide d'acide 2-naphtalènecarboxylique ;
le 2-[(3-chlorophényl)sulfonyl]-hydrazide d'acide 2-naphtalènecarboxylique ;
le 2-[(2-chlorophényl)sulfonyl]-hydrazide d'acide 2-naphtalènecarboxylique ;
le 2-[(3-fluorophényl)sulfonyl]-hydrazide d'acide 2-naphtalènecarboxylique ;
le 2-(2-thiénylsulfonyl]hydrazide d'acide 2-naphtalènecarboxylique ;
le 2-[(2,3-dichlorophényl)sulfonyl]-hydrazide d'acide 2-naphtalènecarboxylique ;
le 2-[(4-éthylphényl)sulfonyl]-hydrazide d'acide 2-)naphtalènecarboxylique ;
le 2-[(4-pentylphényl)sulfonyl)-hydrazide d'acide 3-quinoléinecarboxylique ;
le 2-[[2(trifluorométhyl)phényl]sulfonyl]hydrazide d'acide 3-quinoléinecarboxylique ;
le 2-[[2,5-bis(2,2,2-trifluoroéthoxyl)phényl]sulfonyl]hydrazide d'acide 3-quinoléinecarboxylique ;
le 2-[(3-méthylphényl)sulfonyl]-hydrazide d'acide 3-quinoléinecarboxylique ;
le 2-[[2-(trifluorométhoxy)phényl]sulfonyl]hydrazide d'acide 3-quinoléinecarboxylique ;
le 2-[(4-méthylphényl)sulfonyl]-hydrazide d'acide 3-quinoléinecarboxylique ;
le 2-[(3-méthylphényl)sulfonyl]-hydrazide d'acide 6-quinoléinecarboxylique ;
le 2-[(3-méthylphényl)sulfonyl]-hydrazide d'acide 3-quinoléinecarboxylique ;
le 2-[(3-chlorophényl)sulfonyl]-hydrazide d'acide 6-quinoléinecarboxylique ;
le 2-[(3-chlorophényl)sulfonyl]-hydrazide d'acide 3-quinoléinecarboxylique ;
le 2-[(3-fluorophényl)sulfonyl]-hydrazide d'acide 6-quinoléinecarboxylique ;
le 2-[(3-fluorophényl)sulfonyl]-hydrazide d'acide 3-quinoléinecarboxylique ;
le 2-[(2-trifluorométhylphényl)sulfonyl]-hydrazide d'acide 6-quinoléinecarboxylique ;
le 2-[(2-trifluorométhylphényl)sulfonyl]-hydrazide d'acide 3-quinoléinecarboxylique ;
le 2-[(2-trifluorométhoxyphényl)sulfonyl]-hydrazide d'acide 6-quinoléinecarboxylique ;
le 2-[(2-trifluorométhoxyphényl)sulfonyl]-hydrazide d'acide 3-quinoléinecarboxylique ;
le 2-[(3,5-dichlorophényl)sulfonyl]-hydrazide d'acide 6-quinoléinecarboxylique ;
le 2-[(3,5-dichlorophényl)sulfonyl]-hydrazide d'acide 3-quinoléinecarboxylique ;
le 2-[(3-chloro-4-fluorophényl)sulfonyl]-hydrazide d'acide 6-quinoléinecarboxylique ;
le 2-[(3-chloro-4-fluorophényl)sulfonyl]-hydrazide d'acide 3-quinoléinecarboxylique ;
le 2-[(3-méthoxyphényl)sulfonyl]-hydrazide d'acide 6-quinoléinecarboxylique ;
le 2-[(3-méthoxyphényl)sulfonyl]-hydrazide d'acide 3-quinoléinecarboxylique ;
le 2-[(3-bromophényl)sulfonyl]-hydrazide d'acide 6-quinoléinecarboxylique ;
le 2-[(3-bromophényl)sulfonyl]-hydrazide d'acide 3-quinoléinecarboxylique ;
le 2-(phénylsulfonyl)hydrazide d'acide benzofuranne-2-carboxylique ;
le 2-(phénylsulfonyl)-hydrazide d'acide 7-éthoxy-2-)benzofurannecarboxylique ;
le .2-(phénylsulfonyl)-hydrazide d'acide 7-chlorobenzofuranne-2-carboxylique ;
le 2-(phénylsulfonyl)-hydrazide d'acide 2,3-dihydrobenzofuranne-5-carboxylique ;
le 2-(phénylsulfonyl)-hydrazide d'acide 5-chlorobenzofuranne-2-carboxylique ;
le 2-(phénylsulfonyl)hydrazide d'acide isoquinoléine-1-carboxylique ;
le 2-(phénylsulfonyl)hydrazide d'acide 1H-indole-5-carboxylique ;
le 2-(phénylsulfonyl)hydrazide d'acide quinoléine-3-carboxylique ;
le 2-(phénylsulfonyl)hydrazide d'acide quinoléine-4-carboxylique ;
le 2-[(3-chlorophényl)sulfonyl]-hydrazide d'acide 1H-indole-5-carboxylique ;
le 2-[(2-chlorophényl)sulfonyl]-hydrazide d'acide 1H-indole-5-carboxylique ;
le 2-[(3-fluorophényl)sulfonyl]-hydrazide d'acide 1H-indole-5-carboxylique ;
le 2-[(3-trifluorométhylphényl)sulfonyl]hydra zide d'acide 1H-indole-5-carboxylique ;
le 2-[(2-trifluorométhylphényl)sulfonyl]hydrazide d'acide 1H-indole-5-carboxylique ;
le 2- [(2-trifluorométhoxyphényl)sulfonyl]hydrazide d'acide 1H-indole-5-carboxylique ;
le 2-[(3-trifluorométhoxyphényl)sulfonyl]hydrazide d'acide 1H-indole-5-carboxylique ;
le 2-[(3-bromophényl)sulfonyl]-hydrazide d'acide 1H-indole-5-carboxylique ;
le 2-[(3-méthylphényl)sulfonyl]-hydrazide d'acide 1H-indole-5-carboxylique ;
le 2-[(2-méthylphényl)sulfonyl]-hydrazide d'acide 1H-indole-5-carboxylique ;
le 2-[(2-chlorophényl)sulfonyl]-hydrazide d'acide 6-quinoléinecarboxylique ;
le 2- [(3-trifluorométhylphényl)sulfonyl]hydrazide d'acide 6-quinoléinecarboxylique ;
le 2- [(3-trifluorométhoxyphényl)sulfonyl]hydra zide d'acide 6-quinoléinecarboxylique ;
le 2-[(2-méthylphényl)sulfonyl]-hydrazide d'acide 6-quinoléinecarboxylique ;
le 2-(4-fluoro-3-méthylphénylsulfonyl)hydrazide d'acide dibenzofuranne-2-carboxylique ;
le 2-(phénylsulfonyl)hydrazide d'acide 2-naphtalènecarboxylique.

12. Utilisation d'un composé suivant la revendication 1, pour la production d'un médicament destiné à inhiber les aminotransférases dépendant d'aminoacides à chaîne ramifiée chez un patient, par administration au patient d'une quantité thérapeutiquement efficace.

13. Utilisation suivant la revendication 12, dans laquelle Ar représente un groupe hétéroaryle bicyclique.

14. Utilisation suivant la revendication 12, dans laquelle Ar représente un groupe hétéroaryle tricyclique.

15. Utilisation suivant la revendication 12, dans laquelle R₁, R₂, R₃, R₄ et R₅ représentent des atomes d'hydrogène.

16. Utilisation suivant la revendication 12, dans laquelle le composé est :
le 2-(phénylsulfonyl)hydrazide d'acide dibenzofuranne-2-carboxylique ;
le 2-(phénylsulfonyl)hydrazide d'acide 2,3,4,9-tétrahydro-1H-carbazole-6-carboxylique ;
le 2-(phénylsulfonyl)hydrazide d'acide benzofuranne-2-carboxylique ;
le 2-(phénylsulfonyl)-hydrazide d'acide 5-méthoxy-2-benzofurannecarboylique ;
le 2-(phénylsulfonyl)-hydrazide d'acide 7-méthoxy-2-benzofurannecarboxylique ;
le 2-(phénylsulfonyl)-hydrazide d'acide 7-éthoxy-2-benzofurannecarboxylique ;
le 2-(phénylsulfonyl)-hydrazide d'acide 7-chlorobenzofuranne-2-carboxylique ;
le 2-(phénylsulfonyl)-hydrazide d'acide 5-chlorobenzofuranne-2-carboxylique ;
le 2-(phénylsulfonyl)-hydrazide d'acide 2,3-dihydrobenzofuranne-5-carboxylique ;
le 2-(phénylsulfonyl)hydrazide d'acide benzo[1,3]dioxole-5-carboxylique ;
le 2-(phénylsulfonyl)hydrazide d'acide 1H-indole-5-carboxylique ;
le 2-(phénylsulfonyl)hydrazide d'acide 1-dibenzofurannecarboxylique ;
le 2-(phénylsulfonyl)hydrazide d'acide quinoléine-6-carboxylique ;
le 2-[(2-méthylphényl)sulfonyl]-hydrazide d'acide 2-dibenzofurannecarboxylique ;
le 2-[(3-méthylphényl)sulfonyl]-hydrazide d'acide 2-dibenzofurannecarboxylique ;
le 2-[(3-chlorophényl)sulfonyl]-hydrazide d'acide 2-dibenzofurannecarboxylique ;
le 2-[(2-chlorophényl)sulfonyl]-hydrazide d'acide 2-dibenzofurannecarboxylique ;
le 2-(3,5-dichlorophénylsulfonyl)-hydrazide d'acide dibenzofuranne-2-carboxylique ;
le 2- (3-chloro-2-méthylphénylsulfonyl)hydrazide d'acide dibenzofuranne-2-carboxylique ;
le 2-(phénylsulfonyl)hydrazide d'acide quinoléine-6-carboxylique ;
le 2-(phénylsulfonyl)hydrazide d'acide quinoléine-8-carboxylique ;
le 5-chloro-2-[(4-fluorophényl)]-sulfonylhydrazide d'acide 2-benzofurannecarboxylique ;
le 7-méthoxy-,2-[(4-fluorophényl)sulfonyl]hydrazide d'acide 2-benzofurannecarboxylique ;
le 2-[(4-fluorophényl)sulfonyl]hydrazide d'acide 2-dibenzofurannecarboxylique ;
le 2-[(2-chloro-4-fluorophényl)sulfonyl]hydrazide d'acide 2-dibenzofurannecarboxylique ;
le 2-[(3-cyanophényl)sulfonyl]-hydrazide d'acide 2-dibenzofurannecarboxylique ;
le 5-chloro-,2-[(3-cyanophényl)-sulfonyl]hydrazide d'acide 2-benzofurannecarboxylique ;
le 2-[(2,6-dichlorophényl)sulfonyl]-hydrazide d'acide 2-dibenzofurannecarboxylique ;
le 5-bromo-,2-(phénylsulfonyl)-hydrazide d'acide 2-benzofurannecarboxylique ;
le 5-chloro-,2-[(2-chloro-4-fluorophényl)sulfonyl]-hydrazide d'acide 2-benzofurannecarboxylique ;
le 5-chloro-,2-[(3,5-dichlorophényl)sulfonyl]hydrazide d'acide 2-benzofurannecarboxylique ;
le 2-[[3,5-bis(trifluorométhyl)-phényl]sulfonyl]hydrazide d'acide 2-dibenzofurannecarboxylique ;
le 5-chloro, 2- [(3-chloro-4-fluorophényl)sulfonyl]hydrazide d'acide 2-benzofurannecarboxylique ;
le 5-nitro,2-(phénylsulfonyl)-hydrazide d'acide 2-benzofurannecarboxylique ;
le 2-[(3-fluorophényl)sulfonyl]-hydrazide d'acide 2-dibenzofurannecarboxylique ;
le 2-[(5-fluoro-2-méthylphényl)sulfonyl]hydrazide d'acide 2-dibenzofurannecarboxylique ;
le 2-[(3,4-difluorophényl)sulfonyl]-hydrazide d'acide 2-dibenzofurannecarboxylique ;
le 2-[(3-chloro-4-fluorophényl)sulfonyl]-hydrazide d'acide 2-dibenzofurannecarboxylique ;
le 2-[(3-méthoxyphényl)sulfonyl]hydrazide d'acide 2-dibenzofurannecarboxylique ;
le 5-chloro-2-[(3-fluorophényl)sulfonyl]hydrazide d'acide 2-benzofurannecarboxylique ;
le 5-chloro-2-[(3,4-difluorophényl)sulfonyl]hydrazide d'acide 2-benzofurannecarboxylique ;
le 5-chloro-2-[(2-chlorophényl)sulfonyl]hydrazide d'acide 2-benzofurannecarboxylique ;
le 5-chloru-2-[(3-chlorophényl)sulfonyl]hydrazide d'acide 2-benzofurannecarboxylique ;
le 5-chloro-2- [(2-bromophényl)sulfonyl]hydrazide d'acide 2-benzofurannecarboxylique ;
le 5-chloro-2-[(3-bromophényl)sulfonyl]hydrazide d'acide 2-benzofurannecarboxylique ;
le 5-chloro-2-[(2-méthylphényl)sulfonyl]hydrazide d'acide 2-benzofurannecarboxylique ;
le 5-chloro-2-[(3-méthylphényl)sulfonyl]hydrazide d'acide 2-benzofurannecarboxylique ;
le 5-chloro-2-[(3-méthoxyphényl)sulfonyl]hydrazide d'acide 2-benzofurannecarboxylique ;
le 5-chloro-2-[(3-chloro-2-méthylphényl)sulfonyl]hydrazide d'acide 2-benzofurannecarboxylique ;
le 5-chloro-2- [(5-fluoro-2-méthylphényl)sulfonyl]hydrazide d'acide 2-benzofurannecarboxylique ;
le 5-chloro-2-[[(2-(trifluorométhyl)phényl]sulfonyl]hydrazide d'acide 2-benzofurannecarboxylique ;
le 5-chloro-2-[[(2-(trifluorométhoxy)phényl]sulfonyl])hydrazide d'acide 2-benzofurannecarboxylique ;
le 7-méthoxy-,2-[[(2-(trifluorométhyl)phényl]sulfonyl]hydrazide d'acide 2-benzofurannecarboxylique ;
le 7-méthoxy-,2-[(3-méthylphényl)sulfonyl]hydrazide d'acide 2-benzofurannecarboxylique ;
le 6-méthoxy-,2-(phénylsulfonyl)hydrazide d'acide 2-benzofurannecarboxylique ;
le 5-méthoxy-3-(1-méthyléthoxy),2-(phénylsulfonyl)hydrazide d'acide 2-benzofurannecarboxylique ;
le 3,5-diméthaxy-,2-(phénylsulfonyl)hydrazide d'acide 2-benzofurannecarboxylique ;
le 3,6-diméthoxy-,2-(phénylsulfonyl)hydrazide d'acide 2-benzofurannecarboxylique ;
le 4,6-diméthoxy-,2-(phénylsulfonyl)hydrazide d'acide 2-benzofurannecarboxylique ;
le 3,7-diméthoxy-,2-(phénylsulfonyl)hydrazide d'acide 2-benzofurannecarboxylique ;
le 3,7-diméthoxy-,2-[(2-trifluorométhylphényl)-sulfonyl]hydrazide d'acide 2-benzofurannecarboxylique ;
le 3,7-diméthoxy-,2-[(4-fluorophényl)sulfonyl]-hydrazide d'acide 2-benzofurannecarboxylique ;
le 3,7-diméthoxy-,2-[(3-méthylphényl)sulfonyl]-hydrazide d'acide 2-benzofurannecarboxylique ;
le 3-méthoxy-5-phényl-,2-(phénylsulfonyl)hydrazide d'acide 2-benzofurannecarboxylique ;
le 3-éthoxy-5-phényl-,2-(phénylsulfonyl)hydrazide d'acide 2-benzofurannecarboxylique ;
le 3-méthoxy-7-phényl-,2-(phénylsulfonyl)hydrazide d'acide 2-benzofurannecarboxylique ;
le 3-méthoxy-,2-(phénylsulfonyl)hydrazide d'acide naphto[2,3-b]furanne-2-carboxylique ;
le 5,7-dichloro-2-(phénylsulfonyl)-sulfonylhydrazide d'acide 2-benzofurannecarboxylique ;
le 5,7-dichloro-2-[[2-(trifluorométhyl)phényl)]-sulfonyl]hydrazide d'acide 2-benzofurannecarboxylique ;
le 5,7-dichloro-2-[(2-méthylphényl)sulfonyl]hydrazide d'acide 2-benzofurannecarboxylique ;
le 5,7-dichloro,2-[(3-méthylphényl)sulfonyl]hydrazide d'acide 2-benzofurannecarboxylique ;
le 5,7-dichloro,2-[(2-bromophényl)sulfonyl]hydrazide d'acide 2-benzofurannecarboxylique ;
le 5,7-dichloro-2, [(3-bromophényl)sulfonyl]hydrazide d'acide 2-benzofurannecarboxylique ;
le 5,7-dichloro-2, [(3-chlorophényl)sulfonyl]hydrazide d'acide 2-benzofurannecarboxylique ;
le 5,7-dichloro,2-[[2-(trifluorométhoxy)phényl]-sulfonyl]hydrazide d'acide 2-benzofurannecarboxylique ;
le 5-méthyl-,2-(phénylsulfonyl)-hydrazide d'acide benzofurannecarboxylique ;
le 5-chloro-3-méthoxy,2-(phénylsulfonyl)hydrazide d'acide 2-benzofurannecarboxylique ;
le 5-méthyl-,2-[[2-(trifluorométhyl)phényl]sulfonyl]hydrazide d'acide 2-benzofurannecarboxylique ;
le 5-méthyl-,2-([3-méthylphényl]sulfonyl]hydrazide d'acide 2-benzofurannecarboxylique ;
le 5-méthyl-,2-[2-(méthylphényl)sulfonyl]hydrazide d'acide 2-benzofurannecarboxylique ;
le 5-méthyl-, 2- [(3-chloro-2-méthylphényl)sulfonyl]hydrazide d'acide 2-benzofurannecarboxylique ;
le 5-méthyl-,2-[3-(bromophényl)sulfonyl]hydrazide d'acide 2-benzofurannecarboxylique ;
le 5-méthyl-,2-[(3-chlorophényl)sulfonyl]hydrazide d'acide 2-benzofurannecarboxylique ;
le 5-méthyl-,2-[[2-(trifluorométhoxy)phényl]-sulfonyl]hydrazide d'acide 2-benzofurannecarboxylique ;
le 7-méthyl-,2-(phénylsulfonyl)-hydrazide d'acide 2-benzofurannecarboxylique ;
le 4-méthyl-,2-(phénylsulfonyl)-hydrazide d'acide 2-benzofurannecarboxylique ;
le 3-méthyl-,2-(phénylsulfonyl)-hydrazide d'acide 2-benzofurannecarboxylique ;
le 3,5-diméthyl-,2-(phénylsulfonyl)-hydrazide d'acide 2-benzofurannecarboxylique ;
le 5-chloro-3-méthyl,2-(phénylsulfonyl)hydrazide d'acide 2-benzofurannecarboxylique ;
le 5-amino,2-(phénylsulfonyl)-hydrazide d'acide 2-benzofurannecarboxylique ;
le 2-[(2-fluorophényl)sulfonyl]hydrazide d'acide 2-dibenzofurannecarboxylique ;
le 2-(2,6-difluorophénylsulfonyl)hydrazide d'acide 2-dibenzofurannecarboxylique ;
le 2-[(2-bromophényl)sulfonyl]hydrazide d'acide 2-dibenzofurannecarboxylique ;
le 2-[(3-bromophényl)sulfonyl]hydrazide d'acide 2-dibenzofurannecarboxylique ;
le 2-[(4-méthyl-3-nitrophényl)sulfonyl]hydrazide d'acide 2-dibenzofurannecarboxylique ;
le 2-[2-chloro-5-(trifluorométhyl)phényl]sulfonyl)-hydrazide d'acide 2-dibenzofurannecarboxylique ;
le 2-[(2-trifluorométhoxyphényl)sulfonyl]hydrazide d'acide 2-dibenzofurannecarboxylique ;
le 2-[(4-bromo-2-trifluorométhoxyphényl)sulfonyl]hydrazide d'acide 2-dibenzofurannecarboxylique ;
le 2-[(2-nitrophényl)sulfonyl]-hydrazide d'acide 2-dibenzofurannecarboxylique ;
le 2-[(4-carboxyphényl)sulfonyl]hydrazide d'acide 2-dibenzofurannecarboxylique ;
le 2-[(4,5-dichloro-2-thiényl)sulfonyl]hydrazide d'acide 2-dibenzofurannecarboxylique ;
le 7-méthoxy-,2-[(3-chlorophényl)sulfonyl]hydrazide d'acide 2-benzofurannecarboxylique ;
le 7-méthoxy-,2-[(3-fluorophényl)sulfonyl]hydrazide d'acide 2-benzofurannecarboxylique ;
le 7-méthoxy-,2-[(2,3-dichlororophényl)sulfonyl]hydrazide d'acide 2-benzofurannecarboxylique ;
le 7-méthoxy-,2-[(3-chloro-4-fluorophényl)sulfonyl]hydrazide d'acide 2-benzofurannecarboxylique ;
le 7-méthoxy-,2-[(5-chloro-2-méthoxyphényl)sulfonyl]hydrazide d'acide 2-benzofurannecarboxylique ;
le 7-méthoxy-,2-[(5-bromo-2-méthoxyphényl)sulfonyl]hydrazide d'acide 2-benzofurannecarboxylique ;
le 7-méthoxy-,2-[(4-éthylphényl)sulfonyl]hydrazide d'acide 2-benzofurannecarboxylique ;
le 7-méthoxy-,2-[(3-méthoxyphényl)sulfonyl]hydrazide d'acide 2-benzofurannecarboxylique ;
le 2-[(2,3-dichlorophényl)sulfonyl]hydrazide d'acide 2-dibenzofurannecarboxylique ;
le 2-[(3-méthylphényl)sulfonyl]-hydrazide d'acide 2-naphtalènecarboxylique ;
le 2-[(4-méthylphényl)sulfonyl]-hydrazide d'acide 2-naphtalènecarboxylique ;
le 2-[[(2-(trifluorométhyl)phényl]sulfonyl]hydrazide d'acide 2-naphtalènecarboxylique ;
le 2-[[(4-(trifluorométhyl)phényl]sulfonyl]hydrazide d'acide 2-naphtalènecarboxylique ;
le 2-[(3-chloro-4-fluorophényl)sulfonyl]hydrazide d'acide 2-naphtalènecarboxylique ;
le 2-[(3-méthoxyphényl)sulfonyl]hydrazide d'acide 2-naphtalènecarboxylique ;
le 2-[(3-chlorophényl)sulfonyl]-hydrazide d'acide 2-naphtalènecarboxylique ;
le 2-[(2-chlorophényl)sulfonyl]-hydrazide d'acide 2-naphtalènecarboxylique ;
le 2-[(3-fluorophényl)sulfonyl]-hydrazide d'acide 2-naphtalènecarboxylique ;
le 2-[(2,3-dichlorophényl)sulfonyl]-hydrazide d'acide 2-naphtalènecarboxylique ;
le 2-[(4-éthylphényl)sulfonyl]-hydrazide d'acide 2-naphtalènecarboxylique ;
le 2-[(4-pentylphényl)sulfonyl)-hydrazide d'acide 3-quinoléinecarboxylique ;
le 2-[[2(trifluorométhyl)phényl]sulfonyl]hydrazide d'acide 3-quinoléinecarboxylique ;
le 2-[[2,5-bis(2,2,2-trifluoroéthoxyl)phényl]sulfonyl]-hydrazide d'acide 3-quinoléinecarboxylique ;
le 2-[(3-méthylphényl)sulfonyl]-hydrazide d'acide 3-quinoléinecarboxylique ;
le 2-[[2-(trifluorométhoxy)phényl]sulfonyl]hydrazide d'acide 3-quinoléinecarboxylique ;
le 2-[(4-méthylphényl)sulfényl]-hydrazide d'acide 3-quinoléinecarboxylique ;
le 2-[(3-méthylphényl)sulfonyl]-hydrazide d'acide 6-quinoléinecarboxylique ;
le 2-[(3-méthylphényl)sulfonyl]-hydrazide d'acide 3-quinoléinecarboxylique ;
le 2-[(3-chlorophényl)sulfonyl]-hydrazide d'acide 6-quinoléinecarboxylique ;
le 2-[(3-chlorophényl)sulfonyl]-hydrazide d'acide 3-quinoléinecarboxylique ;
le 2-[(3-fluorophényl)sulfonyl]-hydrazide d'acide 6-quinoléinecarboxylique ;
le 2-[(3-fluorophényl)sulfonyl]-hydrazide d'acide 3-quinoléinecarboxylique ;
le 2-[(2-trifluorométhylphényl)sulfonyl]-hydrazide d'acide 6-quinoléinecarboxylique ;
le 2-[(2-trifluorométhylphényl)sulfonyl]-hydrazide d'acide 3-quinoléinecarboxylique ;
le 2-[(2-trifluorométhoxyphényl)sulfonyl]-hydrazide d'acide 6-quinoléinecarboxylique ;
le 2-[(2-trifluorométhoxyphényl)sulfonyl]-hydrazide d'acide 3-quinoléinecarboxylique ;
le 2-[(3,5-dichlorophényl)sulfonyl]-hydrazide d'acide 6-quinoléinecarboxylique ;
le 2-[(3,5-dichlorophényl)sulfonyl]-hydrazide d'acide 3-quinoléinecarboxylique ;
le 2-[(3-chloro-4-fluorophényl)sulfonyl]-hydrazide d'acide 6-quinoléinecarboxylique ;
le 2-[(3-chloro-4-fluorophényl)sulfonyl]-hydrazide d'acide 3-quinoléinecarboxylique ;
le 2-[(3-méthoxyphényl)sulfonyl]-hydrazide d'acide 6-quinoléinecarboxylique ;
le 2-[(3-méthoxyphényl)sulfonyl]-hydrazide d'acide 3-quinoléinecarboxylique ;
le 2-[(3-bromophényl)sulfonyl]-hydrazide d'acide 6-quinoléinecarboxylique ;
le 2-[(3-bromophényl)sulfonyl]-hydrazide d'acide 3-quinoléinecarboxylique ;
le 2-(phénylsulfonyl)hydrazide d'acide benzofuranne-2-carboxylique ;
le 2-(phénylsulfonyl)-hydrazide d'acide 7-éthoxy-2-benzofurannecarboxylique ;
le 2-(phénylsulfonyl)-hydrazicie d'acide 7-chlorobenzofuranne-2-carboxylique ;
le 2- (phénylsulfonyl) -hydrazide d'acide 2,3-dihydro-benzofuranne-5-carboxylique ;
le 2-(phénylsulfonyl)-hydrazide d'acide 5-chlorobenzofuranne-2-carboxylique ;
le 2-(phénylsulfonyl)hydrazide d'acide isoquinoléine-1-carboxylique ;
le 2-(phénylsulfonyl)hydrazide d'acide 1H-indole-5-carboxylique ;
le 2-(phénylsulfonyl)hydrazide d'acide quinoléine-3-carboxylique ;
le 2-(phénylsulfonyl)hydrazide d'acide quinoléine-4-carboxylique ;
le 2-[(3-chlorophényl)sulfonyl]-hydrazide d'acide 1H-indole-5-carboxylique ;
le 2-[(2-chlorophényl)sulfonyl]-hydrazide d'acide 1H-indole-5-carboxylique ;
le 2-[(3-fluororophényl)sulfonyl]-hydrazide d'acide 1H-indole-5-carboxylique ;
le 2-[(3-trifluorométhylphényl)sulfonyl]hydrazide d'acide 1H-indole-5-carboxylique ;
le 2-[(2-trifluorométhylphényl)sulfonyl]hydrazide d'acide 1H-indole-5-carboxyliqué ;
le 2-[(2-trifluorométhoxyphényl)sulfonyl]hydrazide d'acide 1H-indvle-5-carboxylique ;
le 2-[(3-trifluorométhoxyphényl)sulfonyl]hydrazide d'acide 1H-indole-5-carboxylique ;
le 2-[(3-bromophényl)sulfonyl]-hydrazide d'acide 1H-indole-5-carboxylique ;
le 2-[(3-méthylphényl)sulfonyl]-hydrazide d'acide 1H-indole-5-carboxylique ;
le 2-[(2-méthylphényl)sulfonyl]-hydrazide d'acide 1H-indole-5-carboxylique ;
le 2-[(2-chlorophényl)sulfonyl]-hydrazide d'acide 6-quinoléinecarboxylique ;
le 2-[(3-trifluorométhylphényl)sulfonyl]hydrazide d'acide 6-quinoléinecarboxylique ;
le 2-[(3-trifluorométhoxyphényl)sulfonyl]hydrazide d'acide 6-quinoléinecarboxylique ;
le 2-[(2-méthylphényl)sulfonyl]-hydrazide d'acide 6-quinoléinecarboxylique ;
le 2-(4-fluoro-3-méthylphénylsulfonyl)hydrazide d'acide dibenzofuranne-2-carboxylique ;
le 2-(phénylsulfonyl)hydrazide d'acide 2-naphtalènecarboxylique.

17. Procédé pour la préparation d'un composé de formule I 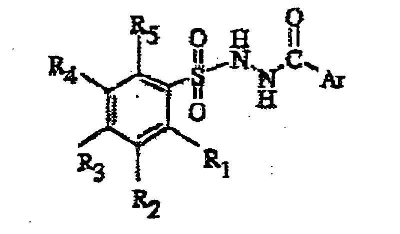
R₃ représente H ou un groupe F, Br, alkyle, carboxy, alkoxy ou alkoxy substitué,
R₁, R₂, R₄ et Rs représentent indépendamment H, un atome d'halogène, un groupe alkyle, alkyle substitué, alkoxy, alkoxy substitué, cyano, nitro, amino, alkylamine ou thioalkyle, Ar représente un groupe hétéroaryle bicyclique, hétéroaryle tricyclique, hétéroaryle bicyclique substitué, à l'exception de (6-méthoxybenzofuranne)-2-nyle, 3-quinolinyle, qui comprend la réaction d'un hydrazide de formule ArC(O)NH₂NH₂ avec un halogénure de sulfonyle de formule : 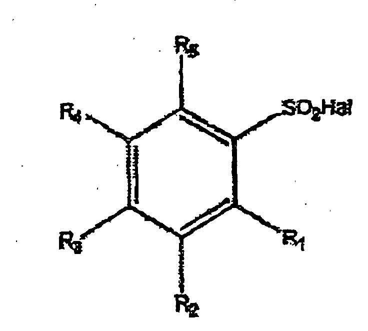 dans laquelle R₁, R₂, R₃, R₄, R₅ et Ar répondent aux définitions précitées et Hal représente un atome d'halogène.

18. Agent médicamenteux pour le traitement ou la prévention de la perte neuronale associée à un ictus, une ischémie, un traumatisme du SNC, l'hypoglycémie ou une intervention chirurgicale, ou pour le traitement d'une maladie neurodégénérative, ou pour le traitement ou la prévention des conséquences néfastes de la stimulation excessive des aminoacides excitateurs, ou pour le traitement de l'anxiété, de la psychose, du glaucome, de la rétinite provoquée par le CMV, de rétinopathie diabétique, de l'incontinence urinaire, des céphalées de la migraine, des convulsions, de la perte d'audition induite par les antibiotiques aminoglycosidiques, de la maladie de Parkinson, de la douleur chronique, de la douleur neuropathique, ou pour induire une anesthésie, une tolérance ou un sevrage des opioïdes, ou pour améliorer la cognition, comprenant une quantité thérapeutiquement efficace d'un composé suivant la revendication 1.

19. Composé de formule I répondant à la définition figurant dans la revendication 1 ou d'un de ses sels, esters ou amides pharmaceutiquement acceptables, destiné à être utilisé comme médicament.
